# EUROPEAN PATENT SPECIFICATION

(11) **EP 3 752 492 B1**
(45) Date of publication and mention of the grant of the patent: **21.12.2022**
(21) Application number: 19705158.4
(22) Date of filing: 11.02.2019
(51) Int. Cl.: C07D 271/06, C07D 413/04, A01N 43/82

(54) **FUNGICIDAL OXADIAZOLES**
FUNGIZIDOXADIAZOLE
OXADIAZOLES FONGICIDES

(30) Priority: 12.02.2018 EP 18156338
(43) Date of publication of application: 23.12.2020
(73) Proprietor: Bayer Aktiengesellschaft, 51373 Leverkusen (DE)
(72) Inventor: BRUNET, Stéphane, 01390 Saint André de Corcy (FR); DESBORDES, Philippe, 69006 Lyon (FR); DUCERF, Sophie, 69380 Chasselay (FR); DUFOUR, Jérémy, 69003 Lyon (FR); GÖRTZ, Andreas, 41541 Dormagen (DE); GOURGUES, Mathieu, 69009 Lyon (FR); HILT, Emmanuelle, 38460 Dizimieu (FR); NAUD, Sébastien, 69660 Collonges au Mont d'Or (FR); REBSTOCK, Anne-Sophie, 69410 Champagne au Mont d'or (FR); THOMAS, Vincent, 69003 Lyon (FR); VERNAY, Aurélia, 69006 Lyon (FR); VILLALBA, François, 69250 Albigny-sur-Saône (FR)
(74) Representative: BIP Patents
(86) International application number: PCT/EP2019/053328
(87) International publication number: WO 2019/155066

(56) References cited:
- EP-A1- 3 050 884
- WO-A1-2015/185485
- WO-A1-2017/014170
- WO-A1-2017/014321
- WO-A1-2017/110863
- WO-A1-2017/222950
- CN-A- 103 087 045
- GB-A- 2 170 199
- NICOLAIDES D N ET AL: "Synthesis and biological evaluation of several coumarin-4-carboxamidoxime and 3-(coumarin-4-yl)-1,2,4-oxadiazole derivatives", EUROPEAN JOURNAL OF MEDICINAL CHEMISTRY, EDITIONS SCIENTIFIQUE ELSEVIER, PARIS, FR, vol. 33, no. 9, 1 September 1998 (1998-09-01), pages 715-724, XP004140826, ISSN: 0223-5234, DOI: 10.1016/S0223-5234(98)80030-5
- MOGHIMI ABOLGHASEM ET AL: "A new library of 4(3H)- and 4,4'(3H,3H')-quinazolinones and 2-(5-alkyl-1,2,4-oxadiazol-3-yl)quinazolin -4(3H)-one obtained from diamino", TETRAHEDRON LETTERS, ELSEVIER, AMSTERDAM, NL, vol. 54, no. 30, 23 May 2013 (2013-05-23), pages 3956-3959, XP028573290, ISSN: 0040-4039, DOI: 10.1016/J.TETLET.2013.05.065

## Description

### TECHNICAL FIELD

The present invention relates to oxadiazole derivatives that may be used as fungicides for controlling phytopathogenic fungi.

### BACKGROUND

Oxadiazole derivatives are known to be useful in the pharmaceutical fied. For instance, WO 2017/222950, WO 2017/014321, EP 3 050 884, GB 2 170 199 and CN 103 087 045 disclose some heterocyclic or carbocyclic ring substituted 5-trifluoromethyl oxadiazole compounds (further examples are disclosed in: D.N. Nicolaides et al., Eur. J. Med. Chem. 33 (1998), 715-724; M. Abolghasem et al., Tet. Lett. 54 (2013), 3956-3959; CA2158910, WO94/29290, CN103087044, US2007/0155738, WO96/09822, RU2550346, WO2004/069162). The use of oxadiazole substituted naphthalene-1-acetic acid derivatives as pesticides is known from EP0538097.

Oxadiazole derivatives are also known to be useful as crop protection agents to combat or prevent microorganisms' infestations. For instance, WO2017/110863, WO2018/118781 and WO2018/187553 discloses oxadiazole derivatives that may be used as fungicides. WO2018/118781 and WO2018/187553 discloses some bicyclic ring substituted 5-trifluomethyl oxadiazole compounds. Numerous fungicidal agents have been developed until now. However, the need remains for the development of new fungicidal compounds as such, so as to provide compounds being effective against a broad spectrum of fungi, having lower toxicity, higher selectivity, being used at lower dosage rate to reduce or avoid unfavorable environmental or toxicological effects whilst still allowing effective pest control. It may also be desired to have new compounds to prevent the emergence of fungicides resistances.

The present invention provides new fungicidal compounds which have advantages over known compounds and compositions in at least some of these aspects.

### SUMMARY

The invention relates to a compound of formula (I') or a salt, N-oxide or solvate thereof: 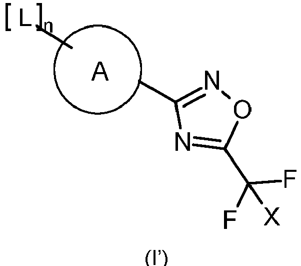 wherein
**X** is F or Cl;
**A** is an aromatic or partially unsaturated fused bicyclic C₉-C₁₀-carbocyclyl ring or an aromatic or partially unsaturated fused bicyclic 9- or 10-membered heterocyclyl ring selected from the group consisting of 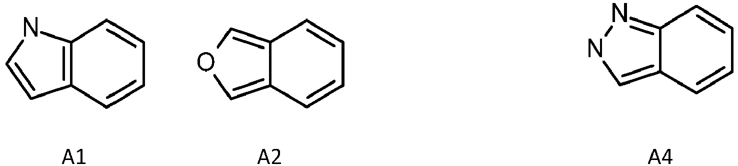 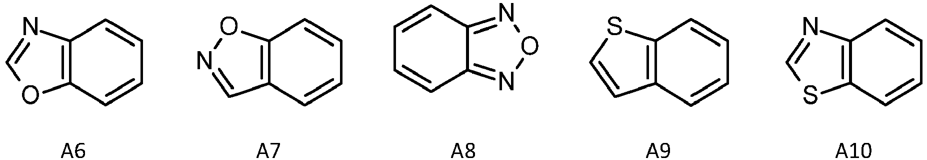 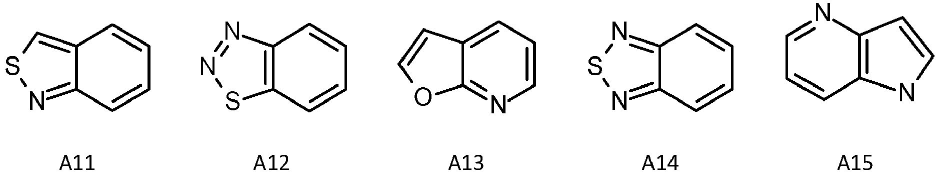 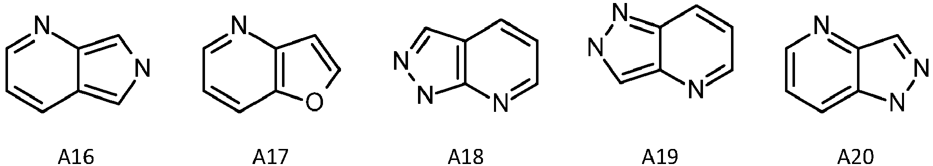 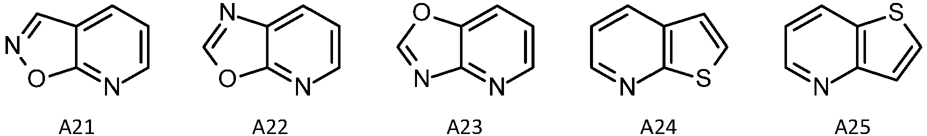 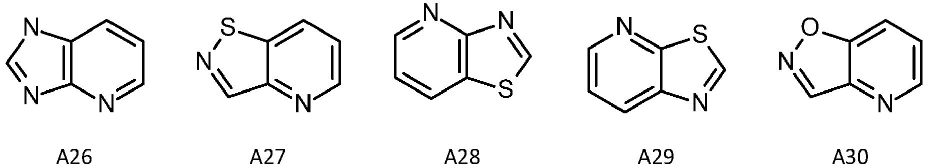 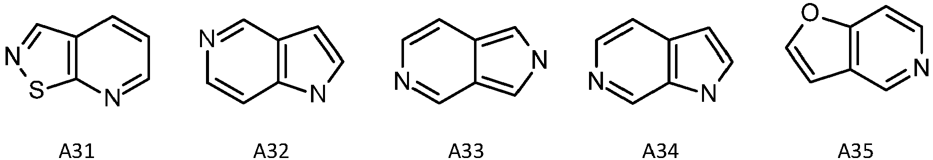 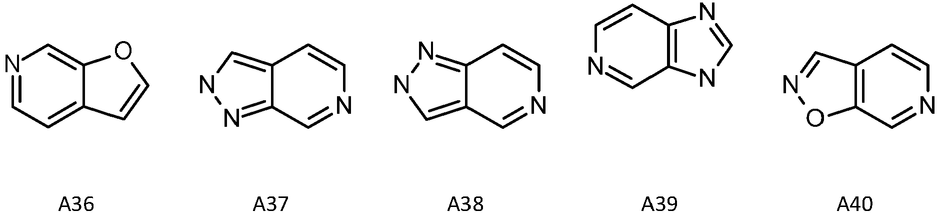 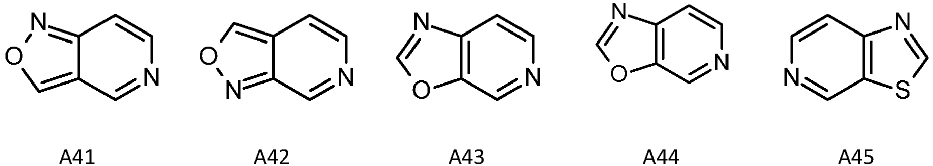 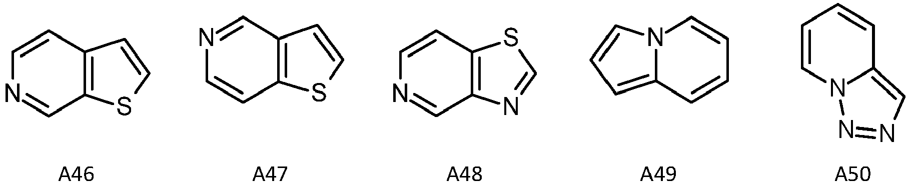 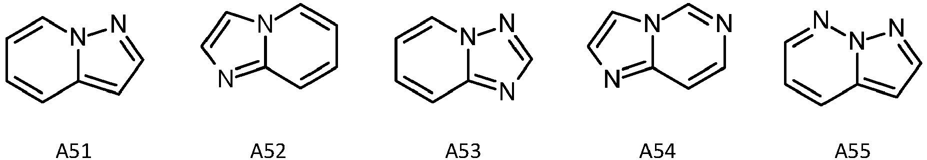 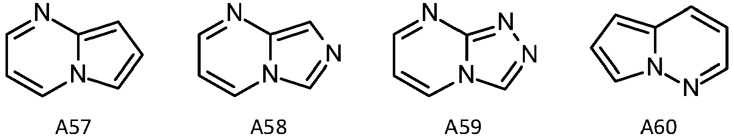 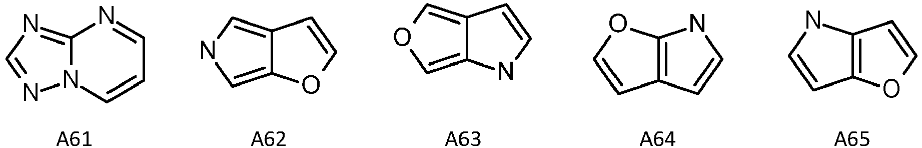 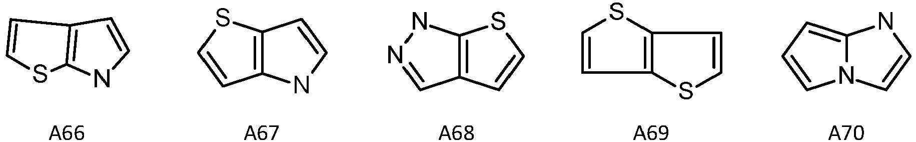 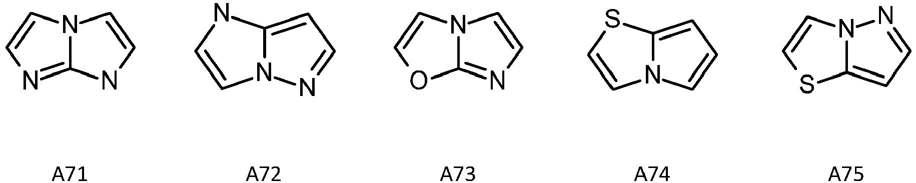 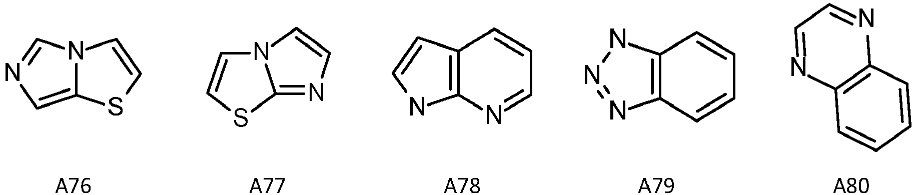 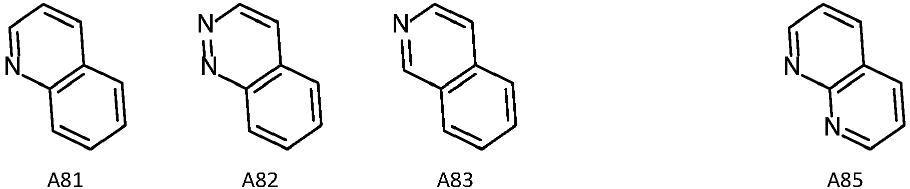 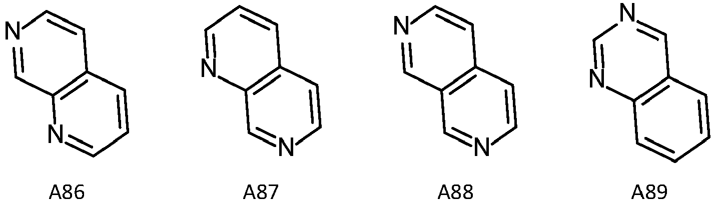 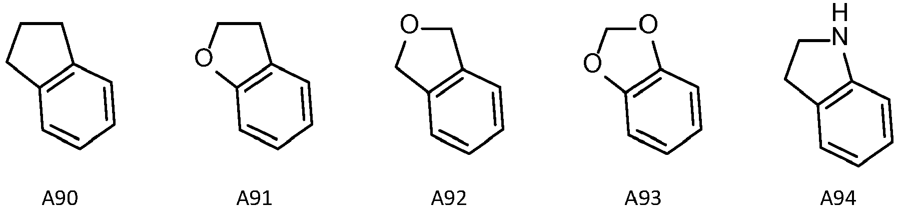 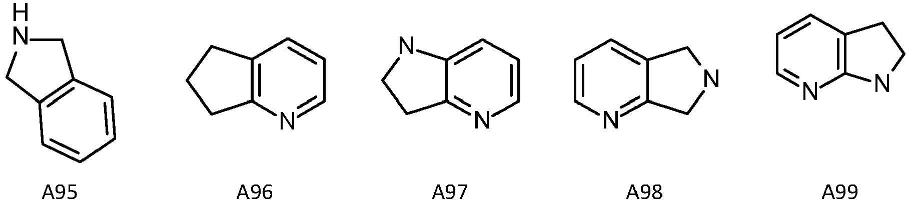 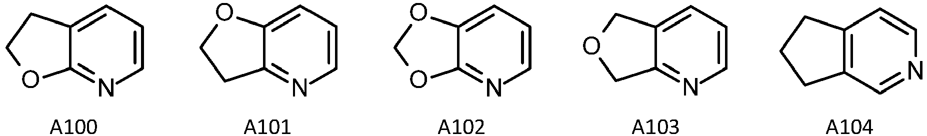 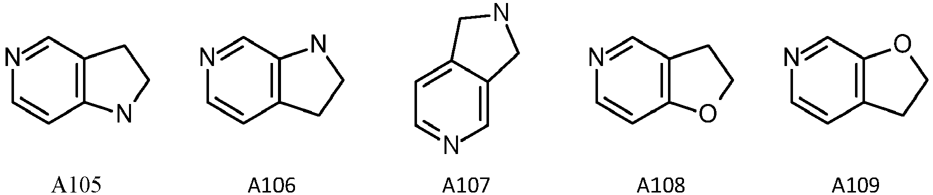 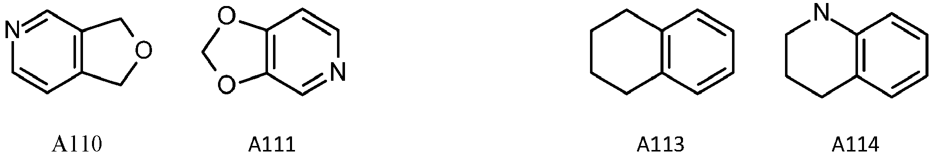 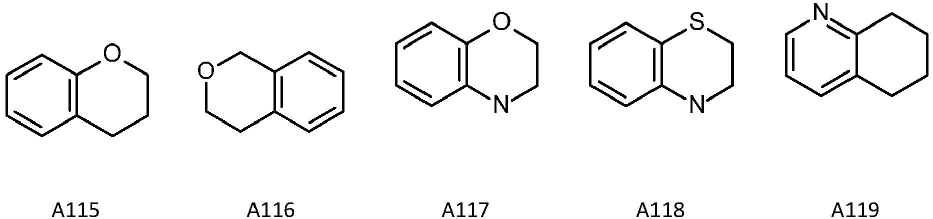 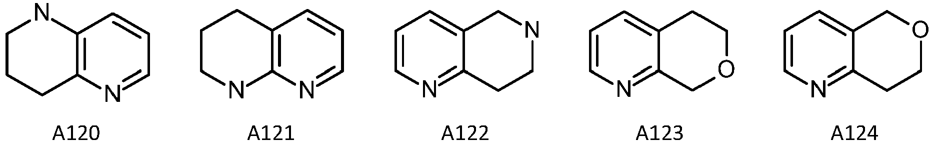 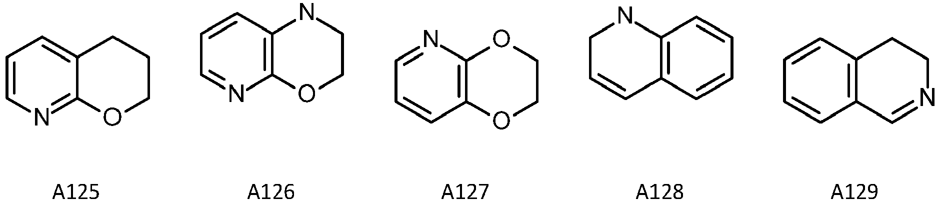 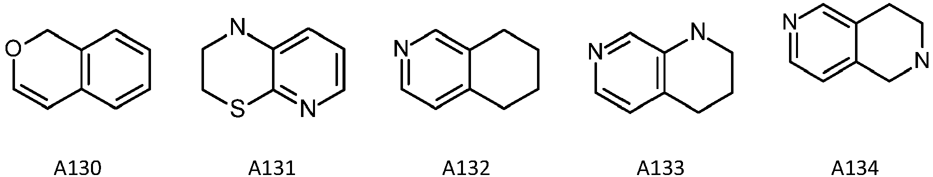 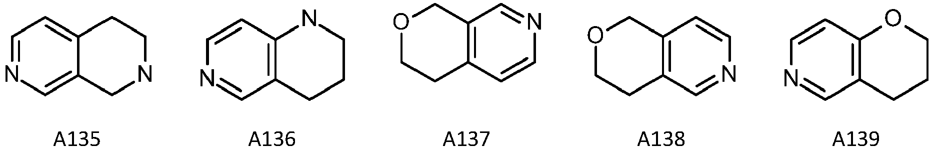 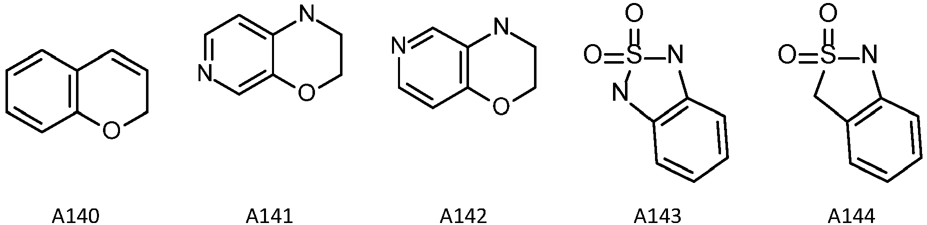 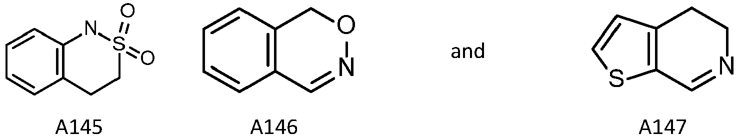 wherein, when **n** is 1, said C₉-C₁₀-carbocyclyl ring or 9- or 10-membered heterocyclyl ring may be substituted with up to four substituents in addition to **L**, said substituents being independently selected from the group consisting of halogen and C₁-C₃-alkyl;
**n** is 0 or 1;
**L** is a substituent selected from the group consisting of halogen, cyano, C₁-C₆-alkyl, C₁-C₆-haloalkyl, C₁-C₆-alkoxy, C₁-C₆-alkylthio, arylsulfenyl, C₁-C₆-alkylsulfinyl, arylsulfinyl, C₁-C₆-alkylsulfonyl, arylsulfonyl, C₃-C₁₀-carbocyclyl, 3- to 10-membered-heterocyclyl, aryl, heteroaryl, =N(OR^{a}), N(OR^{a})C(=O)OR^{a}, N(OR^{a})C(=O)R^{a}, -SO₂R^{a}, -SO₂N(R^{a})₂, -OC(=O)R^{a}, -N(R^{a})₂, - NR^{a}C(=O)OR^{a}, -NR^{a}C(=O)N(R^{a})₂, -NR^{a}C(=O)R^{a}, -NR^{a}C(=S)R^{a}, -NR^{a}C(=S)N(R^{a})₂, -NR^{a}C(=NR^{a})R^{a}, - N=C-N(R^{a})₂, -NR^{a}S(=O)₂R^{a}, -OC(=O)N(R^{a})₂, -C(=NR^{a})R^{a}, -C(=O)R^{a}, -C(=O)(OR^{a}), -C(=O)N(R^{a})₂, - C(=O)NR^{a}N(R^{a})₂, -C(=O)N(OR^{a})R^{a}, -C=NOR^{a}, -C(=S)N(R^{a})₂, , -C₁-Cr₆-alkyl-N(R^{a})₂, -C₁-C₈-alkyl-C(=O)N(R^{a})₂, -C₁-C₆-alkyl-NR^{a}C(=O)OR^{a}, -C₁-C₆-alkyl-NR^{a}C(=O)R^{a}, -C₁-C₆-alkyl-NR^{a}C(=O)N(R^{a})₂, - C₁-C₆-alkyl-N(OR^{a})C(=O)R^{a}, -C₁-C₆-alkyl-C(=O)N(OR^{a})R^{a}, -C₁-C₆-alkyl-NR^{a}C(=S)N(R^{a})₂, -C₁-C₆-alkyl-NR^{a}C(=O)R^{a} and -C₁-C₆-alkyl-NR^{a}S(=O)₂R^{a}, wherein said C₁-C₆-alkyl, C₁-C₆-haloalkyl, C₁-C₆-alkoxy, C₁-C₆-alkylthio, arylsulfenyl, C₁-C₆-alkylsulfinyl, arylsulfinyl, C₁-C₆-alkylsulfonyl, arylsulfonyl, C₃-C₁₀-carbocyclyl, 3- to 10-membered-heterocyclyl, aryl, heteroaryl, -C₁-Cr₆-alkyl-N(R^{a})₂, -C₁-C₆-alkyl-C(=O)N(R^{a})₂, -C₁-C₆-alkyl-NR^{a}C(=O)OR^{a}, -C₁-C₆-alkyl-NR^{a}C(=O)R^{a}, -C₁-C₆-alkyl-NR^{a}C(=O)N(R^{a})₂, - C₁-C₆-alkyl-N(OR^{a})C(=O)R^{a}, -C₁-C₆-alkyl-C(=O)N(OR^{a})R^{a}, -C₁-C₆-alkyl-NR^{a}C(=S)N(R^{a})₂, -C₁-C₆-alkyl-NR^{a}C(=O)R^{a} and -C₁-C₆-alkyl-NR^{a}S(=O)₂R^{a} substituent is itself optionally substituted, one or more times, in the same way or differently, with R5;
**R5** is a substituent selected from the group consisting of halogen, cyano, hydroxy, mercapto, sulfinyl, sulfonyl, amino, C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy, nitro, - C(=O)R^{a}, -C(=O)OR^{a}, -C(=O)N(R^{a})₂, -C(=S)N(R^{a})₂, -C(=NR^{a})R^{a}, -NR^{a}C(=O)OR^{a}, -NR^{a}C(=O)N(R^{a})₂, - NR^{a}C(=O)R^{a}, -NR^{a}C(=S)R^{a}, -NR^{a}C(=S)N(R^{a})₂, -NR^{a}C(=NR^{a})R^{a}, -OC(=O)R^{a}, -OC(=O)N(R^{a})₂, - NR^{a}S(=O)₂R^{a}, -S(=O)₂R^{a}, -S(=O)₂N(R^{a})₂ and -P(=O)(OR^{a})₂; wherein said C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b} ; when two R5 substituents are bound to a common carbon, they may form together C=O, C₃-C₁₀-carbocyclyl or 3- to 10-membered-heterocyclyl;
R^{a} is, independently from each other, a substituent, which is identical or different, selected from the group consisting of hydrogen, halogen, cyano, hydroxy, mercapto, sulfinyl, sulfonyl, amino, C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy, nitro, -C₁-C₆-alkyl-C₃-C₁₀-carbocyclyl, -C₁-C₆-alkyl-aryl, -C₁-C₆-alkyl-C₁-C₆-alkoxy, -C(=O)R^{b}, -C(=O)OR^{b}, -C(=O)N(R^{b})₂, -C(=S)N(R^{b})₂, - NR^{b}C(=O)OR^{b}, -NR^{b}C(=O)N(R^{b})₂, -NR^{b}C(=O)R^{b}, -NR^{b}C(=S)R^{b}, -OC(=O)N(R^{b})₂, -NR^{b}S(=O)₂R^{b}, - S(=O)₂R^{b}, -S(=O)₂N(R^{b})₂ and -P(=O)(OR^{b})₂ ; wherein said C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b};
R^{b} is, independently from each other, a substituent, which is identical or different, selected from the group consisting of hydrogen, halogen, cyano, -OR^{c}, -N(R^{c})₂, -SR^{c}, -S(=O)R^{c}, -S(=O)OR^{C}, - S(=O)₂R^{c}, -S(=O)₂OR^{c}, C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy, nitro, - C(=O)R^{c}, -C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, - NR^{c}C(=S)R^{c}, -OC(=O)N(R^{c})₂, -NR^{c}S(=O)₂R^{c} -S(=O)₂N(R^{c})₂ and -P(=O)(OR^{c})₂; wherein said C₃-C₁₀-carbocyclyl, 3- to 10-membered-heterocyclyl, aryl, heteroaryl substituent is itself optionally substituted, one or more times, in the same way or differently with CN, halogen, C₁-C₆-alkyl, C₁-C₆-haloalkyl or C₁-C₆-alkoxy;
R^{c} is, independently from each other, a substituent, which is identical or different, selected from the group consisting of hydrogen, aryl and C₁-C₆-alkyl,
   provided that
   (a) when **X**is **F**, **n** is **1** and **A** is 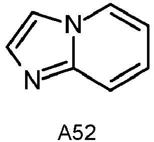 **L** is not a substituent selected from the group consisting of C₁-C₆-alkyl, aryl, 3- to 10-membered heterocyclyl and -C(=O)R^{a} with R^{a} being a 3- to 10-membered heterocyclyl,
   (b) when **X** is F and **n** is 0, **A** is not selected from the group consisting of: 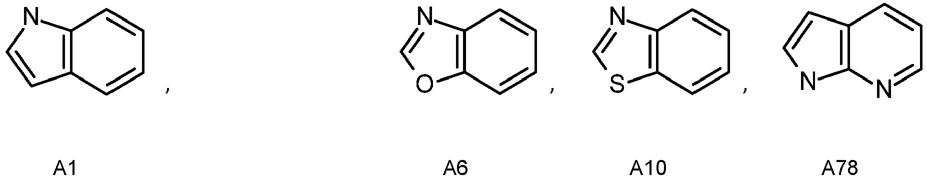 and 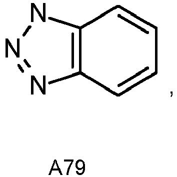
   (c) when **X** is F, **n** is 1and **A** is selected from the group consisting of 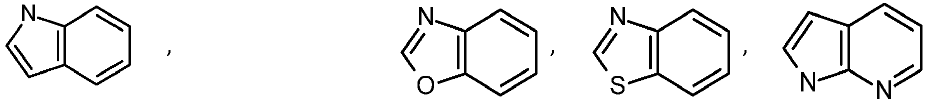 and 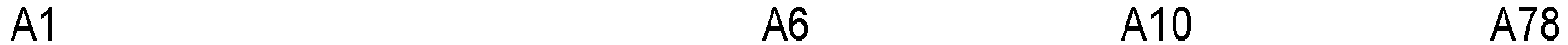 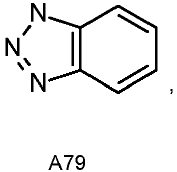 **L** is not a substituent selected from the group consisting of C₁-C₆-alkyl, -C(=O)(OR^{a}) with R^{a} being a C₁-C₆-alkyl and -SO₂R^{a} with R^{a} being an aryl,
   (d) when **X** is F and n is 0, **A** is not 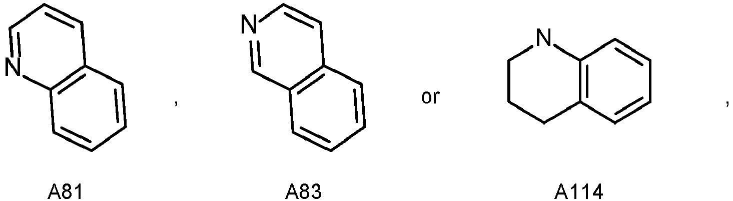
   (e) when **X** is **F**, n is 1 and **A** is 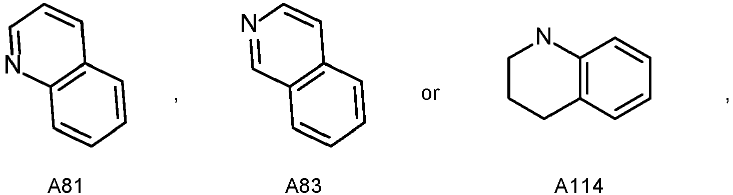 **L** is not a substituent selected from the group consisting of unsubstituted C₁-C₆-alkyl; C₁-C₆-alkyl substituted by one or more **R5** with **R5** being selected from the group consisting of unsubtituted aryl, unsubstituted heteroaryl, aryl substituted with one to three Rb with Rb being unsubstituted C₁-C₆-alkyl, and heteroaryl substituted with one to three Rb with Rb being unsubstituted C₁-C₆-alkyl; C₁-C₆-haloalkyl; halogen; cyano; -SO₂R^{a} with R^{a} being an unsubstituted C₁-C₆-alkyl; unsubstituted C₃-C₁₀-carbocyclyl; unsubstituted aryl; unsubstituted hereroaryl; aryl substituted by one or three **R5** with **R5** being an unsubstituted C₁-C₆-alkyl; and heteroaryl substituted by one or three **R5** with **R5** being an unsubstituted C₁-C₆-alkyl,
   (f) when **L** is -N(Ra)₂ with the two Ra being hydrogen, L is not attached to the atom of **A** that bonds **A** to the oxadiazole moiety,
   (g) compound of formula (I') is not
2-Pyridinecarboxamide,N-methyl-4-[[3-methyl-2-[5-(trifluoromethyl)-1,2,4-oxadiazol-3-yl]benzo[b]thien-6-yl]oxy]- (CAS-2222313-38-2),
Benzothiazole, 6-methoxy-2-[5-(trifluoromethyl)-1,2,4-oxadiazol-3-yl]- (CAS-897805-25-3), Thieno[2,3-c]pyridine, 4-(4-chlorophenoxy)-2-[5-(trifluoromethyl)-1,2,4-oxadiazol-3-yl]- (CAS-251995-30-9),
Pyrazolo[1,5-a]pyrimidine, 3-[5-(trifluoromethyl)-1,2,4-oxadiazol-3-yl]-7-[3-(trifluoromethyl)phenyl]-(CAS-159224-00-7),
1H-Indole, 3-(2-chloro-3-thienyl)-5-[5-(trifluoromethyl)-1,2,4-oxadiazol-3-yl]- (CAS-2252389-71-0), Imidazo[1,2-a]pyridine, 2-(phenylmethyl)-7-[5-(trifluoromethyl)-1,2,4-oxadiazol-3-yl]- (CAS-2170611-35-3),
1H-Pyrrolo[2,3-b]pyridine, 5-[5-(trifluoromethyl)-1,2,4-oxadiazol-3-yl]- (CAS-2170611-21-7),
1H-Pyrrolo[2,3-b]pyridine, 1-(phenylsulfonyl)-5-[5-(trifluoromethyl)-1,2,4-oxadiazol-3-yl]- (CAS-2170611-01-3),
2,1,3-Benzoxadiazole, 4-[3-(4-morpholinylmethyl)-1-azetidinyl]-6-[5-(trifluoromethyl)-1,2,4-oxadiazol-3-yl]- (CAS-1807986-73-7),
Tricyclo[3.3.1.13,7]decan-1-ol, 4-[[5-[5-(trifluoromethyl)-1,2,4-oxadiazol-3-yl]-1H-pyrrolo[2,3-b]pyridin-4-yl]amino]- (CAS-944120-75-6),
1H-Inden-1-amine, 2,3-dihydro-5-[5-(trifluoromethyl)-1,2,4-oxadiazol-3-yl]-, (1S)- (CAS-916303-83-8), 1H-Inden-1-amine, 2,3-dihydro-5-[5-(trifluoromethyl)-1,2,4-oxadiazol-3-yl]-, hydrochloride (1:1), (1S)-(CAS-916210-97-4),
Carbamic acid, N-[(1S)-2,3-dihydro-5-[5-(trifluoromethyl)-1,2,4-oxadiazol-3-yl]-1H-inden-1-yl]-, 1,1-dimethylethyl ester (CAS-916209-96-6),
1H-Pyrrolo[2,3-b]pyridine, 2-[1-methyl-5-[5-(trifluoromethyl)-1,2,4-oxadiazol-3-yl]-1H-indol-3-yl]-( CAS-479551-10-5),
1H-Indole, 2,3-dihydro-7-methyl-1-[3-(3-methyl-5-isoxazolyl)propyl]-5-[5-(trifluoromethyl)-1,2,4-oxadiazol-3-yl]- (CAS-178396-27-5),
1H-Indole, 7-methyl-1-[5-(3-methyl-5-isoxazolyl)pentyl]-5-[5-(trifluoromethyl)-1,2,4-oxadiazol-3-yl]-(CAS-178396-24-2),
1H-Indole, 7-methyl-1-[4-(3-methyl-5-isoxazolyl)butyl]-5-[5-(trifluoromethyl)-1,2,4-oxadiazol-3-yl]-(CAS-178396-22-0),
1H-Indole, 1-[3-(3-methyl-5-isoxazolyl)propyl]-5-[5-(trifluoromethyl)-1,2,4-oxadiazol-3-yl]- (CAS-178396-20-8),
Pyrazino[2,3-c]pyridazine, 5-[(1R)-1-(2,5-dichlorophenyl)ethyl]-5,6,7,8-tetrahydro-3-[5-(trifluoromethyl)-1,2,4-oxadiazol-3-yl]-(CAS-1690421-46-5),
Pyrazino[2,3-c]pyridazine, 5-[(2,5-dichlorophenyl)methyl]-5,6,7,8-tetrahydro-3-[5-(trifluoromethyl)-1,2,4-oxadiazol-3-yl]-(CAS-1690421-40-9),
1,2,4-Oxadiazole, 3-[7-(bromomethyl)-2-naphthalenyl]-5-(trifluoromethyl)- (CAS-1172849-62-5), 1,6-Naphthyridine, 5,6,7,8-tetrahydro-3-[5-(trifluoromethyl)-1,2,4-oxadiazol-3-yl]-, 2,2,2-trifluoroacetate (1:1) (CAS-741736-97-0),
1,6-Naphthyridine, 5,6,7,8-tetrahydro-3-[5-(trifluoromethyl)-1,2,4-oxadiazol-3-yl]- (CAS-741736-96-9), 1-Butanone, 3-amino-4-(2,5-difluorophenyl)-1-[7,8-dihydro-3-[5-(trifluoromethyl)-1,2,4-oxadiazol-3-yl]-1,6-naphthyridin-6(5H)-yl]-, (3R)- (CAS-741736-75-4),
Phthalazine, 1-phenyl-4-[5-(trifluoromethyl)-1,2,4-oxadiazol-3-yl]- (CAS-863601-14-3).
1H-Indole, 7-methyl-1-[4-(3-methyl-5-isoxazolyl)butyl]-5-[5-(trifluoromethyl)-1 ,2,4-oxadiazol-3-yl]-(CAS-178396-22-0),
1H-Indole, 1-[3-(3-methyl-5-isoxazolyl)propyl]-5-[5-(trifluoromethyl)-1,2,4-oxadiazol-3-yl]- (CAS-178396-20-8),
1,2,4-Oxadiazole, 3-[7-methyl-2-[4-(3-methyl-5-isoxazolyl)butyl]-5-benzofuranyl]-5-(trifluoromethyl)-(CAS-178396-09-3),
1H-Benzimidazole, 2-(1-piperazinyl)-7-[5-(trifluoromethyl)-1,2,4-oxadiazol-3-yl]- (CAS-1436430-11-3),
1H-Benzimidazol-2-amine, N-(1-methylethyl)-7-[5-(trifluoromethyl)-1,2,4-oxadiazol-3-yl]- (CAS-1436430-10-2),
1H-Benzimidazole, 2-(2-pyridinyl)-7-[5-(trifluoromethyl)-1,2,4-oxadiazol-3-yl]- (CAS-1436430-09-9),
1H-Benzimidazole, 2-ethoxy-7-[5-(trifluoromethyl)-1,2,4-oxadiazol-3-yl]-(CAS-1436430-08-8), Pyrazino[2,3-c]pyridazine, 5-[(1R)-1-(2,5-dichlorophenyl)ethyl]-5,6,7,8-tetrahydro-3-[5-(trifluoromethyl)-1,2,4-oxadiazol-3-yl]- (CAS-1690421-46-5),
Pyrazino[2,3-c]pyridazine, 5-[(2,5-dichlorophenyl)methyl]-5,6,7,8-tetrahydro-3-[5-(trifluoromethyl)-1,2,4-oxadiazol-3-yl]- (CAS-1690421-40-9),
1,2,4-Oxadiazole, 3-[7-(bromomethyl)-2-naphthalenyl]-5-(trifluoromethyl)- (CAS-1172849-62-5), 1,6-Naphthyridine, 5,6,7,8-tetrahydro-3-[5-(trifluoromethyl)-1,2,4-oxadiazol-3-yl]-, 2,2,2-trifluoroacetate (1:1) (CAS-741736-97-0),
1,6-Naphthyridine, 5,6,7,8-tetrahydro-3-[5-(trifluoromethyl)-1,2,4-oxadiazol-3-yl]- (CAS-741736-96-9), 1-Butanone, 3-amino-4-(2,5-difluorophenyl)-1-[7,8-dihydro-3-[5-(trifluoromethyl)-1,2,4-oxadiazol-3-yl]-1,6-naphthyridin-6(5H)-yl]-, (3R)- (CAS-741736-75-4),
1-Naphthaleneacetic acid, 7-[5-(trifluoromethyl)-1,2,4-oxadiazol-3-yl]-, methyl ester (CAS-149169-37-9),
1-Naphthaleneacetic acid, .alpha.-(methoxymethylene)-7-[5-(trifluoromethyl)-1,2,4-oxadiazol-3-yl]-, methyl ester, (E)- (9CI) (CAS-149169-14-2), and
Phthalazine, 1-phenyl-4-[5-(trifluoromethyl)-1,2,4-oxadiazol-3-yl]- (CAS-863601-14-3).

The invention relates to a composition comprising at least one compound of formula (I') as defined herein and at least one agriculturally suitable auxiliary.

The invention also relates to the use of a compound of formula (I) or (I') as defined herein for controlling phytopathogenic fungi.

The invention relates to a method for controlling unwanted phytopathogenic microorganisms which comprises the step of applying at least one compound of formula (I) or (I') as defined herein or a composition as defined herein to the plants, plant parts, seeds, fruits or to the soil in which the plants grow.

The invention also relates to processes for preparing compounds of formula (I') as disclosed herein.

These and other features of the present teachings are set forth herein.

### DETAILED DESCRIPTION

### Definitions

The term "alkyl" as used herein in the context of alkyl or alkylsulfonyl, alkylsulfinyl, alkylthio, alkylamino, for example, is to be understood as preferably meaning branched and unbranched alkyl, meaning e.g. methyl, ethyl, n-propyl, iso-propyl, n-butyl, iso-butyl, tert-butyl, sec-butyl, pentyl, *iso*pentyl, hexyl, heptyl, octyl, nonyl and decyl and the isomers thereof.

The term "haloalkyl" as used herein is to be understood as preferably meaning branched and unbranched alkyl, as defined *supra,* in which one or more of the hydrogen substituents is replaced in the same way or differently with halogen. Particularly preferably, said haloalkyl is, e.g. chloromethyl, fluoropropyl, fluoromethyl, difluoromethyl, trichloromethyl, 2,2,2-trifluoroethyl, pentafluoroethyl, bromobutyl, trifluoromethyl, iodoethyl, and isomers thereof.

The term "alkoxy" as used herein is to be understood as preferably meaning branched and unbranched alkoxy, meaning e.g. methoxy, ethoxy, propyloxy, iso-propyloxy, butyloxy, iso-butyloxy, tert-butyloxy, sec-butyloxy, pentyloxy, iso-pentyloxy, hexyloxy, heptyloxy, octyloxy, nonyloxy, decyloxy, undecyloxy and dodecyloxy and the isomers thereof.

The term "haloalkoxy" as used herein is to be understood as preferably meaning branched and unbranched alkoxy, as defined *supra,* in which one or more of the hydrogen substituents is replaced in the same way or differently with halogen, e.g. chloromethoxy, fluoromethoxy, pentafluoroethoxy, fluoropropyloxy, difluoromethyloxy, trichloromethoxy, 2,2,2-trifluoroethoxy, bromobutyloxy, trifluoromethoxy, iodoethoxy, and isomers thereof.

Unless provided differently, the term "carbocyclyl" as used herein refers to a non-aromatic mono- or polycyclic (fused, spiro or bridged) carbon containing ring, which may be saturated or unsaturated, having 3 to 10 ring carbon atoms. Examples of carbocyclyl include cycloalkyl and cycloalkenyl groups. Examples of saturated cycloalkyl include but are not limited to cyclopropyl, cyclobutyl, cyclopentyl, cyclohexyl, cycloheptyl, cyclooctyl, cyclononyl and cyclodecyl group. Examples of unsaturated carbocyclyl group include but are not limited to cyclopropenyl, cyclobutenyl, cyclopentenyl, cyclohexenyl, cycloheptenyl, cyclooctenyl, cyclononenyl, or cyclodecenyl group, wherein the linkage of said cyclolaklyl group to the rest of the molecule can be provided to the double or single bond.

Unless provided differently, the term "heterocyclyl" as used herein refers to three- to ten-membered, preferably three- to nine-membered, saturated or partially unsaturated heterocycles (including mono-, bi- or tricyclic heterocycles) containing one to four heteroatoms independently selected from the group of oxygen, nitrogen and sulphur. If the ring contains more than one oxygen atom, they are not directly adjacent. Examples of heterocyclyl group include but are not limited to oxiranyl, aziridinyl, 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl, 1,2,4-hexahydrotriazin-3-yl. This definition also applies to heterocyclyl as part of a composite substituent, for example heterocyclylalkyl etc., unless defined elsewhere.

The term "halogen" or "Hal" as used herein is to be understood as meaning fluorine, chlorine, bromine or iodine.

The term "alkenyl" as used herein is to be understood as preferably meaning branched and unbranched alkenyl, e.g. a vinyl, propen-1-yl, propen-2-yl, but-1-en-1-yl, but-1-en-2-yl, but-2-en-1-yl, but-2-en-2-yl, but-1-en-3-yl, 2-methyl-prop-2-en-1-yl, or 2-methyl-prop-1-en-1-yl group.

The term "alkynyl" as used herein is to be understood as preferably meaning branched and unbranched alkynyl, e.g. an ethynyl, prop-1-yn-1-yl, but-1-yn-1-yl, but-2-yn-1-yl,or but-3-yn-1-yl group.

The term "aryl" as used herein refers to an aromatic, hydrocarbon, ring system, comprising from 6 to 12 carbon atoms, preferably from 6 to 10 carbon atoms. The ring system may be monocyclic or fused polycyclic (e.g. bicyclic or tricyclic) aromatic ring system. Examples of aryl include but are not limited to phenyl, azulenyl, naphthyl, biphenyl and fluorenyl. It is further understood that when said aryl group is substituted with one or more substituents, said substituent(s) may be at any positions on said aryl ring(s). Particularly, in the case of aryl being a phenyl group, said substituent(s) may occupy one or both ortho positions, one or both meta positions, or the para position, or any combination of these positions. This definition also applies to aryl as part of a composite substituent (e.g. aryloxy).

The term "heteroaryl" as used herein refers to an aromatic ring system containing from 5 to 12 member atoms, of which carbons and one or more heteroatoms which may be identical or different selected from O, N and S. If the ring contains more than one oxygen atom, they are not directly adjacent. Heteroaryl may be monocyclic or polycyclic (e.g. bicyclic or tricyclic). A monocyclic heteroaryl may have 1 to 4 heteroatoms in the ring, while a polycyclic heteroaryl ring may have 1 to 10 heteroatoms. Bicyclic heteroaryl rings may contain from 8 to 12 member atoms (carbon and heteroatoms). Monocyclic heteroaryl may contain from 5 to 8 member atoms. Examples of heteroaryl include but are not limited to thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl *etc.,* and benzo derivatives thereof, such as, e.g., benzofuranyl, benzothienyl, benzoxazolyl, benzimidazolyl, benzotriazolyl, indazolyl, indolyl, isoindolyl, *etc.*; or pyridyl, pyridazinyl, pyrimidinyl, pyrazinyl, triazinyl, *etc.,* and benzo derivatives thereof, such as, for example, quinolinyl, isoquinolinyl, *etc.*; or azocinyl, indolizinyl, purinyl, *etc.,* and benzo derivatives thereof; or cinnolinyl, phthalazinyl, quinazolinyl, quinoxalinyl, naphthpyridinyl, pteridinyl, carbazolyl, acridinyl, phenazinyl, phenothiazinyl, phenoxazinyl, xanthenyl, or oxepinyl, etc. It is further understood that in the case in which said heteroaryl group is substituted with one or more substituents, said substituent(s) may occupy any one or more positions on said heteroaryl ring(s). Particularly, in the case of heteroaryl being a pyridyl group, for example, said substituent(s) may occupy any one or more of positions 2, 3, 4, 5, and/or 6 with respect to the nitrogen atom in the pyridine ring. This definition also applies to heteroaryl as part of a composite substituent (e.g. heteroaryloxy).

As used herein, the term "C₁-C₆", e.g. in the context of the definition of "C₁-C₆-alkyl", or "C₁-C₆-alkoxy", is to be understood as meaning a group having a finite number of carbon atoms of 1 to 6, *i.e.* 1, 2, 3, 4, 5, or 6 carbon atoms.

As used herein, when a group is said to be "substituted", the group may be substituted with one or more substituents. It is understood that this does not apply to moieties such as hydrogen, halogen, CN or the like. The expression "one or more substituents" refers to a number of substituents that ranges from one to the maximum number of substituents possible based on the number of available bonding sites, provided that the conditions of stability and chemical feasibility are met.

The term "leaving group" as used herein is to be understood as meaning a group which is displaced from a compound in a substitution or an elimination reaction, for example a halogen atom, a trifluoromethanesulphonate ("triflate") group, alkoxy, methanesulphonate, p-toluenesulphonate, etc..

The present invention relates to the use of compounds of the formula (I) or salts, N-oxides or solvates thereof for controlling unwanted phytopathogenic microorganisms, in particular for controlling phytopathogenic fungi (i.e. as fungicides): 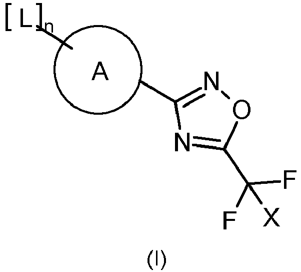 wherein
X is F or CI;
A is an aromatic or partially unsaturated fused bicyclic C₇-C₁₂-carbocyclyl ring or an aromatic or partially unsaturated fused bicyclic 7- to 12-membered heterocyclyl ring, wherein, when n is 1, said C₇-C₁₂-carbocyclyl or 7- to 12-membered heterocyclyl ring may be substituted with up to four substituents in addition to L, said substituents being independently selected from the group consisting of halogen and C₁-C₃-alkyl;
**n** is 0 or 1;
**L** is a substituent selected from the group consisting of halogen, cyano, C₁-C₆-alkyl, C₁-C₆-haloalkyl, C₁-C₆-alkoxy, C₁-C₆-alkylthio, arylsulfenyl, C₁-C₆-alkylsulfinyl, arylsulfinyl, C₁-C₆-alkylsulfonyl, arylsulfonyl, C₃-C₁₀-carbocyclyl, 3- to 10-membered-heterocyclyl, aryl, heteroaryl, =N(OR^{a}), N(OR^{a})C(=O)OR^{a}, N(OR^{a})C(=O)R^{a}, -SO₂R^{a}, -SO₂N(R^{a})₂, -OC(=O)R^{a}, -N(R^{a})₂, - NR^{a}C(=O)OR^{a}, -NR^{a}C(=O)N(R^{a})₂, -NR^{a}C(=O)R^{a}, -NR^{a}C(=S)R^{a}, -NR^{a}C(=S)N(R^{a})₂, -NR^{a}C(=NR^{a})R^{a}, - N=C-N(R^{a})₂, -NR^{a}S(=O)₂R^{a}, -OC(=O)N(R^{a})₂, -C(=NR^{a})R^{a}, -C(=O)R^{a}, -C(=O)(OR^{a}), -C(=O)N(R^{a})₂, - C(=O)NR^{a}N(R^{a})₂, -C(=O)N(OR^{a})R^{a}, -C=(NOR^{a})R^{a}, -C(=S)N(R^{a})₂, , -C₁-C₆-alkyl-N(R^{a})₂, -C₁-C₆-alkyl-C(=O)N(R^{a})₂, -C₁-C₆-alkyl-NR^{a}C(=O)OR^{a}, -C₁-C₆-alkyl-NR^{a}C(=O)R^{a}, -C₁-C₆-alkyl-NR^{a}C(=O)N(R^{a})₂, - C₁-C₆-alkyl-N(OR^{a})C(=O)R^{a}, -C₁-C₆-alkyl-C(=O)N(OR^{a})R^{a}, -C₁-C₆-alkyl-NR^{a}C(=S)N(R^{a})₂, -C₁-C₆-alkyl-NR^{a}C(=O)R^{a} and -C₁-C₆-alkyl-NR^{a}S(=O)₂R^{a}, wherein said C₁-C₆-alkyl, C₁-C₆-haloalkyl, C₁-C₆-alkoxy, C₁-C₆-alkylthio, arylsulfenyl, C₁-C₆-alkylsulfinyl, arylsulfinyl, C₁-C₆-alkylsulfonyl, arylsulfonyl, C₃-C₁₀-carbocyclyl, 3- to 10-membered-heterocyclyl, aryl, heteroaryl, -C₁-C₆-alkyl-N(R^{a})₂, -C₁-C₆-alkyl-C(=O)N(R^{a})₂, -C₁-C₆-alkyl-NR^{a}C(=O)OR^{a}, -C₁-C₆-alkyl-NR^{a}C(=O)R^{a}, -C₁-C₆-alkyl-NR^{a}C(=O)N(R^{a})₂, - C₁-C₆-alkyl-N(OR^{a})C(=OR^{a}, -C₁-C₆-alkyl-C(=O)N(OR^{a})R^{a}, -C₁-C₆-alkyl-NR^{a}C(=S)N(R^{a})₂, -C₁-C₆-alkyl-NR^{a}C(=O)R^{a} and -C₁-C₆-alkyl-NR^{a}S(=O)₂R^{a} substituent is itself optionally substituted, one or more times, in the same way or differently, with **R5**;
**R5** is a substituent selected from the group consisting of halogen, cyano, hydroxy, mercapto, sulfinyl, sulfonyl, amino, C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy, nitro, - C(=O)R^{a}, -C(=O)OR^{a}, -C(=O)N(R^{a})₂, -C(=S)N(R^{a})₂, -C(=NR^{a})R^{a}, -NR^{a}C(=O)OR^{a}, -NR^{a}C(=O)N(R^{a})₂, - NR^{a}C(=O)R^{a}, -NR^{a}C(=S)R^{a}, -NR^{a}C(=S)N(R^{a})₂, -NR^{a}C(=NR^{a})R^{a}, -OC(=O)R^{a}, -OC(=O)N(R^{a})₂, - NR^{a}S(=O)₂R^{a}, -S(=O)₂R^{a}, -S(=O)₂N(R^{a})₂ and -P(=O)(OR^{a})₂ ; wherein said C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b} ; when two **R5** substituents are bound to a common carbon, they may form together C=O, C₃-C₁₀-carbocyclyl or 3- to 10-membered-heterocyclyl;
R^{a} is, independently from each other, a substituent, which is identical or different, selected from the group consisting of hydrogen, halogen, cyano, hydroxy, mercapto, sulfinyl, sulfonyl, amino, C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy, nitro, -C₁-C₆-alkyl-C₃-C₁₀-carbocyclyl, -C₁-C₆-alkyl-aryl, -C₁-C₆-alkyl-C₁-C₆-alkoxy, -C(=O)R^{b}, -C(=O)OR^{b}, -C(=O)N(R^{a})₂, -C(=S)N(R^{b})₂, - NR^{b}C(=O)OR^{b}, -NR^{b}C(=O)N(R^{b})₂, -NR^{b}C(=O)R^{b}, -NR^{b}C(=S)R^{b}, -OC(=O)N(R^{b})₂, -NR^{b}S(=O)₂R^{b}, - S(=O)₂R^{b}, -S(=O)₂N(R^{b})₂ and -P(=O)(OR^{b})₂ ; wherein said C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b};
R^{b} is, independently from each other, a substituent, which is identical or different, selected from the group consisting of hydrogen, halogen, cyano, -OR^{c}, -N(R^{c})₂, -SR^{c}, -S(=O)R^{c}, -S(=O)OR^{c}, - S(=O)₂R^{c}, -S(=O)₂OR^{c}, C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy, nitro, - C(=O)R^{c}, -C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, - NR^{c}C(=S)R^{c}, -OC(=O)N(R^{c})₂, -NR^{c}S(=O)₂R^{c}, -S(=O)₂N(R^{c})₂ and -P(=O)(OR^{c})₂; wherein said C₃-C₁₀-carbocyclyl, 3- to 10-membered-heterocyclyl, aryl, heteroaryl substituent is itself optionally substituted, one or more times, in the same way or differently with CN, halogen, C₁-C₆-alkyl, C₁-C₆-haloalkyl or C₁-C₆-alkoxy;
R^{c} is, independently from each other, a substituent, which is identical or different, selected from the group consisting of hydrogen, aryl and C₁-C₆-alkyl,
   provided that when **A** is naphthyl, **n** is 1 and **X** is F, **L** is not a substituent selected from the group consisting of
   - C₁-C₆-alkyl substituted with one or more **R5**, wherein at least one of said **R5** is selected from the group consisting of C₁-C₆-alkoxy, aryl, heteroaryl, C₃-C₁₀-carbocyclyl, 3- to 10-membered heterocyclyl, aryloxy and heteroaryloxy,
   - C₁-C₆-alkoxy substituted with or more **R5**, wherein at least one of said **R5** is selected from the group consisting of aryl, heteroaryl, C₃-C₁₀-carbocyclyl, 3- to 10-membered heterocyclyl, aryloxy and heteroaryloxy,
   - -N(R^{a})₂ wherein at least one R^{a} is aryl, heteroaryl, C₃-C₁₀-carbocyclyl or 3- to 10-membered heterocyclyl,
   - N(R^{a})₂ wherein at least one R^{a} is C₁-C₆-alkyl substituted with one or more R^{b}, at least one of said R^{b} being an aryl, heteroaryl, C₃-C₁₀-carbocyclyl or 3- to 10-membered heterocyclyl,
   - C₁-C₆-alkyl-N(R^{a})₂ wherein at least one R^{a} is aryl, heteroaryl, C₃-C₁₀-carbocyclyl or 3-to 10-membered heterocyclyl,
   - C₃-C₁₀-carbocyclyl,
   - 3- to 10-membered heterocyclyl and
   - heteroaryl.

As used herein, in connection with the definition of A of the formula (I) or (I'), the term "carbocyclyl ring" designates a ring composed of carbon atoms.

As used herein, in connection with the definition of A of the formula (I) or (I'), the term "heterocyclcyl ring" designates a ring composed of carbon atoms and heteroatoms (one to four heteroatoms independently selected from the group of oxygen, nitrogen and sulphur).

In the above formula (I), A is preferably an aromatic or partially unsaturated fused bicyclic 9 or 10-membered carbocyclyl ring (i.e. C₉-C₁₀-carbocyclyl ring) or an aromatic or partially unsaturated fused bicyclic 8- to 10-membered, preferably 9 or 10-membered, heterocyclyl ring comprising one to four heteroatoms independently selected in the list consisting of N, O and S.

In some embodiments, the present invention provides for the use as fungicides of compounds of formula (I), *supra,* wherein **L** is a substituent selected from the group consisting of halogen, C₁-C₆-alkyl, C₁-C₆-alkoxy, C₁-C₆-alkylsulfonyl, aryl, -N(R^{a})₂, =N(OR^{a}), -N(OR^{a})C(=O)OR^{a}, -NR^{a}C(=O)OR^{a}, - N(OR^{a})C(=O)R^{a}, -NR^{a}C(=O)R^{a}, -OC(=O)N(R^{a})₂, -C(=O)R^{a}, -C(=O)(OR^{a}), -C(=O)N(R^{a})₂, wherein said C₁-C₆-alkyl, C₁-C₆-alkoxy, C₁-C₆-alkylsulfonyl or aryl substituent is itself optionally substituted, one or more times, in the same way or differently, with **R5**, **R5** being as disclosed above, preferably **R5** is halogen, C₁-C₆-alkoxy or aryl.

In the above formula (I), **A**, **L**, **R^{a}** and **R^{b}** substituents may be as disclosed in relation to compounds of formula (I') below.

The present invention also relates to compounds of the formula (I'): 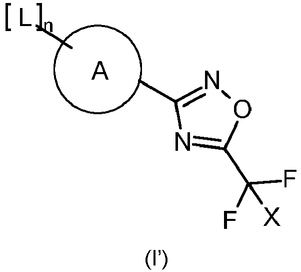 wherein
**X** is F or Cl;
**A** is an aromatic, or partially unsaturated, fused bicyclic C₉-C₁₀-carbocyclyl ring or an aromatic, or partially unsaturated, fused bicyclic 9- or 10-membered heterocyclyl ring selected from the group consisting of: 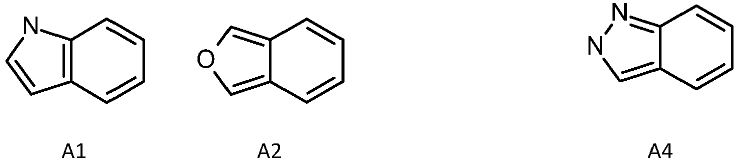 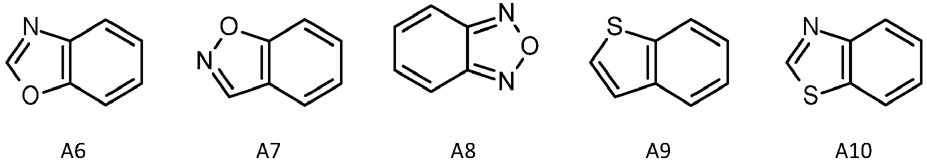 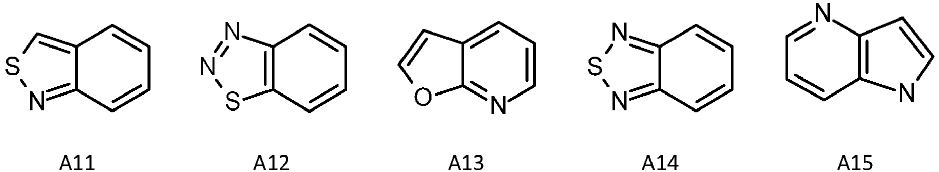 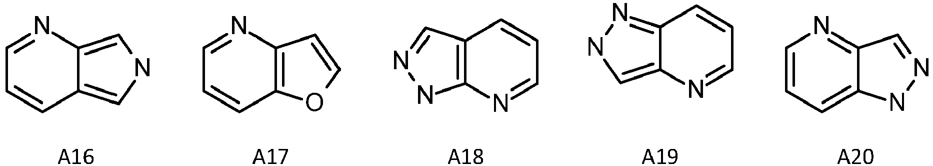 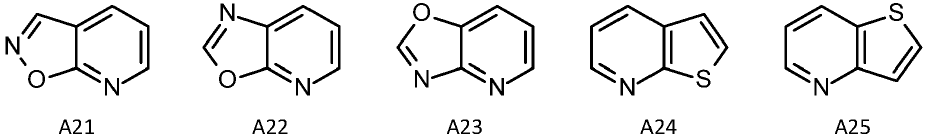 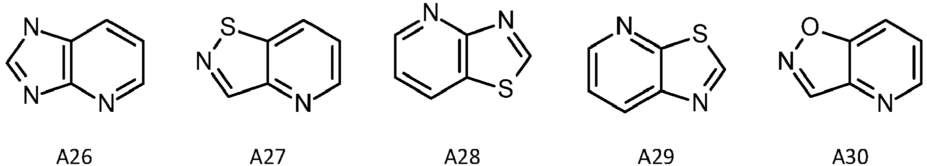 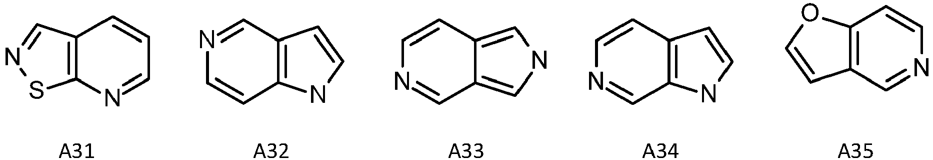 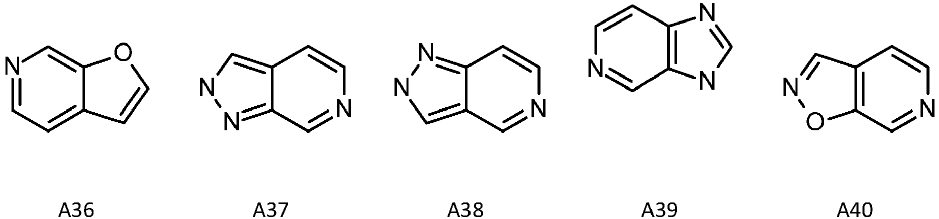 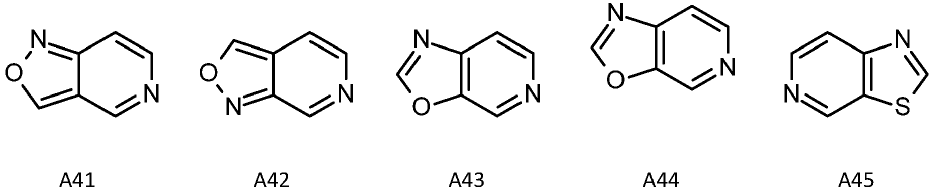 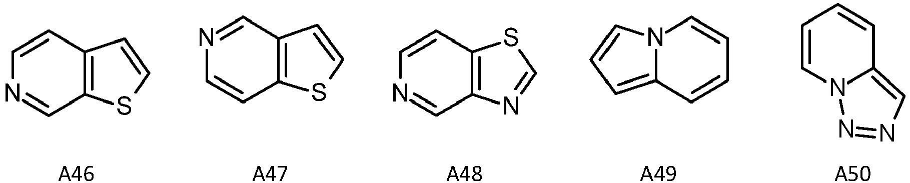 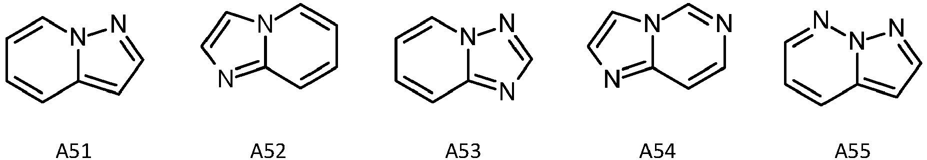 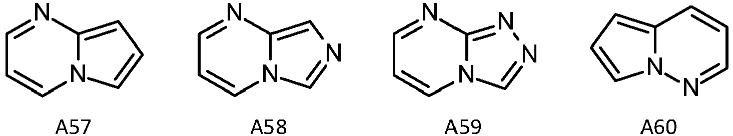 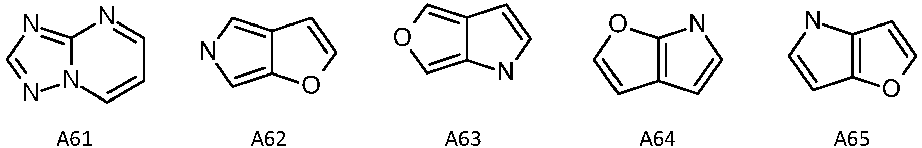 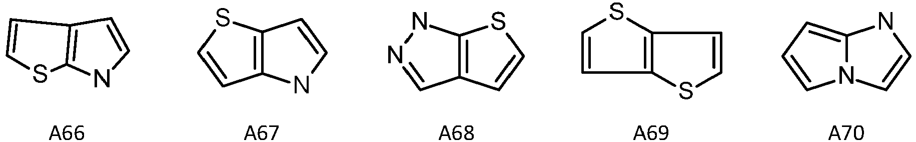 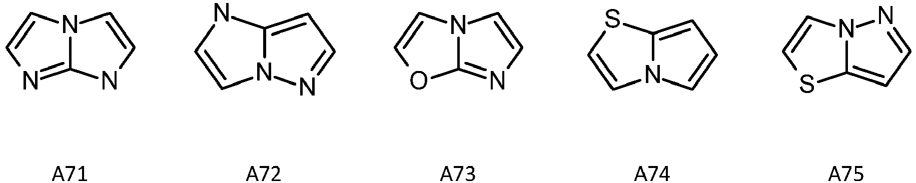 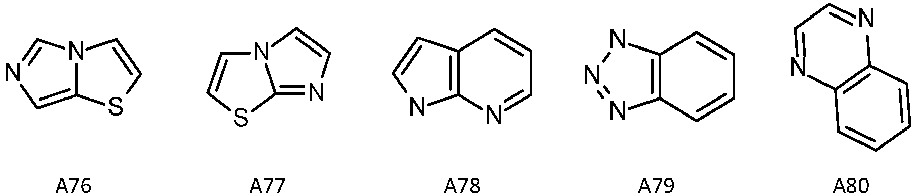 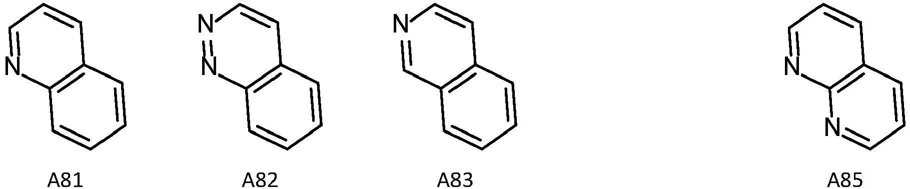 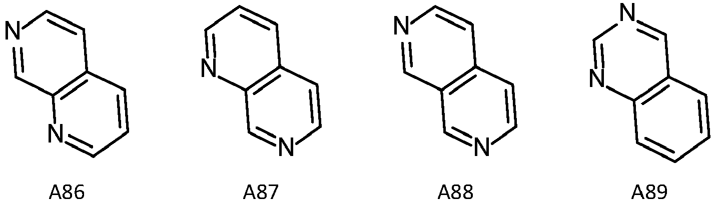 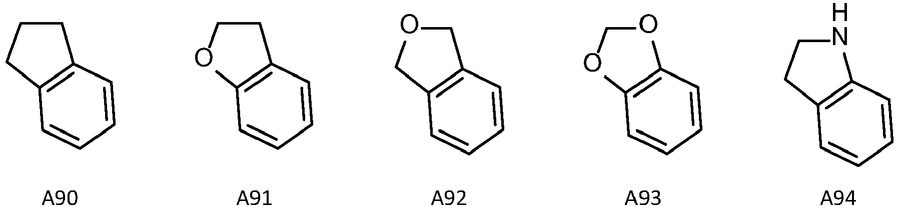 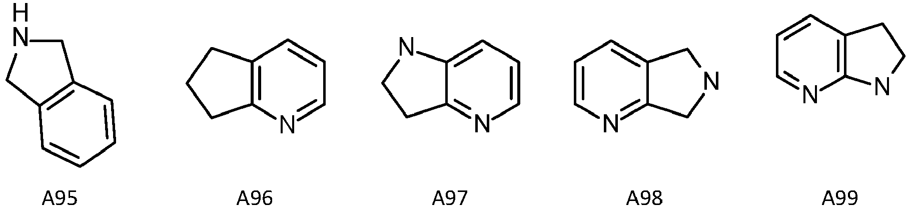 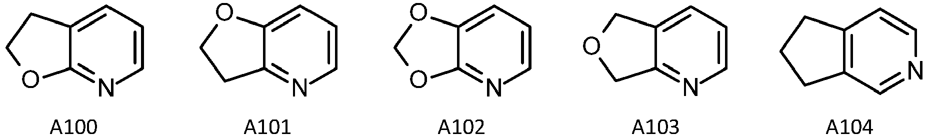 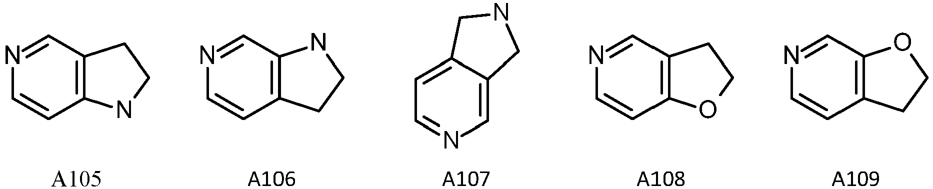 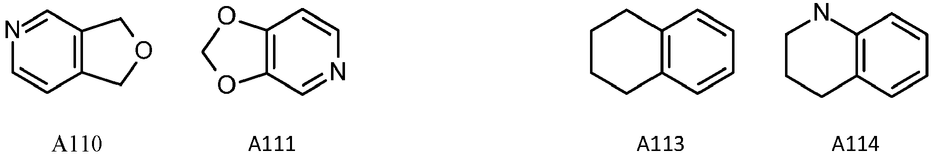 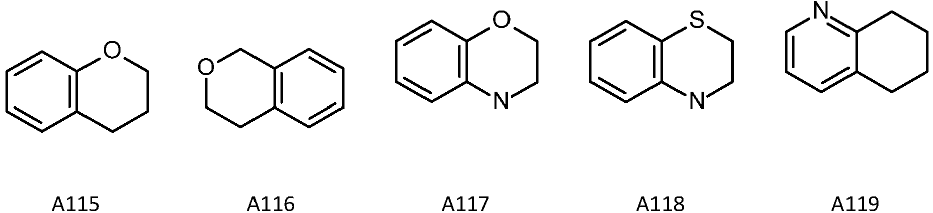 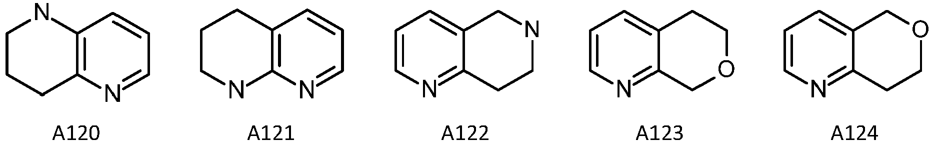 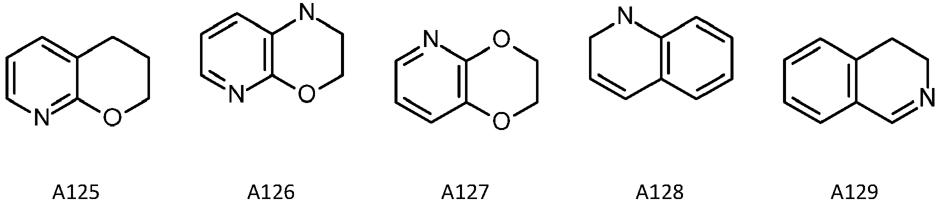 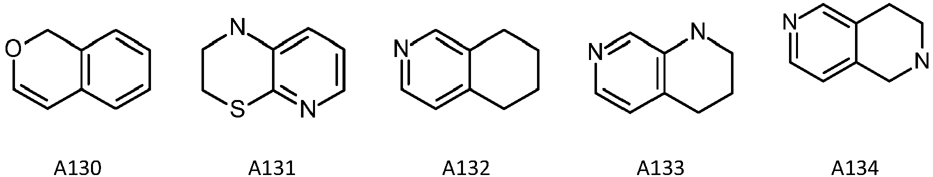 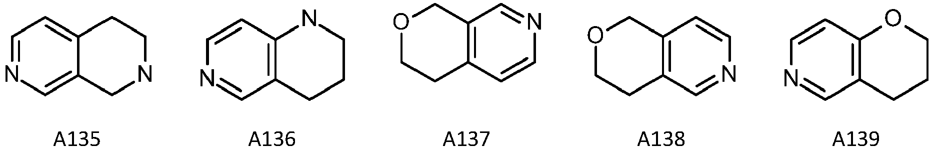 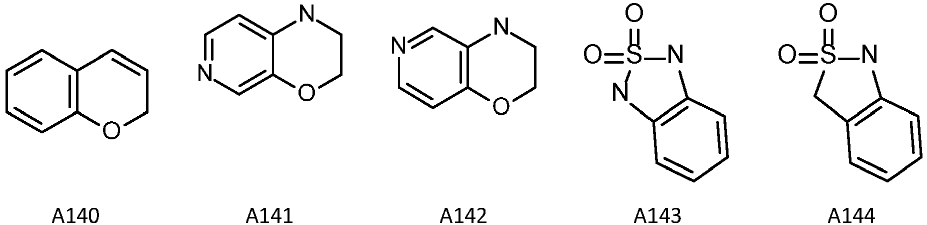 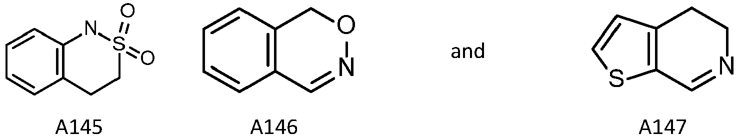 wherein, when **n** is 1, said C₉-C₁₀-carbocyclyl ring or 9- or 10-membered heterocyclyl ring may be substituted with up to four substituents in addition to **L**, said substituents being independently selected from the group consisting of halogen and C₁-C₃-alkyl;
**n** is 0 or 1;
**L** is a substituent selected from the group consisting of halogen, cyano, C₁-C₆-alkyl, C₁-C₆-haloalkyl, C₁-C₆-alkoxy, C₁-C₆-alkylthio, arylsulfenyl, C₁-C₆-alkylsulfinyl, arylsulfinyl, C₁-C₆-alkylsulfonyl, arylsulfonyl, C₃-C₁₀-carbocyclyl, 3- to 10-membered-heterocyclyl, aryl, heteroaryl, =N(OR^{a}), N(OR^{a})C(=O)OR^{a}, N(OR^{a})C(=O)R^{a}, -SO₂R^{a}, -SO₂N(R^{a})₂, -OC(=O)R^{a}, -N(R^{a})₂, - NR^{a}C(=O)OR^{a}, -NR^{a}C(=O)N(R^{a})₂, -NR^{a}C(=O)R^{a}, -NR^{a}C(=S)R^{a}, -NR^{a}C(=S)N(R^{a})₂, -NR^{a}C(=NR^{a})R^{a}, - N=C-N(R^{a})₂, -NR^{a}S(=O)₂R^{a}, -OC(=O)N(R^{a})₂, -C(=NR^{a})R^{a}, -C(=O)R^{a}, -C(=O)(OR^{a}), -C(=O)N(R^{a})₂, - C(=O)NR^{a}N(R^{a})₂, -C(=O)N(OR^{a})R^{a}, -C=NOR^{a}, -C(=S)N(R^{a})₂, , -C₁-C₆-alkyl-N(R^{a})₂, -C₁-C₆-alkyl-C(=O)N(R^{a})₂, -C₁-C₆-alkyl-NR^{a}C(=O)OR^{a}, -C₁-C₆-alkyl-NR^{a}C(=O)R^{a}, -C₁-C₆-alkyl-NR^{a}C(=O)N(R^{a})₂, - C₁-C₆-alkyl-N(OR^{a})C(=O)R^{a}, -C₁-C₆-alkyl-C(=O)N(OR^{a})R^{a}, -C₁-C₆-alkyl-NR^{a}C(=S)N(R^{a})₂, -C₁-C₆-alkyl-NR^{a}C(=O)R^{a} and -C₁-C₆-alkyl-NR^{a}S(=O)₂R^{a}, wherein said C₁-C₆-alkyl, C₁-C₆-haloalkyl, C₁-C₆-alkoxy, C₁-C₆-alkylthio, arylsulfenyl, C₁-C₆-alkylsulfinyl, arylsulfinyl, C₁-C₆-alkylsulfonyl, arylsulfonyl, C₃-C₁₀-carbocyclyl, 3- to 10-membered-heterocyclyl, aryl, heteroaryl, -C₁-Cr₆-alkyl-N(R^{a})₂, -C₁-C₆-alkyl-C(=O)N(R^{a})₂, -C₁-C₆-alkyl-NR^{a}C(=O)OR^{a}, -C₁-C₆-alkyl-NR^{a}C(=O)R^{a}, -C₁-C₆-alkyl-NR^{a}C(=O)N(R^{a})₂, - C₁-C₆-alkyl-N(OR^{a})C(=O)R^{a}, -C₁-C₆-alkyl-C(=O)N(OR^{a})R^{a}, -C₁-C₆-alkyl-NR^{a}C(=S)N(R^{a})₂, -C₁-C₈-alkyl-NR^{a}C(=O)R^{a} and -C₁-C₆-alkyl-NR^{a}S(=O)₂R^{a} substituent is itself optionally substituted, one or more times, in the same way or differently, with R5;
**R5** is a substituent selected from the group consisting of halogen, cyano, hydroxy, mercapto, sulfinyl, sulfonyl, amino, C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy, nitro, - C(=O)R^{a}, -C(=O)OR^{a}, -C(=O)N(R^{a})₂, -C(=S)N(R^{a})₂, -C(=NR^{a})R^{a}, -NR^{a}C(=O)OR^{a}, -NR^{a}C(=O)N(R^{a})₂, - NR^{a}C(=O)R^{a}, -NR^{a}C(=S)R^{a}, -NR^{a}C(=S)N(R^{a})₂, -NR^{a}C(=NR^{a})R^{a}, -OC(=O)R^{a}, -OC(=O)N(R^{a})₂, - NR^{a}S(=O)₂R^{a}, -S(=O)₂R^{a}, -S(=O)₂N(R^{a})₂ and -P(=O)(OR^{a})₂; wherein said C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b} ; when two **R5** substituents are bound to a common carbon, they may form together C=O, C₃-C₁₀-carbocyclyl or 3- to 10-membered-heterocyclyl;
R^{a} is, independently from each other, a substituent, which is identical or different, selected from the group consisting of hydrogen, halogen, cyano, hydroxy, mercapto, sulfinyl, sulfonyl, amino, C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy, nitro, -C₁-C₆-alkyl-C₃-C₁₀-carbocyclyl, -C₁-C₆-alkyl-aryl, -C₁-C₆-alkyl-C₁-C₆-alkoxy, -C(=O)R^{b}, -C(=O)OR^{b}, -C(=O)N(R^{b})₂, -C(=S)N(R^{b})₂, - NR^{b}C(=O)OR^{b}, -NR^{b}C(=O)N(R^{b})₂, -NR^{b}C(=O)R^{b}, -NR^{b}C(=S)R^{b}, -OC(=O)N(R^{b})₂, -NR^{b}S(=O)₂R^{b}, - S(=O)₂R^{b}, -S(=O)₂N(R^{b})₂ and -P(=O)(OR^{b})₂ ; wherein said C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b};
R^{b} is, independently from each other, a substituent, which is identical or different, selected from the group consisting of hydrogen, halogen, cyano, -OR^{c}, -N(R^{c})₂, -SR^{c}, -S(=O)R^{c}, -S(=O)OR^{c}, - S(=O)₂R^{c}, -S(=O)₂OR^{c}, C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy, nitro, - C(=O)R^{c}, -C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, - NR^{c}C(=S)R^{c}, -OC(=O)N(R^{c})₂, -NR^{c}S(=O)₂R^{c}, -S(=O)₂N(R^{c})₂ and -P(=O)(OR^{c})₂; wherein said C₃-C₁₀-carbocyclyl, 3- to 10-membered-heterocyclyl, aryl, heteroaryl substituent is itself optionally substituted, one or more times, in the same way or differently with CN, halogen, C₁-C₆-alkyl, C₁-C₆-haloalkyl or C₁-C₆-alkoxy;
R^{c} is, independently from each other, a substituent, which is identical or different, selected from the group consisting of hydrogen, aryl and C₁-C₆-alkyl,
   provided that
   (a) when **X** is F, **n** is 1 and **A** is 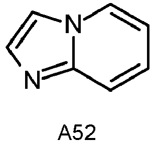 **L** is not a substituent selected from the group consisting of C₁-C₆-alkyl, aryl, 3- to 10-membered heterocyclyl and -C(=O)R^{a} with R^{a} being a 3- to 10-membered heterocyclyl,
   (b) when **X** is F and n is 0, **A** is not selected from the group consisting of: 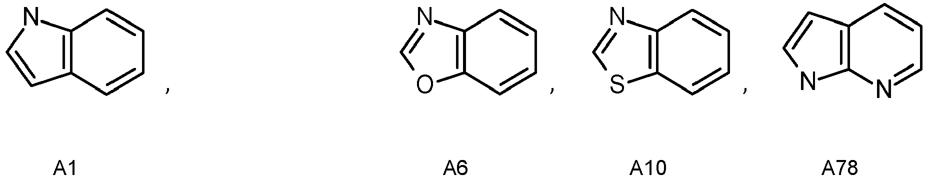 and 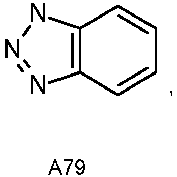
   (c) when **X** is **F**, **n** is 1and **A** is selected from the group consisting of: 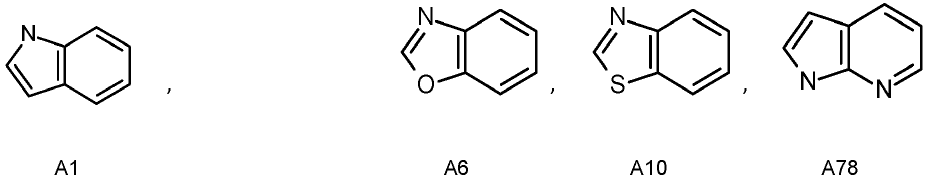 and 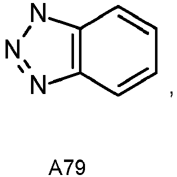 L is not a substituent selected from the group consisting of C₁-C₆-alkyl, -C(=O)(OR^{a}) with R^{a} being a C₁-C₆-alkyl and -SO₂R^{a} with R^{a} being an aryl,
   (d) when **X** is F and **n** is 0, **A** is not 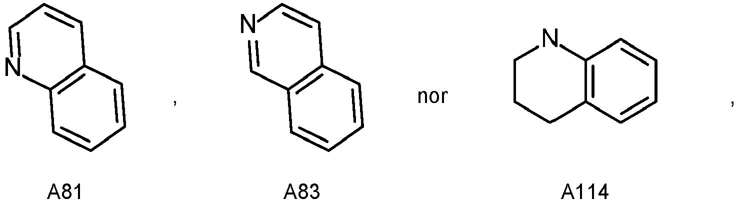
   (e) when **X** is **F**, n is 1 and **A** is 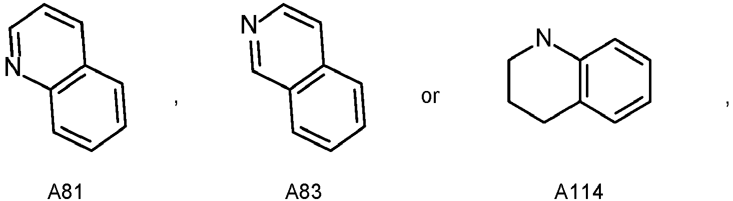 **L** is not a substituent selected from the group consisting of unsubstituted C₁-C₆-alkyl; C₁-C₆-alkyl substituted by one or more **R5** with **R5** being selected from the group consisting of unsubtituted aryl, unsubstituted heteroaryl, aryl substituted with one to three Rb with Rb being unsubstituted C₁-C₆-alkyl, and heteroaryl substituted with one to three Rb with Rb being unsubstituted C₁-C₆-alkyl; C₁-C₆-haloalkyl; halogen; cyano; -SO₂R^{a} with R^{a} being an unsubstituted C₁-C₆-alkyl; unsubstituted C₃-C₁₀-carbocyclyl; unsubstituted aryl; unsubstituted hereroaryl; aryl substituted by one or three **R5** with **R5** being an unsubstituted C₁-C₆-alkyl; and heteroaryl substituted by one or three **R5** with **R5** being an unsubstituted C₁-C₆-alkyl,
   (f) when **L** is -N(Ra)₂ with the two Ra being hydrogen, L is not attached to the atom of **A** that bonds **A** to the rest of the molecule (i.e. to the oxadiazole moiety),
   (g) compound of formula (I') is not
2-Pyridinecarboxamide,N-methyl-4-[[3-methyl-2-[5-(trifluoromethyl)-1,2,4-oxadiazol-3-yl]benzo[b]thien-6-yl]oxy]- (CAS-2222313-38-2),
Benzothiazole, 6-methoxy-2-[5-(trifluoromethyl)-1,2,4-oxadiazol-3-yl]- (CAS-897805-25-3), Thieno[2,3-c]pyridine, 4-(4-chlorophenoxy)-2-[5-(trifluoromethyl)-1,2,4-oxadiazol-3-yl]- (CAS-251995-30-9),
Pyrazolo[1,5-a]pyrimidine, 3-[5-(trifluoromethyl)-1,2,4-oxadiazol-3-yl]-7-[3-(trifluoromethyl)phenyl]-(CAS-159224-00-7),
1H-Indole, 3-(2-chloro-3-thienyl)-5-[5-(trifluoromethyl)-1,2,4-oxadiazol-3-yl]- (CAS-2252389-71-0), Imidazo[1,2-a]pyridine, 2-(phenylmethyl)-7-[5-(trifluoromethyl)-1,2,4-oxadiazol-3-yl]- (CAS-2170611-35-3),
1H-Pyrrolo[2,3-b]pyridine, 5-[5-(trifluoromethyl)-1,2,4-oxadiazol-3-yl]- (CAS-2170611-21-7), 1H-Pyrrolo[2,3-b]pyridine, 1-(phenylsulfonyl)-5-[5-(trifluoromethyl)-1,2,4-oxadiazol-3-yl]- (CAS-2170611-01-3),
2,1,3-Benzoxadiazole, 4-[3-(4-morpholinylmethyl)-1-azetidinyl]-6-[5-(trifluoromethyl)-1,2,4-oxadiazol-3-yl]- (CAS-1807986-73-7),
Tricyclo[3.3.1.13,7]decan-1-ol, 4-[[5-[5-(trifluoromethyl)-1,2,4-oxadiazol-3-yl]-1H-pyrrolo[2,3-b]pyridin-4-yl]amino]- (CAS-944120-75-6),
1H-Inden-1-amine, 2,3-dihydro-5-[5-(trifluoromethyl)-1,2,4-oxadiazol-3-yl]-, (1S)-(CAS-916303-83-8), 1H-Inden-1-amine, 2,3-dihydro-5-[5-(trifluoromethyl)-1,2,4-oxadiazol-3-yl]-, hydrochloride (1:1), (1S)-(CAS-916210-97-4),
Carbamic acid, N-[(1S)-2,3-dihydro-5-[5-(trifluoromethyl)-1,2,4-oxadiazol-3-yl]-1H-inden-1-yl]-, 1,1-dimethylethyl ester (CAS-916209-96-6),
1H-Pyrrolo[2,3-b]pyridine, 2-[1-methyl-5-[5-(trifluoromethyl)-1,2,4-oxadiazol-3-yl]-1H-indol-3-yl]-( CAS-479551-10-5),
1H-Indole, 2,3-dihydro-7-methyl-1-[3-(3-methyl-5-isoxazolyl)propyl]-5-[5-(trifluoromethyl)-1,2,4-oxadiazol-3-yl]- (CAS-178396-27-5),
1H-Indole, 7-methyl-1-[5-(3-methyl-5-isoxazolyl)pentyl]-5-[5-(trifluoromethyl)-1,2,4-oxadiazol-3-yl]-(CAS-178396-24-2),
1H-Indole, 7-methyl-1-[4-(3-methyl-5-isoxazolyl)butyl]-5-[5-(trifluoromethyl)-1,2,4-oxadiazol-3-yl]-(CAS-178396-22-0),
1H-Indole, 1-[3-(3-methyl-5-isoxazolyl)propyl]-5-[5-(trifluoromethyl)-1,2,4-oxadiazol-3-yl]- (CAS-178396-20-8),
Pyrazino[2,3-c]pyridazine, 5-[(1R)-1-(2,5-dichlorophenyl)ethyl]-5,6,7,8-tetrahydro-3-[5-(trifluoromethyl)-1,2,4-oxadiazol-3-yl]-(CAS-1690421-46-5),
Pyrazino[2,3-c]pyridazine, 5-[(2,5-dichlorophenyl)methyl]-5,6,7,8-tetrahydro-3-[5-(trifluoromethyl)-1,2,4-oxadiazol-3-yl]- (CAS-1690421-40-9),
1,2,4-Oxadiazole, 3-[7-(bromomethyl)-2-naphthalenyl]-5-(trifluoromethyl)- (CAS-1172849-62-5), 1,6-Naphthyridine, 5,6,7,8-tetrahydro-3-[5-(trifluoromethyl)-1,2,4-oxadiazol-3-yl]-, 2,2,2-trifluoroacetate (1:1) (CAS-741736-97-0),
1,6-Naphthyridine, 5,6,7,8-tetrahydro-3-[5-(trifluoromethyl)-1,2,4-oxadiazol-3-yl]- (CAS-741736-96-9), 1-Butanone, 3-amino-4-(2,5-difluorophenyl)-1-[7,8-dihydro-3-[5-(trifluoromethyl)-1,2,4-oxadiazol-3-yl]-1,6-naphthyridin-6(5H)-yl]-, (3R)- (CAS-741736-75-4),
Phthalazine, 1-phenyl-4-[5-(trifluoromethyl)-1,2,4-oxadiazol-3-yl]- (CAS-863601-14-3).

The compound of Fomula (I') can be in a free form, salt form, N-oxide form or solvate form (e.g. hydrate).

It is to be understood that the L group and the oxadiazole moiety can be bound to any position on the bicyclic ring **A**, provided the conditions of chemical stability and chemical feasibility are met.

In some embodiments, **A** is naphthyl or tetrahydronaphthyl.

In some embodiments, **A** is an aromatic fused bicyclic C₉-C₁₀-carbocyclyl carbocyclyl or 9- or 10-membered heterocyclyl ring comprising one to four heteroatoms independently selected in the list consisting of N, O and S, wherein said ring is selected from the group consisting of A1, A2, A4, A6 to A55, A57 to A83 and A85 to A89 as disclosed above.

In some embodiments, **A** is an aromatic fused bicyclic C₉-C₁₀-carbocyclyl carbocyclyl or 9- or 10-membered heterocyclyl ring comprising one to four heteroatoms independently selected in the list consisting of N, O and S, wherein said ring is selected from the group consisting of A1, A4, A10, A52, A77, A79 and A83, preferably A1, A4, A10, A52, A79 and A83 as disclosed above.

In some embodiments, **A** is an aromatic fused bicyclic C₉-C₁₀-carbocyclyl carbocyclyl or 9- or 10-membered heterocyclyl ring comprising one to four heteroatoms independently selected in the list consisting of N, O and S, wherein said ring is selected from the group consisting of:

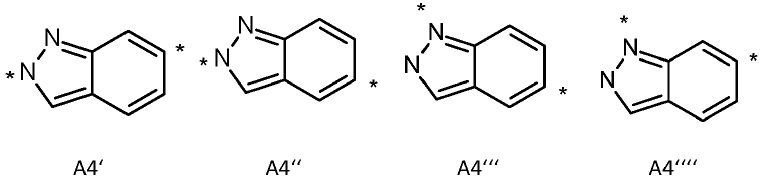

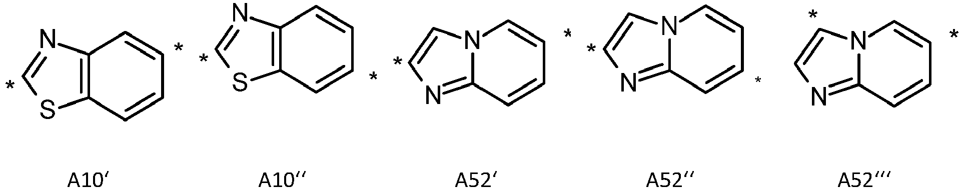

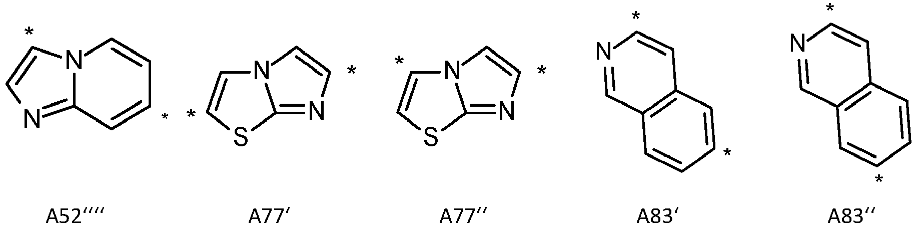

In the above, the symbol "*" denotes the preferred positions on the bicyclic ring at which the L group and the oxadiazole moiety may be bound.

In some embodiments, **A** is a partially unsaturated bicyclic C₉-C₁₀-carbocyclyl carbocyclyl or 9- or 10-membered heterocyclyl ring comprising one to four heteroatoms independently selected in the list consisting of N, O and S, wherein said ring is selected from the group consisting of A90 to A111 and A113 to A147 as disclosed above.

In some embodiments, **A** is a partially unsaturated bicyclic C₉-C₁₀-carbocyclyl carbocyclyl or 9- or 10-membered heterocyclyl ring comprising one to four heteroatoms independently selected in the list consisting of N, O and S, wherein said ring is selected from the group consisting of A90, A91, A93, A113, A114 and A140, preferably A90, A91, A113 and A114 as disclosed above.

In some embodiments, **A** is a partially unsaturated bicyclic C₉-C₁₀-carbocyclyl carbocyclyl or 9- or 10-membered heterocyclyl ring comprising one to four heteroatoms independently selected in the list consisting of N, O and S, wherein said ring is selected in the list consisting of: 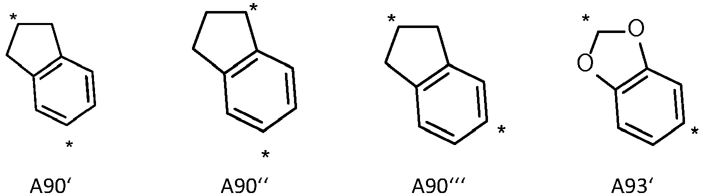 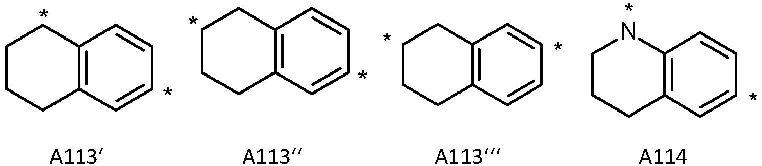 and

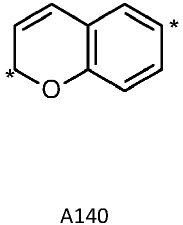

In the above, the symbol "* " denotes the preferred positions on the bicyclic ring at which the **L** group and the oxadiazole moiety may be bound.

In some embodiments, **A** is A1, A4, A10, A52, A77, A79, A83, A90, A91, A93, A113, A114 and A140 as disclosed above.

In some embodiments, **A** is A1, A4, A10, A52, A79, A83, A90, A91, A113 and A114 as disclosed above.

In some embodiments, **A** is A4, A10, A52, A77, A83, A90, A93, A113, A114 or A140 as disclosed above.

In the above formula (I'), **L** is preferably a substituent selected from the group consisting of halogen, C₁-C₆-alkyl, C₁-C₆-alkoxy, C₁-C₆-alkylsulfonyl, aryl, -N(R^{a})₂, =N(OR^{a}), -N(OR^{a})C(=O)OR^{a}, - NR^{a}C(=O)OR^{a}, -N(OR^{a})C(=O)R^{a}, -NR^{a}C(=O)R^{a}, -OC(=O)N(R^{a})₂, -C(=O)R^{a}, -C(=O)(OR^{a}), - C(=O)N(R^{a})₂, wherein said C₁-C₆-alkyl, C₁-C₆-alkoxy, C₁-C₆-alkylsulfonyl and aryl substituent is itself optionally substituted, one or more times, in the same way or differently, with **R5**, **R5** being as disclosed above, preferably **R5** is halogen, C₁-C₆-alkoxy or aryl.

In the above formula (I'), R^{a} is preferably a substituent selected from the group consisting of hydrogen, C₁-C₆-alkyl, C₁-C₆-alkoxy, C₃-C₁₀-carbocyclyl (e.g. cyclopropyl) and aryl (e.g. phenyl).

In the above formula (I'), R^{b} is preferably halogen, or C₁-C₆-alkoxy.

In some embodiments, **n** is 1 and **X** is F.

In some embodiments, **n** is 1 and **X** is Cl.

In some embodiments, **n** is 0 and **X** is F.

In some embodiments, **n** is 0 and **X** is Cl.

The present invention also relates to any compound of formula (I) disclosed in table 1.

In the above disclaimers, when a substituent is excluded from the scope of the formula, unless expressly provided differently, the exclusion encompasses the substituted and unsubstituted substituent.

For instance, when a **L** substituent is excluded from the scope of the formula, unless expressly provided differently, the exclusion encompasses unsubstituted **L** and **L** substituted with one or more **R5**.

Exclusion (a) should then be understood as follows:
(a) when X is F, n is 1 and A is

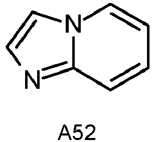

**L** is not a substituent selected from the group consisting of C₁-C₆-alkyl (unsubstituted or substituted with one or more **R5**), aryl (unsubstituted or substituted with one or more **R5**), 3- to 10-membered heterocyclyl (unsubstituted or substituted with one or more **R5**) and -C(=O)R^{a} with R^{a} being a 3- to 10-membered heterocyclyl (said heterocyclyl being optionally substituted with one or more R^{b}).

In some embodiments, compounds of formula (I') wherein **X** is F, **n** is 1, **A** is A52 and **L** is -C(=O)R^{a} with R^{a} being a C₁-C₆-alkyl substituted by one or more Rb, with at least one Rb being a 3- to 10-membered heterocyclyl are excluded from the scope of the formula.

Exclusion (c) should then be understood as follows:
(c) when **X** is F, **n** is 1and **A** is selected from the group consisting of 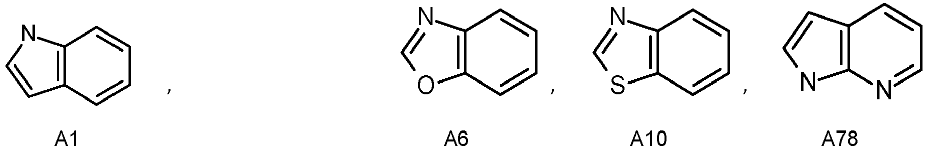 and

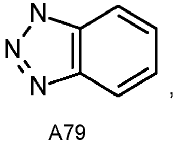

**L** is not a substituent selected from the group consisting of C₁-C₆-alkyl (unsubstituted or substituted with one or more R5), -C(=O)(OR^{a}) with R^{a} being a C₁-C₆-alkyl (unsubstituted or substituted with one or more R^{b}) and -SO₂R^{a} with R^{a} being an aryl (unsubstituted or substituted with one or more R^{b}).

Exclusion (f) should then be understood as follows: 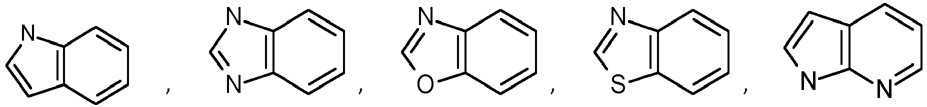 and
(f) when X is F, n is 1 and A is 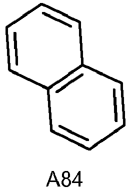 **L** is not a substituent selected from the group consisting of
- C₁-C₆-alkyl substituted with one or more **R5**, wherein at least one of said **R5** is selected from the group consisting of C₁-C₆-alkoxy, aryl, heteroaryl, C₃-C₁₀-carbocyclyl, 3- to 10-membered heterocyclyl, aryloxy and heteroaryloxy (unsubstituted or substituted with one or more R^{b}),
- C₁-C₆-alkoxy substituted with or more **R5**, wherein at least one of said **R5** is selected from the group consisting of aryl, heteroaryl, C₃-C₁₀-carbocyclyl, 3- to 10-membered heterocyclyl, aryloxy and heteroaryloxy (unsubstituted or substituted with one or more Rb),
- -N(R^{a})₂ wherein at least one R^{a} is aryl, heteroaryl, C₃-C₁₀-carbocyclyl or 3- to 10-membered heterocyclyl (unsubstituted or substituted with one or more Rb),
- -N(R^{a})₂ wherein at least one R^{a} is C₁-C₆-alkyl substituted with one or more R^{b}, at least one of said R^{b} being an aryl, heteroaryl, C₃-C₁₀-carbocyclyl or 3- to 10-membered heterocyclyl (unsubstituted or substituted),
- C₁-C₆-alkyl-N(R^{a})₂ wherein at least one R^{a} is aryl, heteroaryl, C₃-C₁₀-carbocyclyl or 3-to 10-membered heterocyclyl (unsubstituted or substituted with one or more Rb) (the C₁-C₆-alkyl part being unsubstituted or substituted with one or more R5),
- C₃-C₁₀-carbocyclyl (unsubstituted or substituted with one or more R5),
- 3- to 10-membered heterocyclyl (unsubstituted or substituted with one or more R5), and- heteroaryl (unsubstituted or substituted with one or more R5).

The compounds of formula (I') may be used as fungicides, in particular in methods for controlling phytopathogenic fungi which comprises the step of applying one or more compounds of formula (I') to to the plants, plant parts, seeds, fruits or to the soil in which the plants grow).

### Compositions and formulations

The present invention further relates to a composition, in particular a composition for controlling unwanted microorganisms, comprising one or more compounds of formula (I) or (I'). The composition is preferably is a fungicidal composition.

The composition typically comprises one or more compounds of formula (I) or (I') and one or more acceptable carriers, in particular one or more agriculturally acceptable carriers.

A carrier is a solid or liquid, natural or synthetic, organic or inorganic substance that is generally inert. The carrier generally improves the application of the compounds, for instance, to plants, plants parts or seeds. Examples of suitable *solid carriers* include, but are not limited to, ammonium salts, natural rock flours, such as kaolins, clays, talc, chalk, quartz, attapulgite, montmorillonite and diatomaceous earth, and synthetic rock flours, such as finely divided silica, alumina and silicates. Examples of typically useful solid carriers for preparing granules include, but are not limited to crushed and fractionated natural rocks such as calcite, marble, pumice, sepiolite and dolomite, synthetic granules of inorganic and organic flours and granules of organic material such as paper, sawdust, coconut shells, maize cobs and tobacco stalks. Examples of suitable *liquid carriers* include, but are not limited to, water, organic solvents and combinations thereof. Examples of suitable *solvents* include polar and nonpolar organic chemical liquids, for example from the classes of aromatic and nonaromatic hydrocarbons (such as cyclohexane, paraffins, alkylbenzenes, xylene, toluene alkylnaphthalenes, chlorinated aromatics or chlorinated aliphatic hydrocarbons such as chlorobenzenes, chloroethylenes or methylene chloride), alcohols and polyols (which may optionally also be substituted, etherified and/or esterified, such as butanol or glycol), ketones (such as acetone, methyl ethyl ketone, methyl isobutyl ketone or cyclohexanone), esters (including fats and oils) and (poly)ethers, unsubstituted and substituted amines, amides (such as dimethylformamide), lactams (such as N-alkylpyrrolidones) and lactones, sulfones and sulfoxides (such as dimethyl sulphoxide). The carrier may also be a liquefied gaseous extender, i.e. liquid which is gaseous at standard temperature and under standard pressure, for example aerosol propellants such as halohydrocarbons, butane, propane, nitrogen and carbon dioxide. The amount of carrier typically ranges from 1 to 99.99%, preferably from 5 to 99.9%, more preferably from 10 to 99.5%, and most preferably from 20 to 99 % by weight of the composition.

The composition may further comprise one or more acceptable auxiliaries which are customary for formulating compositions (e.g. agrochemical compositions), such as one or more surfactants.

The surfactant can be an ionic (cationic or anionic) or non-ionic surfactant, such as ionic or non-ionic emulsifier(s), foam former(s), dispersant(s), wetting agent(s) and any mixtures thereof. Examples of suitable surfactants include, but are not limited to, salts of polyacrylic acid, salts of lignosulfonic acid, salts of phenolsulfonic acid or naphthalenesulfonic acid, polycondensates of ethylene and/or propylene oxide with fatty alcohols, fatty acids or fatty amines (polyoxyethylene fatty acid esters, polyoxyethylene fatty alcohol ethers, for example alkylaryl polyglycol ethers), substituted phenols (preferably alkylphenols or arylphenols), salts of sulfosuccinic esters, taurine derivatives (preferably alkyl taurates), phosphoric esters of polyethoxylated alcohols or phenols, fatty esters of polyols and derivatives of compounds containing sulfates, sulfonates, phosphates (for example, alkylsulfonates, alkyl sulfates, arylsulfonates) and protein hydrolysates, lignosulfite waste liquors and methylcellulose. A surfactant is typically used when the compound of the formula (I) and/or the carrier is insoluble in water and the application is made with water. Then, the amount of surfactants typically ranges from 5 to 40 % by weight of the composition.

Further examples of auxiliaries which are customary for formulating agrochemical compositions include water repellents, siccatives, binders (adhesive, tackifier, fixing agent, such as carboxymethylcellulose, natural and synthetic polymers in the form of powders, granules or latices, such as gum arabic, polyvinyl alcohol and polyvinyl acetate, natural phospholipids such as cephalins and lecithins and synthetic phospholipids, polyvinylpyrrolidone, polyvinyl acetate, polyvinyl alcohol and tylose), thickeners, stabilizers (e.g. cold stabilizers, preservatives, antioxidants, light stabilizers, or other agents which improve chemical and/or physical stability), dyes or pigments (such as inorganic pigments, e.g. iron oxide, titanium oxide and Prussian Blue ; organic dyes, e.g. alizarin, azo and metal phthalocyanine dyes), antifoams (e.g. silicone antifoams and magnesium stearate), preservatives (e.g. dichlorophene and benzyl alcohol hemiformal), secondary thickeners (cellulose derivatives, acrylic acid derivatives, xanthan, modified clays and finely divided silica), stickers, gibberellins and processing auxiliaries, mineral and vegetable oils, perfumes, waxes, nutrients (including trace nutrients, such as salts of iron, manganese, boron, copper, cobalt, molybdenum and zinc), protective colloids, thixotropic substances, penetrants, sequestering agents and complex formers.

The choice of the auxiliaries is related to the intended mode of application of the compound of the formula (I) or (I') and/or on the physical properties. Furthermore, the auxiliaries may be chosen to impart particular properties (technical, physical and/or biological properties) to the compositions or use forms prepared therefrom. The choice of auxiliaries may allow customizing the compositions to specific needs.

The composition of the invention may be in any customary form, such as solutions (e.g aqueous solutions), emulsions, wettable powders, water- and oil-based suspensions, powders, dusts, pastes, soluble powders, soluble granules, granules for broadcasting, suspoemulsion concentrates, natural or synthetic products impregnated with the compoundof theinvention, fertilizers and also microencapsulations in polymeric substances. The compound of the invention may be present in a suspended, emulsified or dissolved form.

The composition of the invention may be provided to the end user as ready-for-use formulation, i.e. the compositions may be directly applied to the plants or seeds by a suitable device, such as a spraying or dusting device. Alternatively, the compositions may be provided to the end user in the form of concentrates which have to be diluted, preferably with water, prior to use.

The composition of the invention can be prepared in conventional manners, for example by mixing the compound of the invention with one or more suitable auxiliaries, such as disclosed herein above.

The composition according to the invention contains generally from 0.01 to 99% by weight, from 0.05 to 98% by weight, preferably from 0.1 to 95% by weight, more preferably from 0.5 to 90% by weight, most preferably from 1 to 80 % by weight of the compound of the invention.

### Mixtures/Combinations

The compound of formula (I) or (I') and compositions comprising thereof can be mixed with other active ingredients like fungicides, bactericides, acaricides, nematicides, insecticides, herbicides, fertilizers, growth regulators, safeners or semiochemicals. This may allow to broaden the activity spectrum or to prevent development of resistance. Examples of known fungicides, insecticides, acaricides, nematicides and bactericides are disclosed in the Pesticide Manual, 17th Edition.

Examples of especially preferred fungicides which could be mixed with the compound of formula (I) or (I') and the compositions are:
1) Inhibitors of the ergosterol biosynthesis, for example (1.001) cyproconazole, (1.002) difenoconazole, (1.003) epoxiconazole, (1.004) fenhexamid, (1.005) fenpropidin, (1.006) fenpropimorph, (1.007) fenpyrazamine, (1.008) fluquinconazole, (1.009) flutriafol, (1.010) imazalil, (1.011) imazalil sulfate, (1.012) ipconazole, (1.013) metconazole, (1.014) myclobutanil, (1.015) paclobutrazol, (1.016) prochloraz, (1.017) propiconazole, (1.018) prothioconazole, (1.019) Pyrisoxazole, (1.020) spiroxamine, (1.021) tebuconazole, (1.022) tetraconazole, (1.023) triadimenol, (1.024) tridemorph, (1.025) triticonazole, (1.026) (1R,2S,5S)-5-(4-chlorobenzyl)-2-(chloromethyl)-2-methyl-1-(1H-1,2,4-triazol-1-ylmethyl)cyclopentanol, (1.027) (1S,2R,5R)-5-(4-chlorobenzyl)-2-(chloromethyl)-2-methyl-1-(1H-1,2,4-triazol-1-ylmethyl)cyclopentanol, (1.028) (2R)-2-(1-chlorocyclopropyl)-4-[(1R)-2,2-dichlorocyclopropyl]-1-(1H-1,2,4-triazol-1-yl)butan-2-ol, (1.029) (2R)-2-(1-chlorocyclopropyl)-4-[(1S)-2,2-dichlorocyclopropyl]-1-(1H-1,2,4-triazol-1-yl)butan-2-ol, (1.030) (2R)-2-[4-(4-chlorophenoxy)-2-(trifluoromethyl)phenyl]-1-(1H-1,2,4-triazol-1-yl)propan-2-ol, (1.031) (2S)-2-(1-chlorocyclopropyl)-4-[(1R)-2,2-dichlorocyclopropyl]-1-(1H-1,2,4-triazol-1-yl)butan-2-ol, (1.032) (2S)-2-(1-chlorocyclopropyl)-4-[(1S)-2,2-dichlorocyclopropyl]-1-(1H-1,2,4-triazol-1-yl)butan-2-ol, (1.033) (2S)-2-[4-(4-chlorophenoxy)-2-(trifluoromethyl)phenyl]-1-(1H-1,2,4-triazol-1-yl)propan-2-ol, (1.034) (R)-[3-(4-chloro-2-fluorophenyl)-5-(2,4-difluorophenyl)-1,2-oxazol-4-yl](pyridin-3-yl)methanol, (1.035) (S)-[3-(4-chloro-2-fluorophenyl)-5-(2,4-difluorophenyl)-1,2-oxazol-4-yl](pyridin-3-yl)methanol, (1.036) [3-(4-chloro-2-fluorophenyl)-5-(2,4-difluorophenyl)-1,2-oxazol-4-yl](pyridin-3-yl)methanol, (1.037) 1-({(2R,4S)-2-[2-chloro-4-(4-chlorophenoxy)phenyl]-4-methyl-1,3-dioxolan-2-yl}methyl)-1H-1,2,4-triazole, (1.038) 1-({(2S,4S)-2-[2-chloro-4-(4-chlorophenoxy)phenyl]-4-methyl-1,3-dioxolan-2-yl}methyl)-1H-1,2,4-triazole, (1.039) 1-{[3-(2-chlorophenyl)-2-(2,4-difluorophenyl)oxiran-2-yl]methyl}-1H-1,2,4-triazol-5-yl thiocyanate, (1.040) 1-{[rel(2R,3R)-3-(2-chlorophenyl)-2-(2,4-difluorophenyl)oxiran-2-yl]methyl}-1H-1,2,4-triazol-5-yl thiocyanate, (1.041) 1-{[rel(2R,3S)-3-(2-chlorophenyl)-2-(2,4-difluorophenyl)oxiran-2-yl]methyl}-1H-1,2,4-triazol-5-yl thiocyanate, (1.042) 2-[(2R,4R,5R)-1-(2,4-dichlorophenyl)-5-hydroxy-2,6,6-trimethylheptan-4-yl]-2,4-dihydro-3H-1,2,4-triazole-3-thione, (1.043) 2-[(2R,4R,5S)-1-(2,4-dichlorophenyl)-5-hydroxy-2,6,6-trimethylheptan-4-yl]-2,4-dihydro-3H-1,2,4-triazole-3-thione, (1.044) 2-[(2R,4S,5R)-1-(2,4-dichlorophenyl)-5-hydroxy-2,6,6-trimethylheptan-4-yl]-2,4-dihydro-3H-1,2,4-triazole-3-thione, (1.045) 2-[(2R,4S,5S)-1-(2,4-dichlorophenyl)-5-hydroxy-2,6,6-trimethylheptan-4-yl]-2,4-dihydro-3H-1,2,4-triazole-3-thione, (1.046) 2-[(2S,4R,5R)-1-(2,4-dichlorophenyl)-5-hydroxy-2,6,6-trimethylheptan-4-yl]-2,4-dihydro-3H-1,2,4-triazole-3-thione, (1.047) 2-[(2S,4R,5S)-1-(2,4-dichlorophenyl)-5-hydroxy-2,6,6-trimethylheptan-4-yl]-2,4-dihydro-3H-1,2,4-triazole-3-thione, (1.048) 2-[(2S,4S,5R)-1-(2,4-dichlorophenyl)-5-hydroxy-2,6,6-trimethylheptan-4-yl]-2,4-dihydro-3H-1,2,4-triazole-3-thione, (1.049) 2-[(2S,4S,5S)-1-(2,4-dichlorophenyl)-5-hydroxy-2,6,6-trimethylheptan-4-yl]-2,4-dihydro-3H-1,2,4-triazole-3-thione, (1.050) 2-[1-(2,4-dichlorophenyl)-5-hydroxy-2,6,6-trimethylheptan-4-yl]-2,4-dihydro-3H-1,2,4-triazole-3-thione, (1.051) 2-[2-chloro-4-(2,4-dichlorophenoxy)phenyl]-1-(1H-1,2,4-triazol-1-yl)propan-2-ol, (1.052) 2-[2-chloro-4-(4-chlorophenoxy)phenyl]-1-(1H-1,2,4-triazol-1-yl)butan-2-ol, (1.053) 2-[4-(4-chlorophenoxy)-2-(trifluoromethyl)phenyl]-1-(1H-1,2,4-triazol-1-yl)butan-2-ol, (1.054) 2-[4-(4-chlorophenoxy)-2-(trifluoromethyl)phenyl]-1-(1H-1,2,4-triazol-1-yl)pentan-2-ol, (1.055) Mefentrifluconazole, (1.056) 2-{[3-(2-chlorophenyl)-2-(2,4-difluorophenyl)oxiran-2-yl]methyl}-2,4-dihydro-3H-1,2,4-triazole-3-thione, (1.057) 2-{[rel(2R,3R)-3-(2-chlorophenyl)-2-(2,4-difluorophenyl)oxiran-2-yl]methyl}-2,4-dihydro-3H-1,2,4-triazole-3-thione, (1.058) 2-{[rel(2R,3S)-3-(2-chlorophenyl)-2-(2,4-difluorophenyl)oxiran-2-yl]methyl}-2,4-dihydro-3H-1,2,4-triazole-3-thione, (1.059) 5-(4-chlorobenzyl)-2-(chloromethyl)-2-methyl-1-(1H-1,2,4-triazol-1-ylmethyl)cyclopentanol, (1.060) 5-(allylsulfanyl)-1-{[3-(2-chlorophenyl)-2-(2,4-difluorophenyl)oxiran-2-yl]methyl}-1H-1,2,4-triazole, (1.061) 5-(allylsulfanyl)-1-{[rel(2R,3R)-3-(2-chlorophenyl)-2-(2,4-difluorophenyl)oxiran-2-yl]methyl}-1H-1,2,4-triazole, (1.062) 5-(allylsulfanyl)-1-{[rel(2R,3S)-3-(2-chlorophenyl)-2-(2,4-difluorophenyl)oxiran-2-yl]methyl}-1H-1,2,4-triazole, (1.063) N'-(2,5-dimethyl-4-{[3-(1,1,2,2-tetrafluoroethoxy)phenyl]sulfanyl}phenyl)-N-ethyl-N-methylimidoformamide, (1.064) N'-(2,5-dimethyl-4-{[3-(2,2,2-trifluoroethoxy)phenyl]sulfanyl}phenyl)-N-ethyl-N-methylimidoformamide, (1.065) N'-(2,5-dimethyl-4-{[3-(2,2,3,3-tetrafluoropropoxy)phenyl]sulfanyl}phenyl)-N-ethyl-N-methylimidoformamide, (1.066) N'-(2,5-dimethyl-4-{[3-(pentafluoroethoxy)phenyl]sulfanyl}phenyl)-N-ethyl-N-methylimidoformamide, (1.067) N'-(2,5-dimethyl-4-{3-[(1,1,2,2-tetrafluoroethyl)sulfanyl]phenoxy}phenyl)-N-ethyl-N-methylimidoformamide, (1.068) N'-(2,5-dimethyl-4-{3-[(2,2,2-trifluoroethyl)sulfanyl]phenoxy}phenyl)-N-ethyl-N-methylimidoformamide, (1.069) N'-(2,5-dimethyl-4-{3-[(2,2,3,3-tetrafluoropropyl)sulfanyl]phenoxy}phenyl)-N-ethyl-N-methylimidoformamide, (1.070) N'-(2,5-dimethyl-4-{3-[(pentafluoroethyl)sulfanyl]phenoxy}phenyl)-N-ethyl-N-methylimidoformamide, (1.071) N'-(2,5-dimethyl-4-phenoxyphenyl)-N-ethyl-N-methylimidoformamide, (1.072) N'-(4-{[3-(difluoromethoxy)phenyl]sulfanyl}-2,5-dimethylphenyl)-N-ethyl-N-methylimidoformamide, (1.073) N'-(4-{3-[(difluoromethyl)sulfanyl]phenoxy}-2,5-dimethylphenyl)-N-ethyl-N-methylimidoformamide, (1.074) N'-[5-bromo-6-(2,3-dihydro-1H-inden-2-yloxy)-2-methylpyridin-3-yl]-N-ethyl-N-methylimidoformamide, (1.075) N'-{4-[(4,5-dichloro-1,3-thiazol-2-yl)oxy]-2,5-dimethylphenyl}-N-ethyl-N-methylimidoformamide, (1.076) N'-{5-bromo-6-[(1R)-1-(3,5-difluorophenyl)ethoxy]-2-methylpyridin-3-yl}-N-ethyl-N-methylimidoformamide, (1.077) N'-{5-bromo-6-[(1S)-1-(3,5-difluorophenyl)ethoxy]-2-methylpyridin-3-yl}-N-ethyl-N-methylimidoformamide, (1.078) N'-{5-bromo-6-[(cis-4-isopropylcyclohexyl)oxy]-2-methylpyridin-3-yl}-N-ethyl-N-methylimidoformamide, (1.079) N'-{5-bromo-6-[(trans-4-isopropylcyclohexyl)oxy]-2-methylpyrid in-3-yl}-N-ethyl-N-methylimidoformamide, (1.080) N'-{5-bromo-6-[1-(3,5-difluorophenyl)ethoxy]-2-methylpyridin-3-yl}-N-ethyl-N-methylimidoformamide, (1.081) Ipfentrifluconazole.
2) Inhibitors of the respiratory chain at complex I or II, for example (2.001) benzovindiflupyr, (2.002) bixafen, (2.003) boscalid, (2.004) carboxin, (2.005) fluopyram, (2.006) flutolanil, (2.007) fluxapyroxad, (2.008) furametpyr, (2.009) Isofetamid, (2.010) isopyrazam (anti-epimeric enantiomer 1R,4S,9S), (2.011) isopyrazam (anti-epimeric enantiomer 1S,4R,9R), (2.012) isopyrazam (anti-epimeric racemate 1RS,4SR,9SR), (2.013) isopyrazam (mixture of syn-epimeric racemate 1RS,4SR,9RS and anti-epimeric racemate 1RS,4SR,9SR), (2.014) isopyrazam (syn-epimeric enantiomer 1R,4S,9R), (2.015) isopyrazam (syn-epimeric enantiomer 1S,4R,9S), (2.016) isopyrazam (syn-epimeric racemate 1RS,4SR,9RS), (2.017) penflufen, (2.018) penthiopyrad, (2.019) pydiflumetofen, (2.020) Pyraziflumid, (2.021) sedaxane, (2.022) 1,3-dimethyl-N-(1,1,3-trimethyl-2,3-dihydro-1H-inden-4-yl)-1H-pyrazole-4-carboxamide, (2.023) 1,3-dimethyl-N-[(3R)-1,1,3-trimethyl-2,3-dihydro-1H-inden-4-yl]-1H-pyrazole-4-carboxamide, (2.024) 1,3-dimethyl-N-[(3S)-1,1,3-trimethyl-2,3-dihydro-1H-inden-4-yl]-1H-pyrazole-4-carboxamide, (2.025) 1-methyl-3-(trifluoromethyl)-N-[2'-(trifluoromethyl)biphenyl-2-yl]-1H-pyrazole-4-carboxamide, (2.026) 2-fluoro-6-(trifluoromethyl)-N-(1,1,3-trimethyl-2,3-dihydro-1H-inden-4-yl)benzamide, (2.027) 3-(difluoromethyl)-1-methyl-N-(1,1,3-trimethyl-2,3-dihydro-1H-inden-4-yl)-1H-pyrazole-4-carboxamide, (2.028) 3-(difluoromethyl)-1-methyl-N-[(3R)-1,1,3-trimethyl-2,3-dihydro-1H-inden-4-yl]-1H-pyrazole-4-carboxamide, (2.029) 3-(difluoromethyl)-1-methyl-N-[(3S)-1,1,3-trimethyl-2,3-dihydro-1H-inden-4-yl]-1H-pyrazole-4-carboxamide, (2.030) Fluindapyr, (2.031) 3-(difluoromethyl)-N-[(3R)-7-fluoro-1,1,3-trimethyl-2,3-dihydro-1H-inden-4-yl]-1-methyl-1H-pyrazole-4-carboxamide, (2.032) 3-(difluoromethyl)-N-[(3S)-7-fluoro-1,1,3-trimethyl-2,3-dihydro-1H-inden-4-yl]-1-methyl-1H-pyrazole-4-carboxamide, (2.033) 5,8-difluoro-N-[2-(2-fluoro-4-{[4-(trifluoromethyl)pyridin-2-yl]oxy}-phenyl)ethyl]quinazolin-4-amine, (2.034) N-(2-cyclopentyl-5-fluorobenzyl)-N-cyclopropyl-3-(difluoromethyl)-5-fluoro-1-methyl-1H-pyrazole-4-carboxamide, (2.035) N-(2-tert-butyl-5-methylbenzyl)-N-cyclopropyl-3-(difluoromethyl)-5-fluoro-1-methyl-1H-pyrazole-4-carboxamide, (2.036) N-(2-tert-butylbenzyl)-N-cyclopropyl-3-(difluoromethyl)-5-fluoro-1-methyl-1H-pyrazole-4-carboxamide, (2.037) N-(5-chloro-2-ethylbenzyl)-N-cyclopropyl-3-(difluoromethyl)-5-fluoro-1-methyl-1H-pyrazole-4-carboxamide, (2.038) isoflucypram, (2.039) N-[(1R,4S)-9-(dichloromethylene)-1,2,3,4-tetrahydro-1,4-methanonaphthalen-5-yl]-3-(difluoromethyl)-1-methyl-1H-pyrazole-4-carboxamide, (2.040) N-[(1S,4R)-9-(dichloromethylene)-1,2,3,4-tetrahydro-1,4-methanonaphthalen-5-yl]-3-(difluoromethyl)-1-methyl-1H-pyrazole-4-carboxamide, (2.041) N-[1-(2,4-dichlorophenyl)-1-methoxypropan-2-yl]-3-(difluoromethyl)-1-methyl-1H-pyrazole-4-carboxamide, (2.042) N-[2-chloro-6-(trifluoromethyl)benzyl]-N-cyclopropyl-3-(difluoromethyl)-5-fluoro-1-methyl-1H-pyrazole-4-carboxamide, (2.043) N-[3-chloro-2-fluoro-6-(trifluoromethyl)benzyl]-N-cyclopropyl-3-(difluoromethyl)-5-fluoro-1-methyl-1H-pyrazole-4-carboxamide, (2.044) N-[5-chloro-2-(trifluoromethyl)benzyl]-N-cyclopropyl-3-(difluoromethyl)-5-fluoro-1-methyl-1H-pyrazole-4-carboxamide, (2.045) N-cyclopropyl-3-(difluoromethyl)-5-fluoro-1-methyl-N-[5-methyl-2-(trifluoromethyl)benzyl]-1H-pyrazole-4-carboxamide, (2.046) N-cyclopropyl-3-(difluoromethyl)-5-fluoro-N-(2-fluoro-6-isopropylbenzyl)-1-methyl-1H-pyrazole-4-carboxamide, (2.047) N-cyclopropyl-3-(difluoromethyl)-5-fluoro-N-(2-isopropyl-5-methylbenzyl)-1-methyl-1H-pyrazole-4-carboxamide, (2.048) N-cyclopropyl-3-(difluoromethyl)-5-fluoro-N-(2-isopropylbenzyl)-1-methyl-1H-pyrazole-4-carbothioamide, (2.049) N-cyclopropyl-3-(difluoromethyl)-5-fluoro-N-(2-isopropylbenzyl)-1-methyl-1H-pyrazole-4-carboxamide, (2.050) N-cyclopropyl-3-(difluoromethyl)-5-fluoro-N-(5-fluoro-2-isopropylbenzyl)-1-methyl-1H-pyrazole-4-carboxamide, (2.051) N-cyclopropyl-3-(difluoromethyl)-N-(2-ethyl-4,5-dimethylbenzyl)-5-fluoro-1-methyl-1H-pyrazole-4-carboxamide, (2.052) N-cyclopropyl-3-(difluoromethyl)-N-(2-ethyl-5-fluorobenzyl)-5-fluoro-1-methyl-1H-pyrazole-4-carboxamide, (2.053) N-cyclopropyl-3-(difluoromethyl)-N-(2-ethyl-5-methylbenzyl)-5-fluoro-1-methyl-1H-pyrazole-4-carboxamide, (2.054) N-cyclopropyl-N-(2-cyclopropyl-5-fluorobenzyl)-3-(difluoromethyl)-5-fluoro-1-methyl-1H-pyrazole-4-carboxamide, (2.055) N-cyclopropyl-N-(2-cyclopropyl-5-methylbenzyl)-3-(difluoromethyl)-5-fluoro-1-methyl-1H-pyrazole-4-carboxamide, (2.056) N-cyclopropyl-N-(2-cyclopropylbenzyl)-3-(difluoromethyl)-5-fluoro-1-methyl-1H-pyrazole-4-carboxamide, (2.057) pyrapropoyne.
3) Inhibitors of the respiratory chain at complex III, for example (3.001) ametoctradin, (3.002) amisulbrom, (3.003) azoxystrobin, (3.004) coumethoxystrobin, (3.005) coumoxystrobin, (3.006) cyazofamid, (3.007) dimoxystrobin, (3.008) enoxastrobin, (3.009) famoxadone, (3.010) fenamidone, (3.011) flufenoxystrobin, (3.012) fluoxastrobin, (3.013) kresoxim-methyl, (3.014) metominostrobin, (3.015) orysastrobin, (3.016) picoxystrobin, (3.017) pyraclostrobin, (3.018) pyrametostrobin, (3.019) pyraoxystrobin, (3.020) trifloxystrobin, (3.021) (2E)-2-{2-[({[(1E)-1-(3-{[(E)-1-fluoro-2-phenylvinyl]oxy}phenyl)ethylidene]amino}oxy)methyl]phenyl}-2-(methoxyimino)-N-methylacetamide, (3.022) (2E,3Z)-5-{[1-(4-chlorophenyl)-1H-pyrazol-3-yl]xy}-2-(methoxyimino)-N,3-dimethylpent-3-enamide, (3.023) (2R)-2-{2-[(2,5-dimethylphenoxy)methyl]phenyl}-2-methoxy-N-methylacetamide, (3.024) (2S)-2-{2-[(2,5-dimethylphenoxy)methyl]phenyl}-2-methoxy-N-methylacetamide, (3.025) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, (3.026) mandestrobin, (3.027) N-(3-ethyl-3,5,5-trimethylcyclohexyl)-3-formamido-2-hydroxybenzamide, (3.028) (2E,3Z)-5-{[1-(4-chloro-2-fluorophenyl)-1H-pyrazol-3-yl]oxy}-2-(methoxyimino)-N,3-dimethylpent-3-enamide, (3.029) methyl {5-[3-(2,4-dimethylphenyl)-1H-pyrazol-1-yl]-2-methylbenzyl}carbamate, (3.030) metyltetraprole, (3.031) florylpicoxamid.
4) Inhibitors of the mitosis and cell division, for example (4.001) carbendazim, (4.002) diethofencarb, (4.003) ethaboxam, (4.004) fluopicolide, (4.005) pencycuron, (4.006) thiabendazole, (4.007) thiophanate-methyl, (4.008) zoxamide, (4.009) 3-chloro-4-(2,6-difluorophenyl)-6-methyl-5-phenylpyridazine, (4.010) 3-chloro-5-(4-chlorophenyl)-4-(2,6-difluorophenyl)-6-methylpyridazine, (4.011) 3-chloro-5-(6-chloropyridin-3-yl)-6-methyl-4-(2,4,6-trifluorophenyl)pyridazine, (4.012) 4-(2-bromo-4-fluorophenyl)-N-(2,6-difluorophenyl)-1,3-dimethyl-1H-pyrazol-5-amine, (4.013) 4-(2-bromo-4-fluorophenyl)-N-(2-bromo-6-fluorophenyl)-1,3-dimethyl-1H-pyrazol-5-amine, (4.014) 4-(2-bromo-4-fluorophenyl)-N-(2-bromophenyl)-1,3-dimethyl-1H-pyrazol-5-amine, (4.015) 4-(2-bromo-4-fluorophenyl)-N-(2-chloro-6-fluorophenyl)-1,3-dimethyl-1H-pyrazol-5-amine, (4.016) 4-(2-bromo-4-fluorophenyl)-N-(2-chlorophenyl)-1,3-dimethyl-1H-pyrazol-5-amine, (4.017) 4-(2-bromo-4-fluorophenyl)-N-(2-fluorophenyl)-1,3-dimethyl-1H-pyrazol-5-amine, (4.018) 4-(2-chloro-4-fluorophenyl)-N-(2,6-difluorophenyl)-1,3-dimethyl-1H-pyrazol-5-amine, (4.019) 4-(2-chloro-4-fluorophenyl)-N-(2-chloro-6-fluorophenyl)-1 ,3-dimethyl-1H-pyrazol-5-amine, (4.020) 4-(2-chloro-4-fluorophenyl)-N-(2-chlorophenyl)-1,3-dimethyl-1H-pyrazol-5-amine, (4.021) 4-(2-chloro-4-fluorophenyl)-N-(2-fluorophenyl)-1 ,3-dimethyl-1H-pyrazol-5-amine, (4.022) 4-(4-chlorophenyl)-5-(2,6-difluorophenyl)-3,6-dimethylpyridazine, (4.023) N-(2-bromo-6-fluorophenyl)-4-(2-chloro-4-fluorophenyl)-1,3-dimethyl-1H-pyrazol-5-amine, (4.024) N-(2-bromophenyl)-4-(2-chloro-4-fluorophenyl)-1,3-dimethyl-1H-pyrazol-5-amine, (4.025) N-(4-chloro-2,6-difluorophenyl)-4-(2-chloro-4-fluorophenyl)-1,3-dimethyl-1H-pyrazol-5-amine.
5) Compounds capable to have a multisite action, for example (5.001) bordeaux mixture, (5.002) captafol, (5.003) captan, (5.004) chlorothalonil, (5.005) copper hydroxide, (5.006) copper naphthenate, (5.007) copper oxide, (5.008) copper oxychloride, (5.009) copper(2+) sulfate, (5.010) dithianon, (5.011) dodine, (5.012) folpet, (5.013) mancozeb, (5.014) maneb, (5.015) metiram, (5.016) metiram zinc, (5.017) oxine-copper, (5.018) propineb, (5.019) sulfur and sulfur preparations including calcium polysulfide, (5.020) thiram, (5.021) zineb, (5.022) ziram, (5.023) 6-ethyl-5,7-dioxo-6,7-dihydro-5H-pyrrolo[3',4':5,6][1,4]dithiino[2,3-c][1,2]thiazole-3-carbonitrile.
6) Compounds capable to induce a host defence, for example (6.001) acibenzolar-S-methyl, (6.002) isotianil, (6.003) probenazole, (6.004) tiadinil.
7) Inhibitors of the amino acid and/or protein biosynthesis, for example (7.001) cyprodinil, (7.002) kasugamycin, (7.003) kasugamycin hydrochloride hydrate, (7.004) oxytetracycline, (7.005) pyrimethanil, (7.006) 3-(5-fluoro-3,3,4,4-tetramethyl-3,4-dihydroisoquinolin-1-yl)quinoline.
8) Inhibitors of the ATP production, for example (8.001) silthiofam.
9) Inhibitors of the cell wall synthesis, for example (9.001) benthiavalicarb, (9.002) dimethomorph, (9.003) flumorph, (9.004) iprovalicarb, (9.005) mandipropamid, (9.006) pyrimorph, (9.007) valifenalate, (9.008) (2E)-3-(4-tert-butylphenyl)-3-(2-chloropyridin-4-yl)-1-(morpholin-4-yl)prop-2-en-1-one, (9.009) (2Z)-3-(4-tert-butylphenyl)-3-(2-chloropyridin-4-yl)-1-(morpholin-4-yl)prop-2-en-1-one.
10) Inhibitors of the lipid and membrane synthesis, for example (10.001) propamocarb, (10.002) propamocarb hydrochloride, (10.003) tolclofos-methyl.
11) Inhibitors of the melanin biosynthesis, for example (11.001) tricyclazole, (11.002) 2,2,2-trifluoroethyl {3-methyl-1-[(4-methylbenzoyl)amino]butan-2-yl}carbamate.
12) Inhibitors of the nucleic acid synthesis, for example (12.001) benalaxyl, (12.002) benalaxyl-M (kiralaxyl), (12.003) metalaxyl, (12.004) metalaxyl-M (mefenoxam).
13) Inhibitors of the signal transduction, for example (13.001) fludioxonil, (13.002) iprodione, (13.003) procymidone, (13.004) proquinazid, (13.005) quinoxyfen, (13.006) vinclozolin.
14) Compounds capable to act as an uncoupler, for example (14.001) fluazinam, (14.002) meptyldinocap.
15) Further compounds, for example (15.001) Abscisic acid, (15.002) benthiazole, (15.003) bethoxazin, (15.004) capsimycin, (15.005) carvone, (15.006) chinomethionat, (15.007) cufraneb, (15.008) cyflufenamid, (15.009) cymoxanil, (15.010) cyprosulfamide, (15.011) flutianil, (15.012) fosetyl-aluminium, (15.013) fosetyl-calcium, (15.014) fosetyl-sodium, (15.015) methyl isothiocyanate, (15.016) metrafenone, (15.017) mildiomycin, (15.018) natamycin, (15.019) nickel dimethyldithiocarbamate, (15.020) nitrothal-isopropyl, (15.021) oxamocarb, (15.022) oxathiapiprolin, (15.023) oxyfenthiin, (15.024) pentachlorophenol and salts, (15.025) phosphorous acid and its salts, (15.026) propamocarb-fosetylate, (15.027) pyriofenone (chlazafenone), (15.028) tebufloquin, (15.029) tecloftalam, (15.030) tolnifanide, (15.031) 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.032) 1-(4-{4-[(5S)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.033) 2-(6-benzylpyridin-2-yl)quinazoline, (15.034) dipymetitrone, (15.035) 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone, (15.036) 2-[3,5-bis(difluoromethyl)-1H-pyrazol-1-yl]-1-[4-(4-{5-[2-chloro-6-(prop-2-yn-1-yloxy)phenyl]-4,5-dihydro-1,2-oxazol-3-yl}-1,3-thiazol-2-yl)piperidin-1-yl]ethanone, (15.037) 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl]ethanone, (15.038) 2-[6-(3-fluoro-4-methoxyphenyl)-5-methylpyridin-2-yl]quinazoline, (15.039) 2-{(5R)-3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.040) 2-{(5S)-3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.041) Ipflufenoquin, (15.042) 2-{2-fluoro-6-[(8-fluoro-2-methylquinolin-3-yl)oxy]phenyl}propan-2-ol, (15.043) 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.044) 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}phenyl methanesulfonate, (15.045) 2-phenylphenol and salts, (15.046) 3-(4,4,5-trifluoro-3,3-dimethyl-3,4-dihydroisoquinolin-1-yl)quinoline, (15.047) quinofumelin, (15.048) 4-amino-5-fluoropyrimidin-2-ol (tautomeric form: 4-amino-5-fluoropyrimidin-2(1H)-one), (15.049) 4-oxo-4-[(2-phenylethyl)amino]butanoic acid, (15.050) 5-amino-1,3,4-thiadiazole-2-thiol, (15.051) 5-chloro-N'-phenyl-N'-(prop-2-yn-1-yl)thiophene-2-sulfonohydrazide, (15.052) 5-fluoro-2-[(4-fluorobenzyl)oxy]pyrimidin-4-amine, (15.053) 5-fluoro-2-[(4-methylbenzyl)oxy]pyrimidin-4-amine, (15.054) 9-fluoro-2,2-dimethyl-5-(quinolin-3-yl)-2,3-dihydro-1,4-benzoxazepine, (15.055) but-3-yn-1-yl {6-[({[(Z)-(1-methyl-1H-tetrazol-5-yl)(phenyl)methylene]amino}oxy)methyl]pyrid in-2-yl}carbamate, (15.056) ethyl (2Z)-3-amino-2-cyano-3-phenylacrylate, (15.057) phenazine-1-carboxylic acid, (15.058) propyl 3,4,5-trihydroxybenzoate, (15.059) quinolin-8-ol, (15.060) quinolin-8-ol sulfate (2:1), (15.061) tert-butyl {6-[({[(1-methyl-1H-tetrazol-5-yl)(phenyl)methylene]amino}oxy)methyl]pyridin-2-yl}carbamate, (15.062) 5-fluoro-4-imino-3-methyl-1-[(4-methylphenyl)sulfonyl]-3,4-dihydropyrimidin-2(1H)-one, (15.063) aminopyrifen.

All named mixing partners of the classes (1) to (15) as described here above can be present in the form of the free compound and/or, if their functional groups enable this, an agriculturally acceptable salt thereof.

The compound of formula (I) or (I') and the compositions may also be combined with one or more biological control agents.

Examples of biological control agents which may be combined with the compound of formula (I) or (I') and compositions comprising thereof are:
(A) Antibacterial agents selected from the group of:
   (A1) bacteria, such as (A1.1) *Bacillus subtilis,* in particular strain QST713/AQ713 (available as SERENADE OPTI or SERENADE ASO from Bayer CropScience LP, US, having NRRL Accession No. B21661and described in U.S. Patent No. 6,060,051); (A1.2) *Bacillus amyloliquefaciens,* in particular strain D747 (available as Double Nickel^{™} from Certis, US, having accession number FERM BP-8234 and disclosed in US Patent No. 7,094,592); (A1.3) *Bacillus pumilus,* in particular strain BU F-33 (having NRRL Accession No. 50185); (A1.4) *Bacillus subtilis var. amyloliquefaciens* strain FZB24 (available as Taegro^{®} from Novozymes, US); (A1.5) a *Paenibacillus* sp. strain having Accession No. NRRL B-50972 or Accession No. NRRL B-67129 and described in International Patent Publication No. WO 2016/154297; and
   (A2) fungi, such as (A2.1) *Aureobasidium pullulans,* in particular blastospores of strain DSM14940;
   (A2.2) *Aureobasidium pullulans* blastospores of strain DSM 14941; (A2.3) *Aureobasidium pullulans,* in particular mixtures of blastospores of strains DSM14940 and DSM14941;
(B) Fungicides selected from the group of:
   (B1) bacteria, for example (B1.1) *Bacillus subtilis,* in particular strain QST713/AQ713 (available as SERENADE OPTI or SERENADE ASO from Bayer CropScience LP, US, having NRRL Accession No. B21661and described in U.S. Patent No. 6,060,051); (B1.2) *Bacillus pumilus,* in particular strain QST2808 (available as SONATA^{®} from Bayer CropScience LP, US, having Accession No. NRRL B-30087 and described in U.S. Patent No. 6,245,551); (B1.3) *Bacillus pumilus,* in particular strain GB34 (available as Yield Shield^{®} from Bayer AG, DE); (B1.4) *Bacillus pumilus,* in particular strain BU F-33 (having NRRL Accession No. 50185); (B1.5) *Bacillus amyloliquefaciens,* in particular strain D747 (available as Double Nickel^{™} from Certis, US, having accession number FERM BP-8234 and disclosed in US Patent No. 7,094,592); (B1.6) *Bacillus subtilis* Y1336 (available as BIOBAC^{®} WP from Bion-Tech, Taiwan, registered as a biological fungicide in Taiwan under Registration Nos. 4764, 5454, 5096 and 5277); (B1.7) *Bacillus amyloliquefaciens* strain MBI 600 (available as SUBTILEX from BASF SE); (B1.8) *Bacillus subtilis* strain GB03 (available as Kodiak^{®} from Bayer AG, DE); (B1.9) *Bacillus subtilis var. amyloliquefaciens* strain FZB24 (available from Novozymes Biologicals Inc., Salem, Virginia or Syngenta Crop Protection, LLC, Greensboro, North Carolina as the fungicide TAEGRO^{®} or TAEGRO^{®} ECO (EPA Registration No. 70127-5); (B1.10) *Bacillus mycoides,* isolate J (available as BmJ TGAI or WG from Certis USA); (B1.11) *Bacillus licheniformis,* in particular strain SB3086 (available as EcoGuard TM Biofungicide and Green Releaf from Novozymes); (B1.12) a *Paenibacillus* sp. strain having Accession No. NRRL B-50972 or Accession No. NRRL B-67129 and described in International Patent Publication No. WO 2016/154297.

In some embodiments, the biological control agent is a *Bacillus subtilis* or *Bacillus amyloliquefaciens* strain that produces a fengycin or plipastatin-type compound, an iturin-type compound, and/or a surfactin-type compound. For background, see the following review article: Ongena, M., et al., "Bacillus Lipopeptides: Versatile Weapons for Plant Disease Biocontrol," Trends in Microbiology, Vol 16, No. 3, March 2008, pp. 115-125. *Bacillus* strains capable of producing lipopeptides include *Bacillus subtilis* QST713 (available as SERENADE OPTI or SERENADE ASO from Bayer CropScience LP, US, having NRRL Accession No. B21661and described in U.S. Patent No. 6,060,051), *Bacillus amyloliquefaciens* strain D747 (available as Double Nickel^{™} from Certis, US, having accession number FERM BP-8234 and disclosed in US Patent No. 7,094,592); *Bacillus subtilis* MBI600 (available as SUBTILEX^{®} from Becker Underwood, US EPA Reg. No. 71840-8); *Bacillus subtilis* Y1336 (available as BIOBAC^{®} WP from Bion-Tech, Taiwan, registered as a biological fungicide in Taiwan under Registration Nos. 4764, 5454, 5096 and 5277); *Bacillus amyloliquefaciens,* in particular strain FZB42 (available as RHIZOVITAL^{®} from ABiTEP, DE); and *Bacillus subtilis* var. *amyloliquefaciens* FZB24 (available from Novozymes Biologicals Inc., Salem, Virginia or Syngenta Crop Protection, LLC, Greensboro, North Carolina as the fungicide TAEGRO^{®} or TAEGRO^{®} ECO (EPA Registration No. 70127-5); and
(B2) fungi, for example: (B2.1) *Coniothyrium minitans,* in particular strain CON/M/91-8 (Accession No. DSM-9660; e.g. Contans ^{®} from Bayer); (B2.2) *Metschnikowia fructicola,* in particular strain NRRL Y-30752 (e.g. Shemer^{®}); (B2.3) *Microsphaeropsis ochracea* (e.g. Microx^{®} from Prophyta); (B2.5) *Trichoderma spp.,* including *Trichoderma atroviride,* strain SC1 described in International Application No. PCT/IT2008/000196); (B2.6) *Trichoderma harzianum rifai* strain KRL-AG2 (also known as strain T-22, /ATCC 208479, e.g. PLANTSHIELD T-22G, Rootshield^{®}, and TurfShield from BioWorks, US); (B2.14) *Gliocladium roseum,* strain 321U from W.F. Stoneman Company LLC; (B2.35) *Talaromyces flavus,* strain V117b; (B2.36) *Trichoderma asperellum,* strain ICC 012 from Isagro; (B2.37) *Trichoderma asperellum,* strain SKT-1 (e.g. ECO-HOPE^{®} from Kumiai Chemical Industry); (B2.38) *Trichoderma atroviride,* strain CNCM I-1237 (e.g. Esquive^{®} WP from Agrauxine, FR); (B2.39) *Trichoderma atroviride,* strain no. V08/002387; (B2.40) *Trichoderma atroviride,* strain NMI no. V08/002388; (B2.41) *Trichoderma atroviride,* strain NMI no. V08/002389; (B2.42) *Trichoderma atroviride,* strain NMI no. V08/002390; (B2.43) *Trichoderma atroviride,* strain LC52 (e.g. Tenet by Agrimm Technologies Limited); (B2.44) *Trichoderma atroviride,* strain ATCC 20476 (IMI 206040); (B2.45) *Trichoderma atroviride,* strain T11 (IMI352941/ CECT20498); (B2.46) *Trichoderma harmatum;* (B2.47) *Trichoderma harzianum;* (B2.48) *Trichoderma harzianum rifai T39* (e.g. Trichodex^{®} from Makhteshim, US); (B2.49) *Trichoderma harzianum,* in particular, strain KD (e.g. Trichoplus from Biological Control Products, SA (acquired by Becker Underwood)); (B2.50) *Trichoderma harzianum,* strain ITEM 908 (e.g. Trianum-P from Koppert); (B2.51) *Trichoderma harzianum,* strain TH35 (e.g. Root-Pro by Mycontrol); (B2.52) *Trichoderma virens* (also known as *Gliocladium virens*)*,* in particular strain GL-21 (e.g. SoilGard 12G by Certis, US); (B2.53) *Trichoderma viride,* strain TV1(e.g. Trianum-P by Koppert); (B2.54) *Ampelomyces quisqualis,* in particular strain AQ 10 (e.g. AQ 10^{®} by IntrachemBio Italia); (B2.56) *Aureobasidium pullulans,* in particular blastospores of strain DSM14940; (B2.57) *Aureobasidium pullulans,* in particular blastospores of strain DSM 14941; (B2.58) *Aureobasidium pullulans,* in particular mixtures of blastospores of strains DSM14940 and DSM 14941 (e.g. Botector^{®} by bio-ferm, CH); (B2.64) *Cladosporium cladosporioides,* strain H39 (by Stichting Dienst Landbouwkundig Onderzoek); (B2.69) *Gliocladium catenulatum* (Synonym: *Clonostachys rosea f. catenulate*) strain J1446 (e.g. Prestop ^{®} by AgBio Inc. and also e.g. Primastop^{®} by Kemira Agro Oy); (B2.70) *Lecanicillium lecanii* (formerly known as *Verticillium lecanii*) *conidia* of strain KV01 (e.g. Vertalec^{®} by Koppert/Arysta); (B2.71) *Penicillium vermiculatum;* (B2.72) *Pichia anomala,* strain WRL-076 (NRRL Y-30842); (B2.75) *Trichoderma atroviride,* strain SKT-1 (FERM P-16510); (B2.76) *Trichoderma atroviride,* strain SKT-2 (FERM P-16511); (B2.77) *Trichoderma atroviride,* strain SKT-3 (FERM P-17021); (B2.78) *Trichoderma gamsii* (formerly *T. viride*), strain ICC080 (IMI CC 392151 CABI, e.g. BioDerma by AGROBIOSOL DE MEXICO, S.A. DE C.V.); (B2.79) *Trichoderma harzianum,* strain DB 103 (e.g., T-Gro 7456 by Dagutat Biolab); (B2.80) *Trichoderma polysporum,* strain IMI 206039 (e.g. Binab TF WP by BINAB Bio-Innovation AB, Sweden); (B2.81) *Trichoderma stromaticum* (e.g. Tricovab by Ceplac, Brazil); (B2.83) *Ulocladium oudemansii,* in particular strain HRU3 (e.g. Botry-Zen^{®} by Botry-Zen Ltd, NZ); (B2.84) *Verticillium albo-atrum* (formerly V. *dahliae*)*,* strain WCS850 (CBS 276.92; e.g. Dutch Trig by Tree Care Innovations); (B2.86) *Verticillium chlamydosporium;* (B2.87) mixtures of *Trichoderma asperellum* strain ICC 012 and *Trichoderma gamsii* strain ICC 080 (product known as e.g. BIO-TAM^{™}from Bayer CropScience LP, US).

Further examples of biological control agents which may be combined with the compound of formula (I) or (I') and compositions comprising thereof are:
bacteria selected from the group consisting of *Bacillus cereus,* in particular B. *cereus* strain CNCM I-1562 and *Bacillus firmus,* strain I-1582 (Accession number CNCM I-1582), *Bacillus subtilis strain* OST 30002 (Accession No. NRRL B-50421), *Bacillus thuringiensis,* in particular B. *thuringiensis* subspecies *israelensis* (serotype H-14), strain AM65-52 (Accession No. ATCC 1276), B. *thuringiensis subsp. aizawai,* in particular strain ABTS-1857 (SD-1372), B. *thuringiensis subsp. kurstaki* strain HD-1, B. *thuringiensis subsp. tenebrionis* strain NB 176 (SD-5428), *Pasteuria penetrans, Pasteuria spp.* (Rotylenchulus reniformis nematode)-PR3 (Accession Number ATCC SD-5834), *Streptomyces microflavus* strain AQ6121 (= QRD 31.013, NRRL B-50550), and *Streptomyces galbus* strain AQ 6047 (Acession Number NRRL 30232);
fungi and yeasts selected from the group consisting of *Beauveria bassiana,* in particular strain ATCC 74040, *Lecanicillium spp.,* in particular strain HRO LEC 12, *Metarhizium anisopliae,* in particular strain F52 (DSM3884 or ATCC 90448), *Paecilomyces fumosoroseus* (now: *Isaria fumosorosea*)*,* in particular strain IFPC 200613, or strain Apopka 97 (Accesion No. ATCC 20874), and *Paecilomyces lilacinus,* in particular *P*. *lilacinus* strain 251 (AGAL 89/030550);
viruses selected from the group consisting of *Adoxophyes orana* (summer fruit tortrix) granulosis virus (GV), *Cydia pomonella* (codling moth) granulosis virus (GV), *Helicoverpa armigera* (cotton bollworm) nuclear polyhedrosis virus (NPV), *Spodoptera exigua* (beet armyworm) mNPV, *Spodoptera frugiperda* (fall armyworm) mNPV, and *Spodoptera littoralis* (African cotton leafworm) NPV.
bacteria and fungi which can be added as 'inoculant' to plants or plant parts or plant organs and which, by virtue of their particular properties, promote plant growth and plant health. Examples are: *Agrobacterium spp., Azorhizobium caulinodans, Azospirillum spp., Azotobacter spp., Bradyrhizobium spp., Burkholderia spp.,* in particular *Burkholderia cepacia* (formerly known as *Pseudomonas cepacia*)*, Gigaspora spp.,* or *Gigaspora monosporum, Glomus spp., Laccaria spp., Lactobacillus buchneri, Paraglomus spp., Pisolithus tinctorus, Pseudomonas spp., Rhizobium spp.,* in particular *Rhizobium trifolii, Rhizopogon spp., Scleroderma spp., Suillus spp.,* and *Streptomyces spp.*
plant extracts and products formed by microorganisms including proteins and secondary metabolites which can be used as biological control agents, such as *Allium sativum, Artemisia absinthium,* azadirachtin, Biokeeper WP, *Cassia nigricans, Celastrus angulatus, Chenopodium anthelminticum,* chitin, Armour-Zen, *Dryopteris filix-mas, Equisetum arvense,* Fortune Aza, Fungastop, Heads Up (*Chenopodium quinoa* saponin extract), *Pyrethrum*/*Pyrethrins, Quassia amara, Quercus, Quillaja,* Regalia, "Requiem ^{™} Insecticide", rotenone, ryania/ryanodine, *Symphytum officinale, Tanacetum vulgare,* thymol, Triact 70, TriCon, *Tropaeulum majus, Urtica dioica,* Veratrin, *Viscum album, Brassicaceae* extract, in particular oilseed rape powder or mustard powder.

Examples of insecticides, acaricides and nematicides, respectively, which could be mixed with the compound of formula (I) or (I') and compositions comprising thereof are:
(1) Acetylcholinesterase (AChE) inhibitors, such as, for example, carbamates, for example alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC and xylylcarb; or organophosphates, for example acephate, azamethiphos, azinphos-ethyl, azinphos-methyl, cadusafos, chlorethoxyfos, chlorfenvinphos, chlormephos, chlorpyrifos-methyl, coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothiophosphoryl) salicylate, isoxathion, malathion, mecarbam, methamidophos, methidathion, mevinphos, monocrotophos, naled, omethoate, oxydemeton-methyl, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, triclorfon and vamidothion.
(2) GABA-gated chloride channel blockers, such as, for example, cyclodiene-organochlorines, for example chlordane and endosulfan or phenylpyrazoles (fiproles), for example ethiprole and fipronil.
(3) Sodium channel modulators, such as, for example, pyrethroids, e.g. acrinathrin, allethrin, d-cis-trans allethrin, d-trans allethrin, bifenthrin, bioallethrin, bioallethrin s-cyclopentenyl isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin [(1R)-trans-isomer], deltamethrin, empenthrin [(EZ)-(1R)-isomer], esfenvalerate, etofenprox, fenpropathrin, fenvalerate, flucythrinate, flumethrin, tau-fluvalinate, halfenprox, imiprothrin, kadethrin, momfluorothrin, permethrin, phenothrin [(1R)-trans-isomer], prallethrin, pyrethrins (pyrethrum), resmethrin, silafluofen, tefluthrin, tetramethrin, tetramethrin [(1R)-isomer)], tralomethrin and transfluthrin or DDT or methoxychlor.
(4) Nicotinic acetylcholine receptor (nAChR) competitive modulators, such as, for example, neonicotinoids, e.g. acetamiprid, clothianidin, dinotefuran, imidacloprid, nitenpyram, thiacloprid and thiamethoxam or nicotine or sulfoxaflor or flupyradifurone.
(5) Nicotinic acetylcholine receptor (nAChR) allosteric modulators, such as, for example, spinosyns, e.g. spinetoram and spinosad.
(6) Glutamate-gated chloride channel (GluCI) allosteric modulators, such as, for example, avermectins/milbemycins, for example abamectin, emamectin benzoate, lepimectin and milbemectin.
(7) Juvenile hormone mimics, such as, for example, juvenile hormone analogues, e.g. hydroprene, kinoprene and methoprene or fenoxycarb or pyriproxyfen.
(8) Miscellaneous non-specific (multi-site) inhibitors, such as, for example, alkyl halides, e.g. methyl bromide and other alkyl halides; or chloropicrine or sulphuryl fluoride or borax or tartar emetic or methyl isocyanate generators, e.g. diazomet and metam.
(9) Modulators of Chordotonal Organs, such as, for example pymetrozine or flonicamid.
(10) Mite growth inhibitors, such as, for example clofentezine, hexythiazox and diflovidazin or etoxazole.
(11) Microbial disruptors of the insect gut membrane, such as, for example *Bacillus thuringiensis* subspecies *israelensis, Bacillus sphaericus, Bacillus thuringiensis* subspecies *aizawai, Bacillus thuringiensis* subspecies *kurstaki, Bacillus thuringiensis* subspecies *tenebrionis,* and *B.t.* plant proteins: Cry1Ab, Cry1Ac, Cry1Fa, Cry1A.105, Cry2Ab, Vip3A, mCry3A, Cry3Ab, Cry3Bb, Cry34Ab1/35Ab1.
(12) Inhibitors of mitochondrial ATP synthase, such as, ATP disruptors such as, for example, diafenthiuron or organotin compounds, for example azocyclotin, cyhexatin and fenbutatin oxide or propargite or tetradifon.
(13) Uncouplers of oxidative phosphorylation via disruption of the proton gradient, such as, for example, chlorfenapyr, DNOC and sulfluramid.
(14) Nicotinic acetylcholine receptor channel blockers, such as, for example, bensultap, cartap hydrochloride, thiocylam, and thiosultap-sodium.
(15) Inhibitors of chitin biosynthesis, type 0, such as, for example, bistrifluron, chlorfluazuron, diflubenzuron, flucycloxuron, flufenoxuron, hexaflumuron, lufenuron, novaluron, noviflumuron, teflubenzuron and triflumuron.
(16) Inhibitors of chitin biosynthesis, type 1, for example buprofezin.
(17) Moulting disruptor (in particular for Diptera, i.e. dipterans), such as, for example, cyromazine.
(18) Ecdysone receptor agonists, such as, for example, chromafenozide, halofenozide, methoxyfenozide and tebufenozide.
(19) Octopamine receptor agonists, such as, for example, amitraz.
(20) Mitochondrial complex III electron transport inhibitors, such as, for example, hydramethylnone or acequinocyl or fluacrypyrim.
(21) Mitochondrial complex I electron transport inhibitors, such as, for example from the group of the METI acaricides, e.g. fenazaquin, fenpyroximate, pyrimidifen, pyridaben, tebufenpyrad and tolfenpyrad or rotenone (Derris).
(22) Voltage-dependent sodium channel blockers, such as, for example indoxacarb or metaflumizone.
(23) Inhibitors of acetyl CoA carboxylase, such as, for example, tetronic and tetramic acid derivatives, e.g. spirodiclofen, spiromesifen and spirotetramat.
(24) Mitochondrial complex IV electron transport inhibitors, such as, for example, phosphines, e.g. aluminium phosphide, calcium phosphide, phosphine and zinc phosphide or cyanides, e.g. calcium cyanide, potassium cyanide and sodium cyanide.
(25) Mitochondrial complex II electron transport inhibitors, such as, for example, beta-ketonitrile derivatives, e.g. cyenopyrafen and cyflumetofen and carboxanilides, such as, for example, pyflubumide.
(28) Ryanodine receptor modulators, such as, for example, diamides, e.g. chlorantraniliprole, cyantraniliprole and flubendiamide,
   further active compounds such as, for example, Afidopyropen, Afoxolaner, Azadirachtin, Benclothiaz, Benzoximate, Bifenazate, Broflanilide, Bromopropylate, Chinomethionat, Chloroprallethrin, Cryolite, Cyclaniliprole, Cycloxaprid, Cyhalodiamide, Dicloromezotiaz, Dicofol, epsilon-Metofluthrin, epsilon-Momfluthrin, Flometoquin, Fluazaindolizine, Fluensulfone, Flufenerim, Flufenoxystrobin, Flufiprole, Fluhexafon, Fluopyram, Fluralaner, Fluxametamide, Fufenozide, Guadipyr, Heptafluthrin, Imidaclothiz, Iprodione, kappa-Bifenthrin, kappa-Tefluthrin, Lotilaner, Meperfluthrin, Paichongding, Pyridalyl, Pyrifluquinazon, Pyriminostrobin, Spirobudiclofen, Tetramethylfluthrin, Tetraniliprole, Tetrachlorantraniliprole, Tigolaner, Tioxazafen, Thiofluoximate, Triflumezopyrim and iodomethane; furthermore preparations based on *Bacillus firmus* (l-1582, BioNeem, Votivo), and also the following compounds: 1-{2-fluoro-4-methyl-5-[(2,2,2-trifluoroethyl)su Iphinyl]phenyl}-3-(trifluoromethyl)-1H-1,2,4-triazole-5-amine (known from WO2006/043635) (CAS 885026-50-6), {1'-[(2E)-3-(4-chlorophenyl)prop-2-en-1-yl]-5-fluorospiro[indol-3,4'-piperidin]-1(2H)-yl}(2-chloropyridin-4-yl)methanone (known from WO2003/106457) (CAS 637360-23-7), 2-chloro-N-[2-{1-[(2E)-3-(4-chlorophenyl)prop-2-en-1-yl]piperidin-4-yl}-4-(trifluoromethyl)phenyl]isonicotinamide (known from WO2006/003494) (CAS 872999-66-1), 3-(4-chloro-2,6-dimethylphenyl)-4-hydroxy-8-methoxy-1,8-diazaspiro[4.5]dec-3-en-2-one (known from WO 2010052161) (CAS 1225292-17-0), 3-(4-chloro-2,6-dimethylphenyl)-8-methoxy-2-oxo-1,8-diazaspiro[4.5]dec-3-en-4-yl ethyl carbonate (known from EP2647626) (CAS 1440516-42-6) , 4-(but-2-yn-1-yloxy)-6-(3,5-dimethylpiperidin-1-yl)-5-fluoropyrimidine (known from WO2004/099160) (CAS 792914-58-0), PF1364 (known from JP2010/018586) (CAS 1204776-60-2), N-[(2E)-1-[(6-chloropyridin-3-yl)methyl]pyridin-2(1H)-ylidene]-2,2,2-trifluoroacetamide (known from WO2012/029672) (CAS 1363400-41-2), (3E)-3-[1-[(6-chloro-3-pyridyl)methyl]-2-pyridylidene]-1,1,1-trifluoro-propan-2-one (known from WO2013/144213) (CAS 1461743-15-6), , N-[3-(benzylcarbamoyl)-4-chlorophenyl]-1-methyl-3-(pentafluoroethyl)-4-(trifluoromethyl)-1H-pyrazole-5-carboxamide (known from WO2010/051926) (CAS 1226889-14-0), 5-bromo-4-chloro-N-[4-chloro-2-methyl-6-(methylcarbamoyl)phenyl]-2-(3-chloro-2-pyridyl)pyrazole-3-carboxamide (known from CN103232431) (CAS 1449220-44-3), 4-[5-(3,5-dichlorophenyl)-4,5-dihydro-5-(trifluoromethyl)-3-isoxazolyl]-2-methyl-N-(cis-1-oxido-3-thietanyl)-benzamide, 4-[5-(3,5-dichlorophenyl)-4,5-dihydro-5-(trifluoromethyl)-3-isoxazolyl]-2-methyl-N-( trans-1-oxido-3-thietanyl)-benzamide and 4-[(5S)-5-(3,5-dichlorophenyl)-4,5-dihydro-5-(trifluoromethyl)-3-isoxazolyl]-2-methyl-N-(eis-1-oxido-3-thietanyl)benzamide (known from WO 2013/050317 A1) (CAS 1332628-83-7), *N*-[3-chloro-1-(3-pyridinyl)-1*H*-pyrazol-4-yl]-*N*-ethyl-3-[(3, 3,3-trifluoropropyl)sulfinyl]-propanamide, (+)-*N*-[3-chloro-1-(3-pyridinyl)-1*H*-pyrazol-4-yl]-*N*-ethyl-3-[(3, 3,3-trifluoropropyl)sulfinyl]-propanamide and (-)-N-[3-chloro-1-(3-pyridinyl)-1H-pyrazol-4-yl]-N-ethyl-3-[(3,3,3-trifluoropropyl)sulfinyl]-propanamide (known from WO 2013/162715 A2, WO 2013/162716 A2, US 2014/0213448 A1) (CAS 1477923-37-7), 5-[[(2E)-3-chloro-2-propen-1-yl]amino]-1-[2,6-dichloro-4-(trifluoromethyl)phenyl]-4-[(trifluoromethyl)sulfinyl]-1H-pyrazole-3-carbonitrile (known from CN 101337937 A) (CAS 1105672-77-2), 3-bromo-N-[4-chloro-2-methyl-6-[(methylamino)thioxomethyl] phenyl]-1-(3-chloro-2-pyrdinyl)-1H-pyrazole-5-carboxamide, (Liudaibenjiaxuanan, known from CN 103109816 A) (CAS 1232543-85-9); N-[4-chloro-2-[[(1,1-dimethylethyl)amino]carbonyl]-6-methylphenyl]-1-(3-chloro-2-pyridinyl)-3-(fluoromethoxy)-1H-Pyrazole-5-carboxamide (known from WO 2012/034403 A1) (CAS 1268277-22-0), N-[2-(5-amino-1,3,4-thiadiazol-2-yl)-4-chloro-6-methylphenyl]-3-bromo-1-(3-chloro-2-pyridinyl)-1H-pyrazole-5-carboxamide (known from WO 2011/085575 A1) (CAS 1233882-22-8), 4-[3-[2,6-dichloro-4-[(3,3-dichloro-2-propen-1-yl)oxy] phenoxy]propoxy]-2-methoxy-6-(trifluoromethyl)-pyrimidine (known from CN 101337940 A) (CAS 1108184-52-6); (2E)- and 2(Z)-2-[2-(4-cyanophenyl)-1-[3-(trifluoromethyl)phenyl]ethylidene]-A/-[4-(difluoromethoxy)phenyl]-hydrazinecarboxamide (known from CN 101715774 A) (CAS 1232543-85-9); 3-(2,2-dichloroethenyl)-2,2-dimethyl-4-(1H-benzimidazol-2-yl)phenyl-cyclopropanecarboxylic acid ester (known from CN 103524422 A) (CAS 1542271-46-4); (4aS)-7-chloro-2,5-dihydro-2-[[(methoxycarbonyl)[4-[(trifluoromethyl)thio]phenyl]amino]carbonyl]-indeno[1,2-e][1,3,4]oxadiazine-4a(3H)-carboxylic acid methyl ester (known from CN 102391261 A) (CAS 1370358-69-2); 6-deoxy-3-O-ethyl-2,4-di-O-methyl-, 1-[N-[4-[1-[4-(1, 1,2,2,2-pentafluoroethoxy)phenyl]-1 H-1,2,4-triazol-3-yl] phenyl]carbamate]-a-L-mannopyranose (known from US 2014/0275503 A1) (CAS 1181213-14-8); 8-(2-cyclopropylmethoxy-4-trifluoromethyl-phenoxy)-3-(6-trifluoromethyl-pyridazin-3-yl)-3-aza-bicyclo[3.2.1 ]octane (CAS 1253850-56-4), (8-anti)-8-(2-cyclopropylmethoxy-4-trifluoromethyl-phenoxy)-3-(6-trifluoromethyl-pyridazin-3-yl)-3-aza-bicyclo[3.2.1 ]octane (CAS 933798-27-7), (8-*syn*)-8-(2-cyclopropylmethoxy-4-trifluoromethyl-phenoxy)-3-(6-trifluoromethyl-pyridazin-3-yl)-3-aza-bicyclo[3.2.1 ]octane (known from WO 2007040280 A1, WO 2007040282 A1) (CAS 934001-66-8), N-[3-chloro-1-(3-pyridinyl)-1H-pyrazol-4-yl]-N-ethyl-3-[(3,3,3-trifluoropropyl)thio]-propanamide (known from WO 2015/058021 A1, WO 2015/058028 A1) (CAS 1477919-27-9) and N-[4-(aminothioxomethyl)-2-methyl-6-[(methylamino)carbonyl]phenyl]-3-bromo-1-(3-chloro-2-pyridinyl)-1H-pyrazole-5-carboxamide (known from CN 103265527 A) (CAS 1452877-50-7), 5-(1,3-dioxan-2-yl)-4-[[4-(trifluoromethyl)phenyl]methoxy]-pyrimidine (known from WO 2013/115391 A1) (CAS 1449021-97-9), 3-(4-chloro-2,6-dimethylphenyl)-4-hydroxy-8-methoxy-1-methyl-1,8-diazaspiro[4.5]dec-3-en-2-one (known from WO 2010/066780 A1, WO 2011/151146 A1) (CAS 1229023-34-0), 3-(4-chloro-2,6-dimethylphenyl)-8-methoxy-1-methyl-1,8-diazaspiro[4.5]decane-2,4-dione (known from WO 2014/187846 A1) (CAS 1638765-58-8), 3-(4-chloro-2,6-dimethylphenyl)-8-methoxy-1-methyl-2-oxo-1, 8-diazaspiro[4.5]dec-3-en-4-yl-carbonic acid ethyl ester (known from WO 2010/066780 A1, WO 2011151146 A1) (CAS 1229023-00-0), N-[1-[(6-chloro-3-pyridinyl)methyl]-2(1H)-pyridinylidene]-2,2,2-trifluoro-acetamide (known from DE 3639877 A1, WO 2012029672 A1) (CAS 1363400-41-2), [N(E)]-N-[1-[(6-chloro-3-pyridinyl)methyl]-2(1H)-pyridinylidene]-2,2,2-trifluoro-acetamide, (known from WO 2016005276 A1) (CAS 1689566-03-7), [N(Z)]-N-[1-[(6-chloro-3-pyridinyl)methyl]-2(1H)-pyridinylidene]-2,2,2-trifluoro-acetamide, (CAS 1702305-40-5), 3-endo-3-[2-propoxy-4-(trifluoromethyl)phenoxy]-9-[[5-(trifluoromethyl)-2-pyridinyl]oxy]-9-azabicyclo[3.3.1]nonane (known from WO 2011/105506 A1, WO 2016/133011 A1) (CAS 1332838-17-1).

Examples of safeners which could be mixed with the compound of formula (I) or (I') and compositions comprising thereof are, for example, benoxacor, cloquintocet (-mexyl), cyometrinil, cyprosulfamide, dichlormid, fenchlorazole (-ethyl), fenclorim, flurazole, fluxofenim, furilazole, isoxadifen (-ethyl), mefenpyr
(-diethyl), naphthalic anhydride, oxabetrinil, 2-methoxy-N-({4-[(methylcarbamoyl)amino]phenyl}-sulphonyl)benzamide (CAS 129531-12-0), 4-(dichloroacetyl)-1-oxa-4-azaspiro[4.5]decane (CAS 71526-07-3), 2,2,5-trimethyl-3-(dichloroacetyl)-1,3-oxazolidine (CAS 52836-31-4).

Examples of herbicides which could be mixed with the compound of formula (I) or (I') and compositions comprising thereof are:
Acetochlor, acifluorfen, acifluorfen-sodium, aclonifen, alachlor, allidochlor, alloxydim, alloxydim-sodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlor-potassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bromacil, bromobutide, bromofenoxim, bromoxynil, bromoxynil-butyrate, -potassium, -heptanoate, and - octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, chlorfenac, chlorfenac-sodium, chlorfenprop, chlorflurenol, chlorflurenol-methyl, chloridazon, chlorimuron, chlorimuron-ethyl, chlorophthalim, chlorotoluron, chlorthal-dimethyl, chlorsulfuron, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyrimorate, cyclosulfamuron, cycloxydim, cyhalofop, cyhalofop-butyl, cyprazine, 2,4-D, 2,4-D-butotyl, -butyl, -dimethylammonium, -diolamin, -ethyl, -2-ethylhexyl, -isobutyl, -isooctyl, -isopropylammonium, -potassium, -triisopropanolammonium, and -trolamine, 2,4-DB, 2,4-DB-butyl, -dimethylammonium, -isooctyl, -potassium, and -sodium, daimuron (dymron), dalapon, dazomet, n-decanol, desmedipham, detosyl-pyrazolate (DTP), dicamba, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-P, diclofop, diclofop-methyl, diclofop-P-methyl, diclosulam, difenzoquat, diflufenican, diflufenzopyr, diflufenzopyr-sodium, dimefuron, dimepiperate, dimethachlor, dimethametryn, dimethenamid, dimethenamid-P, dimetrasulfuron, dinitramine, dinoterb, diphenamid, diquat, diquat-dibromid, dithiopyr, diuron, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-9600, F-5231, i.e. N-{2-chloro-4-fluoro-5-[4-(3-fluoropropyl)-5-oxo-4,5-dihydro-1 H-tetrazol-1-yl]phenyl}ethanesulfonamide, F-7967, i. e. 3-[7-chloro-5-fluoro-2-(trifluoromethyl)-1H-benzimidazol-4-yl]-1-methyl-6-(trifluoromethyl)pyrimidine-2,4(1H,3H)-dione, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, -dimethylammonium and -methyl, fluoroglycofen, fluoroglycofen-ethyl, flupropanate, flupyrsulfuron, flupyrsulfuron-methyl-sodium, fluridone, flurochloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosate-ammonium, -isopropylammonium, -diammonium, -dimethylammonium, -potassium, -sodium, and -trimesium, H-9201, i.e. O-(2,4-dimethyl-6-nitrophenyl) O-ethyl isopropylphosphoramidothioate, halauxifen, halauxifen-methyl ,halosafen, halosulfuron, halosulfuron-methyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(dimethoxyphosphoryl) ethyl-(2,4-dichlorophenoxy)acetate, imazamethabenz, imazamethabenz-methyl, imazamox, imazamox-ammonium, imazapic, imazapic-ammonium, imazapyr, imazapyr-isopropylammonium, imazaquin, imazaquin-ammonium, imazethapyr, imazethapyr-immonium, imazosulfuron, indanofan, indaziflam, iodosulfuron, iodosulfuron-methyl-sodium, ioxynil, ioxynil-octanoate, -potassium and -sodium, ipfencarbazone, isoproturon, isouron, isoxaben, isoxaflutole, karbutilate, KUH-043, i.e. 3-({[5-(difluoromethyl)-1-methyl-3-(trifluoromethyl)-1H-pyrazol-4-yl]methyl}sulfonyl)-5,5-dimethyl-4,5-dihydro-1,2-oxazole, ketospiradox, lactofen, lenacil, linuron, MCPA, MCPA-butotyl, -dimethylammonium, -2-ethylhexyl, -isopropylammonium, -potassium, and -sodium, MCPB, MCPB-methyl, -ethy,l and -sodium, mecoprop, mecoprop-sodium, and -butotyl, mecoprop-P, mecoprop-P-butotyl, -dimethylammonium, - 2-ethylhexyl, and -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinat, mono-linuron, monosulfuron, monosulfuron-ester, MT-5950, i.e. N-(3-chloro-4-isopropylphenyl)-2-methylpentan amide, NGGC-011, napropamide, NC-310, i.e. [5-(benzyloxy)-1-methyl-1H-pyrazol-4-yl](2,4-dichlorophenyl)methanone, neburon, nicosulfuron, nonanoic acid (pelargonic acid), norflurazon, oleic acid (fatty acids), orbencarb, orthosulfamuron, oryzalin, oxadiargyl, oxadiazon, oxasulfuron, oxaziclomefon, oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, pro-sulfuron, pyraclonil, pyraflufen, pyraflufen-ethyl, pyrasulfotole, pyrazolynate (pyrazolate), pyrazosulfuron, pyrazosulfuron-ethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, rimsulfuron, saflufenacil, sethoxydim, siduron, simazine, simetryn, SL-261, sulcotrion, sulfentrazone, sulfometuron, sulfometuron-methyl, sulfosulfuron, SYN-523, SYP-249, i.e. 1-ethoxy-3-methyl-1-oxobut-3-en-2-yl 5-[2-chloro-4-(trifluoromethyl)phenoxy]-2-nitrobenzoate, SYP-300, i.e. 1-[7-fluoro-3-oxo-4-(prop-2-yn-1-yl)-3,4-dihydro-2H-1,4-benzoxazin-6-yl]-3-propyl-2-thioxoimidazolidine-4,5-dione, 2,3,6-TBA, TCA (trichloroacetic acid), TCA-sodium, tebuthiuron, tefuryltrione, tembotrione, tepraloxydim, terbacil, terbucarb, terbumeton, terbuthylazin, terbutryn, thenylchlor, thiazopyr, thiencarbazone, thiencarbazone-methyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-dichloro-N-{2-[(4,6-dimethoxypyrimidin-2-yl)oxy]benzyl}aniline, and the following compounds:

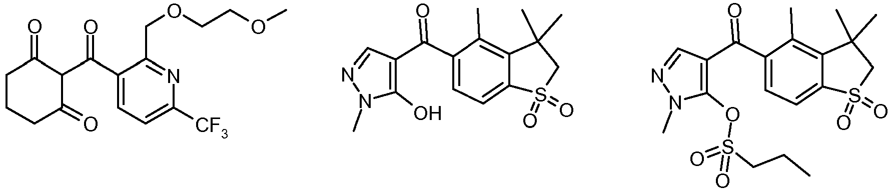

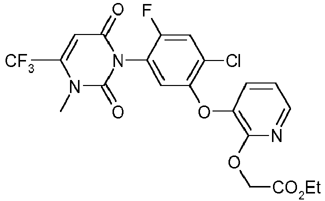

Examples for plant growth regulators are:
Acibenzolar, acibenzolar-S-methyl, 5-aminolevulinic acid, ancymidol, 6-benzylaminopurine, Brassinolid, catechine, chlormequat chloride, cloprop, cyclanilide, 3-(cycloprop-1-enyl) propionic acid, daminozide, dazomet, n-decanol, dikegulac, dikegulac-sodium, endothal, endothal-dipotassium, -disodium, and -mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, jasmonic acid, maleic hydrazide, mepiquat chloride, 1-methyl-cyclopropene, methyl jasmonate, 2-(1-naphthyl)acetamide, 1-naphthylacetic acid, 2- naphthyloxyacetic acid, nitrophenolate-mixture, paclobutrazol, N-(2-phenylethyl)-beta-alanine, N-phenylphthalamic acid, prohexadione, prohexadione-calcium, prohydrojasmone, salicylic acid, strigolactone, tecnazene, thidiazuron, triacontanol, trinexapac, trinexapac-ethyl, tsitodef, uniconazole, uniconazole-P.

### Methods and uses

The compound of formula (I) or (I') and compositions comprising thereof comprising thereof have potent microbicidal activity and/or plant defense modulating potential. They can be used for controlling unwanted microorganisms, such as unwanted fungi and bacteria. They can be particularly useful in crop protection (they control microorganisms that cause plants diseases) or for protecting materials (e.g. industrial materials, timber, storage goods) as described in more details herein below. More specifically, the compound of formula (I) or (I') and compositions comprising thereof can be used to protect seeds, germinating seeds, emerged seedlings, plants, plant parts, fruits, harvest goods and/or the soil in which the plants grow from unwanted microorganisms.

Control or controlling as used herein encompasses protective, curative and eradicative treatment of unwanted microorganisms. Unwanted microorganisms may be pathogenic bacteria, pathogenic virus, pathogenic oomycetes or pathogenic fungi, more specifically phytopathogenic bacteria, phytopathogenic virus, phytopathogenic oomycetes or phytopathogenic fungi. As detailed herein below, these phytopathogenic microorganims are the causal agents of a broad spectrum of plants diseases.

Thus, the invention relates to a method for controlling unwanted phytopathogenic microorganisms which comprises the step of applying at least one compound of formula (I) or (I') as defined herein or a composition as defined herein to the plants, plant parts, seeds, fruits or to the soil in which the plants grow. The compounds of formula (I) or (I') may be used for controlling phytopathogenic microorganisms causing rust diseases.

More specifically, the compound of formula (I) or (I') and compositions comprising thereof can be used as fungicides. For the purpose of the specification, the term "fungicide" refers to a compound or composition that can be used in crop protection for the control of unwanted fungi, such as Plasmodiophoromycetes, Chytridiomycetes, Zygomycetes, Ascomycetes, Basidiomycetes and Deuteromycetes and/or for the control of Oomycetes.

The compound of formula (I) or (I') and compositions comprising thereof may also be used as antibacterial agent. In particular, they may be used in crop protection, for example for the control of unwanted bacteria, such as Pseudomonadaceae, Rhizobiaceae, Xanthomonadaceae, Enterobacteriaceae, Corynebacteriaceae and Streptomycetaceae.

The compound of formula (I) or (I') and compositions comprising thereof may also be used as antiviral agent in crop protection. For example the compound of formula (l) or (I') and compositions comprising thereof may have effects on diseases from plant viruses, such as the tobacco mosaic virus (TMV), tobacco rattle virus, tobacco stunt virus (TStuV), tobacco leaf curl virus (VLCV), tobacco nervilia mosaic virus (TVBMV), tobacco necrotic dwarf virus (TNDV), tobacco streak virus (TSV), potato virus X (PVX), potato viruses Y, S, M, and A, potato acuba mosaic virus (PAMV), potato mop-top virus (PMTV), potato leaf-roll virus (PLRV), alfalfa mosaic virus (AMV), cucumber mosaic virus (CMV), cucumber green mottlemosaic virus (CGMMV), cucumber yellows virus (CuYV), watermelon mosaic virus (WMV), tomato spotted wilt virus (TSWV), tomato ringspot virus (TomRSV), sugarcane mosaic virus (SCMV), rice drawf virus, rice stripe virus, rice black-streaked drawf virus, strawberry mottle virus (SMoV), strawberry vein banding virus (SVBV), strawberry mild yellow edge virus (SMYEV), strawberry crinkle virus (SCrV), broad beanwilt virus (BBWV), and melon necrotic spot virus (MNSV). The present invention also relates to a method for controlling unwanted microorganisms, such as unwanted fungi, oomycetes and bacteria, comprising the step of applying at least one compound of formula (l) or at least one composition to the microorganisms and/or their habitat (to the plants, plant parts, seeds, fruits or to the soil in which the plants grow).

Typically, when the compound of formula (l) or (I') and compositions comprising thereof are used in curative or protective methods for controlling phytopathogenic fungi and/or phytopathogenic oomycetes, an effective and plant-compatible amount thereof is applied to the plants, plant parts, fruits, seeds or to the soil or substrates in which the plants grow. Suitable substrates that may be used for cultivating plants include inorganic based substrates, such as mineral wool, in particular stone wool, perlite, sand or gravel; organic substrates, such as peat, pine bark or sawdust; and petroleum based substrates such as polymeric foams or plastic beads. Effective and plant-compatible amount means an amount that is sufficient to control or destroy the fungi present or liable to appear on the cropland and that does not entail any appreciable symptom of phytotoxicity for said crops. Such an amount can vary within a wide range depending on the fungus to be controlled, the type of crop, the crop growth stage, the climatic conditions and the respective compound of formula (l) or composition used. This amount can be determined by systematic field trials that are within the capabilities of a person skilled in the art.

### Plants and plant parts

The compound of formula (l) or (I') and compositions comprising thereof may be applied to any plants or plant parts.

Plants mean all plants and plant populations, such as desired and undesired wild plants or crop plants (including naturally occurring crop plants). Crop plants may be plants which can be obtained by conventional breeding and optimization methods or by biotechnological and genetic engineering methods or combinations of these methods, including the genetically modified plants (GMO or transgenic plants) and the plant cultivars which are protectable and non-protectable by plant breeders' rights.

### Genetically modified plants (GMO)

Genetically modified plants (GMO or transgenic plants) are plants in which a heterologous gene has been stably integrated into the genome. The expression "heterologous gene" essentially means a gene which is provided or assembled outside the plant and when introduced in the nuclear, chloroplastic or mitochondrial genome. This gene gives the transformed plant new or improved agronomic or other properties by expressing a protein or polypeptide of interest or by downregulating or silencing other gene(s) which are present in the plant (using for example, antisense technology, cosuppression technology, RNA interference - RNAi - technology or microRNA - miRNA - technology). A heterologous gene that is located in the genome is also called a transgene. A transgene that is defined by its particular location in the plant genome is called a transformation or transgenic event.

Plant cultivars are understood to mean plants which have new properties ("traits") and have been obtained by conventional breeding, by mutagenesis or by recombinant DNA techniques. They can be cultivars, varieties, bio- or genotypes.

Plant parts are understood to mean all parts and organs of plants above and below the ground, such as shoots, leaves, needles, stalks, stems, flowers, fruit bodies, fruits, seeds, roots, tubers and rhizomes. The plant parts also include harvested material and vegetative and generative propagation material, for example cuttings, tubers, rhizomes, slips and seeds.

Plants which may be treated in accordance with the methods described herein include the following: cotton, flax, grapevine, fruit, vegetables, such as *Rosaceae sp.* (for example pome fruits such as apples and pears, but also stone fruits such as apricots, cherries, almonds and peaches, and soft fruits such as strawberries), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (for example banana trees and plantations), *Rubiaceae sp.* (for example coffee), *Theaceae sp., Sterculiceae sp., Rutaceae sp.* (for example lemons, oranges and grapefruit); *Solanaceae sp.* (for example tomatoes), *Liliaceae sp., Asteraceae sp.* (for example lettuce), *Umbelliferae sp., Cruciferae sp., Chenopodiaceae sp., Cucurbitaceae* sp. (for example cucumber), *Alliaceae sp.* (for example leek, onion), *Papilionaceae sp.* (for example peas); major crop plants, such as *Gramineae sp.* (for example maize, turf, cereals such as wheat, rye, rice, barley, oats, millet and triticale), *Asteraceae sp.* (for example sunflower), *Brassicaceae sp.* (for example white cabbage, red cabbage, broccoli, cauliflower, Brussels sprouts, pak choi, kohlrabi, radishes, and oilseed rape, mustard, horseradish and cress), *Fabacae sp.* (for example bean, peanuts), *Papilionaceae sp.* (for example soya bean), *Solanaceae sp.* (for example potatoes), *Chenopodiaceae sp.* (for example sugar beet, fodder beet, swiss chard, beetroot); useful plants and ornamental plants for gardens and wooded areas; and genetically modified varieties of each of these plants.

Plants and plant cultivars which may be treated by the above disclosed methods include plants and plant cultivars which are resistant against one or more biotic stresses, i.e. said plants show a better defense against animal and microbial pests, such as against nematodes, insects, mites, phytopathogenic fungi, bacteria, viruses and/or viroids.

Plants and plant cultivars which may be treated by the above disclosed methods include those plants which are resistant to one or more abiotic stresses. Abiotic stress conditions may include, for example, drought, cold temperature exposure, heat exposure, osmotic stress, flooding, increased soil salinity, increased mineral exposure, ozone exposure, high light exposure, limited availability of nitrogen nutrients, limited availability of phosphorus nutrients, shade avoidance.

Plants and plant cultivars which may be treated by the above disclosed methods include those plants characterized by enhanced yield characteristics. Increased yield in said plants may be the result of, for example, improved plant physiology, growth and development, such as water use efficiency, water retention efficiency, improved nitrogen use, enhanced carbon assimilation, improved photosynthesis, increased germination efficiency and accelerated maturation. Yield may furthermore be affected by improved plant architecture (under stress and non-stress conditions), including but not limited to, early flowering, flowering control for hybrid seed production, seedling vigor, plant size, internode number and distance, root growth, seed size, fruit size, pod size, pod or ear number, seed number per pod or ear, seed mass, enhanced seed filling, reduced seed dispersal, reduced pod dehiscence and lodging resistance. Further yield traits include seed composition, such as carbohydrate content and composition for example cotton or starch, protein content, oil content and composition, nutritional value, reduction in anti-nutritional compounds, improved processability and better storage stability.

Plants and plant cultivars which may be treated by the above disclosed methods include plants and plant cultivars which are hybrid plants that already express the characteristic of heterosis or hybrid vigor which results in generally higher yield, vigor, health and resistance towards biotic and abiotic stresses. Plants and plant cultivars (obtained by plant biotechnology methods such as genetic engineering) which may be treated by the above disclosed methods include plants and plant cultivars which are herbicide-tolerant plants, i.e. plants made tolerant to one or more given herbicides. Such plants can be obtained either by genetic transformation, or by selection of plants containing a mutation imparting such herbicide tolerance.

Plants and plant cultivars (obtained by plant biotechnology methods such as genetic engineering) which may be treated by the above disclosed methods include plants and plant cultivars which are insect-resistant transgenic plants, i.e. plants made resistant to attack by certain target insects. Such plants can be obtained by genetic transformation, or by selection of plants containing a mutation imparting such insect resistance.

Plants and plant cultivars (obtained by plant biotechnology methods such as genetic engineering) which may be treated by the above disclosed methods include plants and plant cultivars which are disease-resistant transgenic plants, i.e. plants made resistant to attack by certain target insects. Such plants can be obtained by genetic transformation, or by selection of plants containing a mutation imparting such insect resistance.

Plants and plant cultivars (obtained by plant biotechnology methods such as genetic engineering) which may be treated by the above disclosed methods include plants and plant cultivars which are tolerant to abiotic stresses. Such plants can be obtained by genetic transformation, or by selection of plants containing a mutation imparting such stress resistance.

Plants and plant cultivars (obtained by plant biotechnology methods such as genetic engineering) which may be treated by the above disclosed methods include plants and plant cultivars which show altered quantity, quality and/or storage-stability of the harvested product and/or altered properties of specific ingredients of the harvested product.

Plants and plant cultivars (obtained by plant biotechnology methods such as genetic engineering) which may be treated by the above disclosed methods include plants and plant cultivars, such as cotton plants, with altered fiber characteristics. Such plants can be obtained by genetic transformation, or by selection of plants contain a mutation imparting such altered fiber characteristics.

Plants and plant cultivars (obtained by plant biotechnology methods such as genetic engineering) which may be treated by the above disclosed methods include plants and plant cultivars, such as oilseed rape or related Brassica plants, with altered oil profile characteristics. Such plants can be obtained by genetic transformation, or by selection of plants contain a mutation imparting such altered oil profile characteristics.

Plants and plant cultivars (obtained by plant biotechnology methods such as genetic engineering) which may be treated by the above disclosed methods include plants and plant cultivars, such as oilseed rape or related Brassica plants, with altered seed shattering characteristics. Such plants can be obtained by genetic transformation, or by selection of plants contain a mutation imparting such altered seed shattering characteristics and include plants such as oilseed rape plants with delayed or reduced seed shattering.

Plants and plant cultivars (obtained by plant biotechnology methods such as genetic engineering) which may be treated by the above disclosed methods include plants and plant cultivars, such as Tobacco plants, with altered post-translational protein modification patterns.

### Pathogens

Non-limiting examples of pathogens of fungal diseases which may be treated in accordance with the invention include:
diseases caused by powdery mildew pathogens, for example *Blumeria* species, for example *Blumeria graminis; Podosphaera* species, for example *Podosphaera leucotricha; Sphaerotheca* species, for example *Sphaerotheca fuliginea; Uncinula* species, for example *Uncinula necator;*
diseases caused by rust disease pathogens, for example *Gymnosporangium* species, for example *Gymnosporangium sabinae; Hemileia* species, for example *Hemileia vastatrix; Phakopsora* species, for example *Phakopsora pachyrhizi* or *Phakopsora meibomiae; Puccinia* species, for example *Puccinia recondita, Puccinia graminis* oder *Puccinia striiformis; Uromyces* species, for example *Uromyces appendiculatus;*
diseases caused by pathogens from the group of the Oomycetes, for example *Albugo* species, for example *Albugo Candida; Bremia* species, for example *Bremia lactucae; Peronospora* species, for example *Peronospora pisi* or *P*. *brassicae; Phytophthora* species, for example *Phytophthora infestans; Plasmopara* species, for example *Plasmopara viticola; Pseudoperonospora* species, for example *Pseudoperonospora humuli* or *Pseudoperonospora cubensis; Pythium* species, for example *Pythium ultimum;*
leaf blotch diseases and leaf wilt diseases caused, for example, by *Alternaria* species, for example *Alternaria solani; Cercospora* species, for example *Cercospora beticola; Cladiosporium* species, for example *Cladiosporium cucumerinum; Cochliobolus* species, for example Cochliobolus sativus (conidial form: *Drechslera,* syn: *Helminthosporium*) or *Cochliobolus miyabeanus; Colletotrichum* species, for example *Colletotrichum lindemuthanium; Corynespora* species, for example *Corynespora cassiicola; Cycloconium* species, for example *Cycloconium oleaginum; Diaporthe* species, for example *Diaporthe citri; Elsinoe* species, for example *Elsinoe fawcettii; Gloeosporium* species, for example *Gloeosporium laeticolor; Glomerella* species, for example *Glomerella cingulata; Guignardia* species, for example *Guignardia bidwelli; Leptosphaeria* species, for example *Leptosphaeria maculans; Magnaporthe* species, for example *Magnaporthe grisea; Microdochium* species, for example *Microdochium nivale; Mycosphaerella* species, for example *Mycosphaerella graminicola, Mycosphaerella arachidicola* or *Mycosphaerella fijiensis; Phaeosphaeria* species, for example *Phaeosphaeria nodorum; Pyrenophora* species, for example *Pyrenophora teres* or *Pyrenophora tritici repentis; Ramularia* species, for example *Ramularia collo-cygni* or *Ramularia areola; Rhynchosporium* species, for example *Rhynchosporium secalis; Septoria* species, for example *Septoria apii* or *Septoria lycopersici; Stagonospora* species, for example *Stagonospora nodorum; Typhula* species, for example *Typhula incarnata; Venturia* species, for example *Venturia inaequalis;*
root and stem diseases caused, for example, by *Corticium* species, for example *Corticium graminearum; Fusarium* species, for example *Fusarium oxysporum; Gaeumannomyces* species, for example *Gaeumannomyces graminis; Plasmodiophora* species, for example *Plasmodiophora brassicae; Rhizoctonia* species, for example *Rhizoctonia solani; Sarocladium* species, for example *Sarocladium oryzae; Sclerotium* species, for example *Sclerotium oryzae; Tapesia* species, for example *Tapesia acuformis; Thielaviopsis* species, for example *Thielaviopsis basicola;*
ear and panicle diseases (including corn cobs) caused, for example, by *Alternaria* species, for example *Alternaria spp.; Aspergillus* species, for example *Aspergillus flavus; Cladosporium* species, for example *Cladosporium cladosporioides; Claviceps* species, for example *Claviceps purpurea; Fusarium* species, for example *Fusarium culmorum; Gibberella* species, for example *Gibberella* zeae; *Monographella* species, for example *Monographella nivalis; Stagnospora* species, for example *Stagnospora nodorum;*
diseases caused by smut fungi, for example *Sphacelotheca* species, for example *Sphacelotheca reiliana; Tilletia* species, for example *Tilletia caries* or *Tilletia controversa; Urocystis* species, for example *Urocystis occulta; Ustilago* species, for example *Ustilago nuda;*
fruit rot caused, for example, by *Aspergillus* species, for example *Aspergillus flavus; Botrytis* species, for example *Botrytis cinerea; Monilinia* species, for example *Monilinia laxa; Penicillium* species, for example *Penicillium expansum* or *Penicillium purpurogenum; Rhizopus* species, for example *Rhizopus stolonifer; Sclerotinia* species, for example *Sclerotinia sclerotiorum; Verticilium* species, for example *Verticilium alboatrum;*
seed- and soil-borne rot and wilt diseases, and also diseases of seedlings, caused, for example, by *Alternaria* species, for example *Alternaria brassicicola; Aphanomyces* species, for example *Aphanomyces euteiches; Ascochyta* species, for example *Ascochyta lentis; Aspergillus* species, for example *Aspergillus flavus; Cladosporium* species, for example *Cladosporium herbarum; Cochliobolus* species, for example *Cochliobolus sativus* (conidial form: *Drechslera,* Bipolaris Syn: *Helminthosporium); Colletotrichum* species, for example *Colletotrichum coccodes; Fusarium* species, for example *Fusarium culmorum; Gibberella* species, for example *Gibberella* zeae; *Macrophomina* species, for example *Macrophomina phaseolina; Microdochium* species, for example *Microdochium nivale; Monographella* species, for example *Monographella nivalis; Penicillium* species, for example *Penicillium expansum; Phoma* species, for example *Phoma lingam; Phomopsis* species, for example *Phomopsis sojae; Phytophthora* species, for example *Phytophthora cactorum; Pyrenophora* species, for example *Pyrenophora graminea; Pyricularia* species, for example *Pyricularia oryzae; Pythium* species, for example *Pythium ultimum; Rhizoctonia* species, for example *Rhizoctonia solani; Rhizopus* species, for example *Rhizopus oryzae; Sclerotium* species, for example *Sclerotium rolfsii; Septoria* species, for example *Septoria nodorum; Typhula* species, for example *Typhula incarnata; Verticillium* species, for example *Verticillium dahliae;*
cancers, galls and witches' broom caused, for example, by *Nectria* species, for example *Nectria galligena;*
wilt diseases caused, for example, by *Verticillium* species, for example *Verticillium longisporum; Fusarium* species, for example *Fusarium oxysporum;*
deformations of leaves, flowers and fruits caused, for example, by *Exobasidium* species, for example *Exobasidium vexans; Taphrina* species, for example *Taphrina deformans;*
degenerative diseases in woody plants, caused, for example, by *Esca* species, for example *Phaeomoniella chlamydospora, Phaeoacremonium aleophilum* or *Fomitiporia mediterranean Ganoderma* species, for example *Ganoderma boninense;*
diseases of plant tubers caused, for example, by *Rhizoctonia* species, for example *Rhizoctonia solani; Helminthosporium* species, for example *Helminthosporium solani;*
diseases caused by bacterial pathogens, for example *Xanthomonas* species, for example *Xanthomonas campestris pv. oryzae; Pseudomonas* species, for example *Pseudomonas syringae pv. lachrymans; Erwinia* species, for example *Erwinia amylovora; Liberibacter* species, for example *Liberibacter asiaticus; Xyella* species, for example *Xylella fastidiosa; Ralstonia* species, for example *Ralstonia solanacearum; Dickeya* species, for example *Dickeya solani; Clavibacter* species, for example *Clavibacter michiganensis; Streptomyces* species, for example *Streptomyces scabies.*
   diseases of soya beans:
   Fungal diseases on leaves, stems, pods and seeds caused, for example, by *Alternaria* leaf spot *(Alternaria spec. atrans tenuissima), Anthracnose (Colletotrichum gloeosporoides dematium var. truncatum*), brown spot (*Septoria glycines*), *cercospora* leaf spot and blight (*Cercospora kikuchii*), *choanephora* leaf blight (*Choanephora infundibulifera trispora (Syn.)*), *dactuliophora* leaf spot (*Dactuliophora glycines*), downy mildew (*Peronospora manshurica*), *drechslera* blight (*Drechslera glycini*), frogeye leaf spot (*Cercospora sojina*), *leptosphaerulina* leaf spot (*Leptosphaerulina trifolii*), *phyllostica* leaf spot (*Phyllosticta sojaecola*), pod and stem blight (*Phomopsis sojae*), powdery mildew (*Microsphaera diffusa*), *pyrenochaeta* leaf spot (*Pyrenochaeta glycines*), rhizoctonia aerial, foliage, and web blight (*Rhizoctonia solani*), rust (P*hakopsora pachyrhizi, Phakopsora meibomiae*), scab (*Sphaceloma glycines*), *stemphylium* leaf blight (*Stemphylium botryosum*), sudden death syndrome (*Fusarium virguliforme*), target spot (*Corynespora cassiicola*).
   Fungal diseases on roots and the stem base caused, for example, by black root rot (*Calonectria crotalariae*), charcoal rot (*Macrophomina phaseolina*), fusarium blight or wilt, root rot, and pod and collar rot (*Fusarium oxysporum, Fusarium orthoceras, Fusarium semitectum, Fusarium equiseti*), mycoleptodiscus root rot (*Mycoleptodiscus terrestris*), neocosmospora (*Neocosmospora vasinfecta*), pod and stem blight (*Diaporthe phaseolorum*), stem canker (*Diaporthe phaseolorum var. caulivora*), phytophthora rot (*Phytophthora megasperma*), brown stem rot (*Phialophora gregata*), pythium rot (*Pythium aphanidermatum, Pythium irregulare, Pythium debaryanum, Pythium myriotylum, Pythium ultimum),* rhizoctonia root rot, stem decay, and damping-off (*Rhizoctonia solani*), sclerotinia stem decay (*Sclerotinia sclerotiorum*), sclerotinia southern blight (*Sclerotinia rolfsii*), thielaviopsis root rot (*Thielaviopsis basicola*)*.*

### Mycotoxin

In addition, the compound of formula (l) or (I') and compositions comprising thereof may reduce the mycotoxin content in the harvested material and the foods and feeds prepared therefrom. Mycotoxins include particularly, but not exclusively, the following: deoxynivalenol (DON), nivalenol, 15-Ac-DON, 3-Ac-DON, T2- and HT2-toxin, fumonisins, zearalenon, moniliformin, fusarin, diaceotoxyscirpenol (DAS), beauvericin, enniatin, fusaroproliferin, fusarenol, ochratoxins, patulin, ergot alkaloids and aflatoxins which can be produced, for example, by the following fungi: *Fusarium* spec., such as *F. acuminatum, F. asiaticum, F. avenaceum, F. crookwellense, F. culmorum, F. graminearum (Gibberella* zeae), *F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides* etc., and also by *Aspergillus* spec., such as *A. flavus, A. parasiticus, A. nomius, A. ochraceus, A. clavatus, A. terreus, A. versicolor, Penicillium* spec., such as *P*. *verrucosum, P. viridicatum, P. citrinum, P. expansum, P. claviforme, P. roqueforti, Claviceps* spec., such as *C*. *purpurea, C. fusiformis, C. paspali, C. africana, Stachybotrys* spec. and others.

### Material Protection

The compound of formula (l) or (I') and compositions comprising thereof may also be used in the protection of materials, especially for the protection of industrial materials against attack and destruction by phytopathogenic fungi.

In addition, the compound of formula (l) or (I') and compositions comprising thereof may be used as antifouling compositions, alone or in combinations with other active ingredients.

Industrial materials in the present context are understood to mean inanimate materials which have been prepared for use in industry. For example, industrial materials which are to be protected from microbial alteration or destruction may be adhesives, glues, paper, wallpaper and board/cardboard, textiles, carpets, leather, wood, fibers and tissues, paints and plastic articles, cooling lubricants and other materials which can be infected with or destroyed by microorganisms. Parts of production plants and buildings, for example cooling-water circuits, cooling and heating systems and ventilation and air-conditioning units, which may be impaired by the proliferation of microorganisms may also be mentioned within the scope of the materials to be protected. Industrial materials within the scope of the present invention preferably include adhesives, sizes, paper and card, leather, wood, paints, cooling lubricants and heat transfer fluids, more preferably wood.

The compound of formula (l) or (I') and compositions comprising thereof may prevent adverse effects, such as rotting, decay, discoloration, decoloration or formation of mould.

In the case of treatment of wood the compound of formula (l) or (I') and compositions comprising thereof may also be used against fungal diseases liable to grow on or inside timber.

Timber means all types of species of wood, and all types of working of this wood intended for construction, for example solid wood, high-density wood, laminated wood, and plywood. In addition, the compound of formula (l) or (I') and compositions comprising thereof may be used to protect objects which come into contact with saltwater or brackish water, especially hulls, screens, nets, buildings, moorings and signalling systems, from fouling.

The compound of formula (l) or (I') and compositions comprising thereof may also be employed for protecting storage goods. Storage goods are understood to mean natural substances of vegetable or animal origin or processed products thereof which are of natural origin, and for which long-term protection is desired. Storage goods of vegetable origin, for example plants or plant parts, such as stems, leaves, tubers, seeds, fruits, grains, may be protected freshly harvested or after processing by (pre)drying, moistening, comminuting, grinding, pressing or roasting. Storage goods also include timber, both unprocessed, such as construction timber, electricity poles and barriers, or in the form of finished products, such as furniture. Storage goods of animal origin are, for example, hides, leather, furs and hairs. The compound of formula (l) and composition comprising thereof may prevent adverse effects, such as rotting, decay, discoloration, decoloration or formation of mould.

Microorganisms capable of degrading or altering industrial materials include, for example, bacteria, fungi, yeasts, algae and slime organisms. The compound of formula (l) or (I') and compositions comprising thereof preferably act against fungi, especially moulds, wood-discoloring and wood-destroying fungi (*Ascomycetes, Basidiomycetes, Deuteromycetes* and *Zygomycetes*), and against slime organisms and algae. Examples include microorganisms of the following genera: *Alternaria,* such as *Alternaria tenuis; Aspergillus,* such as *Aspergillus niger, Chaetomium,* such as *Chaetomium globosum; Coniophora,* such as *Coniophora puetana; Lentinus,* such as *Lentinus tigrinus; Penicillium,* such as *Penicillium glaucum; Polyporus,* such as *Polyporus versicolor, Aureobasidium,* such as *Aureobasidium pullulans; Sclerophoma,* such as *Sclerophoma pityophila; Trichoderma,* such as *Trichoderma viride; Ophiostoma spp., Ceratocystis spp., Humicola spp., Petriella spp., Trichurus spp., Coriolus spp., Gloeophyllum spp., Pleurotus spp., Poria spp., Serpula spp.* and *Tyromyces spp., Cladosporium spp., Paecilomyces spp. Mucor spp., Escherichia,* such as *Escherichia coli; Pseudomonas,* such as *Pseudomonas aeruginosa; Staphylococcus,* such as *Staphylococcus aureus, Candida spp.* and *Saccharomyces spp.,* such as *Saccharomyces cerevisae.*

### Seed Treatment

The compound of formula (I) or (I') and compositions comprising thereofmay also be used to protect seeds from unwanted microorganisms, such as phytopathogenic microorganisms, for instance phytopathogenic fungi or phytopathogenic oomycetes. The term seed(s) as used herein include dormant seeds, primed seeds, pregerminated seeds and seeds with emerged roots and leaves.

Thus, the present invention also relates to a method for protecting seeds from unwanted microorganisms which comprises the step of treating the seeds with the compound of formula (I) or (I') or the composition.

The treatment of seeds with the compound of formula (I) or (I') or the compositions protects the seeds from phytopathogenic microorganisms, but also protects the germinating seeds, the emerging seedlings and the plants after emergence from the treated seeds. Therefore, the present invention also relates to a method for protecting seeds, germinating seeds and emerging seedlings.

The seeds treatment may be performed prior to sowing, at the time of sowing or shortly thereafter.

When the seeds treatment is performed prior to sowing (e.g. so-called on-seed applications), the seeds treatment may be performed as follows: the seeds may be placed into a mixer with a desired amount of the compound of formula (I) or (I') or the composition, the seeds and the compound of formula (I) or (I') or the compositions are mixed until an homogeneous distribution on seeds is achieved. If appropriate, the seeds may then be dried.

The invention also relates to seeds coated with the compound of formula (I) or (I') or compositions comprising thereof.

Preferably, the seeds are treated in a state in which it is sufficiently stable for no damage to occur in the course of treatment. In general, seeds can be treated at any time between harvest and shortly after sowing. It is customary to use seeds which have been separated from the plant and freed from cobs, shells, stalks, coats, hairs or the flesh of the fruits. For example, it is possible to use seeds which have been harvested, cleaned and dried down to a moisture content of less than 15% by weight. Alternatively, it is also possible to use seeds which, after drying, for example, have been treated with water and then dried again, or seeds just after priming, or seeds stored in primed conditions or pregerminated seeds, or seeds sown on nursery trays, tapes or paper.

The amount of the compound of formula (I) or (I') or compositions comprising thereof applied to the seeds is typically such that the germination of the seed is not impaired, or that the resulting plant is not damaged. This must be ensured particularly in case the the compound of formula (I) or (I') would exhibit phytotoxic effects at certain application rates. The intrinsic phenotypes of transgenic plants should also be taken into consideration when determining the amount of the compound of formula (I) or (I') to be applied to the seed in order to achieve optimum seed and germinating plant protection with a minimum amount of compound being employed.

The compound of formula (I) or (I') can be applied as such, directly to the seeds, i.e. without the use of any other components and without having been diluted. Also the composition comprising thereeof can be applied to the seeds.

The compound of formula (I) or (I') and compositions comprising thereof are suitable for protecting seeds of any plant variety. Preferred seeds are that of cereals (such as wheat, barley, rye, millet, triticale, and oats), oilseed rape, maize, cotton, soybean, rice, potatoes, sunflower, beans, coffee, peas, beet (e.g. sugar beet and fodder beet), peanut, vegetables (such as tomato, cucumber, onions and lettuce), lawns and ornamental plants. More preferred are seeds of wheat, soybean, oilseed rape, maize and rice.

The compound of formula (I) or (I') and compositions comprising thereof may be used for treating transgenic seeds, in particular seeds of plants capable of expressing a polypeptide or protein which acts against pests, herbicidal damage or abiotic stress, thereby increasing the protective effect. Seeds of plants capable of expressing a polypeptide or protein which acts against pests, herbicidal damage or abiotic stress may contain at least one heterologous gene which allows the expression of said polypeptide or protein. These heterologous genes in transgenic seeds may originate, for example, from microorganisms of the species Bacillus, Rhizobium, Pseudomonas, Serratia, Trichoderma, Clavibacter, Glomus or Gliocladium. These heterologous genes preferably originate from Bacillus sp., in which case the gene product is effective against the European corn borer and/or the Western corn rootworm. Particularly preferably, the heterologous genes originate from Bacillus thuringiensis.

### Application

The compound of formula (I) or (I') can be applied as such, or for example in the form of as ready-to-use solutions, emulsions, water- or oil-based suspensions, powders, wettable powders, pastes, soluble powders, dusts, soluble granules, granules for broadcasting, suspoemulsion concentrates, natural products impregnated with the compound of formula (I) or (I'), synthetic substances impregnated with the compound of formula (I) or (I'), fertilizers or microencapsulations in polymeric substances.

Application is accomplished in a customary manner, for example by watering, spraying, atomizing, broadcasting, dusting, foaming, spreading-on and the like. It is also possible to deploy the compound of formula (I) or (I') by the ultra-low volume method, via a drip irrigation system or drench application, to apply it in-furrow or to inject it into the soil stem or trunk. It is further possible to apply the compound of formula (I) or (I') by means of a wound seal, paint or other wound dressing.

The effective and plant-compatible amount of the compound of formula (I) or (I') which is applied to the plants, plant parts, fruits, seeds or soil will depend on various factors, such as the compound/composition employed, the subject of the treatment (plant, plant part, fruit, seed or soil), the type of treatment (dusting, spraying, seed dressing), the purpose of the treatment (curative and protective), the type of microorganisms, the development stage of the microorganisms, the sensitivity of the microorganisms, the crop growth stage and the environmental conditions.

When the compound of formula (I) or (I') is used as a fungicide, the application rates can vary within a relatively wide range, depending on the kind of application. For the treatment of plant parts, such as leaves, the application rate may range from 0.1 to 10 000 g/ha, preferably from 10 to 1000 g/ha, more preferably from 50 to 300 g/ha (in the case of application by watering or dripping, it is even possible to reduce the application rate, especially when inert substrates such as rockwool or perlite are used). For the treatment of seeds, the application rate may range from 0.1 to 200 g per 100 kg of seeds, preferably from 1 to 150 g per 100 kg of seeds, more preferably from 2.5 to 25 g per 100 kg of seeds, even more preferably from 2.5 to 12.5 g per 100 kg of seeds. For the treatment of soil, the application rate may range from 0.1 to 10 000 g/ha, preferably from 1 to 5000 g/ha.

These application rates are merely examples and are not intended to limit the scope of the present invention.

### General synthetic routes to the compounds of general formula I

The present invention also relates to processes for the preparation of compounds of formula (I) or (I'). Unless indicated otherwise, the radicals **X**, **A**, **L**, R^{a} have the meanings given above for the compounds of formula (I). These definitions apply not only to the end products of the formula (I) or (I') but likewise to all intermediates.

Compounds of formula (I) or (I') can be prepared, according to process P1, by reacting amidoximes of formula (II) with difluorohaloalkylacetic anhydride or difluorohaloalkylacetyl chloride in a suitable solvent such as tetrahydrofurane or dichloromethane optionally in presence of a base such as triethylamine or pyridine, preferably at room temperature, as previously described in WO2013080120.

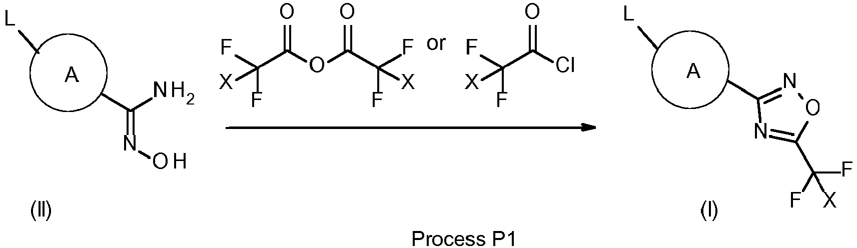

Amidoximes of formula (II) can be prepared according to known procedures (see for examples WO2013080120), as shown in process P2 by treating nitriles of formula (III) with hydroxylamine (or its hydrochloride salt) in the presence of a base such as triethylamine in a solvent such as ethanol.

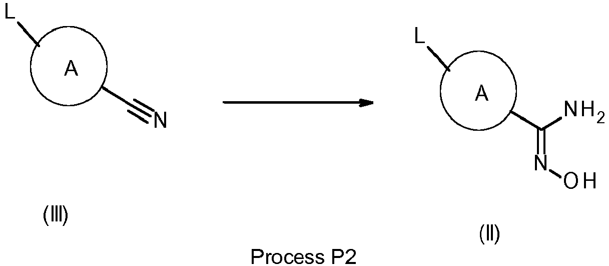

Compounds of formula (III) can be commercially available or may be prepared starting from readily available compounds according to known procedures.

Alternatively compounds of formula (III) can be prepared, according to process P3, from compounds of formula (IV), wherein LG1 is a leaving group as for example bromide with a suitable cyanide reagent such as for example zinc cyanide in presence of palladium (0) in a solvent such as N,N-dimethylformamide as described for example in ACS Medicinal Chemistry Letters, 8(9), 919-924, 2017.

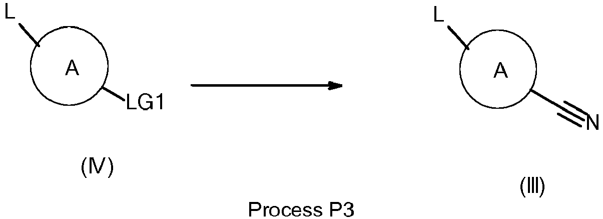

Compounds of formula (IV) can be commercially available or may be prepared starting from readily available compounds according to known procedures.

Alternatively compounds of formula (III-b) can be prepared, according to process P4, from a compound of formula (III-a) and a compound of formula (V) or its salt (as described for example in Organic Letters, 13(3), 540-542; 2011) in a solvent such as for example ethanol with a base such as, for example, potassium acetate or pyridine.

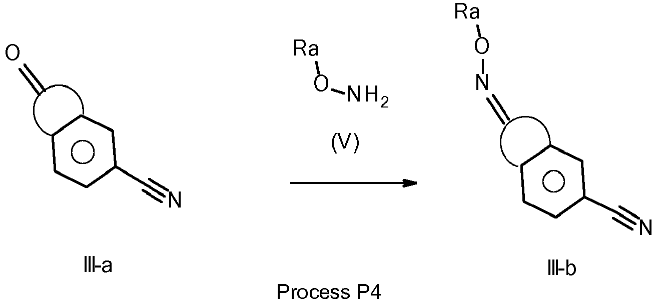

Compounds of formula (V) can be commercially available or may be prepared starting from readily available compounds according to known procedures.

Alternatively compounds of formula (I-c) can be prepared, according to process P5, from a compound of formula (I-b) by reduction with a hydride such as for example sodium cyanoborohydride (as described for example in WO2012093100) in a solvent such as acetic acid.

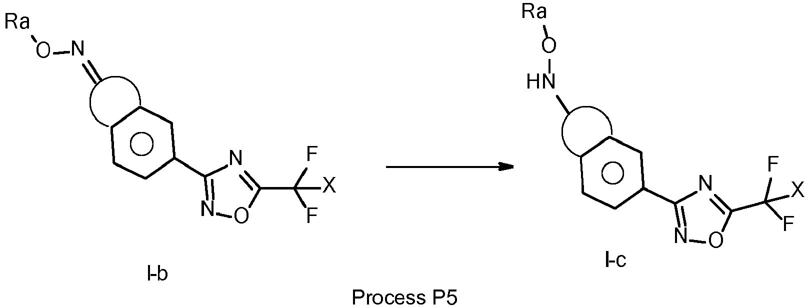

Compounds of formula (I-b) may be prepared starting from compounds of formula (III-b) as described in process P1 and P2.

Alternatively compounds of formula (I-d) can be prepared, according to process P6, from a compound of formula (I-c) with a compound of formula (VI), wherein LG2 is a leaving group, by nucleophilic substitution (as described for example in WO2012093100) optionally in presence of a base (like for example triethylamine), optionally in presence of a solvent such as for example dichloromethane.

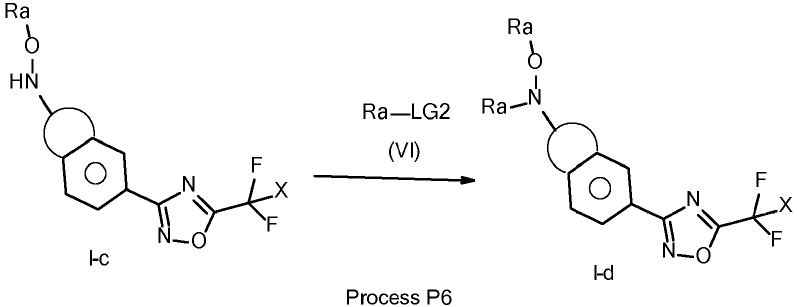

Compounds of formula (VI) can be commercially available or may be prepared starting from readily available compounds according to known procedures.

Alternatively compounds of formula (I-f) can be prepared, according to process P7, from a compound of formula (I-e) with a compound of formula (VI), wherein LG2 is a leaving group, by nucleophilic substitution (as described for example in Journal of Medicinal Chemistry, 59(4), 1556-1564; 2016) optionally in presence of a base (like for example potassium carbonate), optionally in presence of a solvent such as for example N,N-dimethylformamide.

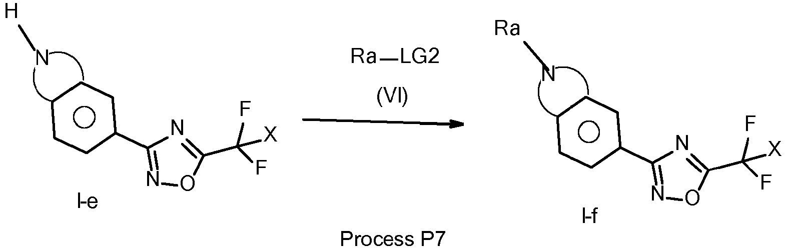

Compounds of formula (VI) can be commercially available or may be prepared starting from readily available compounds according to known procedures.

According to the invention, processes P1 to P7 can be performed if appropriate in the presence of a solvent and if appropriate in the presence of a base.

Suitable solvents for carrying out processes P1 to P7 according to the invention are customary inert organic solvents. Preference is given to using optionally halogenated aliphatic, alicyclic or aromatic hydrocarbons, such as petroleum ether, hexane, heptane, cyclohexane, methylcyclohexane, benzene, toluene, xylene or decalin ; chlorobenzene, dichlorobenzene, dichloromethane, chloroform, carbon tetrachloride, dichlorethane or trichlorethane ; ethers, such as diethyl ether, diisopropyl ether, methyl tert-butyl ether, methyl tert-amyl ether, dioxane, tetrahydrofuran, 1,2-dimethoxyethane, 1,2-diethoxyethane or anisole ; nitriles, such as acetonitrile, propionitrile, n- or iso-butyronitrile or benzonitrile ; amides, such as N,N-dimethylformamide, N,N-dimethylacetamide, N-methylformanilide, N-methylpyrrolidone or hexamethylphosphoric triamide ; esters, such as methyl acetate or ethyl acetate, sulfoxides, such as dimethyl sulfoxide or sulfones, such as sulfolane.

Suitable bases for carrying out processes P1 to P7 according to the invention are inorganic and organic bases which are customary for such reactions. Preference is given to using alkaline earth metal, alkali metal hydride, alkali metal hydroxides or alkali metal alkoxides, such as sodium hydroxide, sodium hydride, calcium hydroxide, potassium hydroxide, potassium tert-butoxide or other ammonium hydroxide, alkali metal carbonates, such as sodium carbonate, potassium carbonate, potassium bicarbonate, sodium bicarbonate, cesium carbonate, alkali metal or alkaline earth metal acetates, such as sodium acetate, potassium acetate, calcium acetate and also tertiary amines, such as triethylamine, diisopropylethylamine, tributylamine, N,N-dimethylaniline, pyridine, *N*-methylpiperidine, *N*,*N*-dimethylaminopyridine, 1,4-diazabicyclo[2.2.2]octane (DABCO), 1,5-diazabicyclo[4.3.0]non-5-ene (DBN) or 1,8-diazabicyclo[5.4.0]undec-7-ene (DBU).

When carrying out processes P1 to P7, according to the invention, the reaction temperature can independently be varied within a relatively wide range. Generally, processes according to the invention are carried out at temperatures between -20°C and 160°C.

Processes P1 to P7 according to the invention are generally independently carried out under atmospheric pressure. However, it is also possible to operate under elevated or reduced pressure.

Work-up is carried out by customary methods. Generally, the reaction mixture is treated with water and the organic phase is separated off and, after drying, concentrated under reduced pressure. If appropriate, the remaining residue can be freed by customary methods, such as chromatography or recrystallization, from any impurities that can still be present.

Compounds according to the invention can be prepared according to the above described processes. It will nevertheless be understood that, on the basis of his general knowledge and of available publications, the skilled worker will be able to adapt these processes according to the specifics of each of the compounds according to the invention that is desired to be synthesized.

Aspects of the present teaching may be further understood in light of the following examples, which should not be construed as limiting the scope of the present teaching in any way.

### EXAMPLES

The following examples illustrate in a non-limiting manner the preparation and efficacy of the compounds of formula (I) or (I') according to the invention.

### Synthesis of intermediate of formula (III-b)

### 5-(Methoxyimino)-5,6,7,8-tetrahydronaphthalene-2-carbonitrile

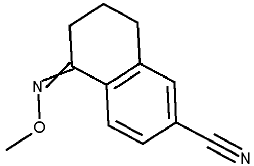

To a suspension of 5-oxo-5,6,7,8-tetrahydronaphthalene-2-carbonitrile (0.68 g, 4.0 mmol) in absolute ethanol (10 ml) were added methoxylamine hydrochloride (0.50 g, 5.9 mmol) and sodium acetate (0.49 g, 5.9 mmol). The suspension was refluxed for 3 h. The mixture was then concentrated under reduced pressure and the residue was dissolved in ethyl acetate (40 ml) and water (20 ml). The layers were separated and the organic layer was washed with brine (20 ml, dried over magnesium sulfate and concentrated over reduced pressure to afford 5-(methoxyimino)-5,6,7,8-tetrahydronaphthalene-2-carbonitrile (0.75 g, 92% yield, mixture 87:13 of 2 stereoisomers) as a yellow solid. This compound was used in the next step without further purification.

MS (ESI): 201 ([M+H]+)

### Synthesis of compounds of formula (I)

### O-Methyl-N-{6-[5-(trifluoromethyl)-1,2,4-oxadiazol-3-yl]-3,4-dihydronaphthalen-1(2H)-ylidenelhydroxylamine

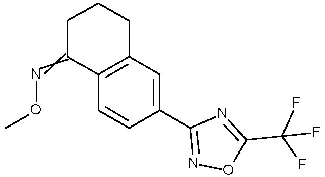

To a suspension of 5-(methoxyimino)-5,6,7,8-tetrahydronaphthalene-2-carbonitrile (0.74 g, 3.7 mmol) in ethanol (13 ml) was added hydroxylamine hydrochloride (0.51 g, 7.3 mmol), followed by triethylamine (2.3 ml, 17 mmol). The orange solution was stirred at room temperature for 48 h. The mixture was concentrated under reduced pressure to afford N'-hydroxy-5-(methoxyimino)-5,6,7,8-tetrahydronaphthalene-2-carboximidamide (1.93g, purity 44%, 99% yield) as a yellow solid. The crude residue was suspended in tetrahydrofuran (15 ml) and trifluoroacetic anhydride (1.0 ml, 7.3 mmol) was then added. The yellow suspension was then stirred at room temperature for 5 h. The mixture was concentrated under reduced pressure and ethyl acetate (30 ml) and water (20 ml) were added to the residue. The layers were separated and the organic layer was washed with a saturated sodium hydrogen carbonate solution (15 ml) and brine (15 ml), dried over magnesium sulfate and evaporated under reduced pressure to afford O-methyl-N-{6-[5-(trifluoromethyl)-1,2,4-oxadiazol-3-yl]-3,4-dihydronaphthalen-1(2H)-ylidene}hydroxylamine (1.1 g, 91% yield) as an orange solid which was used in the next step without further purification.

### MS (ESI): 312 ([M+H]+)

### O-Methyl-N-{6-[5-(trifluoromethyl)-1,2,4-oxadiazol-3-yl]-1,2,3,4-tetrahydronaphthalen-1-yl}hydroxlamine (Compound 18)

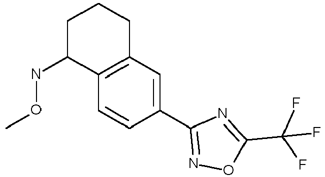

To a solution of O-methyl-N-{6-[5-(trifluoromethyl)-1,2,4-oxadiazol-3-yl]-3,4-dihydronaphthalen-1(2H)-ylidene}hydroxylamine (1.0 g, 3.3 mmol) in acetic acid (8 ml) was added portionwise at -15°C sodium cyanoborohydride (0.44 g, 6.9 mmol). The reaction mixture was stirred at room temperature for 66 h and sodium cyanoborohydride (0.10 g, 1.7 mmol) was then added. The mixture was stirred at room temperature for further 8 h and then poured carefully in cold sodium hydroxide 1 M solution (145 ml). The aqueous layer was extracted 3 times with ethyl acetate (60 ml). The organic layers were combined, washed with brine (60 ml), dried over magnesium sulfate and evaporated under reduced pressure. The residue was purified by flash chromatography (eluant heptane:ethyl acetate 1:0 to 3:7) to afford O-methyl-N-{6-[5-(trifluoromethyl)-1,2,4-oxadiazol-3-yl]-1,2,3,4-tetrahydronaphthalen-1-yl}hydroxylamine (0.3 g, 28% yield) as an orange oil.

### MS (ESI): 314([M+H]+)

### N-Ethyl-O-methyl-N-16-[5-(trifluoromethyl)-1,2,4-oxadiazol-3-yl]-1,2,3,4-tetrahydronaphthalen-1- yl}hydroxylamine (Compound 23)

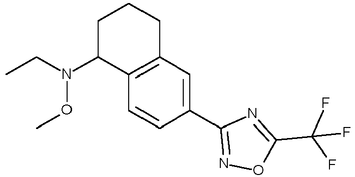

Further purification of the above reaction by preparative high pressure liquid chromatography afforded N-ethyl-O-methyl-N-{6-[5-(trifluoromethyl)-1,2,4-oxadiazol-3-yl]-1,2,3,4-tetrahydronaphthalen-1-yl}hydroxylamine (0.2 g, 17% yield) as a colorless oil.

MS (ESI): 342([M+H]+)

### N-Methoxy-N-16-[5-(trifluoromethyl)-1,2,4-oxadiazol-3-yl]-1,2,3,4-tetrahydronaphthalen-1-yl}acetamide (Compound 20)

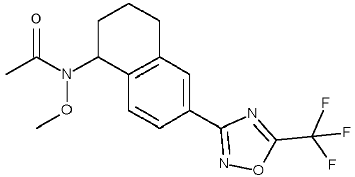

To a solution of O-methyl-N-16-[5-(trifluoromethyl)-1,2,4-oxadiazol-3-yl]-1,2,3,4-tetrahydronaphthalen-1-yl}hydroxylamine (40 mg, 0.12 mmol) in tetrahydrofuran (2 ml) were added triethylamine (27 µl, 0.19 mmol) and acetyl chloride (10 µl, 0.14 mmol). The mixture was stirred at room temperature for 16 h. Water was then added and the mixture was extracted with ethyl acetate. The organic layer was washed with a saturated sodium hydrogen carbonate solution and brine, dried over magnesium sulfate and concentrated over reduced pressure to afford N-methoxy-N-{6-[5-(trifluoromethyl)-1,2,4-oxadiazol-3-yl]-1,2,3,4-tetrahydronaphthalen-1-yl}acetamide (47 mg, 98% yield) as a colorless oil.

### MS (ESI): 356 ([M+H]+)

### 5-[5-(Trifluoromethyl)-1,2,4-oxadiazol-3-yl]-1H-indazole (Compound 5)

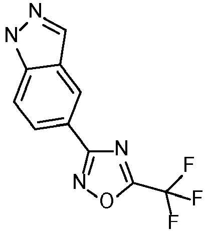

To a suspension of 1H-indazole-5-carbonitrile (1.0 g, 7.2 mmol) in ethanol (25 ml) was added hydroxylamine hydrochloride (1.0 g, 14 mmol), followed by triethylamine (4.5 ml, 33 mmol). The orange solution was stirred at room temperatura for 18 h. The reaction mixture was concentrated under reduced pressure and the residue was suspended in tetrahydrofuran (30 ml). Trifluoroacetic anhydride (1.9 ml, 14 mmol) was then added and the reaction mixture was stirred at room temperature for 5 h. Trifluoroacetic anhydride (1.0 ml, 6.8 mmol) was then added and the reaction mixture was stirred at room temperature for 3 h. The solvent was evaporated under reduced pressure and the residue was diluted in ethyl acetate and washed with water, with a saturated sodium hydrogen carbonate solution and then with brine. The organic layer was dried over magnesium sulfate and concentrated under reduced pressure to afford 5-[5-(trifluoromethyl)-1,2,4-oxadiazol-3-yl]-1H-indazole (0.62 g, 34% yield) as a yellow solid.

### MS (ESI): 255 ([M+H]+)

### 1-(2-Methoxyethyl)-5-[5-(trifluoromethyl)-1,2,4-oxadiazol-3-yl]-1H-indazole (Compound 13)

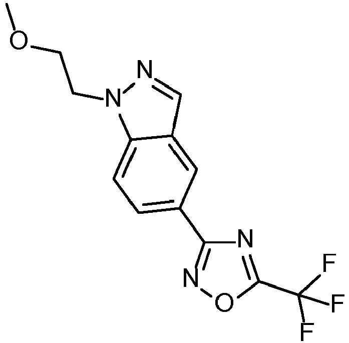

To a solution of 5-[5-(trifluoromethyl)-1,2,4-oxadiazol-3-yl]-1H-indazole (0.12 g, 0.47 mmol) in N,N-dimethylformamide (3 ml) was added potassium carbonate (0.16 g, 1.2 mmol) and the mixture was stirred at room temperature for 30 minutes. 1-Bromo-2-methoxyethane (49 µl, 0.51 mmol) was then added and the mixture was stirred at room temperatura for 2 days. 1-Bromo-2-methoxyethane (25 µl, 0.26 mmol) was then added and the mixture was stirred at room temperatura for 7 h. Water was then added and the reaction mixture was extracted twice with ethyl acetate. The organic layers were combined, washed with brine, dried over magnesium sulfate and concentrated under vacuum. The residue was purified by column chromatography to afford the title compound 1-(2-methoxyethyl)-5-[5-(trifluoromethyl)-1,2,4-oxadiazol-3-yl]-1H-indazole (60 mg, 39% yield) as a yellow oil and its regioisomer 2-(2-methoxyethyl)-5-[5-(trifluoromethyl)-1,2,4-oxadiazol-3-yl]-2H-indazole (30 mg, 19 % yield) as a pale yellow solid.

MS (ESI): 313([M+H]+)

### 2-(2-Methoxyethyl)-5-[5-(trifluoromethyl)-1,2,4-oxadiazol-3-yl]-2H-indazole (Compound 14)

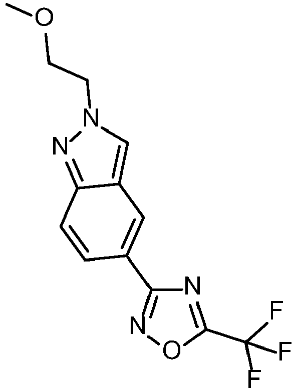

MS (ESI): 313 ([M+H]+)

The compounds as shown in table 1 below were prepared in analogy with the examples provided above

**Table 1: Compounds according to formula (I)**

| 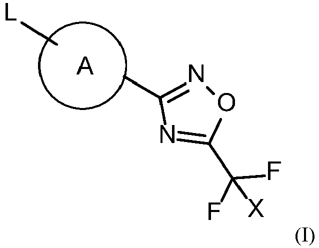 | | | |
|---|---|---|---|
| Ex N° | X | 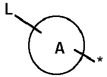 | LogP |
| I-001 | fluoro | 5-hydroxyimino-5,6,7,8-tetrahydronaphthalen-2-yl | 3.62^{[a]} |
| I-002* | fluoro | 3H-benzimidazol-5-yl | 1.46^{[a]} |
| I-003* | fluoro | 1-methyl-1H-benzimidazol-5-yl | 1.70^{[a]} |
| I-004* | fluoro | 3-methyl-3H-benzimidazol-5-yl | 1.67^{[a]} |
| I-005 | fluoro | 1H-indazol-5-yl | 2.68^{[a]} |
| I-006 | fluoro | 1H-indazol-6-yl | 2.70^{[a]} |
| I-007 | fluoro | 1-benzyl-1H-indazol-6-yl | 4.34^{[a]} |
| I-008 | fluoro | 2-benzyl-2H-indazol-6-yl | 4.03^{[a]} |
| I-009 | fluoro | 1-benzyl-1H-indazol-5-yl | 4.39^{[a]} |
| I-010 | fluoro | 2-ethyl-2H-indazol-5-yl | 3.15^{[a]} |
| I-011 | fluoro | 1-ethyl-1H-indazol-5-yl | 3.67^{[a]} |
| I-012 | fluoro | 2-benzyl-2H-indazol-5-yl | 4.03^{[a]} |
| I-013 | fluoro | 1-(2-methoxyethyl)-1H-indazol-5-yl | 3.31^{[a]} |
| I-014 | fluoro | 2-(2-methoxyethyl)-2H-indazol-5-yl | 2.96^{[a]} |
| I-015 | fluoro | 2-ethyl-2H-indazol-6-yl | 3.21^{[a]} |
| I-016 | fluoro | 1-(2-methoxyethyl)-1H-indazol-6-yl | 3.35^{[a]} |
| I-017 | fluoro | 2-(2-methoxyethyl)-2H-indazol-6-yl | 3.00^{[a]} |
| I-018 | fluoro | 5-(methoxyamino)-5,6,7,8-tetrahydronaphthalen-2-yl | 4.08^{[a]} |
| I-019 | fluoro | 5-[(cyclopropylcarbonyl)-methoxyamino]-5,6,7,8-tetrahydronaphthalen-2-yl | 4.44^{[a]} |
| I-020 | fluoro | 5-[acetyl(methoxy)amino]-5,6,7,8-tetrahydronaphthalen-2-yl | 3.79^{[a]} |
| I-021 | fluoro | 5-[methoxy(3-methoxypropanoyl)amino]-5,6,7,8-tetrahydronaphthalen-2-yl | 3.96^{[a]} |
| I-022 | fluoro | 5-[benzoyl(methoxy)amino]-5,6,7,8-tetrahydronaphthalen-2-yl | 4.72^{[a]} |
| I-023 | fluoro | 5-[ethyl(methoxy)amino]-5,6,7,8-tetrahydronaphthalen-2-yl | 6.04^{[a]} |
| I-024 | fluoro | 5-[methoxy-(methoxycarbonyl)amino]-5,6,7,8-tetrahydronaphthalen-2-yl | 4.34^{[a]} |
| I-025 | fluoro | 5-[methoxy-(2-methoxyethoxycarbonyl)amino]-5,6,7,8-tetrahydronaphthalen-2-yl | 4.27^{[a]} |
| I-026* | chloro | 2-(methoxycarbonyl)-1,2,3,4-tetrahydroisoquinolin-7-yl | 3.83^{[a]} |
| I-027 | chloro | 1-(methoxycarbonyl)-1,2,3,4-tetrahydroquinolin-6-yl | 4.27^{[a]} |
| I-028 | fluoro | 1-(methoxycarbonyl)-1,2,3,4-tetrahydroquinolin-6-yl | 4.03^{[a]} |
| I-029 | chloro | 1 -propionyl-1,2,3,4-tetrahydroquinolin-6-yl | 3.90^{[a]} |
| I-030 | fluoro | 1-propionyl-1,2,3,4-tetrahydroquinolin-6-yl | 3.68^{[a]} |
| I-031* | fluoro | 2-propionyl-1,2,3,4-tetrahydroisoquinolin-7-yl | 3.11^{[a]} |
| I-032* | fluoro | 2-(methoxycarbonyl)-1,2,3,4-tetrahydroisoquinolin-7-yl | 3.59^{[a]} |
| I-033* | chloro | 2-propionyl-1,2,3,4-tetrahydroisoquinolin-7-yl | 3.31^{[a]} |
| I-034* | fluoro | 6-(dimethylaminocarbonyl)oxynaphthalen-2-yl | 4.18^{[a]} |
| I-035 | fluoro | 3-(2,2,2-trifluoroacetyl)imidazo[1,2-a]pyridin-6-yl | 3.41^{[a]} |
| I-036 | fluoro | 3-bromoimidazo[1,2-a]pyridin-6-yl | 2.75^{[a]} |
| I-037 | fluoro | 2-[(2-methoxyethylamino)carbonyl]imidazo[1,2-a]pyridin-6-yl | 2.18^{[a]} |
| I-038* | fluoro | 2-(tert-butoxycarbonyl)-1,2,3,4-tetrahydroisoquinolin-6-yl | 4.93^{[a]} |
| I-039 | fluoro | 2-(ethoxycarbonyl)imidazo[1,2-a]pyridin-6-yl | 2.59^{[a]} |
| I-040 | fluoro | 2-carboxyimidazo[1,2-a]pyridin-6-yl | 1.41^{[a]} |
| I-041* | chloro | 3H-benzimidazol-5-yl | 1.57^{[a]} |
| I-042 | fluoro | 6-aminonaphthalen-2-yl | 3.47^{[a]} |
| I-043 | fluoro | 1,3-benzothiazol-6-yl | 3.21^{[a]} |
| I-044 | fluoro | 7-chloro-3H-benzotriazol-5-yl | 2.78^{[a]} |
| I-045* | fluoro | 1-ethyl-2-methyl-1H-benzimidazol-5-yl | 1.60^{[a]} |
| I-046 | chloro | 1H-indol-6-yl | 3.68^{[a]} |
| I-047* | fluoro | 2-(cyclopropylcarbonyl)-1,2,3,4-tetrahydroisoquinolin-6-yl | 3.31^{[a]} |
| I-048* | fluoro | 2-(methoxycarbonyl)-1 ,2,3,4-tetrahydroisoquinolin-6-yl | 3.63^{[a]} |
| I-049* | fluoro | 2-acetyl-1,2,3,4-tetrahydroisoquinolin-6-yl | 2.71^{[a]} |
| I-050* | fluoro | 2-(2-phenylacetyl)-1,2,3,4-tetrahydroisoquinolin-6-yl | 3.83^{[a]} |
| I-051 | fluoro | 3-[(methoxycarbonyl)amino]isoquinolin-6-yl | 3.45^{[a]} |
| I-052 | fluoro | 3-(propionylamino)isoquinolin-6-yl | 3.33^{[a]} |
| I-053 | fluoro | 4-(acetylamino)-2,3-dihydro-1-benzofuran-7-yl | 2.32^{[a]} |
| I-054 | fluoro | 3-(acetylamino)isoquinolin-6-yl | 2.90^{[a]} |
| I-055 | fluoro | 3-aminoisoquinolin-6-yl | 2.31^{[a]} |
| I-056 | fluoro | 7-(acetylamino)indan-4-yl | 3.28^{[a]} |
| I-057 | fluoro | 6-[(2,2,2-trifluoroacetyl)amino]naphthalen-2-yl | 4.27^{[a]} |
| I-058* | chloro | 3-benzyl-3H-benzimidazol-5-yl | 3.23^{[a]} |
| I-059* | fluoro | 3-benzyl-3H-benzimidazol-5-yl | 3.06^{[a]} |
| I-060* | chloro | 1-methyl-1H-benzimidazol-5-yl | 1.86^{[a]} |
| I-061 | fluoro | 6-fluoro-1H-indazol-5-yl | 2.61^{[a]} |
| I-062 | chloro | 6-fluoro-1H-indazol-5-yl | 2.78^{[a]} |
| I-063 | chloro | 1H-indazol-5-yl | 2.84^{[a]} |
| I-064 | fluoro | 1-benzyl-6-fluoro-1H-indazol-5-yl | 4.28^{[a]} |
| I-065 | fluoro | 2-benzyl-6-fluoro-2H-indazol-5-yl | 3.92^{[a]} |
| I-066 | chloro | 2-benzyl-6-fluoro-2H-indazol-5-yl | 4.11^{[a]} |

| | | | |
|---|---|---|---|
| ^{∗}: compound used according to the invention | | | |

Measurement of LogP values was performed according to EEC directive 79/831 Annex V.A8 by HPLC (High Performance Liquid Chromatography) on reversed phase columns with the following methods:
^{[a]} LogP value is determined by measurement of LC-UV, in an acidic range, with 0.1% formic acid in water and acetonitrile as eluent (linear gradient from 10% acetonitrile to 95% acetonitrile).
^{[b]} LogP value is determined by measurement of LC-UV, in a neutral range, with 0.001 molar ammonium acetate solution in water and acetonitrile as eluent (linear gradient from 10% acetonitrile to 95% acetonitrile).
^{[c]} LogP value is determined by measurement of LC-UV, in an acidic range, with 0.1% phosphoric acid and acetonitrile as eluent (linear gradient from 10% acetonitrile to 95% acetonitrile).

If more than one LogP value is available within the same method, all the values are given and separated by "+".

Calibration was done with straight-chain alkan2-ones (with 3 to 16 carbon atoms) with known LogP values (measurement of LogP values using retention times with linear interpolation between successive alkanones). Lambda-max-values were determined using UV-spectra from 200 nm to 400 nm and the peak values of the chromatographic signals.

### NMR-Peak lists

1H-NMR data of selected examples are written in form of 1H-NMR-peak lists. To each signal peak are listed the δ-value in ppm and the signal intensity in round brackets. Between the δ-value - signal intensity pairs are semicolons as delimiters.

The peak list of an example has therefore the form:
δ₁ (intensity₁₎; δ₂ (intense₂);........; δᵢ (intensityᵢ);......; δₙ (intensityₙ)

Intensity of sharp signals correlates with the height of the signals in a printed example of a NMR spectrum in cm and shows the real relations of signal intensities. From broad signals several peaks or the middle of the signal and their relative intensity in comparison to the most intensive signal in the spectrum can be shown.

For calibrating chemical shift for 1H spectra, we use tetramethylsilane and/or the chemical shift of the solvent used, especially in the case of spectra measured in DMSO. Therefore in NMR peak lists, tetramethylsilane peak can occur but not necessarily.

The 1H-NMR peak lists are similar to classical 1H-NMR prints and contains therefore usually all peaks, which are listed at classical NMR-interpretation.

Additionally they can show like classical 1H-NMR prints signals of solvents, stereoisomers of the target compounds, which are also object of the invention, and/or peaks of impurities.

To show compound signals in the delta-range of solvents and/or water the usual peaks of solvents, for example peaks of DMSO in DMSO-D₆ and the peak of water are shown in our 1H-NMR peak lists and have usually on average a high intensity .

The peaks of stereoisomers of the target compounds and/or peaks of impurities have usually on average a lower intensity than the peaks of target compounds (for example with a purity >90%).

Such stereoisomers and/or impurities can be typical for the specific preparation process. Therefore their peaks can help to recognize the reproduction of our preparation process via "side-products-fingerprints".

An expert, who calculates the peaks of the target compounds with known methods (MestreC, ACD-simulation, but also with empirically evaluated expectation values) can isolate the peaks of the target compounds as needed optionally using additional intensity filters. This isolation would be similar to relevant peak picking at classical 1H-NMR interpretation.

Further details of NMR-data description with peak lists you find in the publication "Citation of NMR Peaklist Data within Patent Applications" of the Research Disclosure Database Number 564025.

| |
|---|
| I-001: ¹H-NMR(499.9 MHz, CDCl3): |
| δ= 8.0776 (4.8); 8.0614 (5.7); 7.9402 (5.1); 7.9244 (14.5); 7.4675 (0.6); 7.4344 (2.6); 7.2608 (68.4); 7.0491 (0.4); 2.8695 (5.8); 2.8568 (14.4); 2.8438 (16.0); 2.8300 (7.4); 2.0069 (0.3); 1.9470 (2.1); 1.9340 (5.5); 1.9216 (6.8); 1.9091 (5.1); 1.8959 (1.7); 1.5621 (115.6); 1.4235 (0.3); 1.3189 (0.5); 1.2550 (2.4); 0.9361 (0.5); 0.9212 (0.8); 0.9062 (0.6); 0.8955 (0.7); 0.8807 (0.6); -0.0002 (63.9); -0.0066 (2.4) |
| I-002: ¹H-NMR(300.2 MHz, d₆-DMSO): |
| δ= 12.8685 (0.4); 8.4604 (0.4); 8.4465 (16.0); 8.3062 (6.0); 8.3029 (6.2); 7.9548 (3.6); 7.9495 (3.5); 7.9267 (5.7); 7.9214 (5.6); 7.8361 (6.5); 7.8081 (4.1); 3.3580 (1.4); 2.5343 (2.5); 2.5284 (5.3); 2.5223 (7.3); 2.5163 (5.3); 2.5103 (2.5); 2.0082 (0.4); 0.0170 (7.8) |
| I-003: ¹H-NMR(499.9 MHz, CDCl3): |
| δ= 8.5866 (2.5); 8.1062 (1.5); 8.1043 (1.6); 8.0893 (1.7); 8.0874 (1.7); 7.9734 (2.6); 7.5250 (2.0); 7.5080 (2.0); 7.2650 (2.2); 3.9091 (16.0); 1.7349 (0.5); -0.0002 (2.3) |
| I-004: ¹H-NMR(499.9 MHz, CDCl3): |
| δ= 8.1969 (2.3); 8.0811 (1.4); 8.0783 (1.3); 8.0642 (1.6); 8.0615 (1.5); 7.9981 (1.9); 7.9205 (1.6); 7.9036 (1.3); 7.2653 (2.2); 3.9371 (16.0); 1.7329 (0.6); -0.0002 (2.4) |
| I-005: ¹H-NMR(300.2 MHz, d₆-DMSO): |
| δ= 13.4953 (1.1); 8.5986 (3.5); 8.3103 (3.4); 8.0491 (1.8); 8.0441 (1.7); 8.0199 (2.2); 8.0149 (2.1); 7.7946 (2.7); 7.7654 (2.2); 3.3397 (16.0); 2.5345 (3.7); 2.5285 (7.5); 2.5224 (10.2); 2.5163 (7.2); 2.5104 (3.3); 1.3667 (0.7); 1.2528 (0.7); 0.0306 (0.5); 0.0198 (12.9); 0.0088 (0.5) |
| I-006: ¹H-NMR(300.2 MHz, d₆-DMSO): |
| δ= 13.5020 (1.3); 8.2660 (4.7); 8.0484 (2.0); 8.0202 (2.7); 7.8220 (1.8); 7.8184 (1.7); 7.7934 (1.4); 3.3392 (16.0); 2.5345 (3.2); 2.5285 (6.7); 2.5224 (9.2); 2.5164 (6.6); 2.5104 (3.1); 0.0313 (0.4); 0.0204 (11.5); 0.0094 (0.5) |
| I-007: ¹H-NMR(499.9 MHz, d₆-DMSO): |
| δ= 8.5066 (7.3); 8.3402 (9.5); 8.0809 (5.1); 8.0640 (6.2); 7.8757 (4.3); 7.8736 (4.6); 7.8589 (3.8); 7.8567 (4.1); 7.3758 (2.8); 7.3617 (7.9); 7.3465 (6.8); 7.3165 (3.1); 7.3020 (4.0); 7.2973 (1.3); 7.2873 (1.4); 7.2726 (7.9); 7.2583 (6.2); 5.8947 (16.0); 3.3736 (26.9); 2.5564 (3.2); 2.5530 (4.5); 2.5495 (3.5) |
| I-008: ¹H-NMR(499.9 MHz, d₆-DMSO): |
| δ= 8.6661 (6.1); 8.6646 (5.8); 8.3556 (4.2); 8.3534 (3.9); 7.9787 (3.3); 7.9771 (3.2); 7.9612 (3.7); 7.9596 (3.4); 7.6865 (3.4); 7.6837 (3.2); 7.6690 (3.0); 7.6663 (2.8); 7.3845 (13.0); 7.3757 (16.0); 7.3685 (0.9); 7.3652 (0.6); 7.3607 (0.6); 7.3580 (0.7); 7.3545 (0.7); 7.3468 (1.5); 7.3388 (2.0); 7.3300 (1.8); 7.3266 (0.7); 7.3220 (0.9); 7.3191 (0.6); 7.3122 (0.4); 5.7361 (13.6); 3.3292 (16.4); 2.5165 (1.3); 2.5129 (2.4); 2.5093 (3.2); 2.5056 (2.2); 2.5020 (1.0) |
| I-009: ¹H-NMR(400.1 MHz, d₆-DMSO): |
| δ= 8.5803 (6.6); 8.3355 (9.1); 8.0484 (2.6); 8.0449 (2.7); 8.0264 (4.6); 8.0228 (4.7); 7.9647 (5.6); 7.9425 (3.5); 7.3418 (1.7); 7.3234 (4.7); 7.3062 (7.1); 7.2825 (3.8); 7.2705 (11.0); 7.2513 (5.9); 5.7404 (16.0); 3.3083 (19.8); 2.5067 (4.1); 2.5025 (5.7); 2.4983 (4.5); -0.0002 (2.1) |
| I-010: ¹H-NMR(400.1 MHz, CDCl3): |
| δ= 8.5300 (5.9); 8.1275 (0.4); 8.0930 (0.8); 8.0831 (7.8); 7.9820 (2.8); 7.9786 (2.8); 7.9593 (3.7); 7.9559 (3.6); 7.8230 (4.5); 7.8003 (3.4); 7.2613 (4.8); 4.5485 (2.5); 4.5302 (7.5); 4.5118 (7.6); 4.4935 (2.6); 4.4715 (0.6); 4.4533 (0.5); 1.6969 (8.0); 1.6786 (16.0); 1.6602 (7.8); 1.6063 (4.2); 1.5574 (0.6); 1.5392 (1.1); 1.5211 (0.6); 1.2559 (0.5); -0.0002 (4.8) |
| I-011: ¹H-NMR(400.1 MHz, CDCl3): |
| δ= 8.5623 (11.3); 8.1128 (16.0); 8.0969 (6.7); 8.0940 (6.4); 7.5413 (7.6); 7.5192 (7.1); 7.2600 (8.0); 4.5102 (4.5); 4.4921 (13.6); 4.4739 (13.8); 4.4557 (4.7); 1.5780 (21.0); 1.5604 (28.2); 1.5422 (13.9); 1.3848 (0.3); 1.2551 (0.7); 0.0725 (1.7); -0.0002 (8.3) |
| I-012: ¹H-NMR(499.9 MHz, d₆-DMSO): |
| δ= 8.7477 (8.2); 8.5930 (5.6); 7.8608 (2.3); 7.8576 (2.3); 7.8427 (4.9); 7.8395 (5.2); 7.8142 (5.7); 7.7961 (2.5); 7.3940 (1.4); 7.3838 (16.0); 7.3777 (10.0); 7.3735 (10.0); 7.3617 (1.2); 7.3583 (1.4); 7.3566 (1.5); 7.3519 (0.7); 7.3425 (1.6); 7.3368 (1.9); 7.3326 (1.6); 7.3295 (1.5); 7.3251 (2.0); 7.3189 (1.0); 7.3149 (0.8); 7.3135 (0.7); 7.3081 (0.4); 5.7167 (15.8); 3.3477 (5.7); 3.3441 (5.7); 2.5162 (0.5); 2.5127 (1.1); 2.5091 (1.6); 2.5055 (1.2); 2.5020 (0.6) |
| I-013: ¹H-NMR(499.9 MHz, CDCl3): |
| δ= 8.5497 (2.4); 8.1304 (3.3); 8.1152 (1.5); 8.1123 (1.4); 8.0975 (1.6); 8.0946 (1.4); 7.6168 (1.8); 7.5991 (1.6); 7.2612 (2.7); 4.6021 (2.5); 4.5914 (4.4); 4.5806 (2.3); 3.8716 (2.6); 3.8609 (4.3); 3.8501 (2.3); 3.3014 (16.0); 1.5882 (2.4); -0.0002 (2.6) |
| I-014: ¹H-NMR(499.9 MHz, CDCl3): |
| δ= 8.4690 (3.3); 8.1053 (4.4); 8.0378 (0.4); 7.9074 (1.6); 7.8892 (2.0); 7.7412 (2.3); 7.7231 (1.9); 7.4960 (0.4); 7.1901 (2.8); 4.5553 (2.6); 4.5453 (4.4); 4.5351 (2.8); 4.4974 (0.4); 3.8390 (2.7); 3.8290 (4.4); 3.8189 (2.7); 3.7564 (0.4); 3.2787 (16.0); 3.2048 (1.2); 1.5399 (2.7); -0.0002 (10.7); -0.0722 (2.7) |
| I-015: ¹H-NMR(499.9 MHz, CDCl3): |
| δ= 8.5615 (4.6); 8.5595 (4.5); 8.0017 (5.9); 7.7776 (11.8); 7.7757 (10.6); 7.2617 (4.9); 4.5559 (2.3); 4.5412 (6.9); 4.5265 (7.0); 4.5118 (2.4); 1.6906 (7.8); 1.6758 (16.0); 1.6611 (7.8); 1.6275 (5.0); 1.5566 (0.4); 1.5531 (0.3); 1.5461 (0.4); 1.3409 (0.4); 1.3266 (0.5); -0.0002 (4.9) |
| I-016: ¹H-NMR(499.9 MHz, CDCl3): |
| δ= 8.3035 (2.3); 8.3021 (2.3); 8.0879 (2.9); 8.0866 (2.9); 7.8960 (1.0); 7.8936 (0.9); 7.8791 (2.1); 7.8767 (2.1); 7.8556 (2.4); 7.8551 (2.4); 7.8388 (1.0); 7.8383 (1.0); 7.2609 (2.6); 4.6493 (2.3); 4.6384 (4.3); 4.6275 (2.2); 3.8849 (2.5); 3.8741 (4.3); 3.8632 (2.2); 3.3065 (16.0); 1.5892 (3.0); 0.0719 (0.5); -0.0002 (2.6) |
| I-017: ¹H-NMR(499.9 MHz, CDCl3): |
| δ= 8.5531 (2.1); 8.5512 (2.0); 8.0951 (3.0); 7.8011 (0.5); 7.7994 (0.4); 7.7835 (2.8); 7.7819 (2.7); 7.7773 (2.6); 7.7748 (2.4); 7.7598 (0.4); 7.7573 (0.4); 7.2613 (3.0); 4.6414 (2.2); 4.6313 (3.3); 4.6210 (2.3); 3.9136 (2.3); 3.9033 (3.1); 3.8932 (2.2); 3.3512 (16.0); 1.6129 (2.5); -0.0002 (2.8) |
| I-018: ¹H-NMR(300.2 MHz, CDCl3): |
| δ= 7.9446 (0.7); 7.9390 (0.9); 7.9124 (1.4); 7.9032 (1.8); 7.5823 (1.5); 7.5559 (1.3); 7.2986 (7.2); 5.6128 (0.8); 4.2123 (0.6); 3.6126 (16.0); 2.9433 (0.4); 2.9249 (0.8); 2.9069 (0.4); 2.8908 (0.4); 2.8717 (0.5); 2.8618 (0.4); 2.8421 (0.4); 2.1807 (0.4); 2.1590 (0.5); 2.1348 (0.3); 2.0683 (0.3); 2.0455 (0.4); 1.9689 (0.3); 1.9596 (0.4); 1.9541 (0.4); 1.9438 (0.4); 1.9255 (0.5); 1.9157 (0.4); 1.9102 (0.5); 1.9002 (0.3); 1.8863 (0.3); 1.8821 (0.3); 1.8722 (0.6); 1.8534 (0.4); 1.8341 (0.4); 1.5823 (6.2); 0.1082 (0.8); 0.0492 (0.4); 0.0384 (8.8); 0.0274 (0.3) |
| I-019: ¹H-NMR(300.2 MHz, CDCl3): |
| δ= 7.9283 (1.6); 7.9117 (2.7); 7.4626 (1.7); 7.4477 (0.4); 7.4339 (1.5); 7.2984 (4.0); 5.8032 (0.5); 5.7846 (0.7); 5.7562 (0.6); 3.6497 (16.0); 2.9925 (0.5); 2.9775 (0.5); 2.9590 (0.6); 2.9442 (0.5); 2.9197 (0.5); 2.9061 (0.9); 2.8493 (0.4); 2.2742 (0.4); 2.2427 (1.1); 2.2379 (1.0); 2.2079 (1.0); 2.1908 (1.0); 2.1734 (1.3); 2.1653 (1.5); 2.1475 (1.5); 2.1313 (1.4); 2.1156 (0.7); 1.9235 (0.4); 1.9080 (0.6); 1.8904 (0.6); 1.8808 (0.4); 1.8745 (0.4); 1.8567 (0.4); 1.6093 (1.1); 1.4693 (1.8); 1.2924 (0.5); 1.1949 (0.4); 1.1836 (0.5); 1.1751 (0.7); 1.1630 (1.6); 1.1554 (1.6); 1.1469 (1.9); 1.1397 (1.6); 1.1304 (0.8); 1.1213 (0.6); 1.1083 (0.4); 0.9927 (0.4); 0.9878 (0.4); 0.9781 (0.9); 0.9655 (1.8); 0.9520 (1.3); 0.9386 (1.8); 0.9263 (0.8); 0.9174 (0.4); 0.9117 (0.4); 0.1197 (0.8); 0.1081 (16.5); 0.0959 (0.9); 0.0368 (4.9) |
| I-020: ¹H-NMR(300.2 MHz, CDCl3): |
| δ= 7.9183 (2.0); 7.9024 (1.4); 7.4559 (1.4); 7.4416 (0.4); 7.4272 (1.2); 7.2986 (3.0); 5.7902 (0.3); 3.5407 (7.5); 2.9855 (0.4); 2.9708 (0.4); 2.9524 (0.5); 2.9363 (0.4); 2.9203 (0.4); 2.9053 (0.8); 2.8896 (0.4); 2.3062 (16.0); 2.2207 (0.5); 2.1913 (0.5); 2.1767 (0.6); 2.1700 (0.7); 2.1625 (0.7); 2.1534 (1.4); 2.1435 (1.1); 2.1367 (1.0); 2.1296 (1.0); 2.1198 (1.0); 2.1081 (0.6); 1.9193 (0.4); 1.9039 (0.5); 1.8863 (0.4); 1.8712 (0.3); 1.4684 (1.1); 1.2914 (0.6); 0.1073 (13.2); 0.0950 (0.6); 0.0359 (3.7) |
| I-021: ¹H-NMR(300.2 MHz, CDCl3): |
| δ= 7.9095 (2.0); 7.8925 (1.3); 7.4702 (1.3); 7.4416 (1.2); 7.2984 (1.8); 5.7899 (0.4); 3.8591 (0.6); 3.8502 (0.9); 3.8370 (0.4); 3.8281 (2.2); 3.8059 (2.4); 3.7954 (0.4); 3.7848 (0.9); 3.7747 (0.5); 3.5538 (7.4); 3.4460 (16.0); 3.4357 (0.9); 3.3753 (0.5); 2.9818 (0.4); 2.9666 (0.4); 2.9476 (0.5); 2.9321 (0.4); 2.9150 (0.5); 2.9019 (0.8); 2.8792 (1.2); 2.8575 (2.1); 2.8367 (1.0); 2.2249 (0.4); 2.1900 (0.5); 2.1744 (0.6); 2.1562 (1.0); 2.1500 (1.1); 2.1313 (1.1); 2.1159 (0.9); 2.1001 (0.5); 1.9019 (0.4); 1.8841 (0.4); 1.6596 (0.5); 0.1055 (1.2); 0.0329 (2.1) |
| I-022: ¹H-NMR(300.2 MHz, CDCl3): |
| δ= 7.9863 (4.4); 7.9811 (5.1); 7.9541 (6.6); 7.9357 (10.5); 7.8031 (8.4); 7.7983 (10.0); 7.7777 (10.2); 7.7714 (10.2); 7.6359 (8.4); 7.6089 (7.2); 7.5590 (0.6); 7.5541 (1.3); 7.5488 (1.1); 7.5450 (0.9); 7.5305 (4.5); 7.5281 (4.6); 7.5191 (2.9); 7.5133 (6.6); 7.5074 (12.3); 7.5025 (16.0); 7.4877 (4.1); 7.4784 (11.6); 7.4629 (1.8); 7.4578 (3.2); 7.4488 (2.3); 7.2983 (14.1); 5.7465 (1.4); 5.3345 (1.1); 3.4423 (12.8); 3.0647 (0.8); 3.0468 (0.9); 3.0296 (0.8); 3.0090 (2.2); 2.9912 (2.2); 2.9725 (2.2); 2.9583 (2.0); 2.9355 (2.1); 2.9195 (3.8); 2.9046 (2.1); 2.8796 (0.8); 2.8636 (1.4); 2.8472 (0.7); 2.4008 (0.8); 2.3901 (0.9); 2.3696 (0.9); 2.3584 (2.6); 2.3476 (2.0); 2.3209 (3.6); 2.2908 (2.2); 2.2723 (2.5); 2.2589 (2.4); 2.2514 (2.6); 2.2381 (2.3); 2.2230 (2.7); 2.2133 (3.1); 2.2094 (3.1); 2.2011 (2.5); 2.1939 (2.9); 2.1763 (2.2); 2.1661 (2.0); 2.1477 (1.3); 2.1290 (0.5); 2.0819 (0.6); 1.9545 (0.4); 1.9372 (0.9); 1.9202 (1.1); 1.9046 (1.7); 1.8855 (1.8); 1.8675 (1.5); 1.8510 (1.4); 1.8243 (0.5); 1.6251 (1.0); 1.3200 (0.4); 1.2935 (2.5); 0.1929 (0.3); 0.1758 (0.5); 0.1216 (3.4); 0.1098 (65.8); 0.0974 (2.5); 0.0481 (0.7); 0.0373 (17.6); 0.0264 (0.6) |
| I-023: ¹H-NMR(499.9 MHz, CDCl3): |
| δ= 7.8819 (1.1); 7.8787 (1.2); 7.8658 (1.8); 7.8625 (2.1); 7.8301 (3.2); 7.8220 (2.7); 7.8056 (1.5); 7.2595 (4.1); 4.1359 (0.7); 4.1227 (1.2); 4.1094 (0.6); 3.3860 (16.0); 2.9134 (0.3); 2.9058 (0.4); 2.8920 (0.8); 2.8801 (0.6); 2.8727 (0.7); 2.8615 (0.6); 2.8227 (0.7); 2.8124 (1.3); 2.8018 (0.8); 2.7895 (0.5); 2.7791 (0.9); 2.7685 (0.7); 2.7576 (1.0); 2.7439 (1.8); 2.7301 (1.6); 2.7169 (0.6); 2.7051 (0.3); 2.0872 (0.5); 2.0765 (0.8); 2.0657 (0.8); 2.0618 (0.5); 2.0548 (0.6); 2.0506 (0.6); 2.0404 (0.7); 2.0300 (0.4); 2.0157 (1.0); 2.0048 (2.3); 1.9916 (2.0); 1.9808 (1.0); 1.7481 (0.4); 1.7400 (0.4); 1.7324 (0.6); 1.7298 (0.6); 1.7214 (0.7); 1.7139 (0.5); 1.7066 (0.4); 1.7026 (0.6); 1.5503 (4.2); 1.1831 (4.8); 1.1690 (9.9); 1.1549 (4.6); -0.0002 (4.1) |
| I-024: ¹H-NMR(300.2 MHz, CDCl3): |
| δ= 7.9412 (1.0); 7.9148 (3.7); 7.5403 (1.2); 7.5141 (1.1); 7.2983 (1.8); 5.3824 (0.4); 5.3620 (0.6); 5.3358 (0.4); 3.9076 (16.0); 3.6061 (12.8); 2.9654 (0.4); 2.9509 (0.4); 2.9322 (0.4); 2.9178 (0.4); 2.9005 (0.4); 2.8836 (0.7); 2.8687 (0.4); 2.2118 (0.4); 2.1827 (0.5); 2.1779 (0.5); 2.1588 (1.4); 2.1453 (1.1); 2.1361 (1.4); 2.1190 (0.7); 2.1144 (0.8); 2.1002 (0.3); 1.8710 (0.4); 1.8532 (0.4); 1.6174 (1.1); 0.1078 (0.3); 0.0349 (2.0) |
| I-025: ¹H-NMR(300.2 MHz, CDCl3): |
| δ= 7.9390 (1.1); 7.9123 (4.0); 7.5484 (1.4); 7.5221 (1.2); 7.2985 (2.4); 5.3893 (0.4); 5.3693 (0.7); 5.3420 (0.5); 4.4523 (1.0); 4.4437 (1.3); 4.4382 (1.8); 4.4278 (1.7); 4.4207 (1.3); 4.4128 (1.2); 3.7168 (2.1); 3.7010 (3.9); 3.6855 (1.8); 3.6309 (12.6); 3.4421 (16.0); 3.4303 (1.0); 2.9648 (0.4); 2.9494 (0.4); 2.9289 (0.5); 2.9160 (0.4); 2.9002 (0.4); 2.8829 (0.8); 2.2184 (0.4); 2.2082 (0.4); 2.1891 (0.7); 2.1697 (1.8); 2.1507 (1.8); 2.1294 (0.7); 2.1165 (0.7); 1.9004 (0.3); 1.8859 (0.4); 1.8712 (0.5); 1.8529 (0.4); 1.8367 (0.3); 1.6245 (1.1); 0.0354 (2.9) |
| I-026: ¹H-NMR(300.2 MHz, CDCl3): |
| δ= 7.9581 (0.9); 7.9316 (1.2); 7.9094 (1.2); 7.3404 (1.2); 7.3140 (1.1); 7.2987 (3.4); 4.7419 (2.6); 3.8008 (16.0); 3.7819 (1.2); 2.9836 (1.0); 2.9643 (1.7); 2.9453 (0.9); 1.6212 (0.7); 1.4689 (0.6); 0.1076 (2.6); 0.0366 (4.0) |
| I-027: ¹H-NMR(400.2 MHz, d₆-DMSO): |
| δ= 7.9528 (1.4); 7.9399 (0.4); 7.9297 (2.0); 7.8361 (1.8); 7.8278 (2.1); 7.8230 (2.2); 3.7637 (2.0); 3.7486 (16.0); 3.7335 (2.1); 3.3261 (50.2); 2.8619 (1.4); 2.8459 (2.7); 2.8299 (1.5); 2.5074 (36.7); 2.5034 (45.9); 1.9256 (0.5); 1.9097 (1.5); 1.8943 (1.9); 1.8792 (1.4); 1.8633 (0.4) |
| I-028: ¹H-NMR(400.2 MHz, d₆-DMSO): |
| δ= 8.3085 (0.4); 7.9554 (1.2); 7.9436 (0.4); 7.9323 (1.8); 7.8486 (0.7); 7.8432 (1.6); 7.8368 (1.8); 7.8341 (1.5); 7.8315 (1.7); 7.8258 (1.3); 7.8204 (0.5); 3.7648 (1.7); 3.7490 (16.0); 3.7346 (1.8); 3.3235 (59.0); 2.8624 (1.1); 2.8464 (2.1); 2.8303 (1.2); 2.5256 (1.0); 2.5208 (1.4); 2.5122 (18.3); 2.5077 (37.1); 2.5032 (48.8); 2.4986 (35.3); 2.4940 (17.2); 1.9271 (0.4); 1.9114 (1.2); 1.8959 (1.4); 1.8805 (1.1); 1.8647 (0.4); - 0.0002 (0.4) |
| I-029: ¹H-NMR(400.2 MHz, d₆-DMSO): |
| δ= 8.3112 (0.5); 7.8641 (5.3); 7.8287 (9.0); 3.7568 (4.4); 3.7414 (6.2); 3.7258 (4.5); 3.3265 (155.9); 2.8464 (3.3); 2.8301 (6.8); 2.8136 (3.5); 2.6766 (0.5); 2.6722 (0.7); 2.6678 (0.5); 2.5986 (2.1); 2.5804 (7.0); 2.5621 (7.2); 2.5439 (2.4); 2.5255 (2.1); 2.5206 (3.1); 2.5120 (40.1); 2.5077 (81.1); 2.5032 (106.5); 2.4987 (78.0); 2.4944 (39.1); 2.3345 (0.4); 2.3302 (0.6); 2.3256 (0.5); 1.9443 (1.1); 1.9282 (3.5); 1.9125 (4.6); 1.8969 (3.3); 1.8808 (1.0); 1.2399 (0.8); 1.0738 (7.5); 1.0556 (16.0); 1.0373 (7.2); -0.0001 (0.5) |
| I-030: ¹H-NMR(400.2 MHz, d₆-DMSO): |
| δ= 8.3117 (0.8); 7.8731 (5.3); 7.8346 (8.7); 3.7573 (4.4); 3.7419 (6.0); 3.7263 (4.5); 3.3269 (284.5); 2.8920 (0.4); 2.8468 (3.2); 2.8305 (6.5); 2.8140 (3.4); 2.7323 (0.4); 2.6760 (0.8); 2.6716 (1.2); 2.6671 (0.9); 2.5993 (2.1); 2.5811 (7.0); 2.5628 (7.3); 2.5445 (2.6); 2.5250 (3.6); 2.5202 (5.4); 2.5115 (69.6); 2.5071 (141.1); 2.5026 (185.5); 2.4981 (135.1); 2.4936 (66.6); 2.3339 (0.8); 2.3294 (1.1); 2.3249 (0.8); 1.9448 (1.0); 1.9286 (3.4); 1.9128 (4.5); 1.8973 (3.2); 1.8811 (1.0); 1.2400 (1.4); 1.0733 (7.4); 1.0552 (16.0); 1.0368 (7.2); 0.8537 (0.4); -0.0001 (0.8) |
| I-031: ¹H-NMR(400.1 MHz, d₆-DMSO): |
| δ= 12.0505 (0.8); 8.1714 (0.4); 7.9527 (4.2); 7.9079 (5.5); 7.8898 (2.9); 7.8762 (4.1); 7.8612 (3.6); 7.4849 (0.4); 7.4636 (0.4); 7.4427 (5.7); 7.4229 (5.2); 6.6255 (0.5); 4.7833 (8.4); 4.7260 (10.9); 3.7283 (7.2); 3.7137 (14.6); 3.6988 (8.1); 3.3086 (19.0); 2.9783 (3.2); 2.9644 (5.7); 2.9503 (3.3); 2.8794 (2.7); 2.8650 (4.5); 2.8511 (2.5); 2.5125 (12.2); 2.5086 (15.9); 2.5046 (12.2); 2.4657 (4.7); 2.4473 (14.9); 2.4288 (15.4); 2.4103 (5.2); 1.0521 (8.1); 1.0461 (7.6); 1.0339 (16.0); 1.0157 (8.4) |
| I-032: ¹H-NMR(400.2 MHz, d₆-DMSO): |
| δ= 8.3088 (0.4); 7.9085 (1.3); 7.8790 (0.9); 7.8747 (0.7); 7.8592 (1.0); 7.8548 (0.8); 7.4305 (1.3); 7.4107 (1.2); 4.6677 (1.6); 3.6545 (16.0); 3.6439 (2.4); 3.6291 (1.2); 3.3514 (0.4); 3.3296 (112.5); 2.9140 (0.9); 2.8991 (1.8); 2.8845 (0.9); 2.6723 (0.4); 2.5258 (1.1); 2.5210 (1.6); 2.5125 (22.5); 2.5080 (45.7); 2.5034 (59.4); 2.4988 (42.2); 2.4942 (20.0); 2.3302 (0.4); 1.2407 (0.4) |
| I-033: ¹H-NMR(400.1 MHz, d₆-DMSO): |
| δ= 12.0508 (2.2); 11.8731 (0.4); 8.1631 (1.2); 8.0151 (0.7); 8.0113 (0.7); 7.9953 (0.8); 7.9440 (4.0); 7.8986 (5.2); 7.8850 (2.8); 7.8685 (4.1); 7.8531 (3.3); 7.4808 (1.0); 7.4604 (1.0); 7.4389 (5.3); 7.4192 (4.8); 6.8929 (0.3); 6.6269 (1.4); 4.7832 (8.0); 4.7248 (10.2); 3.7274 (6.6); 3.7127 (13.4); 3.6979 (7.4); 3.3094 (21.3); 3.1954 (0.3); 3.1837 (0.4); 2.9771 (3.1); 2.9632 (5.3); 2.9488 (3.2); 2.8782 (2.7); 2.8637 (4.3); 2.8497 (2.5); 2.8134 (0.4); 2.7260 (0.3); 2.7036 (0.5); 2.6851 (0.5); 2.6283 (0.5); 2.6095 (0.6); 2.5876 (0.5); 2.5125 (8.3); 2.5087 (10.7); 2.5047 (8.4); 2.4651 (4.5); 2.4466 (14.2); 2.4281 (14.7); 2.4097 (4.9); 1.0518 (8.3); 1.0453 (8.0); 1.0338 (16.0); 1.0274 (13.1); 1.0157 (8.4) |
| I-034: ¹H-NMR(300.2 MHz, d₆-DMSO): |
| δ= 8.7918 (1.2); 8.2698 (1.0); 8.2401 (1.0); 8.1484 (2.9); 8.1449 (2.8); 7.8310 (1.0); 7.8232 (1.0); 7.4954 (0.7); 7.4877 (0.7); 7.4658 (0.7); 7.4580 (0.7); 3.3387 (16.0); 3.1368 (3.4); 2.9805 (3.4); 2.5342 (3.0); 2.5283 (6.0); 2.5222 (8.1); 2.5161 (5.8); 2.5102 (2.7); 0.0317 (0.5); 0.0210 (10.8); 0.0100 (0.4) |
| I-035: ¹H-NMR(499.9 MHz, CDCl3): |
| δ= 10.3938 (13.5); 10.3919 (16.0); 10.3905 (15.8); 10.3887 (13.2); 8.6767 (0.3); 8.6668 (5.4); 8.6635 (14.5); 8.6600 (14.8); 8.6565 (5.5); 8.3475 (11.8); 8.3441 (11.6); 8.3289 (13.2); 8.3254 (13.1); 8.0463 (14.1); 8.0446 (13.9); 8.0357 (0.4); 8.0277 (12.4); 8.0260 (12.1); 7.4678 (0.3); 7.2612 (60.7); 7.0495 (0.3); 1.5612 (26.0); 0.0063 (2.1); -0.0002 (61.0); -0.0068 (1.9) |
| I-036: ¹H-NMR(499.9 MHz, d₆-DMSO): |
| δ= 8.8571 (4.4); 8.8546 (7.6); 8.8520 (4.0); 7.9294 (0.5); 7.9175 (13.6); 7.9048 (0.5); 7.9022 (0.6); 7.8857 (16.0); 7.8832 (14.3); 3.3183 (41.2); 2.5126 (2.8); 2.5091 (5.3); 2.5055 (6.8); 2.5019 (4.7); 2.4983 (2.1) |
| I-037: ¹H-NMR(300.2 MHz, CDCl3): |
| δ= 9.0596 (1.5); 9.0562 (1.9); 9.0544 (2.0); 9.0510 (1.6); 8.2970 (3.0); 8.2955 (3.1); 7.9552 (1.1); 7.9496 (1.1); 7.9234 (1.5); 7.9178 (1.5); 7.7679 (1.7); 7.7361 (1.4); 7.7067 (0.5); 7.2987 (7.7); 3.7588 (0.6); 3.7425 (1.7); 3.7251 (2.0); 3.7068 (0.9); 3.7031 (0.8); 3.6476 (2.1); 3.6310 (2.5); 3.6151 (1.0); 3.4509 (16.0); 1.6418 (9.7); 0.0362 (7.0) |
| I-038: ¹H-NMR(300.2 MHz, CDCl3): |
| δ= 7.9713 (0.4); 7.9440 (1.1); 7.3098 (0.5); 7.2986 (1.9); 7.2839 (0.4); 4.6826 (1.4); 3.7468 (0.4); 3.7274 (0.8); 3.7079 (0.4); 2.9770 (0.4); 2.9578 (0.7); 2.9386 (0.4); 2.0820 (0.6); 1.5421 (16.0); 1.2964 (0.4); 0.0368 (2.2) |
| I-039: ¹H-NMR(300.2 MHz, CDCl3): |
| δ= 9.0559 (3.1); 9.0523 (4.2); 9.0508 (4.5); 9.0473 (3.4); 8.3384 (7.1); 7.9651 (1.8); 7.9596 (1.8); 7.9332 (3.8); 7.9277 (3.9); 7.8776 (4.2); 7.8457 (1.9); 7.2990 (11.2); 4.5632 (2.3); 4.5394 (7.4); 4.5156 (7.5); 4.4919 (2.4); 1.6306 (2.5); 1.5188 (7.7); 1.4951 (16.0); 1.4713 (7.5); 1.2893 (0.5); 0.0457 (0.5); 0.0349 (13.9); 0.0258 (0.4); 0.0241 (0.5) |
| I-040: ¹H-NMR(300.2 MHz, d₆-DMSO): |
| δ= 9.5600 (3.5); 9.5553 (4.8); 9.5515 (3.4); 9.3705 (0.4); 8.7529 (0.4); 8.6999 (7.7); 8.5487 (0.6); 7.9182 (1.2); 7.9126 (1.1); 7.8864 (4.8); 7.8809 (4.9); 7.8659 (5.1); 7.8342 (1.2); 4.3783 (0.4); 4.3547 (0.4); 4.1063 (0.4); 4.0912 (0.4); 4.0050 (0.5); 3.9855 (0.5); 3.9653 (0.5); 3.8129 (0.3); 3.1884 (16.0); 2.5340 (8.5); 2.5280 (17.3); 2.5219 (23.4); 2.5159 (16.9); 2.5099 (8.1); 1.3560 (0.6); 0.0308 (1.2); 0.0199 (27.8); 0.0088 (1.2); -0.0419 (0.9) |
| I-041: ¹H-NMR(300.2 MHz, CDCl3): |
| δ= 8.5075 (10.3); 8.2530 (16.0); 8.1452 (5.6); 8.1407 (4.9); 8.1168 (6.4); 8.1122 (5.9); 7.8450 (7.8); 7.8166 (6.5); 7.7826 (0.4); 7.2982 (33.3); 5.3364 (2.7); 4.2000 (0.8); 4.1761 (2.4); 4.1524 (2.5); 4.1287 (0.8); 2.0879 (11.4); 1.4690 (1.9); 1.4424 (0.5); 1.4277 (0.4); 1.4097 (0.4); 1.3934 (0.7); 1.3854 (0.8); 1.3694 (1.2); 1.3612 (0.5); 1.3457 (0.6); 1.3231 (3.1); 1.3126(0.4); 1.2993 (6.2); 1.2903 (1.0); 1.2754 (3.1); 1.2581 (0.4); 1.2431 (0.6); 0.1070 (1.6); 0.0472 (1.8); 0.0364 (43.9); 0.0255 (1.7) |
| I-042: ¹H-NMR(400.2 MHz, d₆-DMSO): |
| δ= 8.4481 (12.2); 8.0261 (0.6); 7.8912 (13.7); 7.8694 (16.0); 7.7660 (0.6); 7.7288 (9.5); 7.7071 (7.5); 7.6759 (0.6); 7.6536 (0.6); 7.5045 (0.4); 7.2242 (0.5); 7.1989 (0.4); 7.1771 (0.4); 7.1031 (5.8); 7.0985 (6.1); 7.0813 (5.6); 7.0765 (6.0); 6.9860 (10.5); 6.5086 (1.5); 2.5063 (11.4); -0.0024 (4.0) |
| I-043: ¹H-NMR(300.2 MHz, CDCl3): |
| δ= 9.1972 (12.6); 8.8284 (4.9); 8.8253 (6.8); 8.8216 (4.2); 8.3522 (0.5); 8.3478 (0.4); 8.3233 (10.2); 8.3195 (16.0); 8.2915 (0.5); 8.0622 (0.3); 7.2982 (21.1); 2.9965 (2.7); 2.9248 (2.4); 1.6634 (0.3); 0.0475 (0.6); 0.0367 (19.0); 0.0257 (0.7) |
| I-044: ¹H-NMR(400.1 MHz, d₆-DMSO): |
| δ= 8.5761 (13.2); 8.1032 (16.0); 3.8077 (0.3); 3.7162 (0.5); 3.3334 (9.5); 2.8987 (0.8); 2.7394 (0.7); 2.5096 (25.9) |
| I-045: ¹H-NMR(400.1 MHz, d₆-DMSO): |
| δ= 8.1911 (3.8); 7.9260 (1.7); 7.9049 (2.2); 7.7677 (3.1); 7.7467 (2.4); 4.3222 (1.1); 4.3042 (3.4); 4.2862 (3.5); 4.2682 (1.2); 3.3239 (83.1); 2.6060 (16.0); 2.5084 (7.0); 1.3563 (3.5); 1.3383 (7.6); 1.3203 (3.7) |
| I-046: ¹H-NMR(300.2 MHz, d₆-DMSO): |
| δ= 11.5477 (1.2); 8.1573 (3.0); 8.1550 (3.3); 8.1528 (3.1); 7.7837 (1.7); 7.7560 (3.9); 7.7187 (3.5); 7.7137 (3.4); 7.6910 (1.6); 7.6861 (1.5); 7.6348 (2.2); 7.6255 (3.2); 7.6163 (2.3); 6.6031 (1.3); 6.6002 (1.6); 6.5968 (1.8); 6.5935 (2.5); 6.5902 (1.7); 6.5868 (1.6); 6.5839 (1.3); 3.3427 (16.0); 2.5339 (1.8); 2.5279 (3.8); 2.5218 (5.2); 2.5156 (3.7); 2.5097 (1.8); 0.0191 (6.3) |
| I-047: ¹H-NMR(400.1 MHz, CDCl3): |
| δ= 8.0501 (0.3); 7.9616 (6.4); 7.9344 (14.2); 7.5208 (0.4); 7.3134 (9.9); 7.2936 (9.5); 7.2621 (61.2); 4.9394 (5.1); 4.8138 (11.4); 3.9548 (7.0); 3.9423 (5.5); 3.8856 (3.8); 3.0469 (6.6); 2.9584 (3.4); 2.9390 (3.5); 2.8855 (1.3); 1.8680 (2.1); 1.8563 (4.6); 1.8479 (5.3); 1.8364 (9.6); 1.8249 (5.9); 1.8165 (5.3); 1.8048 (2.8); 1.5881 (9.0); 1.0689 (3.7); 1.0586 (13.8); 1.0514 (16.0); 1.0478 (15.1); 1.0406 (15.6); 1.0313 (5.8); 1.0045 (0.8); 0.9931 (0.7); 0.8895 (0.4); 0.8414 (9.8); 0.8346 (10.9); 0.8225 (11.2); 0.8161 (9.4); 0.0080 (0.7); -0.0002 (22.0) |
| I-048: ¹H-NMR(300.2 MHz, CDCl3): |
| δ= 7.9817 (0.8); 7.9764 (1.0); 7.9488 (2.3); 7.2985 (2.8); 7.2837 (0.5); 4.7328 (2.5); 3.8579 (0.4); 3.8042 (16.0); 3.7882 (1.0); 3.7776 (1.0); 2.9945 (0.9); 2.9754 (1.6); 2.9560 (0.8); 1.6342 (1.4); 1.2905 (0.9); 0.1069 (0.8); 0.0352 (2.7) |
| I-049: ¹H-NMR(300.2 MHz, CDCl3): |
| δ= 7.9979 (1.8); 7.9721 (3.6); 7.9515 (2.5); 7.3507 (1.7); 7.3239 (1.7); 7.3135 (1.0); 7.2983 (4.4); 7.2874 (0.9); 4.8441 (5.8); 4.7298 (3.3); 3.9231 (1.1); 3.9034 (2.1); 3.8834 (1.2); 3.7858 (2.2); 3.7664 (3.8); 3.7463 (2.5); 3.0548 (1.6); 3.0353 (2.7); 3.0159 (1.5); 2.9927 (1.0); 2.9729 (1.6); 2.9532 (0.9); 2.4132 (0.3); 2.2762 (0.7); 2.2446 (9.6); 2.2373 (16.0); 1.7081 (0.9); 1.2877 (1.2); 0.0323 (4.4) |
| I-050: ¹H-NMR(300.2 MHz, CDCl3): |
| δ= 8.0246 (0.3); 8.0015 (0.5); 7.9765 (2.1); 7.9483 (3.2); 7.9172 (1.0); 7.8776 (2.9); 7.5117 (0.6); 7.4862 (0.5); 7.3977 (1.1); 7.3742 (2.9); 7.3691 (2.9); 7.3601 (2.5); 7.3490 (6.4); 7.3314 (6.8); 7.3181 (6.1); 7.2984 (19.2); 7.2830 (2.2); 7.2725 (2.7); 7.2586 (2.5); 7.1727 (0.9); 7.1464 (0.9); 7.1159 (0.4); 6.5363 (1.0); 5.3359 (3.1); 4.8792 (7.2); 4.6980 (3.5); 4.1107 (0.8); 4.0868 (0.8); 3.9544 (1.8); 3.9344 (2.2); 3.9144 (1.3); 3.8847 (16.0); 3.7663 (2.6); 3.7469 (4.4); 3.7270 (2.8); 3.0034 (0.9); 2.9839 (1.7); 2.9641 (1.0); 2.9380 (0.6); 2.8002 (2.0); 2.7810 (3.2); 2.7618 (1.7); 1.6500 (0.9); 0.0487 (0.4); 0.0380 (13.2); 0.0270 (0.5) |
| I-051: ¹H-NMR(300.1 MHz, d₆-DMSO): |
| δ= 10.4406 (1.9); 9.2471 (3.2); 8.6386 (2.8); 8.3662 (3.6); 8.2796 (1.6); 8.2511 (2.2); 8.0895 (1.8); 8.0845 (1.8); 8.0608 (1.4); 8.0560 (1.4); 3.7394 (16.0); 3.3244 (0.4); 2.5082 (16.4); 2.5024 (21.7); 2.4967 (15.6); 0.0105 (0.5); -0.0005 (11.6) |
| I-052: ¹H-NMR(400.2 MHz, d₆-DMSO): |
| δ= 10.6841 (5.1); 9.2701 (7.9); 8.6510 (7.3); 8.6166 (6.1); 8.2755 (3.7); 8.2541 (4.6); 8.0915 (3.7); 8.0881 (3.6); 8.0703 (3.0); 8.0667 (2.9); 3.3242 (3.8); 2.5065 (13.9); 2.5023 (18.6); 2.4806 (7.4); 2.4618 (7.4); 2.4430 (2.5); 1.1592 (0.3); 1.1385 (7.8); 1.1197 (16.0); 1.1009 (7.4); -0.0021 (7.7) |
| I-053: ¹H-NMR(400.2 MHz, d₆-DMSO): |
| δ= 9.6566 (2.7); 7.7096 (2.1); 7.6879 (2.8); 7.5516 (2.6); 7.5298 (2.0); 4.7563 (2.2); 4.7343 (4.5); 4.7122 (2.3); 3.3238 (1.7); 3.2664 (2.0); 3.2445 (3.9); 3.2225 (1.9); 2.5046 (8.2); 2.5004 (10.6); 2.4963 (7.7); 2.1188 (16.0); -0.0021 (5.6) |
| I-054: ¹H-NMR(300.1 MHz, d₆-DMSO): |
| δ= 10.7436 (2.7); 9.2795 (4.6); 8.6395 (5.6); 8.2895 (2.0); 8.2606 (2.8); 8.1083 (2.2); 8.1038 (2.2); 8.0800 (1.7); 8.0753 (1.7); 3.3219 (6.2); 2.5070 (33.0); 2.5015 (41.8); 2.1660 (16.0); 1.8872 (0.4); -0.0005 (16.8) |
| I-055: ¹H-NMR(400.2 MHz, d₆-DMSO): |
| δ= 9.0640 (16.0); 8.4097 (12.8); 8.2854 (0.4); 8.0920 (8.4); 8.0703 (9.5); 7.9940 (0.4); 7.9727 (0.4); 7.7797 (7.9); 7.7764 (7.4); 7.7582 (6.9); 7.7548 (6.6); 7.6488 (0.4); 7.6246 (0.4); 7.0603 (14.7); 7.0372 (0.6); 4.3788 (0.4); 4.3610 (0.4); 2.5103 (12.6); 2.5061 (16.2); 2.5021 (11.8); 1.3787 (0.4); 1.3611 (0.8); 1.3434 (0.4); -0.0021 (7.7) |
| I-056: ¹H-NMR(400.2 MHz, d₆-DMSO): |
| δ= 9.4860 (2.8); 7.8915 (1.1); 7.8702 (2.4); 7.8382 (3.6); 7.8170 (1.6); 3.2364 (1.9); 3.2177 (3.7); 3.1989 (2.1); 2.9506 (1.8); 2.9319 (3.6); 2.9130 (2.1); 2.5093 (6.5); 2.5050 (8.3); 2.5006 (6.2); 2.1296 (16.0); 2.1131 (2.6); 2.0942 (3.0); 2.0754 (2.0); 2.0564 (0.6); 0.0009 (2.0) |
| I-057: ¹H-NMR(400.2 MHz, d₆-DMSO): |
| δ= 11.6160 (13.1); 8.7213 (15.1); 8.4505 (13.2); 8.4472 (13.6); 8.3208 (0.9); 8.2571 (10.1); 8.2348 (11.4); 8.1550 (4.7); 8.1333 (16.0); 8.1189 (12.6); 8.1157 (13.0); 8.0974 (3.7); 8.0941 (4.0); 7.8609 (8.3); 7.8560 (8.3); 7.8387 (7.7); 7.8337 (7.7); 3.3340 (12.3); 2.6720 (0.4); 2.5072 (44.1); 2.5029 (58.4); 2.4986 (42.9); 2.3300 (0.4); 0.0064 (1.2); -0.0016 (26.6) |
| I-058: ¹H-NMR(499.9 MHz, CDCl3): |
| δ= 8.1306 (6.3); 8.0793 (3.5); 8.0774 (3.3); 8.0625 (11.2); 7.9438 (4.9); 7.9268 (3.9); 7.3906 (1.5); 7.3867 (2.1); 7.3737 (6.3); 7.3587 (7.4); 7.3533 (4.8); 7.3453 (1.6); 7.3397 (2.6); 7.3315 (0.7); 7.3251 (0.7); 7.2609 (5.2); 7.2254 (6.2); 7.2117 (5.5); 5.4395 (16.0); 5.4076 (0.4); 5.3739 (0.4); 1.2562 (0.4); -0.0002 (5.1) |
| I-059: ¹H-NMR(499.9 MHz, d₆-DMSO): |
| δ= 8.6331 (15.6); 8.2472 (7.0); 8.2454 (7.2); 7.9301 (2.7); 7.9270 (2.5); 7.9131 (6.8); 7.9100 (6.8); 7.8922 (9.2); 7.8753 (3.5); 7.3706 (2.8); 7.3675 (1.2); 7.3557 (6.9); 7.3453 (2.4); 7.3413 (7.5); 7.3318 (0.3); 7.3107 (10.0); 7.3057 (2.6); 7.3027 (3.6); 7.2968 (5.9); 7.2908 (2.2); 7.2865 (3.9); 7.2818 (0.9); 7.2750 (0.9); 7.2722 (1.3); 5.6720 (16.0); 3.3267 (39.8); 2.5259 (0.4); 2.5222 (0.6); 2.5181 (0.8); 2.5116 (9.8); 2.5080 (20.5); 2.5044 (27.9); 2.5007 (19.8); 2.4972 (9.0); 2.0763 (1.2); -0.0002 (0.6) |
| I-060: ¹H-NMR(499.9 MHz, d₆-DMSO): |
| δ= 8.3774 (4.5); 8.3101 (2.7); 8.3079 (2.8); 7.9893 (1.5); 7.9863 (1.5); 7.9724 (1.8); 7.9694 (1.8); 7.8185 (2.6); 7.8016 (2.2); 3.9133 (16.0); 3.3302 (8.3); 2.5134 (2.9); 2.5098 (5.8); 2.5062 (7.8); 2.5027 (5.6); 2.4992 (2.5) |
| I-061: ¹H-NMR(300.2 MHz, CDCl3): |
| δ= 10.3308 (0.4); 10.2545 (1.0); 10.2039 (0.7); 10.1633 (0.4); 8.6393 (9.3); 8.6178 (9.3); 8.2420 (11.9); 8.2390 (12.1); 7.6446 (0.6); 7.4312 (6.3); 7.3964 (6.3); 7.3646 (0.4); 7.2988 (99.9); 7.2328 (0.5); 6.9479 (0.6); 4.1979 (1.1); 4.1742 (3.5); 4.1503 (3.5); 4.1266 (1.3); 2.0852 (16.0); 1.5855 (12.4); 1.3225 (4.2); 1.2987 (8.4); 1.2749 (4.1); 0.2333 (0.6); 0.1075 (0.9); 0.0492 (4.4); 0.0384 (134.4); 0.0275 (4.7); -0.0279 (0.8); -0.1601 (0.5) |
| I-062: ¹H-NMR(499.9 MHz, d₆-DMSO): |
| δ= 13.5143 (6.2); 8.5996 (12.5); 8.5862 (12.5); 8.5037 (0.4); 8.4914 (0.4); 8.3091 (16.0); 8.2799 (0.6); 8.1882 (0.4); 8.1535 (0.4); 8.1395 (0.4); 7.6639 (0.5); 7.6394 (11.8); 7.6171 (11.8); 4.0536 (0.5); 4.0394 (1.4); 4.0251 (1.4); 4.0109 (0.4); 3.3310 (36.9); 2.5145 (5.8); 2.5109 (12.5); 2.5072 (17.3); 2.5036 (12.3); 2.4999 (5.5); 1.9915 (6.1); 1.3509 (0.5); 1.2260 (0.5); 1.1917 (1.7); 1.1775 (3.4); 1.1632 (1.7) |
| I-063: ¹H-NMR(499.9 MHz, d₆-DMSO): |
| δ= 13.4731 (5.0); 8.5694 (13.3); 8.5680 (14.3); 8.5666 (13.3); 8.2900 (11.0); 8.2878 (16.0); 8.2856 (11.0); 8.0131 (9.6); 8.0100 (9.4); 8.0025 (0.6); 7.9956 (11.0); 7.9925 (11.1); 7.7642 (11.8); 7.7467 (10.4); 3.3296 (45.8); 2.5145 (3.4); 2.5109 (7.3); 2.5072 (10.0); 2.5036 (7.2); 2.5000 (3.3); 1.3588 (1.5); 1.2256 (0.4) |
| I-064: ¹H-NMR(499.9 MHz, d₆-DMSO): |
| δ= 8.6158 (5.4); 8.6024 (5.5); 8.3561 (9.3); 8.3544 (9.0); 8.0160 (3.8); 7.9932 (3.9); 7.3568 (1.4); 7.3544 (2.2); 7.3513 (0.9); 7.3421 (4.0); 7.3399 (5.8); 7.3375 (4.3); 7.3304 (2.1); 7.3258 (7.8); 7.3163 (0.7); 7.3075 (10.0); 7.3011 (4.0); 7.2939 (4.7); 7.2905 (3.1); 7.2846 (3.4); 7.2788 (0.7); 7.2741 (0.6); 7.2708 (1.0); 7.2676 (0.5); 5.7002 (16.0); 3.3273 (19.5); 2.5171 (1.7); 2.5135 (3.6); 2.5099 (5.0); 2.5062 (3.6); 2.5026 (1.6) |
| I-065: ¹H-NMR(499.9 MHz, d₆-DMSO): |
| δ= 8.8112 (6.0); 8.8098 (6.0); 8.6761 (3.7); 8.6620 (3.8); 8.3321 (0.4); 8.3303 (0.4); 7.6603 (2.8); 7.6360 (2.8); 7.3955 (0.4); 7.3869 (12.2); 7.3780 (16.0); 7.3710 (0.8); 7.3675 (0.5); 7.3631 (0.5); 7.3603 (0.6); 7.3579 (0.6); 7.3501 (1.5); 7.3422 (2.0); 7.3393 (0.9); 7.3333 (1.8); 7.3299 (0.8); 7.3254 (0.9); 7.3223 (0.6); 7.3157 (0.6); 7.2810 (0.4); 5.7066 (12.9); 5.6827 (0.7); 3.3290 (25.9); 2.5172 (1.3); 2.5136 (2.7); 2.5099 (3.8); 2.5062 (2.7); 2.5026 (1.2) |
| I-066: ¹H-NMR(499.9 MHz, CDCl3): |
| δ= 8.5109 (4.5); 8.4974 (4.6); 8.0423 (7.3); 7.5108 (3.6); 7.4876 (3.6); 7.4165 (0.8); 7.4111 (1.1); 7.4078 (0.7); 7.3989 (4.6); 7.3958 (2.6); 7.3844 (7.2); 7.3784 (3.3); 7.3695 (2.0); 7.3575 (0.4); 7.3540 (0.5); 7.3347 (4.9); 7.3309 (4.9); 7.3189 (3.6); 7.2594 (9.0); 5.6051 (16.0); 1.5722 (4.0); 0.0063 (0.4); -0.0002 (11.2); -0.0066 (0.5) |

### BIOLOGICAL DATA

The term "active ingredient" as used herein designates a compound of formula (I) as disclosed in table 1.

### Example A: in vivo preventive test on Puccinia recondita (brown rust on wheat)

| | | |
|---|---|---|
| Solvent: | 5% | by volume of Dimethyl sulfoxide |
| | 10% | by volume of Acetone |
| Emulsifier: | 1µl | of Tween^{®} 80 per mg of active ingredient |

The active ingredients were made soluble and homogenized in a mixture of Dimethyl sulfoxide/Acetone/ /Tween^{®} 80 and then diluted in water to the desired concentration.

The young plants of wheat were treated by spraying the active ingredient prepared as described above. Control plants were treated only with an aqueous solution of Acetone/Dimethyl sulfoxide/ Tween^{®} 80.

After 24 hours, the plants were contaminated by spraying the leaves with an aqueous suspension of *Puccinia recondita* spores. The contaminated wheat plants were incubated for 24 hours at 20°C and at 100% relative humidity and then for 10 days at 20°C and at 70-80% relative humidity.

The test was evaluated 11 days after the inoculation. 0% means an efficacy which corresponds to that of the control plants while an efficacy of 100% means that no disease was observed.

In this test, the following compounds according to the invention and compounds used according to the invention showed efficacy between 70% and 79% at a concentration of 500 ppm of active ingredient: I-015; I-026; I-064.

In this test, the following compounds according to the invention and compounds used according to the invention showed efficacy between 80% and 89% at a concentration of 500 ppm of active ingredient: I-003; I-008; I-030; I-034; I-046; I-052; I-060; I-062.

In this test, the following compounds according to the invention and compounds used according to the invention showed efficacy between 90% and 100% at a concentration of 500 ppm of active ingredient: I-001; I-002; I-005; I-006; 1-012; I-014; I-017; I-031; I-032; I-033; I-038; 1-041; I-045; I-047; I-048; I-049; I-050; I-054; I-055; I-061; I-063.

### Example B : in vivo preventive test on Uromyces appendiculatus (bean rust)

| | | |
|---|---|---|
| Solvent: | 5% | by volume of Dimethyl sulfoxide |
| | 10% | by volume of Acetone |
| Emulsifier: | 1µl | of Tween^{®} 80 per mg of active ingredient |

The active ingredients were made soluble and homogenized in a mixture of Dimethyl sulfoxide/Acetone/ /Tween^{®} 80 and then diluted in water to the desired concentration.

The young plants of bean were treated by spraying the active ingredient prepared as described above. Control plants were treated only with an aqueous solution of Acetone/Dimethyl sulfoxide/ Tween^{®} 80.

After 24 hours, the plants were contaminated by spraying the leaves with an aqueous suspension of *Uromyces appendiculatus* spores. The contaminated bean plants were incubated for 24 hours at 20°C and at 100% relative humidity and then for 10 days at 20°C and at 70-80% relative humidity.

The test was evaluated 11 days after the inoculation. 0% means an efficacy which corresponds to that of the control plants while an efficacy of 100% means that no disease was observed.

In this test, the following compounds according to the invention showed efficacy between 70% and 79% at a concentration of 500 ppm of active ingredient: I-037; I-057.

In this test, the following compounds according to the invention showed efficacy between 80% and 89% at a concentration of 500 ppm of active ingredient: I-011; I-046; I-062; I-065.

In this test the following compounds according to the invention and compounds used according to the invention showed efficacy between 90% and 100% at a concentration of 500 ppm of active ingredient: I-001; I-002; I-003; I-004; I-005; I-006; I-008; I-010; I-012; I-013; I-014; I-015; I-016; I-017; I-026; I-030; I-031; I-032; I-033; I-034; I-038; I-041; I-042; I-045; I-047; I-048; I-049; I-050; I-051; I-052; I-054; I-055; I-060; I-061; I-063; I-064.

### Example C: in vivo preventive test on Phakospora pachyrhizi (soybean rust)

| | | |
|---|---|---|
| Solvent: | 5% | by volume of Dimethyl sulfoxide |
| | 10% | by volume of Acetone |
| Emulsifier: | 1µl | of Tween^{®} 80 per mg of active ingredient |

The active ingredients were made soluble and homogenized in a mixture of Dimethyl sulfoxide/Acetone/ /Tween^{®} 80 and then diluted in water to the desired concentration.

The young plants of soybean were treated by spraying the active ingredient prepared as described above. Control plants were treated only with an aqueous solution of Acetone/Dimethyl sulfoxide/ Tween^{®} 80.

After 24 hours, the plants were contaminated by spraying the leaves with an aqueous suspension of *Phakospora pachyrhizi* spores. The contaminated soybean plants were incubated for 24 hours at 24°C and at 100% relative humidity and then for 11 days at 24°C and at 70-80% relative humidity.

The test was evaluated 12 days after the inoculation. 0% means an efficacy which corresponds to that of the control plants while an efficacy of 100% means that no disease was observed.

In this test, the following compounds according to the invention showed efficacy between 70% and 79% at a concentration of 500 ppm of active ingredient: I-053.

In this test, the following compounds according to the invention and compounds used according to the invention showed efficacy between 90% and 100% at a concentration of 500 ppm of active ingredient: I-001; I-004; I-012; I-030; I-033; I-041; I-052; I-054; I-055; I-056; I-061; I-063; I-064; I-065; I-066.

### Example D: Fungicidal activity against Phakosporra pachirrhizi

Cryo-conserved wild-type spores of the biotroph *Phakosporra pachirrhizi* are humidified in dedicated chamber overnight at 18°C in the dark. The next day, a solution of spores at 7×10³ sp/ml is prepared in a water based growth medium (DH₂O + 0.2 mM MOPS at pH 7 + 0.01% Tween 20) and spores are distributed in a 96-MTPS (final volume of 200µL per well) thanks to a dispenser robot. Each molecule is tested at 10 doses (from 30 to 0.002 ppm final concentration) and accordingly 1.2 µL of each dilution is transferred in dedicated well to end-up with a final concentration of 0.6% DMSO. Wild-type spores and molecules are incubated for 4 hours at 21°C, and 6 images par well are then acquired with Transmitted Light images (Image Xpress Micro microscope, Molecular Devices, Objective 10X, 6 images per well). Detection and quantification of the number of germinated spores per image is performed with a dedicated in-house developed algorithm (MetaXpress software, Molecular Devices). Inhibition of the fungal germination was hence determined by comparing the number of germinated spores in wells containing the active ingredients to the ones without active ingredient.

In this test the following compounds according to the invention and compounds used according to the invention showed efficacy between 70% and 79% at a concentration of 30 ppm of active ingredient: I-004; I-011; I-014; I-016; I-017; I-020; I-021; I-022; I-027; I-040.

In this test, the following compounds according to the invention and compounds used according to the invention showed efficacy between 80% and 89% at a concentration of 30 ppm of active ingredient: I-013; I-019; I-030; I-038.

In this test, the following compounds according to the invention and compounds used according to the invention showed efficacy between 90% and 100% at a concentration of 30 ppm of active ingredient: I-002; I-003; I-005; I-006; I-010; I-015; I-024; I-025; I-029; I-031; I-032; I-033; I-041; I-042.

### Example E: Colletotrichum lindemuthianum in vitro cell test

| | |
|---|---|
| Solvent: | DMSO |
| Culture medium: | 14.6g anhydrous D-glucose (VWR), 7.1g Mycological Peptone (Oxoid), |
| | 1.4g granulated Yeast Extract (Merck), QSP 1liter |
| Inoculum: | spores suspension |

Active ingredients were solubilized in DMSO and the solution used to prepare the required range of concentrations. The final concentration of DMSO used in the assay was ≤ 1%.

A spore suspension of C. *lindemuthianum* was prepared and diluted to the desired spore density.

The active ingredients were evaluated for their ability to inhibit spores germination and mycelium growth in liquid culture assay. The compounds were added in the desired concentration to the culture medium with spores. After 6 days incubation, fungi-toxicity of compounds was determined by spectrometric measurement of mycelium growth. Inhibition of fungal growth was determined by comparing the absorbance values in wells containing the active ingredient with the absorbance in control wells without active ingredient.

In this test, the following compounds according to the invention and compounds used according to the invention showed efficacy between 70% and 79% at a concentration of 20 ppm of active ingredient: I-004; I-028; I-037; I-043; I-054; I-066.

In this test the following compounds according to the invention and compounds used according to the invention showed efficacy between 80% and 89% at a concentration of 20 ppm of active ingredient: I-003; I-008; I-009; I-012; I-013; I-014; I-017; I-019; I-021; I-024; I-025; I-026; I-029; I-030; I-033; I-038; I-041; I-046; I-050; I-055; I-059; I-060; I-064.

In this test the following compounds according to the invention and compounds used according to the invention showed efficacy between 90% and 100% at a concentration of 20 ppm of active ingredient: I-001; I-002; I-005; I-006; I-010; I-020; I-031; I-032; I-036; I-042; I-044; I-045; I-047; I-048; I-049; I-058; I-061; I-062; I-063.

### Example F: in vivo preventive test on Phakopsora test (soybeans)

| | | |
|---|---|---|
| Solvent: | 24.5 | parts by weight of acetone |
| | 24.5 | parts by weight of dimethylacetamide |
| Emulsifier: | 1 | part by weight of alkylaryl polyglycol ether |

To produce a suitable preparation of active ingredient, 1 part by weight of active ingredient was mixed with the stated amounts of solvent and emulsifier, and the concentrate was diluted with water to the desired concentration.

To test for preventive activity, young plants were sprayed with the preparation of active ingredient at the stated rate of application. After the spray coating had dried on, the plants were inoculated with an aqueous spore suspension of the causal agent of soybean rust (***Phakopsora pachyrhizi***) and stay for 24h without light in an incubation cabinet at approximately 24°C and a relative atmospheric humidity of 95%.

The plants remained in the incubation cabinet at approximately 24°C and a relative atmospheric humidity of approximately 80 % and a day / night interval of 12h.

The test was evaluated 7 days after the inoculation. 0% means an efficacy which corresponds to that of the untreated control, while an efficacy of 100% means that no disease is observed.

In this test, the following compounds according to the invention showed efficacy between 80% and 89% at a concentration of 250 ppm of active ingredient: I-014; I-042.

In this test, the following compounds according to the invention and compounds used according to the invention showed efficacy between 90% and 100% at a concentration of 250 ppm of active ingredient: I-001; I-002; I-003; I-005; I-006; I-008; I-009; I-010; I-011; I-012; I-013; 1-015; I-016; I-017; I-026; I-030; I-031; I-032; I-034; I-038; I-041; I-045; I-047; I-048; I-049; I-050; I-052; I-055.

## Claims

1. A compound of the formula (I') or a salt, N-oxide or solvate thereof: 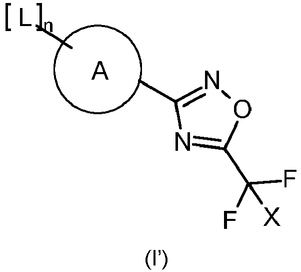 wherein
**X** is F or CI;
**A** is an aromatic or partially unsaturated fused bicyclic C₉-C₁₀-carbocyclyl ring or an aromatic or partially unsaturated fused bicyclic 9- or 10-membered heterocyclyl ring selected from the group consisting of 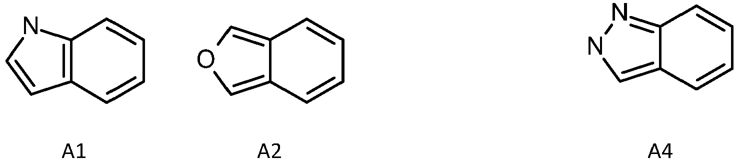 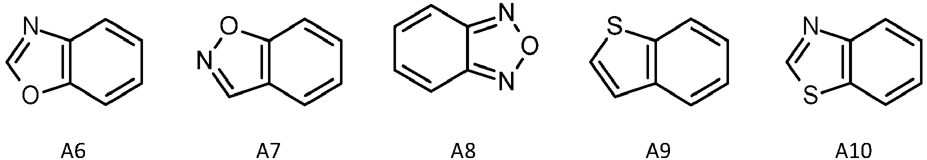 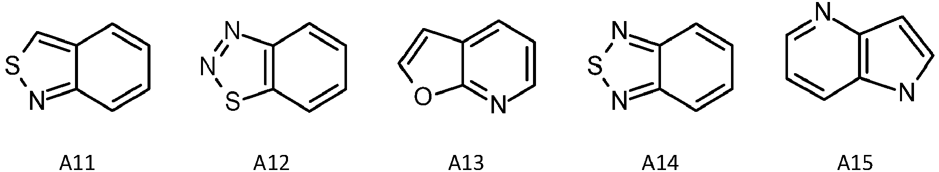 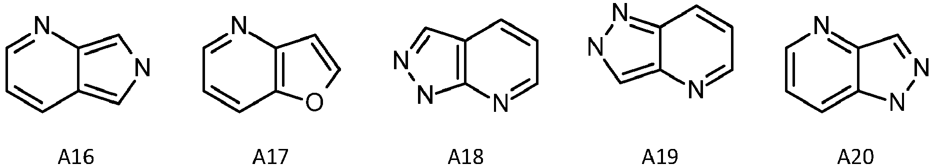 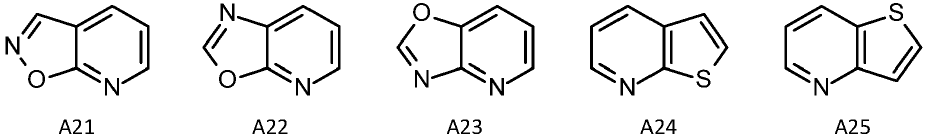 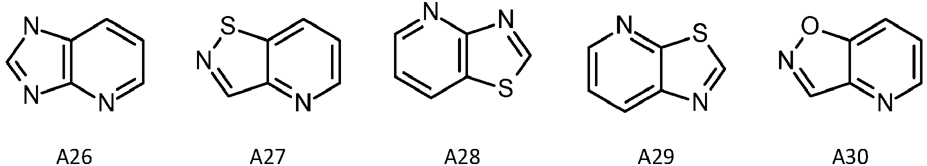 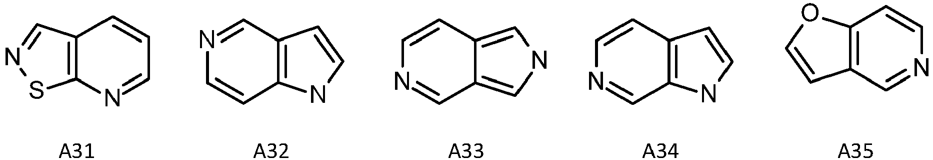 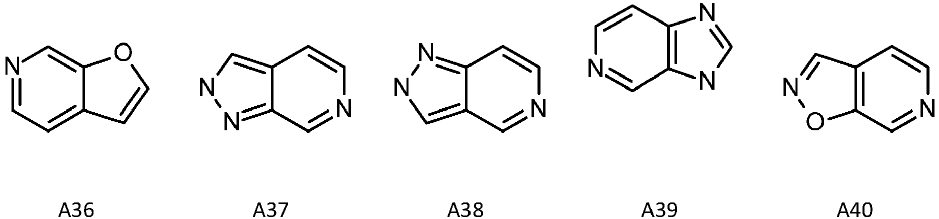 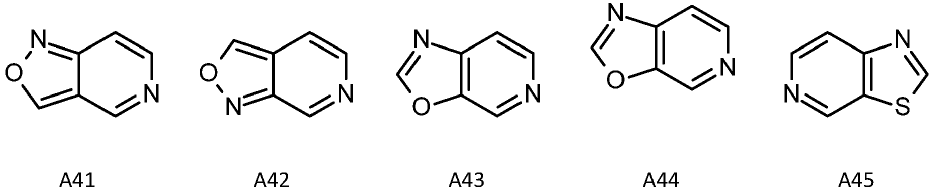 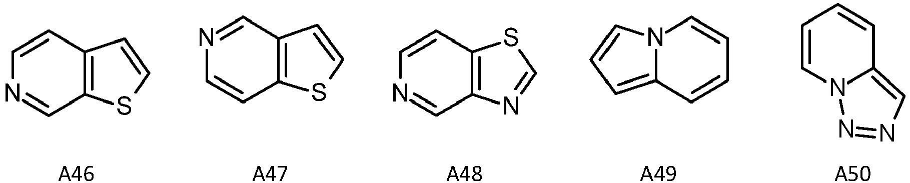 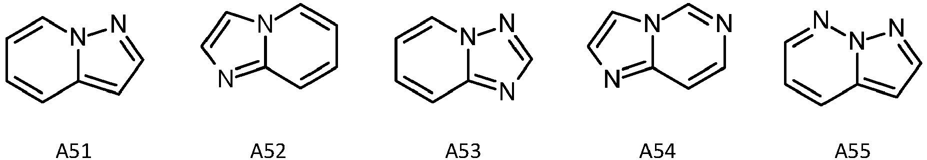 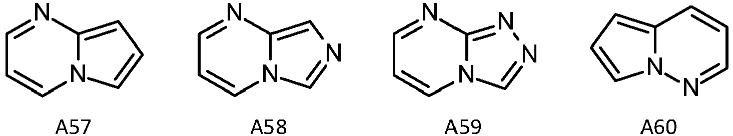 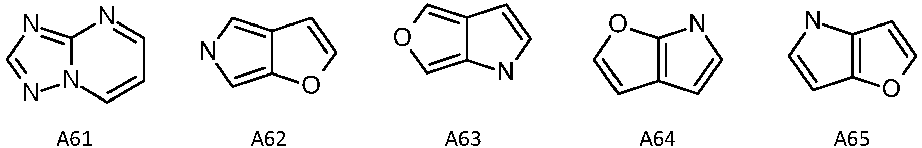 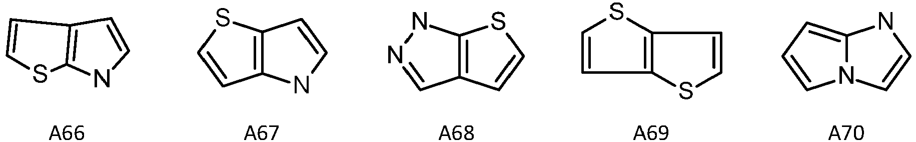 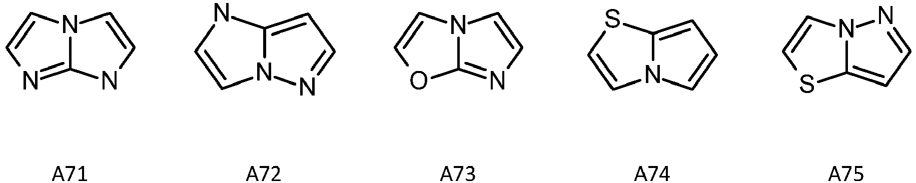 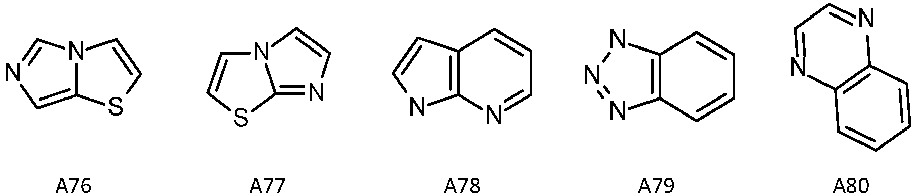 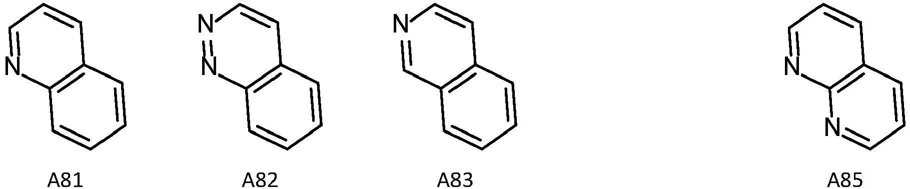 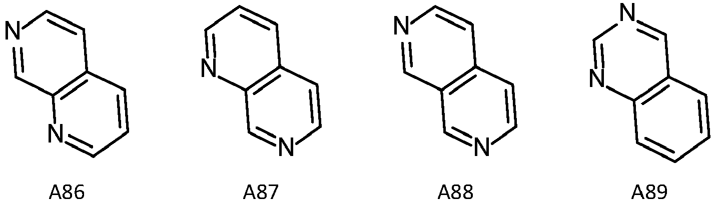 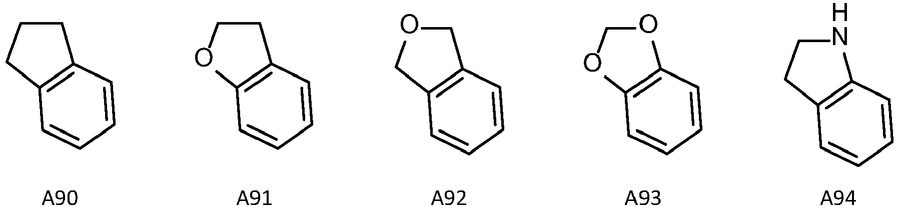 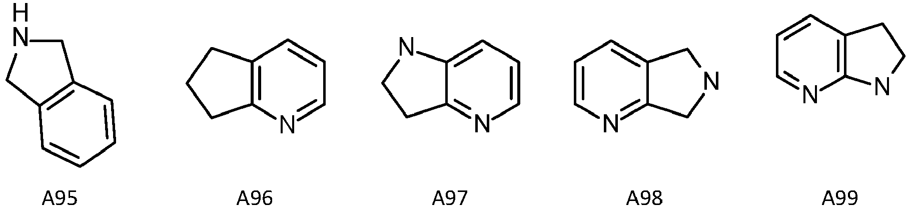 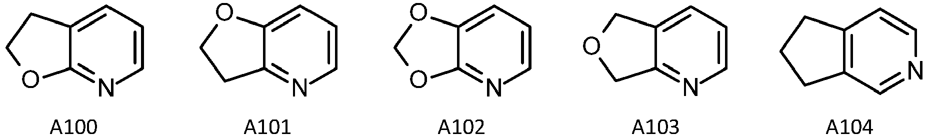 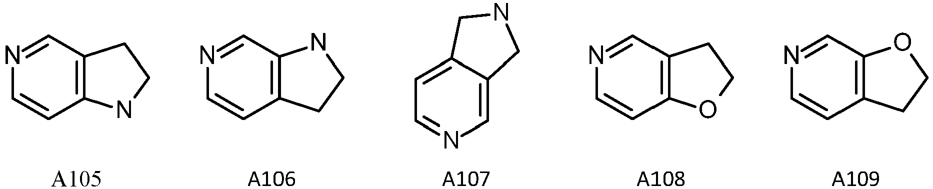 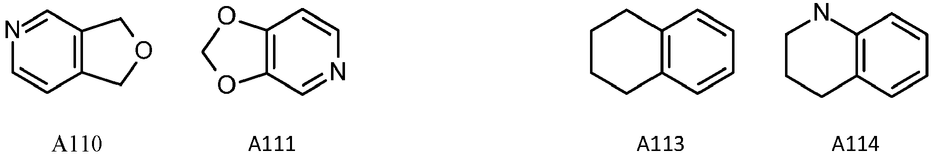 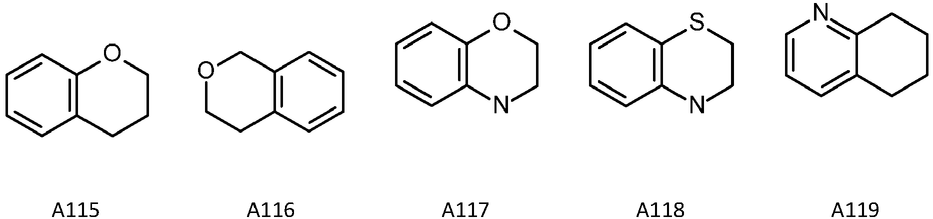 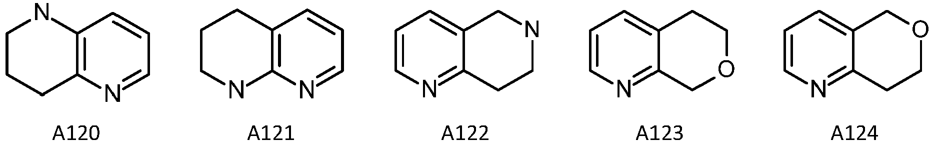 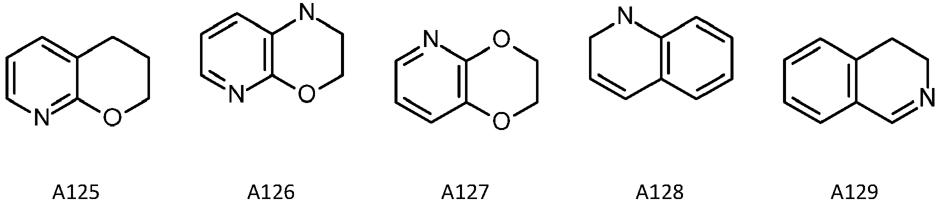 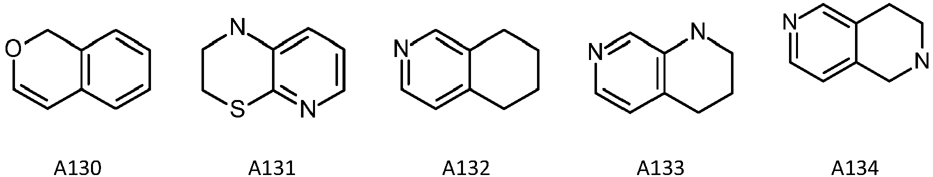 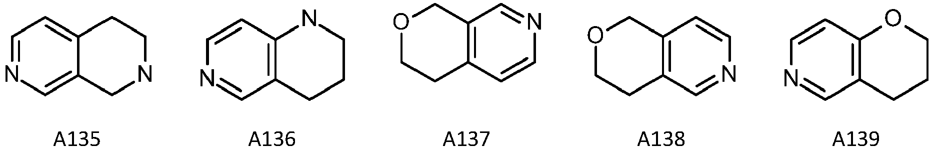 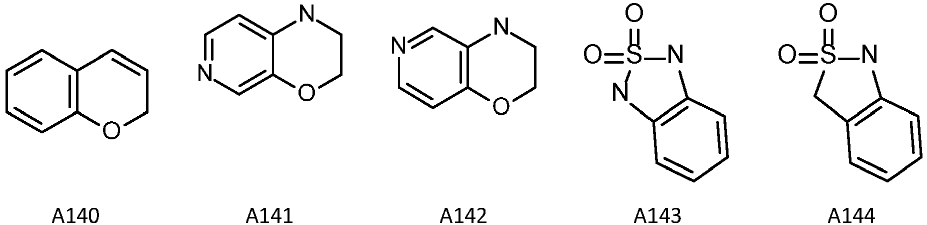 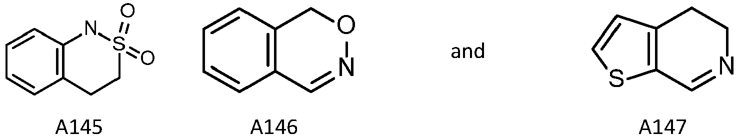 wherein, when **n** is 1, said C₉-C₁₀-carbocyclyl ring or 9- or 10-membered heterocyclyl ring may be substituted with up to four substituents in addition to **L,** said substituents being independently selected from the group consisting of halogen and C₁-C₃-alkyl;
**n** is 0 or 1;
**L** is a substituent selected from the group consisting of halogen, cyano, C₁-C₆-alkyl, C₁-C₆-haloalkyl, C₁-C₆-alkoxy, C₁-C₆-alkylthio, arylsulfenyl, C₁-C₆-alkylsulfinyl, arylsulfinyl, C₁-C₆-alkylsulfonyl, arylsulfonyl, C₃-C₁₀-carbocyclyl, 3- to 10-membered-heterocyclyl, aryl, heteroaryl, =N(OR^{a}), N(OR^{a})C(=O)OR^{a}, N(OR^{a})C(=O)R^{a}, -SO₂R^{a}, -SO₂N(R^{a})₂, -OC(=O)R^{a}, -N(R^{a})₂, - NR^{a}C(=O)OR^{a}, -NR^{a}C(=O)N(R^{a})₂, -NR^{a}C(=O)R^{a}, -NR^{a}C(=S)R^{a}, -NR^{a}C(=S)N(R^{a})₂, -NR^{a}C(=NR^{a})R^{a}, - N=C-N(R^{a})₂, -NR^{a}S(=O)₂R^{a}, -OC(=O)N(R^{a})₂, -C(=NR^{a})R^{a}, -C(=O)R^{a}, -C(=O)(OR^{a}), -C(=O)N(R^{a})₂, - C(=O)NR^{a}N(R^{a})₂, -C(=O)N(OR^{a})R^{a}, -C=NOR^{a}, -C(=S)N(R^{a})₂, , -C₁-C₆-alkyl-N(R^{a})₂, -C₁-C₆-alkyl-C(=O)N(R^{a})₂, -C₁-C₆-alkyl-NR^{a}C(=O)OR^{a}, -C₁-C₆-alkyl-NR^{a}C(=O)R^{a}, -C₁-C₆-alkyl-NR^{a}C(=O)N(R^{a})₂, - C₁-C₆-alkyl-N(OR^{a})C(=O)R^{a}, -C₁-C₆-alkyl-C(=O)N(OR^{a})R^{a}, -C₁-C₆-alkyl-NR^{a}C(=S)N(R^{a})₂, -C₁-C₆-alkyl-NR^{a}C(=O)R^{a} and -C₁-C₆-alkyl-NR^{a}S(=O)₂R^{a}, wherein said C₁-C₆-alkyl, C₁-C₆-haloalkyl, C₁-C₆-alkoxy, C₁-C₆-alkylthio, arylsulfenyl, C₁-C₆-alkylsulfinyl, arylsulfinyl, C₁-C₆-alkylsulfonyl, arylsulfonyl, C₃-C₁₀-carbocyclyl, 3- to 10-membered-heterocyclyl, aryl, heteroaryl, -C₁-C₆-alkyl-N(R^{a})₂, -C₁-C₆-alkyl-C(=O)N(R^{a})₂, -C₁-C₆-alkyl-NR^{a}C(=O)OR^{a}, -C₁-C₆-alkyl-NR^{a}C(=O)R^{a}, -C₁-C₆-alkyl-NR^{a}C(=O)N(R^{a})₂, - C₁-C₆-alkyl-N(OR^{a})C(=O)R^{a}, -C₁-C₆-alkyl-C(=O)N(OR^{a})R^{a}, -C₁-C₆-alkyl-NR^{a}C(=S)N(R^{a})₂, -C₁-C₆-alkyl-NR^{a}C(=O)R^{a} and -C₁-C₆-alkyl-NR^{a}S(=O)₂R^{a} substituent is itself optionally substituted, one or more times, in the same way or differently, with R5;
**R5** is a substituent selected from the group consisting of halogen, cyano, hydroxy, mercapto, sulfinyl, sulfonyl, amino, C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy, nitro, - C(=O)R^{a}, -C(=O)OR^{a}, -C(=O)N(R^{a})₂, -C(=S)N(R^{a})₂, -C(=NR^{a})R^{a}, -NR^{a}C(=O)OR^{a}, -NR^{a}C(=O)N(R^{a})₂, - NR^{a}C(=O)R^{a}, -NR^{a}C(=S)R^{a}, -NR^{a}C(=S)N(R^{a})₂, -NR^{a}C(=NR^{a})R^{a}, -OC(=O)R^{a}, -OC(=O)N(R^{a})₂, - NR^{a}S(=O)₂R^{a}, -S(=O)₂R^{a}, -S(=O)₂N(R^{a})₂ and -P(=O)(OR^{a})₂; wherein said C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b} ; when two R5 substituents are bound to a common carbon, they may form together C=O, C₃-C₁₀-carbocyclyl or 3- to 10-membered-heterocyclyl;
R^{a} is, independently from each other, a substituent, which is identical or different, selected from the group consisting of hydrogen, halogen, cyano, hydroxy, mercapto, sulfinyl, sulfonyl, amino, C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy, nitro, -C₁-C₆-alkyl-C₃-C₁₀-carbocyclyl, -C₁-C₆-alkyl-aryl, -C₁-C₆-alkyl-C₁-C₆-alkoxy, -C(=O)R^{b}, -C(=O)OR^{b}, -C(=O)N(R^{b})₂, -C(=S)N(R^{b})₂, - NR^{b}C(=O)OR^{b}, -NR^{b}C(=O)N(R^{b})₂, -NR^{b}C(=O)R^{b}, -NR^{b}C(=S)R^{b}, -OC(=O)N(R^{b})₂, -NR^{b}S(=O)₂R^{b}, - S(=O)₂R^{b}, -S(=O)₂N(R^{b})₂ and -P(=O)(OR^{b})₂ ; wherein said C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b};
R^{b} is, independently from each other, a substituent, which is identical or different, selected from the group consisting of hydrogen, halogen, cyano, -OR^{c}, -N(R^{c})₂, -SR^{c}, -S(=O)R^{c}, -S(=O)OR^{c}, - S(=O)₂R^{c}, -S(=O)₂OR^{c}, C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy, nitro, - C(=O)R^{c}, -C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, - NR^{c}C(=S)R^{c}, -OC(=O)N(R^{c})₂, -NR^{c}S(=O)₂R^{c}, -S(=O)₂N(R^{c})₂ and -P(=O)(OR^{c})₂; wherein said C₃-C₁₀-carbocyclyl, 3- to 10-membered-heterocyclyl, aryl, heteroaryl substituent is itself optionally substituted, one or more times, in the same way or differently with CN, halogen, C₁-C₆-alkyl, C₁-C₆-haloalkyl or C₁-C₆-alkoxy;
R^{c} is, independently from each other, a substituent, which is identical or different, selected from the group consisting of hydrogen, aryl and C₁-C₆-alkyl,
provided that
(a) when **X** is F, **n** is 1 and **A** is 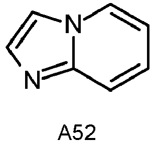 L is not a substituent selected from the group consisting of C₁-C₆-alkyl, aryl, 3- to 10-membered heterocyclyl and -C(=O)R^{a} with R^{a} being a 3- to 10-membered heterocyclyl,
(b) when **X** is F and n is 0, **A** is not selected from the group consisting of: 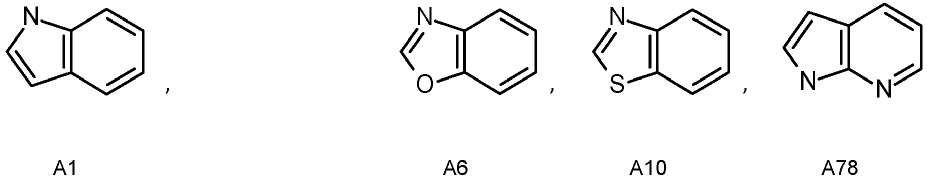 and 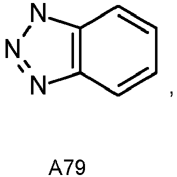
(c) when **X** is F, **n** is 1 and **A** is selected from the group consisting of 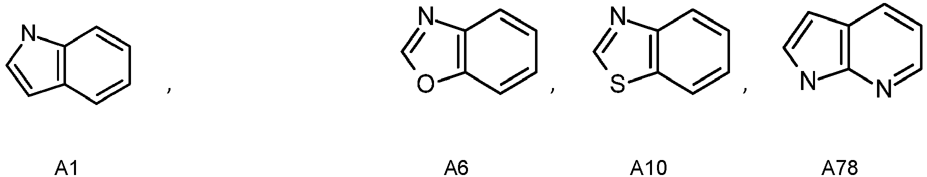 and 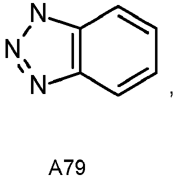 L is not a substituent selected from the group consisting of C₁-C₆-alkyl, -C(=O)(OR^{a}) with R^{a} being a C₁-C₆-alkyl and -SO₂R^{a} with R^{a} being an aryl,
(d) when **X** is F and n is 0, **A** is not 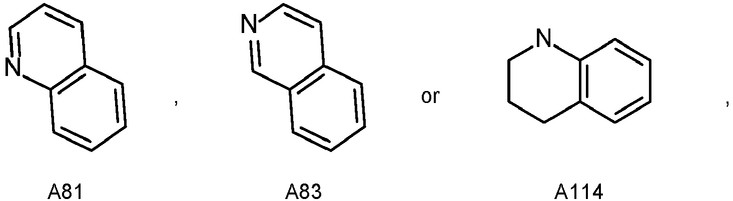
(e) when **X** is F, **n** is 1 and **A** is 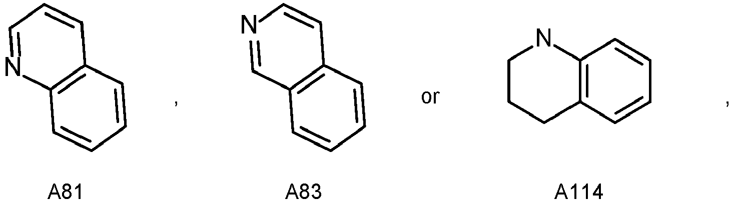 **L** is not a substituent selected from the group consisting of unsubstituted C₁-C₆-alkyl; C₁-C₆-alkyl substituted by one or more **R5** with **R5** being selected from the group consisting of unsubtituted aryl, unsubstituted heteroaryl, aryl substituted with one to three Rb with Rb being unsubstituted C₁-C₆-alkyl, and heteroaryl substituted with one to three Rb with Rb being unsubstituted C₁-C₆-alkyl; C₁-C₆-haloalkyl; halogen; cyano; -SO₂R^{a} with R^{a} being an unsubstituted C₁-C₆-alkyl; unsubstituted C₃-C₁₀-carbocyclyl; unsubstituted aryl; unsubstituted hereroaryl; aryl substituted by one or three **R5** with **R5** being an unsubstituted C₁-C₆-alkyl; and heteroaryl substituted by one or three **R5** with **R5** being an unsubstituted C₁-C₆-alkyl,
(f) when L is -N(Ra)₂ with the two Ra being hydrogen, L is not attached to the atom of **A** that bonds **A** to the oxadiazole moiety,
(g) compound of formula (I') is not
2-Pyridinecarboxamide,N-methyl-4-[[3-methyl-2-[5-(trifluoromethyl)-1,2,4-oxadiazol-3-yl]benzo[b]thien-6-yl]oxy]- (CAS-2222313-38-2),
Benzothiazole, 6-methoxy-2-[5-(trifluoromethyl)-1,2,4-oxadiazol-3-yl]- (CAS-897805-25-3), Thieno[2,3-c]pyridine, 4-(4-chlorophenoxy)-2-[5-(trifluoromethyl)-1,2,4-oxadiazol-3-yl]- (CAS-251995-30-9),
Pyrazolo[1,5-a]pyrimidine, 3-[5-(trifluoromethyl)-1,2,4-oxadiazol-3-yl]-7-[3-(trifluoromethyl)phenyl]-(CAS-159224-00-7),
1H-Indole, 3-(2-chloro-3-thienyl)-5-[5-(trifluoromethyl)-1,2,4-oxadiazol-3-yl]- (CAS-2252389-71-0), Imidazo[1,2-a]pyridine, 2-(phenylmethyl)-7-[5-(trifluoromethyl)-1,2,4-oxadiazol-3-yl]- (CAS-2170611-35-3),
1H-Pyrrolo[2,3-b]pyridine, 5-[5-(trifluoromethyl)-1,2,4-oxadiazol-3-yl]- (CAS-2170611-21-7), 1H-Pyrrolo[2,3-b]pyridine, 1-(phenylsulfonyl)-5-[5-(trifluoromethyl)-1,2,4-oxadiazol-3-yl]- (CAS-2170611-01-3),
2,1,3-Benzoxadiazole, 4-[3-(4-morpholinylmethyl)-1-azetidinyl]-6-[5-(trifluoromethyl)-1,2,4-oxadiazol-3-yl]- (CAS-1807986-73-7),
Tricyclo[3.3.1,13,7]decan-1-ol, 4-[[5-[5-(trifluoromethyl)-1,2,4-oxadiazol-3-yl]-1H-pyrrolo[2,3-b]pyridin-4-yl]amino]- (CAS-944120-75-6),
1H-Inden-1-amine, 2,3-dihydro-5-[5-(trifluoromethyl)-1,2,4-oxadiazol-3-yl]-, (1S)- (CAS-916303-83-8), 1H-Inden-1-amine, 2,3-dihydro-5-[5-(trifluoromethyl)-1,2,4-oxadiazol-3-yl]-, hydrochloride (1:1), (1S)-(CAS-916210-97-4),
Carbamic acid, N-[(1S)-2,3-dihydro-5-[5-(trifluoromethyl)-1,2,4-oxadiazol-3-yl]-1H-inden-1-yl]-, 1,1-dimethylethyl ester (CAS-916209-96-6),
1H-Pyrrolo[2,3-b]pyridine, 2-[1-methyl-5-[5-(trifluoromethyl)-1,2,4-oxadiazol-3-yl]-1H-indol-3-yl]-( CAS-479551-10-5),
1H-Indole, 2,3-dihydro-7-methyl-1-[3-(3-methyl-5-isoxazolyl)propyl]-5-[5-(trifluoromethyl)-1,2,4-oxadiazol-3-yl]- (CAS-178396-27-5),
1H-Indole, 7-methyl-1-[5-(3-methyl-5-isoxazolyl)pentyl]-5-[5-(trifluoromethyl)-1,2,4-oxadiazol-3-yl]-(CAS-178396-24-2),
1H-Indole, 7-methyl-1-[4-(3-methyl-5-isoxazolyl)butyl]-5-[5-(trifluoromethyl)-1,2,4-oxadiazol-3-yl]-(CAS-178396-22-0),
1H-Indole, 1-[3-(3-methyl-5-isoxazolyl)propyl]-5-[5-(trifluoromethyl)-1,2,4-oxadiazol-3-yl]- (CAS-178396-20-8),
Pyrazino[2,3-c]pyridazine, 5-[(1R)-1-(2,5-dichlorophenyl)ethyl]-5,6,7,8-tetrahydro-3-[5-(trifluoromethyl)-1,2,4-oxadiazol-3-yl]- (CAS-1690421-46-5),
Pyrazino[2,3-c]pyridazine, 5-[(2,5-dichlorophenyl)methyl]-5,6,7,8-tetrahydro-3-[5-(trifluoromethyl)-1,2,4-oxadiazol-3-yl]- (CAS-1690421-40-9),
1,2,4-Oxadiazole, 3-[7-(bromomethyl)-2-naphthalenyl]-5-(trifluoromethyl)- (CAS-1172849-62-5), 1,6-Naphthyridine, 5,6,7,8-tetrahydro-3-[5-(trifluoromethyl)-1,2,4-oxadiazol-3-yl]-, 2,2,2-trifluoroacetate (1:1) (CAS-741736-97-0),
1,6-Naphthyridine, 5,6,7,8-tetrahydro-3-[5-(trifluoromethyl)-1,2,4-oxadiazol-3-yl]- (CAS-741736-96-9), 1-Butanone, 3-amino-4-(2,5-difluorophenyl)-1-[7,8-dihydro-3-[5-(trifluoromethyl)-1,2,4-oxadiazol-3-yl]-1,6-naphthyridin-6(5H)-yl]-, (3R)- (CAS-741736-75-4),
Phthalazine, 1-phenyl-4-[5-(trifluoromethyl)-1,2,4-oxadiazol-3-yl]- (CAS-863601-14-3).

2. The compound of formula (I') according to claim 1 wherein **A** is an aromatic fused bicyclic C₉-C₁₀-carbocyclyl ring or an aromatic fused bicyclic 9- or 10-membered heterocyclyl ring selected from the group consisting of A1, A2, A4, A6 to A55, A57 to A83, A85 to A89 as recited in claim 1.

3. The compound of formula (I') according to any one of the preceding claims wherein **A** is selected from the group consisting of A1, A4, A10, A52, A77, A79 and A83 as recited in claim 1.

4. The compound of formula (I') according to claim 1 wherein **A** is partially unsaturated fused bicyclic C₉-C₁₀-carbocyclyl ring or a partially unsaturated fused bicyclic 9- or 10-membered heterocyclyl ring selected from the group consisting of A90 to A111 and A113 to A147 as recited in claim 1.

5. The compound of formula (I') according to claim 1 or 4 wherein **A** is selected from the group consisting of A90, A91, A93, A113, A114 and A140 as recited in claim 1.

6. The compound of formula (I') according to any one of the preceding claims wherein **L** is selected from the group consisting of halogen, C₁-C₆-alkyl, C₁-C₆-alkoxy, C₁-C₆-alkylsulfonyl, aryl, - N(R^{a})₂, =N(OR^{a}), -N(OR^{a})C(=O)OR^{a}, -NR^{a}C(=O)OR^{a}, -N(OR^{a})C(=O)R^{a}, -NR^{a}C(=O)R^{a}, -OC(=O)N(R^{a})₂, -C(=O)R^{a}, -C(=O)(OR^{a}), -C(=O)N(R^{a})₂, wherein said C₁-C₆-alkyl, C₁-C₆-alkoxy, C₁-C₆-alkylsulfonyl and aryl substituent is itself optionally substituted, one or more times, in the same way or differently, with **R5** as recited in claim 1.

7. The compound of formula (I') according to claim 6 wherein **R5** is halogen, C₁-C₆-alkoxy or aryl.

8. A composition comprising one or more compounds of formula (I') according to any one of claims 1 to 7 and one or more acceptable carriers.

9. Non-therapeutic use of a compound of the formula (I) or a salt, N-oxide or solvate thereof for controlling phytopathogenic fungi: 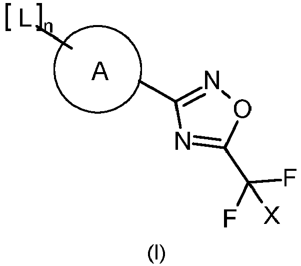 wherein
**X** is F or CI;
**A** is an aromatic or partially unsaturated fused bicyclic C₇-C₁₂-carbocyclyl ring or an aromatic or partially unsaturated fused bicyclic 7- to 12-membered heterocyclyl ring, wherein, when n is 1, said C₇-C₁₂-carbocyclyl or 7- to 12-membered heterocyclyl ring may be substituted with up to four substituents in addition to L, said substituents being independently selected from the group consisting of halogen and C₁-C₃-alkyl;
**n** is 0 or 1;
**L** is a substituent selected from the group consisting of halogen, cyano, C₁-C₆-alkyl, C₁-C₆-haloalkyl, C₁-C₆-alkoxy, C₁-C₆-alkylthio, arylsulfenyl, C₁-C₆-alkylsulfinyl, arylsulfinyl, C₁-C₆-alkylsulfonyl, arylsulfonyl, C₃-C₁₀-carbocyclyl, 3- to 10-membered-heterocyclyl, aryl, heteroaryl, =N(OR^{a}), N(OR^{a})C(=O)OR^{a}, N(OR^{a})C(=O)R^{a}, -SO₂R^{a}, -SO₂N(R^{a})₂, -OC(=O)R^{a}, -N(R^{a})₂, - NR^{a}C(=O)OR^{a}, -NR^{a}C(=O)N(R^{a})₂, -NR^{a}C(=O)R^{a}, -NR^{a}C(=S)R^{a}, -NR^{a}C(=S)N(R^{a})₂, -NR^{a}C(=NR^{a})R^{a}, - N=C-N(R^{a})₂, -NR^{a}S(=O)₂R^{a}, -OC(=O)N(R^{a})₂, -C(=NR^{a})R^{a}, -C(=O)R^{a}, -C(=O)(OR^{a}), -C(=O)N(R^{a})₂, - C(=O)NR^{a}N(R^{a})₂, -C(=O)N(OR^{a})R^{a}, -C=(NOR^{a})R^{a}, -C(=S)N(R^{a})₂, , -C₁-C₆-alkyl-N(R^{a})₂, -C₁-C₆-alkyl-C(=O)N(R^{a})₂, -C₁-C₆-alkyl-NR^{a}C(=O)OR^{a}, -C₁-C₆-alkyl-NR^{a}C(=O)R^{a}, -C₁-C₆-alkyl-NR^{a}C(=O)N(R^{a})₂, - C₁-C₆-alkyl-N(OR^{a})C(=O)R^{a}, -C₁-C₆-alkyl-C(=O)N(OR^{a})R^{a}, -C₁-C₆-alkyl-NR^{a}C(=S)N(R^{a})₂, -C₁-C₆-alkyl-NR^{a}C(=O)R^{a} and -C₁-C₆-alkyl-NR^{a}S(=O)₂R^{a}, wherein said C₁-C₆-alkyl, C₁-C₆-haloalkyl, C₁-C₆-alkoxy, C₁-C₆-alkylthio, arylsulfenyl, C₁-C₆-alkylsulfinyl, arylsulfinyl, C₁-C₆-alkylsulfonyl, arylsulfonyl, C₃-C₁₀-carbocyclyl, 3- to 10-membered-heterocyclyl, aryl, heteroaryl, -C₁-C₆-alkyl-N(R^{a})₂, -C₁-C₆-alkyl-C(=O)N(R^{a})₂, -C₁-C₆-alkyl-NR^{a}C(=O)OR^{a}, -C₁-C₆-alkyl-NR^{a}C(=O)R^{a}, -C₁-C₆-alkyl-NR^{a}C(=O)N(R^{a})₂, - C₁-C₆-alkyl-N(OR^{a})C(=O)R^{a}, -C₁-C₆-alkyl-C(=O)N(OR^{a})R^{a}, -C₁-C₆-alkyl-NR^{a}C(=S)N(R^{a})₂, -C₁-C₆-alkyl-NR^{a}C(=O)R^{a} and -C₁-C₆-alkyl-NR^{a}S(=O)₂R^{a} substituent is itself optionally substituted, one or more times, in the same way or differently, with R5;
**R5** is a substituent selected from the group consisting of halogen, cyano, hydroxy, mercapto, sulfinyl, sulfonyl, amino, C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy, nitro, - C(=O)R^{a}, -C(=O)OR^{a}, -C(=O)N(R^{a})₂, -C(=S)N(R^{a})₂, -C(=NR^{a})R^{a}, -NR^{a}C(=O)OR^{a}, -NR^{a}C(=O)N(R^{a})₂, - NR^{a}C(=O)R^{a}, -NR^{a}C(=S)R^{a}, -NR^{a}C(=S)N(R^{a})₂, -NR^{a}C(=NR^{a})R^{a}, -OC(=O)R^{a}, -OC(=O)N(R^{a})₂, - NR^{a}S(=O)₂R^{a}, -S(=O)₂R^{a}, -S(=O)₂N(R^{a})₂ and -P(=O)(OR^{a})₂; wherein said C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b} ; when two R5 substituents are bound to a common carbon, they may form together C=O, C₃-C₁₀-carbocyclyl or 3- to 10-membered-heterocyclyl;
R^{a} is, independently from each other, a substituent, which is identical or different, selected from the group consisting of hydrogen, halogen, cyano, hydroxy, mercapto, sulfinyl, sulfonyl, amino, C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy, nitro, -C₁-C₆-alkyl-C₃-C₁₀-carbocyclyl, -C₁-C₆-alkyl-aryl, -C₁-C₆-alkyl-C₁-C₆-alkoxy, -C(=O)R^{b}, -C(=O)OR^{b}, -C(=O)N(R^{b})₂, -C(=S)N(R^{b})₂, - NR^{b}C(=O)OR^{b}, -NR^{b}C(=O)N(R^{b})₂, -NR^{b}C(=O)R^{b}, -NR^{b}C(=S)R^{b}, -OC(=O)N(R^{b})₂, -NR^{b}S(=O)₂R^{b}, - S(=O)₂R^{b}, -S(=O)₂N(R^{b})₂ and -P(=O)(OR^{b})₂ ; wherein said C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy substituent is itself optionally substituted, one or more times, in the same way or differently with R^{b};
R^{b} is, independently from each other, a substituent, which is identical or different, selected from the group consisting of hydrogen, halogen, cyano, -OR^{c}, -N(R^{c})₂, -SR^{c}, -S(=O)R^{c}, -S(=O)OR^{c}, - S(=O)₂R^{c}, -S(=O)₂OR^{c}, C₁-C₆-alkyl, C₁-C₆-alkoxy, C₁-C₆-alkylthio, C₁-C₆-alkylsulfinyl, C₁-C₆-alkylsulfonyl, C₁-C₆-alkylamino, C₂-C₆-alkenyl, C₂-C₆-alkynyl, C₃-C₁₀-carbocyclyl, 3- to 10-membered-heterocyclyl, C₁-C₆-haloalkyl, hydroxy-C₁-C₆-alkyl, aryl, aryloxy, heteroaryl, heteroaryloxy, nitro, - C(=O)R^{c}, -C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, - NR^{c}C(=S)R^{c}, -OC(=O)N(R^{c})₂, -NR^{c}S(=O)₂R^{c} -S(=O)₂N(R^{c})₂ and -P(=O)(OR^{c})₂ ; wherein said C₃-C₁₀-carbocyclyl, 3- to 10-membered-heterocyclyl, aryl, heteroaryl substituent is itself optionally substituted, one or more times, in the same way or differently with CN, halogen, C₁-C₆-alkyl, C₁-C₆-haloalkyl or C₁-C₆-alkoxy;
R^{c} is, independently from each other, a substituent, which is identical or different, selected from the group consisting of hydrogen, aryl and C₁-C₆-alkyl,
provided that when A is naphthyl, n is 1 and X is F, L is not a substituent selected from the group consisting of
- C₁-C₆-alkyl substituted with one or more R5, wherein at least one of said R5 is selected from the group consisting of C₁-C₆-alkoxy, aryl, heteroaryl, C₃-C₁₀-carbocyclyl, 3- to 10-membered heterocyclyl, aryloxy and heteroaryloxy,
- C₁-C₆-alkoxy substituted with or more R5, wherein at least one of said R5 is selected from the group consisting of aryl, heteroaryl, C₃-C₁₀-carbocyclyl, 3- to 10-membered heterocyclyl, aryloxy and heteroaryloxy,
- -N(R^{a})₂ wherein at least one R^{a} is aryl, heteroaryl, C₃-C₁₀-carbocyclyl or 3- to 10-membered heterocyclyl,
- N(R^{a})₂ wherein at least one R^{a} is C₁-C₆-alkyl substituted with one or more R^{b}, at least one of said R^{b} being an aryl, heteroaryl, C₃-C₁₀-carbocyclyl or 3- to 10-membered heterocyclyl,
- C₁-C₆-alkyl-N(R^{a})₂ wherein at least one R^{a} is aryl, heteroaryl, C₃-C₁₀-carbocyclyl or 3-to 10-membered heterocyclyl,
- C₃-C₁₀-carbocyclyl,
- 3- to 10-membered heterocyclyl and
- heteroaryl.

10. Non-therapeutic use of a compound of formula (I) for controlling phytopathogenic fungi according to claim 9 wherein A is selected from the group consisting of

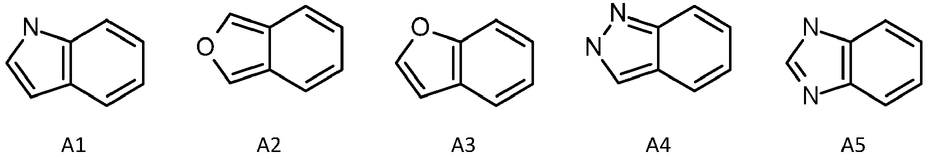

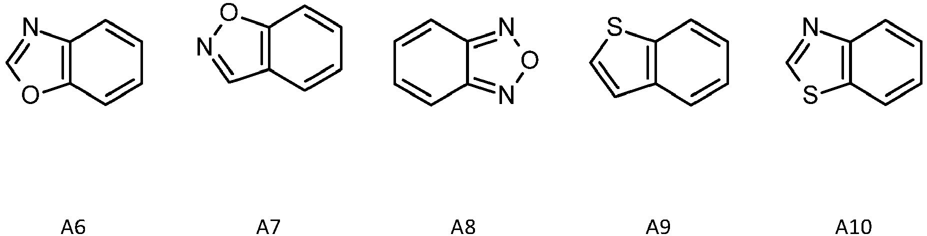

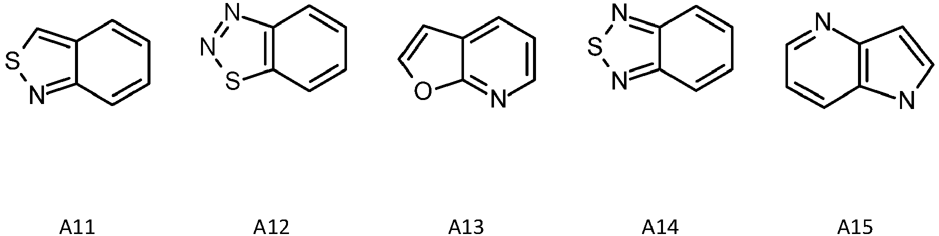

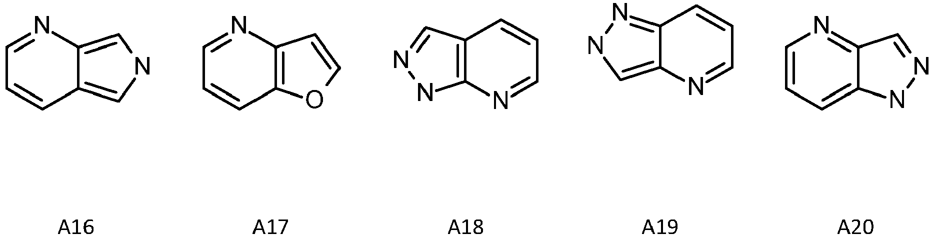

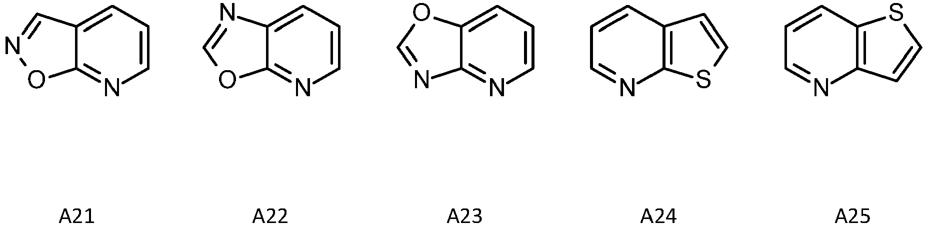

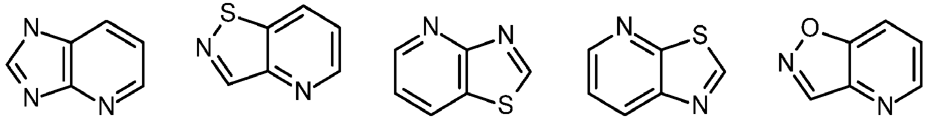

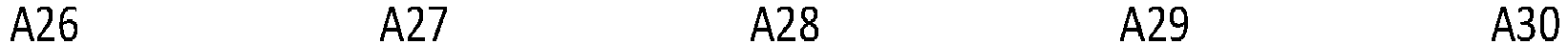

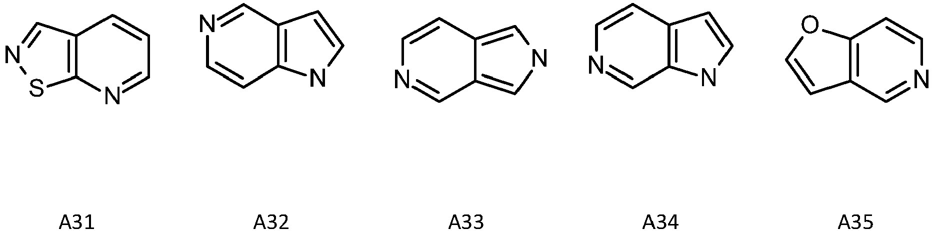

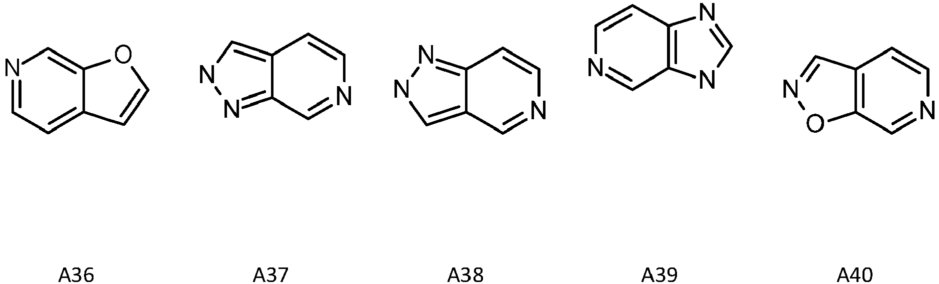

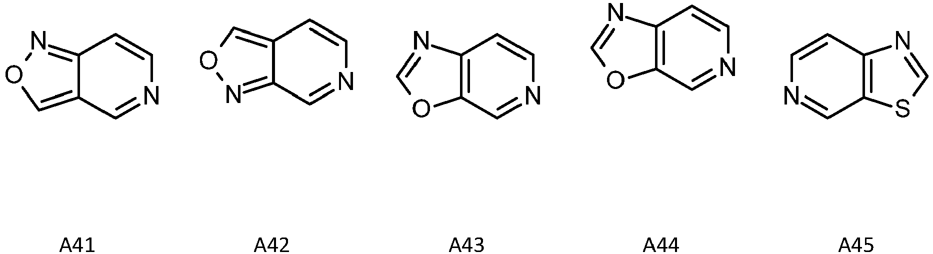

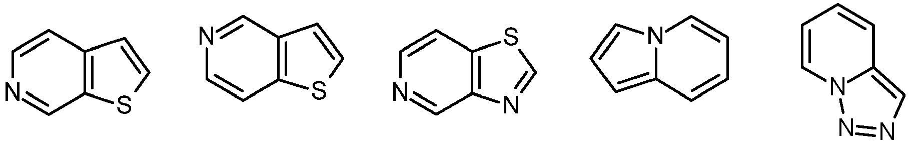

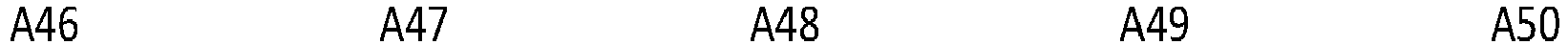

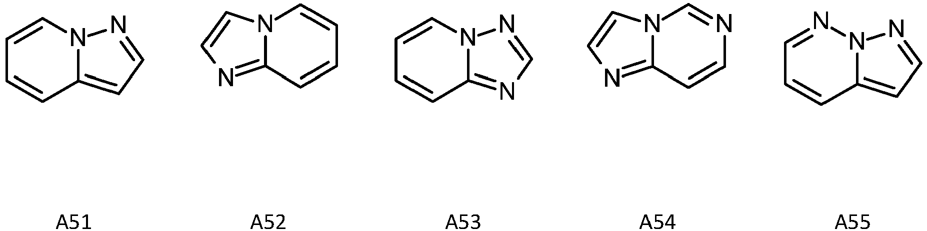

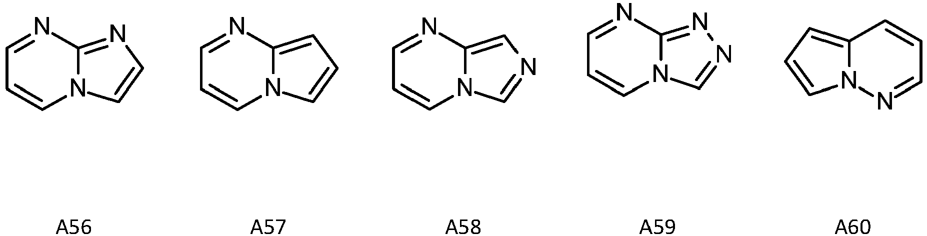

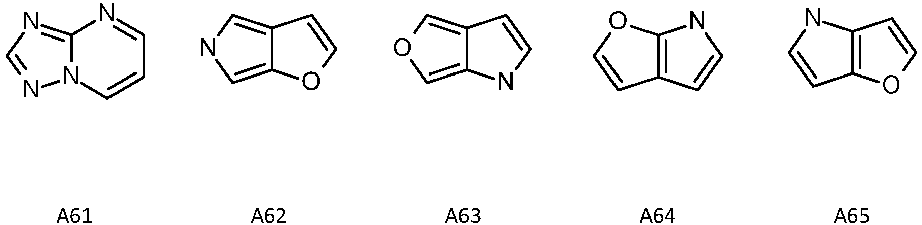

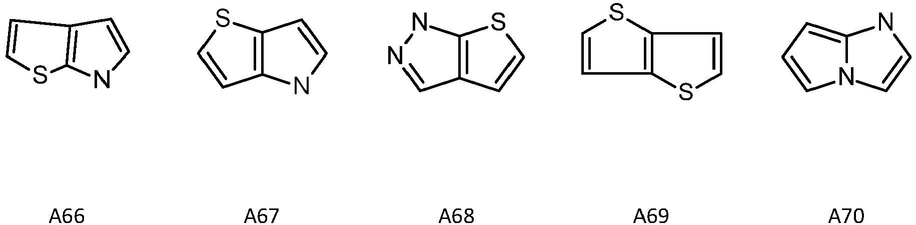

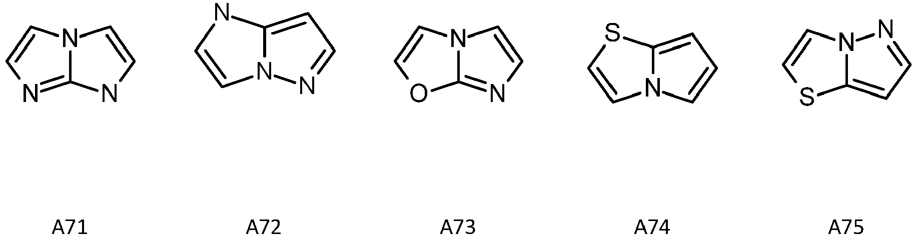

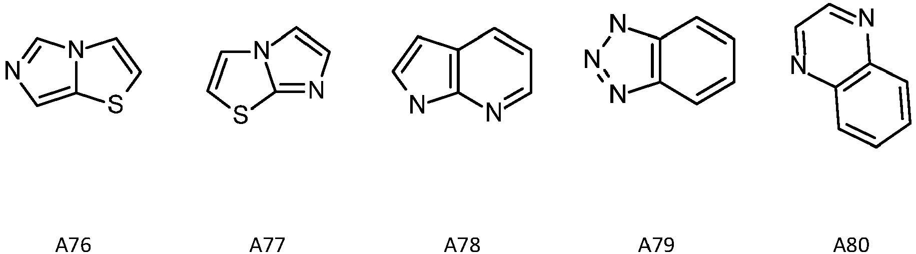

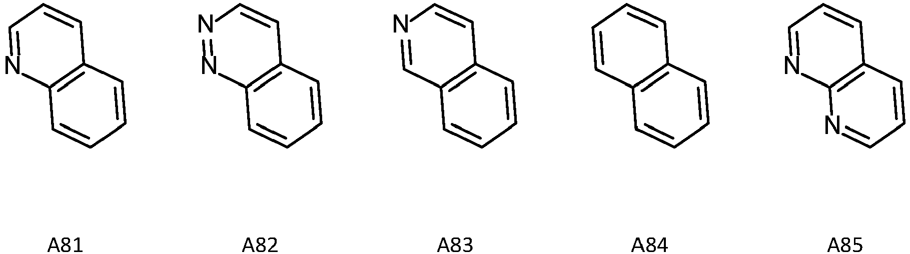

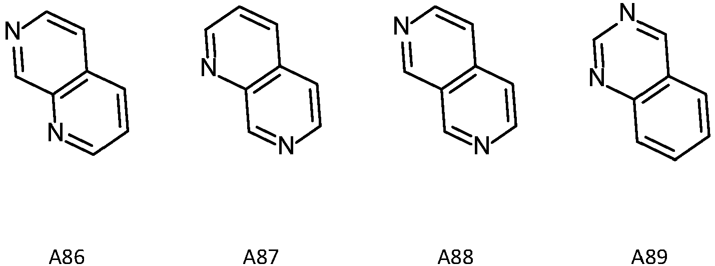

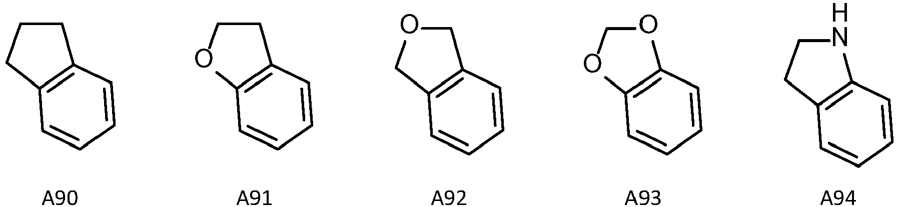

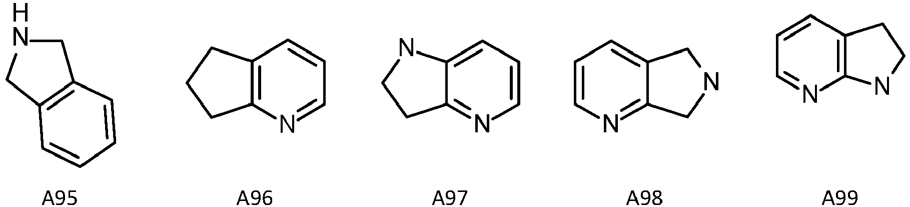

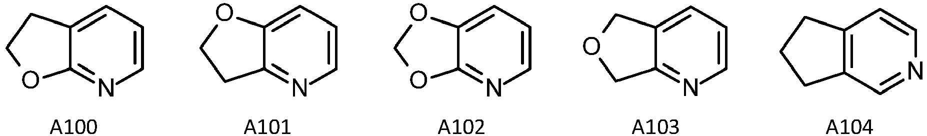

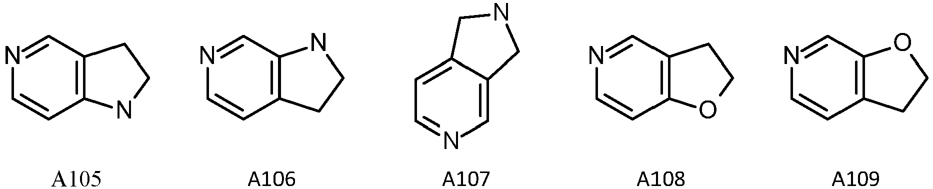

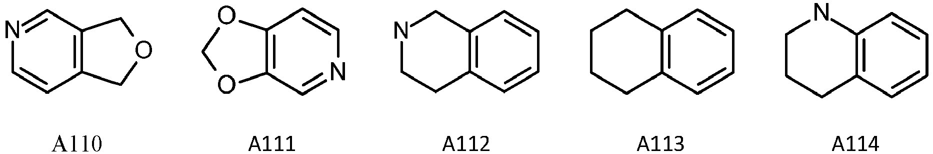

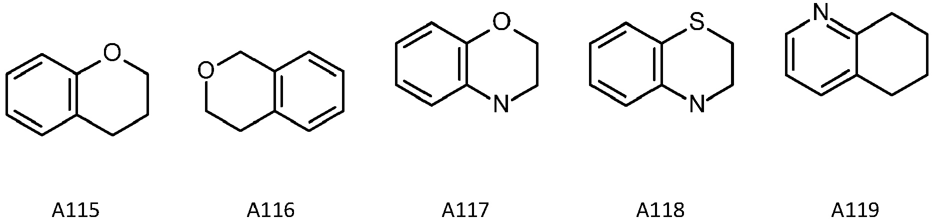

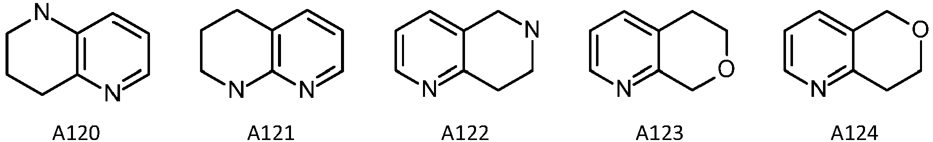

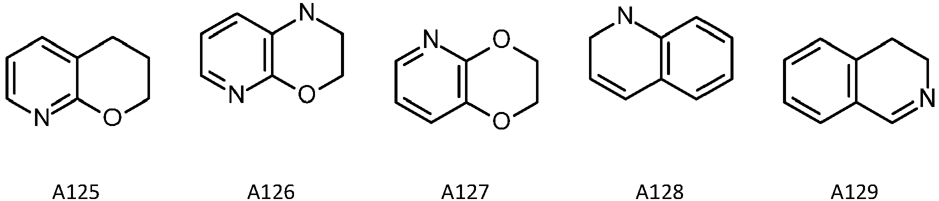

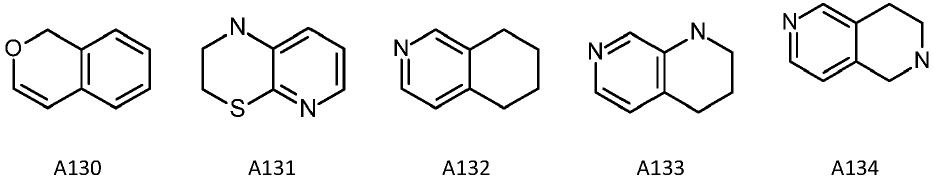

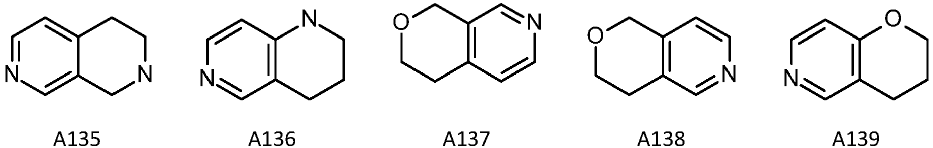

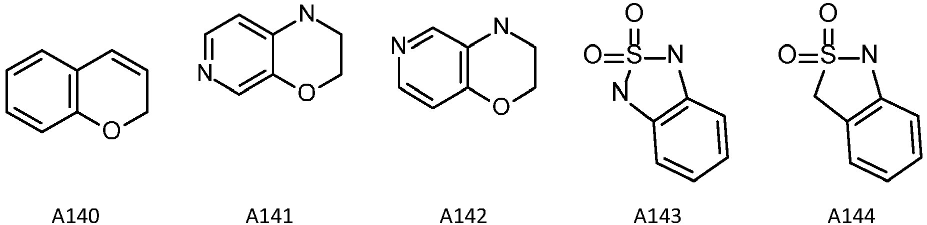

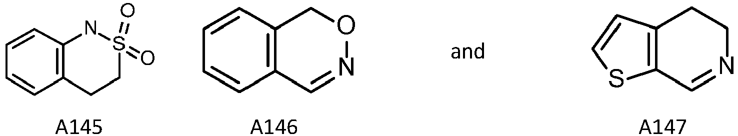

11. A method for controlling unwanted phytopathogenic microorganisms which comprises the step of applying one or more compounds of formula (I) as recited in claim 9 or 10 to the plants, plant parts, seeds, fruits or to the soil in which the plants grow.

12. A process for preparing a compound of formula (I') according to any one of claims 1 to 7, wherein n is 1, which comprises the step of reacting amidoximes of formula (II) with difluorohaloalkylacetic anhydride or difluorohaloalkylacetyl chloride in a solvent to give a compound of formula (I): 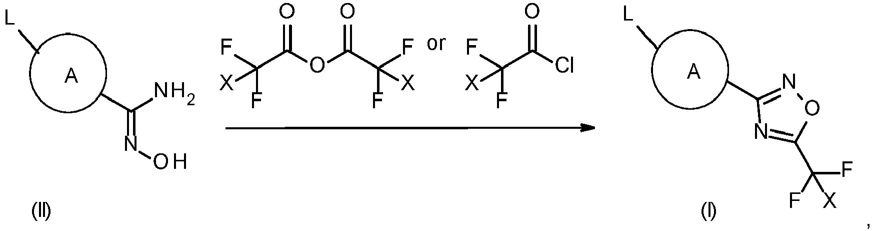 wherein formula (I) corresponds to formula (I'), wherein n is 1, and wherein L, A and X in formulae (II) and (I) are as defined in the compound of formula (I').

## Patentansprüche

1. Verbindung der Formel (I') oder ein Salz, N-Oxid oder Solvat davon: 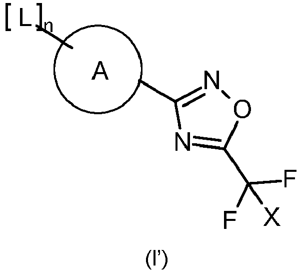 wobei
**X** für F oder Cl steht;
**A** für einen aromatischen oder teilweise ungesättigten anellierten bicyclischen C₉-C₁₀-Carbocyclylring oder einen aromatischen oder teilweise ungesättigten anellierten bicyclischen 9- oder 10-gliedrigen Heterocyclylring aus der Gruppe bestehend aus 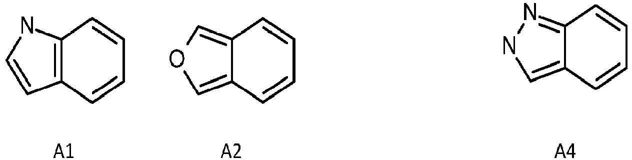 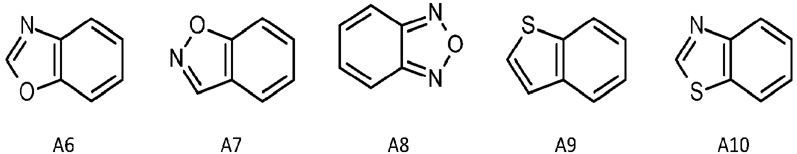 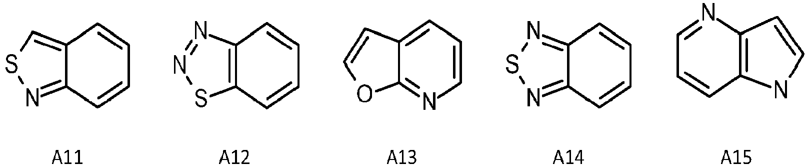 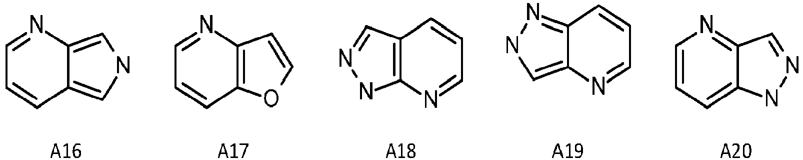 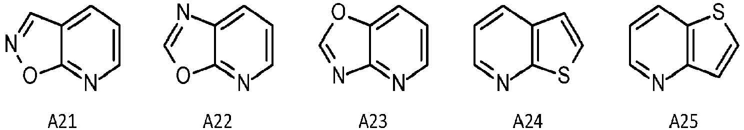 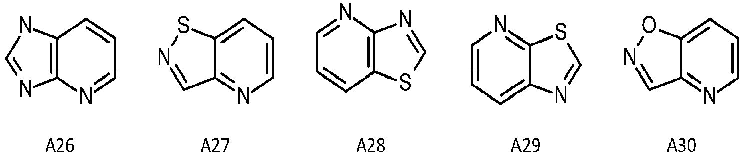 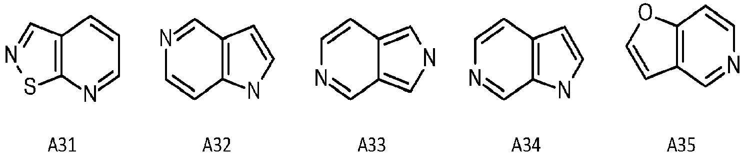 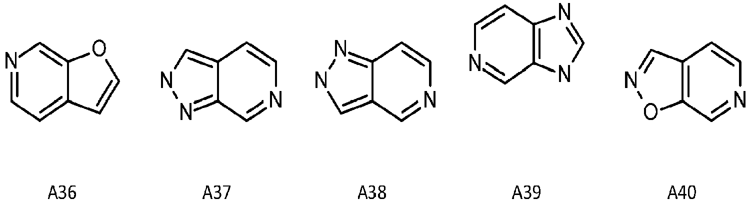 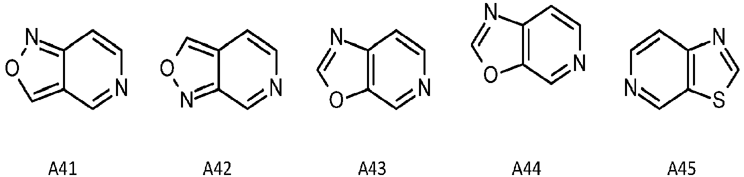 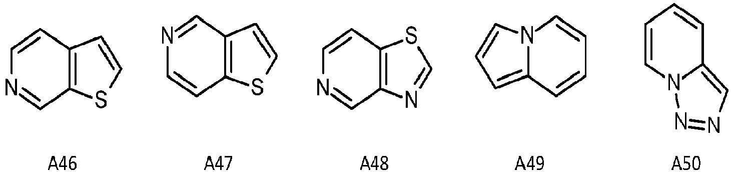 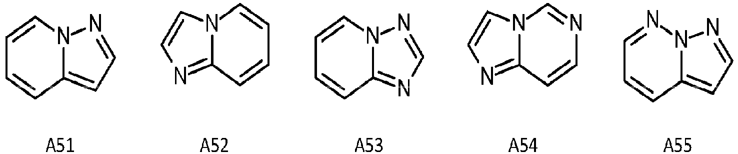 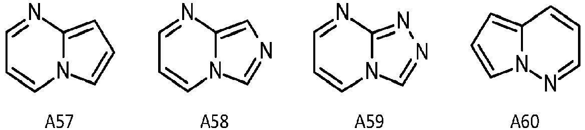 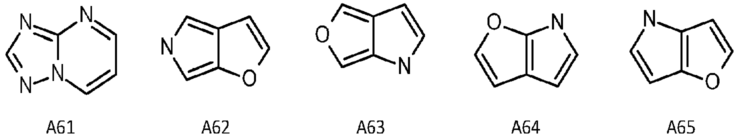 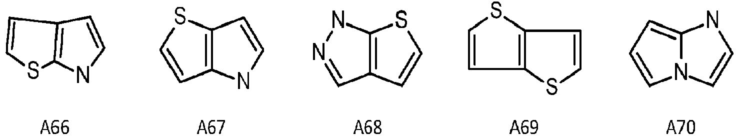 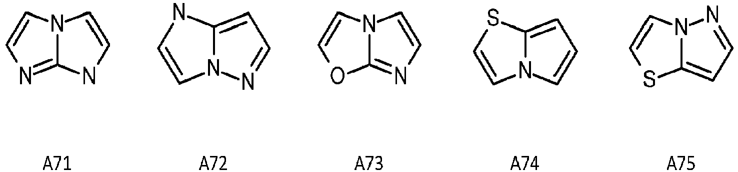 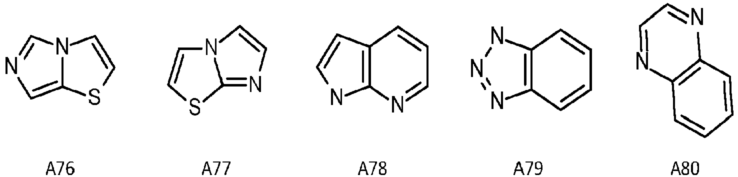 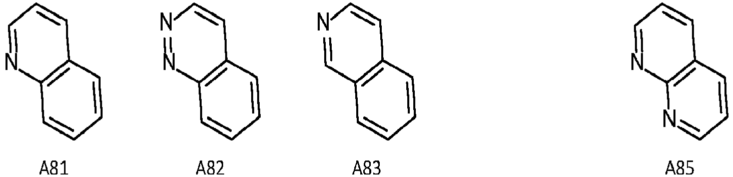 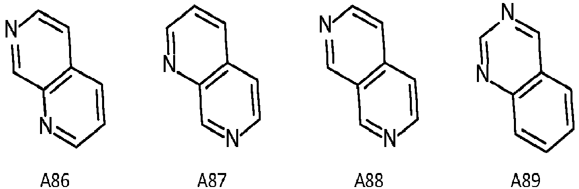 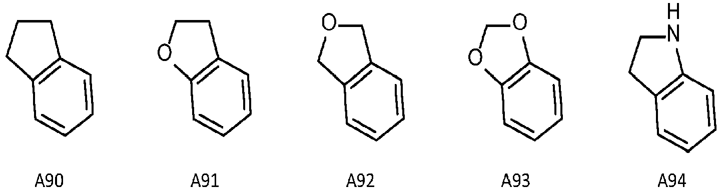 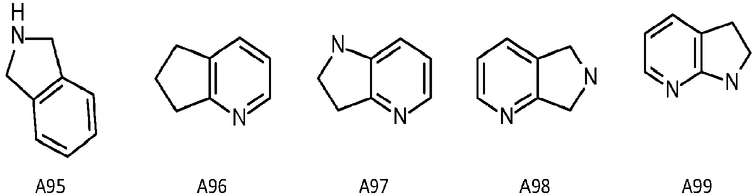 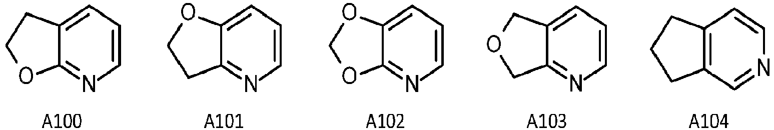 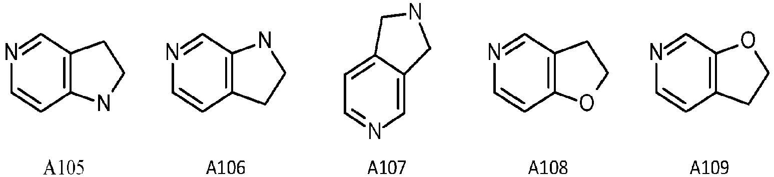 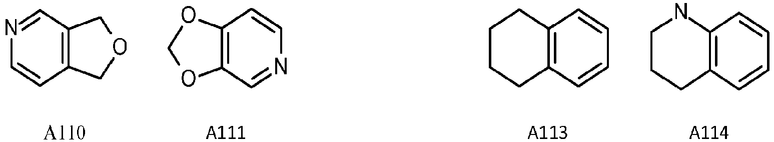 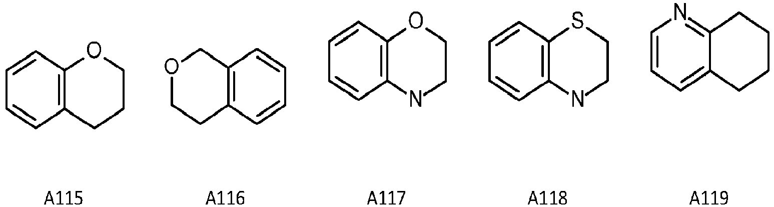 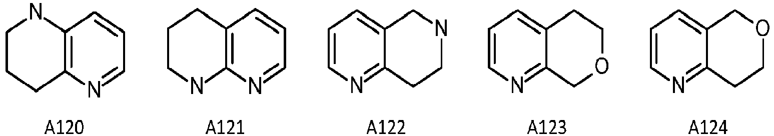 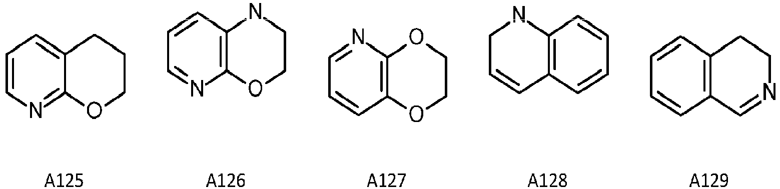 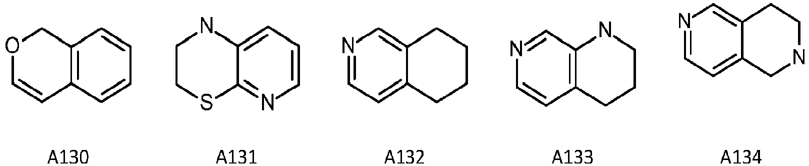 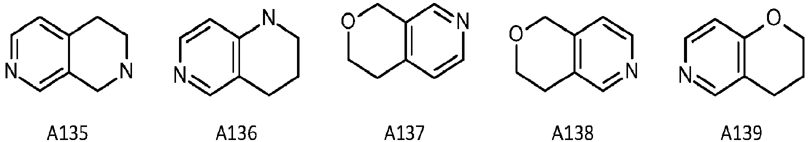 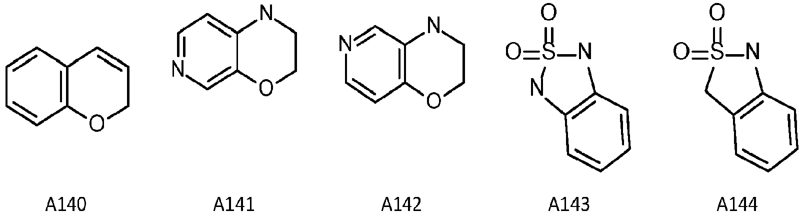 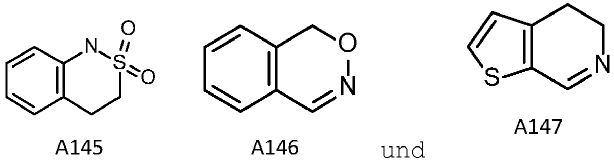
steht, wobei dann, wenn **n** für 1 steht, der C₉-C₁₀-Carbocyclylring oder 9- oder 10-gliedrige Heterocyclylring durch bis zu vier Substituenten zusätzlich zu **L** substituiert sein kann, wobei die Substituenten unabhängig aus der Gruppe bestehend aus Halogen und C₁-C₃-Alkyl ausgewählt sind;
**n** für 0 oder 1 steht;
**L** für einen Substituenten aus der Gruppe bestehend aus Halogen, Cyano, C₁-C₆-Alkyl, C₁-C₆-Halogenalkyl, C₁-C₆-Alkoxy, C₁-C₆-Alkylthio, Arylsulfenyl, C₁-C₆-Alkylsulfinyl, Arylsulfinyl, C₁-C₆-Alkylsulfonyl, Arylsulfonyl, C₃-C₁₀-Carbocyclyl, 3- bis 10-gliedrigem Heterocyclyl, Aryl, Heteroaryl, =N(OR^{a}), N(OR^{a})C(=O)OR^{a}, N(OR^{a})C (=O)R^{a}, -SO₂R^{a}, -SO₂N(R^{a})₂, -OC(=O)R^{a}, -N(R^{a})₂, - NR^{a}C(=O)OR^{a}, -NR^{a}C(=O)N(R^{a})₂, -NR^{a}C(=O)R^{a}, -NR^{a}C(=S)R^{a}, - NR^{a}C(=S)N(R^{a})₂, -NR^{a}C(=NR^{a})R^{a}, -N=C-N(R^{a})₂, -NR^{a}S(=O)₂R^{a}, - OC(=O)N(R^{a})₂, -C(=NR^{a})R^{a}, -C(=O)R^{a}, -C(=O)(OR^{a}), - C (=O)N(R^{a})₂, -C(=O)NR^{a}N(R^{a})₂, -C(=O)N(OR^{a})R^{a}, -C=NOR^{a}, - C (=S)N(R^{a})₂, -C₁-C₆-Alkyl-N(R^{a})₂, -C₁-C₆-Alkyl-C(=O)N(R^{a})₂, -C₁-C₆-Alkyl-NR^{a}C(=O)OR^{a}, -C₁-C₆-Alkyl-NR^{a}C(=O)R^{a}, -C₁-C₆-Alkyl-NR^{a}C(=O)N(R^{a})₂, -C₁-C₆-Alkyl-N(OR^{a})C(=O)R^{a}, -C₁-C₆-Alkyl-C(=O)N(OR^{a})R^{a}, -C₁-C₆-Alkyl-NR^{a}C(=S)N(R^{a})₂, -C₁-C₆-Alkyl-NR^{a}C(=O)R^{a} und -C₁-C₆-Alkyl-NR^{a}S(=O)₂R^{a} steht, wobei der C₁-C₆-Alkyl-, C₁-C₆-Halogenalkyl-, C₁-C₆-Alkoxy-, C₁-C₆-Alkylthio-, Arylsulfenyl-, C₁-C₆-Alkylsulfinyl-, Arylsulfinyl-, C₁-C₆-Alkylsulfonyl-, Arylsulfonyl-, C₃-C₁₀-Carbocyclyl-, 3- bis 10-gliedrige Heterocyclyl-, Aryl-, Heteroaryl-, -C₁-C₆-Alkyl-N(R^{a})₂-, -C₁-C₆-Alkyl-C(=O)N(R^{a})₂-, -C₁-C₆-Alkyl-NR^{a}C(=O)OR^{a}-, -C₁-C₆-Alkyl-NR^{a}C(=O)R^{a}-, -C₁-C₆-Alkyl-NR^{a}C(=O)N(R^{a})₂-, -C₁-C₆-Alkyl-N(OR^{a})C(=O)R^{a}-, -C₁-C₆-Alkyl-C(=O)N(OR^{a})R^{a}-, -C₁-C₆-Alkyl-NR^{a}C(=S)N(R^{a})₂-, -C₁-C₆-Alkyl-NR^{a}C(=O)R^{a}- und -C₁-C₆-Alkyl-NR^{a}S(=O)₂R^{a}-Substituent selbst gegebenenfalls ein-oder mehrfach gleich oder verschieden durch R5 substituiert ist;
R5 für einen Substituenten aus der Gruppe bestehend aus Halogen, Cyano, Hydroxy, Mercapto, Sulfinyl, Sulfonyl, Amino, C₁-C₆-Alkyl, C₁-C₆-Alkoxy, C₁-C₆-Alkylthio, C₁-C₆-Alkylsulfinyl, C₁-C₆-Alkylsulfonyl, C₁-C₆-Alkylamino, C₂-C₆-Alkenyl, C₂-C₆-Alkinyl, C₃-C₁₀-Carbocyclyl, 3- bis 10-gliedrigem Heterocyclyl, C₁-C₆-Halogenalkyl, Hydroxy-C₁-C₆-alkyl, Aryl, Aryloxy, Heteroaryl, Heteroaryloxy, Nitro, -C(=O)R^{a}, -C(=O)OR^{a}, -C(=O)N(R^{a})₂, -C(=S)N(R^{a})₂, - C(=NR^{a})R^{a}, -NR^{a}C(=O)OR^{a}, -NR^{a}C(=O)N(R^{a})₂, -NR^{a}C(=O)R^{a}, - NR^{a}C(=S)R^{a}, -NR^{a}C(=S)N(R^{a})₂, -NR^{a}C(=NR^{a})R^{a}, -OC(=O)R^{a}, - OC(=O)N(R^{a})₂, -NR^{a}S(=O)₂R^{a}, -S(=O)₂R^{a}, -S(=O)₂N(R^{a})₂ und - P(=O)(OR^{a})₂ steht; wobei der C₁-C₆-Alkyl-, C₁-C₆-Alkoxy-, C₁-C₆-Alkylthio-, C₁-C₆-Alkylsulfinyl-, C₁-C₆-Alkylsulfonyl-, C₁-C₆-Alkylamino-, C₂-C₆-Alkenyl-, C₂-C₆-Alkinyl-, C₃-C₁₀-Carbocyclyl-, 3- bis 10-gliedrige Heterocyclyl-, C₁-C₆-Halogenalkyl-, Hydroxy-C₁-C₆-alkyl-, Aryl-, Aryloxy-, Heteroaryl-, Heteroaryloxy-Substituent selbst gegebenenfalls ein- oder mehrfach gleich oder verschieden durch R^{b} substituiert ist; zwei R5-Substituenten dann, wenn sie an ein gemeinsames Kohlenstoffatom gebunden sind, zusammen C=O, C₃-C₁₀-Carbocyclyl oder 3- bis 10-gliedriges Heterocyclyl bilden können;
R^{a} unabhängig voneinander für einen Substituenten steht, der gleich oder verschieden aus der Gruppe bestehend aus Wasserstoff, Halogen, Cyano, Hydroxy, Mercapto, Sulfinyl, Sulfonyl, Amino, C₁-C₆-Alkyl, C₁-C₆-Alkoxy, C₁-C₆-Alkylthio, C₁-C₆-Alkylsulfinyl, C₁-C₆-Alkylsulfonyl, C₁-C₆-Alkylamino, C₂-C₆-Alkenyl, C₂-C₆-Alkinyl, C₃-C₁₀-Carbocyclyl, 3- bis 10-gliedrigem Heterocyclyl, C₁-C₆-Halogenalkyl, Hydroxy-C₁-C₆-alkyl, Aryl, Aryloxy, Heteroaryl, Heteroaryloxy, Nitro, -C₁-C₆-Alkyl-C₃-C₁₀-carbocyclyl, -C₁-C₆-Alkyl-aryl, -C₁-C₆-Alkyl-C₁-C₆-alkoxy, -C(=O)R^{b}, -C(=O)OR^{b}, -C(=O)N(R^{b})₂, -C(=S)N(R^{b})₂, -NR^{b}C(=O)OR^{b}, -NR^{b}C(=O)N(R^{b})₂, -NR^{b}C(=O)R^{b}, -NR^{b}C(=S)R^{b}, - OC(=O)N(R^{b})₂, -NR^{b}S(=O)₂R^{b}, -S(=O)₂R^{b}, -S(=O)₂N(R^{b})₂ und - P(=O)(OR^{b})₂ ausgewählt ist, wobei der C₁-C₆-Alkyl-, C₁-C₆-Alkoxy-, C₁-C₆-Alkylthio-, C₁-C₆-Alkylsulfinyl-, C₁-C₆-Alkylsulfonyl-, C₁-C₆-Alkylamino-, C₂-C₆-Alkenyl-, C₂-C₆-Alkinyl-, C₃-C₁₀-Carbocyclyl-, 3- bis 10-gliedrige Heterocyclyl-, C₁-C₆-Halogenalkyl-, Hydroxy-C₁-C₆-alkyl-, Aryl-, Aryloxy-, Heteroaryl-, Heteroaryloxy-Substituent selbst gegebenenfalls ein- oder mehrfach gleich oder verschieden durch R^{b} substituiert ist;
R^{b} unabhängig voneinander für einen Substituenten steht, der gleich oder verschieden aus der Gruppe bestehend aus Wasserstoff, Halogen, Cyano,-OR^{c}, -N(R^{c})₂, -SR^{c}, - S(=O)R^{c}, -S(=O)OR^{c}, -S(=O)₂R^{c}, -S(=O)₂OR^{c}, C₁-C₆-Alkyl, C₁-C₆-Alkoxy, C₁-C₆-Alkylthio, C₁-C₆-Alkylsulfinyl, C₁-C₆-Alkylsulfonyl, C₁-C₆-Alkylamino, C₂-C₆-Alkenyl, C₂-C₆-Alkinyl, C₃-C₁₀-Carbocyclyl, 3- bis 10-gliedrigem Heterocyclyl, C₁-C₆-Halogenalkyl, Hydroxy-C₁-C₆-alkyl, Aryl, Aryloxy, Heteroaryl, Heteroaryloxy, Nitro, - C(=O)R^{c}, -C(=O)OR^{c}, -C(=O)N(R^{c})₂, -C(=S)N(R^{c})₂, - NR^{c}C(=O)OR^{c}, -NR^{c}C(=O)N(R^{c})₂, -NR^{c}C(=O)R^{c}, -NR^{c}C(=S)R^{c}, - OC(=O)N(R^{c})₂, -NR^{c}S(=O)₂R^{c}, -S(=O)₂N(R^{c})₂ und -P(=O)(OR^{c})₂ ausgewählt ist; wobei der C₃-C₁₀-Carbocyclyl-, 3- bis 10-gliedrige Heterocyclyl-, Aryl-, Heteroaryl-Substituent selbst gegebenenfalls ein-oder mehrfach gleich oder verschieden durch CN, Halogen, C₁-C₆-Alkyl, C₁-C₆-Halogenalkyl oder C₁-C₆-Alkoxy substituiert ist;
R^{c} unabhängig voneinander für einen Substituenten steht, der gleich oder verschieden aus der Gruppe bestehend aus Wasserstoff, Aryl und C₁-C₆-Alkyl ausgewählt ist, mit der Maßgabe, dass
(a) dann, wenn **X** für F steht, **n** für 1 steht 1 und **A** für 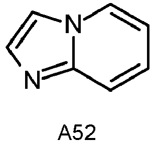 steht, **L** nicht für einen Substituenten aus der Gruppe bestehend aus C₁-C₆-Alkyl, Aryl, 3- bis 10-gliedrigem Heterocyclyl und -C(=O)R^{a}, wobei R^{a} für ein 3- bis 10-gliedriges Heterocyclyl steht, steht,
(b) dann, wenn **X** für F steht und **n** für 0 steht, **A** nicht aus der Gruppe bestehend aus: 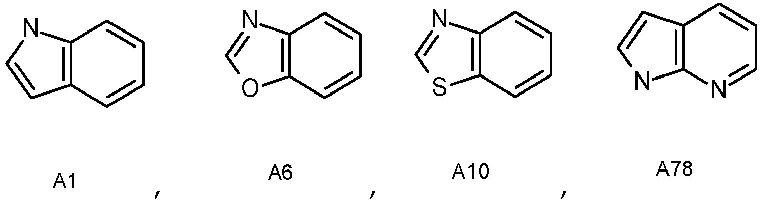 und 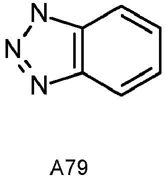 ausgewählt ist,
(c) dann, wenn **X** für F steht, **n** für 1 steht und **A** aus der Gruppe bestehend aus 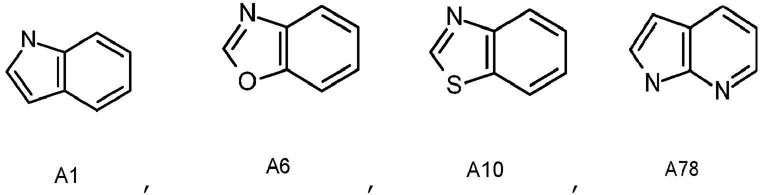 und 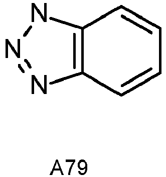 ausgewählt ist, **L** nicht für einen Substituenten aus der Gruppe bestehend aus C₁-C₆-Alkyl, -C (=O)(OR^{a}), wobei R^{a} für ein C₁-C₆-Alkyl steht, und -SO₂R^{a}, wobei R^{a} für ein Aryl steht, steht,
(d) dann, wenn **X** für F steht und n für 0 steht, **A** nicht für 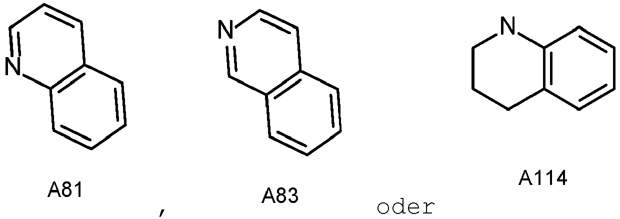 steht,
(e) dann, wenn **X** für F steht, **n** für 1 steht und **A** für 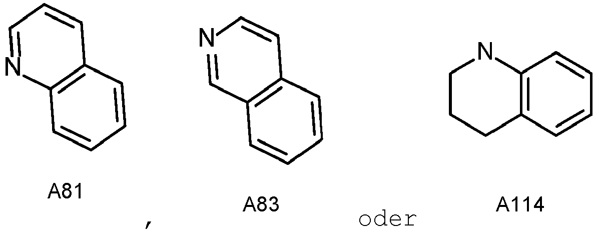 steht,
**L** nicht für einen Substituenten aus der Gruppe bestehend aus unsubstituiertem C₁-C₆-Alkyl; C₁-C₆-Alkyl, das durch ein oder mehrere **R5** substituiert ist, wobei **R5** aus der Gruppe bestehend aus unsubstituiertem Aryl, unsubstituiertem Heteroaryl, Aryl, das durch ein bis drei Rb substituiert ist, wobei Rb für unsubstituiertes C₁-C₆-Alkyl steht, und Heteroaryl, das durch ein bis drei Rb substituiert ist, wobei Rb für unsubstituiertes C₁-C₆-Alkyl steht, ausgewählt ist; C₁-C₆-Halogenalkyl; Halogen; Cyano; -SO₂R^{a}, wobei R^{a} für ein unsubstituiertes C₁-C₆-Alkyl steht; unsubstituiertem C₃-C₁₀-Carbocyclyl; unsubstituiertem Aryl; unsubstituiertem Heteroaryl; Aryl, das durch ein oder drei **R5** substituiert ist, wobei **R5** für ein unsubstituiertes C₁-C₆-Alkyl steht; und Heteroaryl, das durch ein oder drei **R5** substituiert ist, wobei **R5** für ein unsubstituiertes C₁-C₆-Alkyl steht; steht,
(f) dann, wenn **L** für -N(Ra)₂ steht, wobei die beiden Ra für Wasserstoff stehen, L nicht an das Atom von **A** gebunden ist, das **A** mit der Oxadiazolgruppierung verbindet,
(g) es sich bei der Verbindung der Formel (I') nicht um 2-Pyridincarboxamid, N-methyl-4-[[3-methyl-2-[5-(trifluormethyl)-1,2,4-oxadiazol-3-yl]benzo[b]thien-6-yl]oxy]- (CAS-2222313-38-2),
Benzothiazol, 6-methoxy-2-[5-(trifluormethyl)-1,2,4-oxadiazol-3-yl]- (CAS-897805-25-3), Thieno[2,3-c]pyridin, 4-(4-chlorphenoxy)-2-[5-(trifluormethyl)-1,2,4-oxadiazol-3-yl]- (CAS-251995-30-9),
Pyrazolo[1,5-a]pyrimidin, 3-[5-(trifluormethyl)-1,2,4-oxadiazol-3-yl]-7-[3-(trifluormethyl)phenyl]- (CAS-159224-00-7),
1H-Indol, 3-(2-chlor-3-thienyl)-5-[5-(trifluormethyl)-1,2,4-oxadiazol-3-yl]- (CAS-2252389-71-0), Imidazo[1,2-a]pyridin, 2-(phenylmethyl)-7-[5-(trifluormethyl)-1,2,4-oxadiazol-3-yl]- (CAS-2170611-35-3),
1H-Pyrrolo[2,3-b]pyridin, 5-[5-(trifluormethyl)-1,2,4-oxadiazol-3-yl]- (CAS-2170611-21-7), 1H-Pyrrolo[2,3-b]pyridin, 1-(phenylsulfonyl)-5-[5-(trifluormethyl)-1,2,4-oxadiazol-3-yl]- (CAS-2170611-01-3),
2,1,3-Benzoxadiazol, 4-[3-(4-morpholinylmethyl)-1-azetidinyl]-6-[5-(trifluormethyl)-1,2,4-oxadiazol-3-yl]- (CAS-1807986-73-7), Tricyclo[3.3.1.13,7]decan-1-ol, 4-[[5-[5-(trifluormethyl)-1,2,4-oxadiazol-3-yl]-1H-pyrrolo[2,3-b]pyridin-4-yl]amino]- (CAS-944120-75-6),
1H-Inden-1-amin, 2,3-dihydro-5-[5-(trifluormethyl)-1,2,4-oxadiazol-3-yl]-, (1S)- (CAS-916303-83-8), 1H-Inden-1-amin, 2,3-dihydro-5-[5-(trifluormethyl)-1,2,4-oxadiazol-3-yl]-, Hydrochlorid (1:1), (1S)- (CAS-916210-97-4),
Carbamidsäure, N-[(1S)-2,3-dihydro-5-[5-(trifluormethyl)-1,2,4-oxadiazol-3-yl]-1H-inden-1-yl]-, 1,1-dimethylethylester (CAS-916209-96-6),
1H-Pyrrolo[2,3-b]pyridin, 2-[1-methyl-5-[5-(trifluormethyl)-1,2,4-oxadiazol-3-yl]-1H-indol-3-yl]-(CAS-479551-10-5),
1H-Indol, 2,3-dihydro-7-methyl-1-[3-(3-methyl-5-isoxazolyl)propyl]-5-[5-(trifluormethyl)-1,2,4-oxadiazol-3-yl]- (CAS-178396-27-5),
1H-Indol, 7-methyl-1-[5-(3-methyl-5-isoxazolyl)pentyl]-5-[5-(trifluormethyl)-1,2,4-oxadiazol-3-yl]-(CAS-178396-24-2),
1H-Indol, 7-methyl-1-[4-(3-methyl-5-isoxazolyl)butyl]-5-[5-(trifluormethyl)-1,2,4-oxadiazol-3-yl]-(CAS-178396-22-0),
1H-Indol, 1-[3-(3-methyl-5-isoxazolyl)propyl]-5-[5-(trifluormethyl)-1,2,4-oxadiazol-3-yl]- (CAS-178396-20-8),
Pyrazino[2,3-c]pyridazin, 5-[(1R)-1-(2,5-dichlorphenyl)ethyl]-5,6,7,8-tetrahydro-3-[5-(trifluormethyl)-1,2,4-oxadiazol-3-yl]- (CAS-1690421-46-5),
Pyrazino[2,3-c]pyridazin, 5-[(2,5-dichlorphenyl)methyl]-5,6,7,8-tetrahydro-3-[5-(trifluormethyl)-1,2,4-oxadiazol-3-yl]- (CAS-1690421-40-9),
1,2,4-Oxadiazol, 3-[7-(brommethyl)-2-naphthalinyl]-5-(trifluormethyl)- (CAS-1172849-62-5),
1,6-Naphthyridin, 5,6,7,8-tetrahydro-3-[5-(trifluormethyl)-1,2,4-oxadiazol-3-yl]-, 2,2,2-Trifluoracetat (1:1) (CAS-741736-97-0), 1,6-Naphthyridin, 5,6,7,8-tetrahydro-3-[5-(trifluormethyl)-1,2,4-oxadiazol-3-yl]- (CAS-741736-96-9),
1-Butanon, 3-amino-4-(2,5-difluorphenyl)-1-[7,8-dihydro-3-[5-(trifluormethyl)-1,2,4-oxadiazol-3-yl]-1,6-naphthyridin-6(5H)-yl]-, (3R)- (CAS-741736-75-4), Phthalazin, 1-phenyl-4-[5-(trifluormethyl)-1,2,4-oxadiazol-3-yl]- (CAS-863601-14-3)
handelt.

2. Verbindung der Formel (I') nach Anspruch 1, wobei **A** für einen aromatischen anellierten bicyclischen Cg-C₁₀-Carbocyclylring oder einen aromatischen anellierten bicyclischen 9- oder 10-gliedrigen Heterocyclylring aus der Gruppe bestehend aus A1, A2, A4, A6 bis A55, A57 bis A83, A85 bis A89 gemäß Anspruch 1 steht.

3. Verbindung der Formel (I') nach einem der vorhergehenden Ansprüche, wobei **A** aus der Gruppe bestehend aus A1, A4, A10, A52, A77, A79 und A83 gemäß Anspruch 1 ausgewählt ist.

4. Verbindung der Formel (I') nach Anspruch 1, wobei **A** für einen teilweise ungesättigten anellierten bicyclischen C₉-C₁₀-Carbocyclylring oder einen teilweise ungesättigten anellierten bicyclischen 9- oder 10-gliedrigen Heterocyclylring aus der Gruppe bestehend aus A90 bis A111 und A113 bis A147 gemäß Anspruch 1 steht.

5. Verbindung der Formel (I') nach Anspruch 1 oder 4, wobei **A** aus der Gruppe bestehend aus A90, A91, A93, A113, A114 und A140 gemäß Anspruch 1 ausgewählt ist.

6. Verbindung der Formel (I') nach einem der vorhergehenden Ansprüche, wobei **L** aus der Gruppe bestehend aus Halogen, C₁-C₆-Alkyl, C₁-C₆-Alkoxy, C₁-C₆-Alkylsulfonyl, Aryl, -N(R^{a})₂, =N(OR^{a}), -N(OR^{a})C(=O)OR^{a}, - NR^{a}C(=O)OR^{a}, -N(OR^{a})C(=O)R^{a}, -NR^{a}C(=O)R^{a}, -OC(=O)N(R^{a})₂, - C(=O)R^{a}, -C(=O)(OR^{a}), -C(=O)N(R^{a})₂ ausgewählt ist, wobei der C₁-C₆-Alkyl-, C₁-C₆-Alkoxy-, C₁-C₆-Alkylsulfonyl- und Arylsubstituent selbst gegebenenfalls ein- oder mehrfach gleich oder verschieden durch **R5** gemäß Anspruch 1 substituiert ist.

7. Verbindung der Formel (I') nach Anspruch 6, wobei **R5** für Halogen, C₁-C₆-Alkoxy oder Aryl steht.

8. Zusammensetzung, umfassend eine oder mehrere Verbindungen der Formel (I') nach einem der Ansprüche 1 bis 7 und einen oder mehrere unbedenkliche Träger.

9. Nichttherapeutische Verwendung einer Verbindung der Formel (I) oder eines Salzes, N-Oxids oder Solvats davon zur Bekämpfung von phytopathogenen Pilzen: 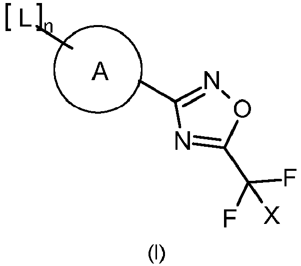 wobei
**X** für F oder Cl steht;
**A** für einen aromatischen oder teilweise ungesättigten anellierten bicyclischen C₇-C₁₂-Carbocyclylring oder einen aromatischen oder teilweise ungesättigten anellierten bicyclischen 7- bis 12-gliedrigen Heterocyclylring steht, wobei dann, wenn n für 1 steht, der C₇-C₁₂-Carbocyclylring oder 7- bis 12-gliedrige Heterocyclylring durch bis zu vier Substituenten zusätzlich zu L substituiert sein kann, wobei die Substituenten unabhängig aus der Gruppe bestehend aus Halogen und C₁-C₃-Alkyl ausgewählt sind;
**n** für 0 oder 1 steht;
**L** für einen Substituenten aus der Gruppe bestehend aus Halogen, Cyano, C₁-C₆-Alkyl, C₁-C₆-Halogenalkyl, C₁-C₆-Alkoxy, C₁-C₆-Alkylthio, Arylsulfenyl, C₁-C₆-Alkylsulfinyl, Arylsulfinyl, C₁-C₆-Alkylsulfonyl, Arylsulfonyl, C₃-C₁₀-Carbocyclyl, 3- bis 10-gliedrigem Heterocyclyl, Aryl, Heteroaryl, =N(OR^{a}), N(OR^{a})C(=O)OR^{a}, N(OR^{a})C (=O)R^{a}, -SO₂R^{a}, -SO₂N(R^{a})₂, -OC(=O)R^{a}, -N(R^{a})₂, - NR^{a}C(=O)OR^{a}, -NR^{a}C(=O)N(R^{a})₂, -NR^{a}C(=O)R^{a}, -NR^{a}C(=S)R^{a}, - NR^{a}C(=S)N(R^{a})₂, -NR^{a}C(=NR^{a})R^{a}, -N=C-N(R^{a})₂, -NR^{a}S(=O)₂R^{a}, - OC(=O)N(R^{a})₂, -C(=NR^{a})R^{a}, -C(=O)R^{a}, -C(=O)(OR^{a}), - C(=O)N(R^{a})₂, -C(=O)NR^{a}N(R^{a})₂, -C(=O)N(OR^{a})R^{a}, -C=NOR^{a}R^{a}, - C(=S)N(R^{a})₂, -C₁-C₆-Alkyl-N(R^{a})₂, -C₁-C₆-Alkyl-C(=O)N(R^{a})₂, -C₁-C₆-Alkyl-NR^{a}C(=O)OR^{a}, -C₁-C₆-Alkyl-NR^{a}C(=O)R^{a}, -C₁-C₆-Alkyl-NR^{a}C(=O)N(R^{a})₂, -C₁-C₆-Alkyl-N(OR^{a})C(=O)R^{a}, -C₁-C₆-Alkyl-C(=O)N(OR^{a})R^{a}, -C₁-C₆-Alkyl-NR^{a}C(=S)N(R^{a})₂, -C₁-C₆-Alkyl-NR^{a}C(=O)R^{a} und -C₁-C₆-Alkyl-NR^{a}S(=O)₂R^{a} steht, wobei der C₁-C₆-Alkyl-, C₁-C₆-Halogenalkyl-, C₁-C₆-Alkoxy-, C₁-C₆-Alkylthio-, Arylsulfenyl-, C₁-C₆-Alkylsulfinyl-, Arylsulfinyl-, C₁-C₆-Alkylsulfonyl-, Arylsulfonyl-, C₃-C₁₀-Carbocyclyl-, 3- bis 10-gliedrige Heterocyclyl-, Aryl-, Heteroaryl-, -C₁-C₆-Alkyl-N(R^{a})₂-, -C₁-C₆-Alkyl-C(=O)N(R^{a})₂-, -C₁-C₆-Alkyl-NR^{a}C(=O)OR^{a}-, -C₁-C₆-Alkyl-NR^{a}C(=O)R^{a}-, -C₁-C₆-Alkyl-NR^{a}C(=O)N(R^{a})₂-, -C₁-C₆-Alkyl-N(OR^{a})C(=O)R^{a}-, -C₁-C₆-Alkyl-C(=O)N(OR^{a})R^{a}-, -C₁-C₆-Alkyl-NR^{a}C(=S)N(R^{a})₂-, -C₁-C₆-Alkyl-NR^{a}C(=O)R^{a}- und -C₁-C₆-Alkyl-NR^{a}S(=O)₂R^{a}-Substituent selbst gegebenenfalls ein-oder mehrfach gleich oder verschieden durch R5 substituiert ist;
**R5** für einen Substituenten aus der Gruppe bestehend aus Halogen, Cyano, Hydroxy, Mercapto, Sulfinyl, Sulfonyl, Amino, C₁-C₆-Alkyl, C₁-C₆-Alkoxy, C₁-C₆-Alkylthio, C₁-C₆-Alkylsulfinyl, C₁-C₆-Alkylsulfonyl, C₁-C₆-Alkylamino, C₂-C₆-Alkenyl, C₂-C₆-Alkinyl, C₃-C₁₀-Carbocyclyl, 3- bis 10-gliedrigem Heterocyclyl, C₁-C₆-Halogenalkyl, Hydroxy-C₁-C₆-alkyl, Aryl, Aryloxy, Heteroaryl, Heteroaryloxy, Nitro, -C(=O)R^{a}, -C(=O)OR^{a}, -C(=O)N(R^{a})₂, -C(=S)N(R^{a})₂, - C(=NR^{a})R^{a}, -NR^{a}C(=O)OR^{a}, -NR^{a}C(=O)N(R^{a})₂, -NR^{a}C(=O)R^{a}, - NR^{a}C(=S)R^{a}, -NR^{a}C(=S)N(R^{a})₂, -NR^{a}C(=NR^{a})R^{a}, -OC(=O)R^{a}, - OC(=O)N(R^{a})₂, -NR^{a}S(=O)₂R^{a}, -S(=O)₂R^{a}, -S(=O)₂N(R^{a})₂ und - P(=O)(OR^{a})₂ steht; wobei der C₁-C₆-Alkyl-, C₁-C₆-Alkoxy-, C₁-C₆-Alkylthio-, C₁-C₆-Alkylsulfinyl-, C₁-C₆-Alkylsulfonyl-, C₁-C₆-Alkylamino-, C₂-C₆-Alkenyl-, C₂-C₆-Alkinyl-, C₃-C₁₀-Carbocyclyl-, 3- bis 10-gliedrige Heterocyclyl-, C₁-C₆-Halogenalkyl-, Hydroxy-C₁-C₆-alkyl-, Aryl-, Aryloxy-, Heteroaryl-, Heteroaryloxy-Substituent selbst gegebenenfalls ein- oder mehrfach gleich oder verschieden durch R^{b} substituiert ist; zwei R5-Substituenten dann, wenn sie an ein gemeinsames Kohlenstoffatom gebunden sind, zusammen C=O, C₃-C₁₀-Carbocyclyl oder 3- bis 10-gliedriges Heterocyclyl bilden können;
R^{a} unabhängig voneinander für einen Substituenten steht, der gleich oder verschieden aus der Gruppe bestehend aus Wasserstoff, Halogen, Cyano, Hydroxy, Mercapto, Sulfinyl, Sulfonyl, Amino, C₁-C₆-Alkyl, C₁-C₆-Alkoxy, C₁-C₆-Alkylthio, C₁-C₆-Alkylsulfinyl, C₁-C₆-Alkylsulfonyl, C₁-C₆-Alkylamino, C₂-C₆-Alkenyl, C₂-C₆-Alkinyl, C₃-C₁₀-Carbocyclyl, 3- bis 10-gliedrigem Heterocyclyl, C₁-C₆-Halogenalkyl, Hydroxy-C₁-C₆-alkyl, Aryl, Aryloxy, Heteroaryl, Heteroaryloxy, Nitro, -C₁-C₆-Alkyl-C₃-C₁₀-carbocyclyl, -C₁-C₆-Alkyl-aryl, -C₁-C₆-Alkyl-C₁-C₆-alkoxy, -C(=O)R^{b}, -C(=O)OR^{b}, -C(=O)N(R^{b})₂, -C(=S)N(R^{b})₂, -NR^{b}C(=O)OR^{b}, -NR^{b}C(=O)N(R^{b})₂, -NR^{b}C(=O)R^{b}, -NR^{b}C(=S)R^{b}, - OC(=O)N(R^{b})₂, -NR^{b}S(=O)₂R^{b}, -S(=O)₂R^{b}, -S(=O)₂N(R^{b})₂ und - P(=O)(OR^{b})₂ ausgewählt ist, wobei der C₁-C₆-Alkyl-, C₁-C₆-Alkoxy-, C₁-C₆-Alkylthio-, C₁-C₆-Alkylsulfinyl-, C₁-C₆-Alkylsulfonyl-, C₁-C₆-Alkylamino-, C₂-C₆-Alkenyl-, C₂-C₆-Alkinyl-, C₃-C₁₀-Carbocyclyl-, 3- bis 10-gliedrige Heterocyclyl-, C₁-C₆-Halogenalkyl-, Hydroxy-C₁-C₆-alkyl-, Aryl-, Aryloxy-, Heteroaryl-, Heteroaryloxy-Substituent selbst gegebenenfalls ein- oder mehrfach gleich oder verschieden durch R^{b} substituiert ist;
R^{b} unabhängig voneinander für einen Substituenten steht, der gleich oder verschieden aus der Gruppe bestehend aus Wasserstoff, Halogen, Cyano,-OR^{c}, -N(R^{c})₂, -SR^{c}, - S(=O)R^{c}, -S(=O)OR^{c}, -S(=O)₂R^{c}, -S(=O)₂OR^{c}, C₁-C₆-Alkyl, C₁-C₆-Alkoxy, C₁-C₆-Alkylthio, C₁-C₆-Alkylsulfinyl, C₁-C₆-Alkylsulfonyl, C₁-C₆-Alkylamino, C₂-C₆-Alkenyl, C₂-C₆-Alkinyl, C₃-C₁₀-Carbocyclyl, 3- bis 10-gliedrigem Heterocyclyl, C₁-C₆-Halogenalkyl, Hydroxy-C₁-C₆-alkyl, Aryl, Aryloxy, Heteroaryl, Heteroaryloxy, Nitro, - C(=O)R^{c}, -C(=O)OR^{c}, -C(=O)N(R^{c})₂, -C(=S)N(R^{c})₂, - NR^{c}C(=O)OR^{c}, -NR^{c}C(=O)N(R^{c})₂, -NR^{c}C(=O)R^{c}, -NR^{c}C(=S)R^{c}, - OC(=O)N(R^{c})₂, -NR^{c}S(=O)₂R^{c}, -S(=O)₂N(R^{c})₂ und -P(=O)(OR^{c})₂ ausgewählt ist; wobei der C₃-C₁₀-Carbocyclyl-, 3- bis 10-gliedrige Heterocyclyl-, Aryl-, Heteroaryl-Substituent selbst gegebenenfalls ein-oder mehrfach gleich oder verschieden durch CN, Halogen, C₁-C₆-Alkyl, C₁-C₆-Halogenalkyl oder C₁-C₆-Alkoxy substituiert ist;
R^{c} unabhängig voneinander für einen Substituenten steht, der gleich oder verschieden aus der Gruppe bestehend aus Wasserstoff, Aryl und C₁-C₆-Alkyl ausgewählt ist, mit der Maßgabe, dass dann, wenn A für Naphthyl steht, n für 1 steht und X für F steht, L nicht für einen Substituenten aus der Gruppe bestehend aus
- C₁-C₆-Alkyl, das durch ein oder mehrere R5 substituiert ist, wobei mindestens eines der R5 aus der Gruppe bestehend aus C₁-C₆-Alkoxy, Aryl, Heteroaryl, C₃-C₁₀-Carbocyclyl, 3- bis 10-gliedrigem Heterocyclyl, Aryloxy und Heteroaryloxy ausgewählt ist,
- C₁-C₆-Alkoxy, das durch ein oder mehrere R5 substituiert ist, wobei mindestens eines der R5 aus der Gruppe bestehend aus Aryl, Heteroaryl, C₃-C₁₀-Carbocyclyl, 3- bis 10-gliedrigem Heterocyclyl, Aryloxy und Heteroaryloxy ausgewählt ist,
- -N(R^{a})₂, wobei mindestens ein R^{a} für Aryl, Heteroaryl, C₃-C₁₀-Carbocyclyl oder 3- bis 10-gliedriges Heterocyclyl steht,
- N(R^{a})₂, wobei mindestens ein R^{a} für C₁-C₆-Alkyl, das durch ein oder mehrere R^{b} substituiert ist, steht, wobei mindestens eines der R^{b} für ein Aryl, Heteroaryl, C₃-C₁₀-Carbocyclyl oder 3- bis 10-gliedriges Heterocyclyl steht,
- C₁-C₆-Alkyl-N(R^{a})₂, wobei mindestens ein R^{a} für Aryl, Heteroaryl, C₃-C₁₀-Carbocyclyl oder 3- bis 10-gliedriges Heterocyclyl steht,
- C₃-C₁₀-Carbocyclyl,
- oder 3- bis 10-gliedriges Heterocyclyl und
- Heteroaryl
steht.

10. Nichttherapeutische Verwendung einer Verbindung der Formel (I) zur Bekämpfung von phytopathogenen Pilzen nach Anspruch 9, wobei A aus der Gruppe bestehend aus 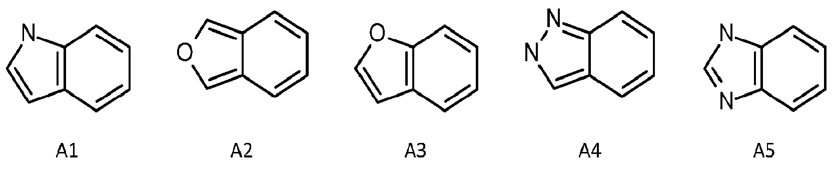 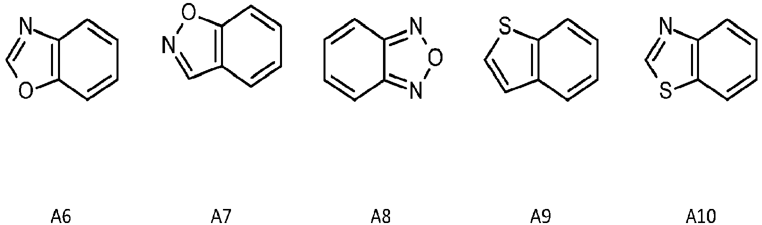 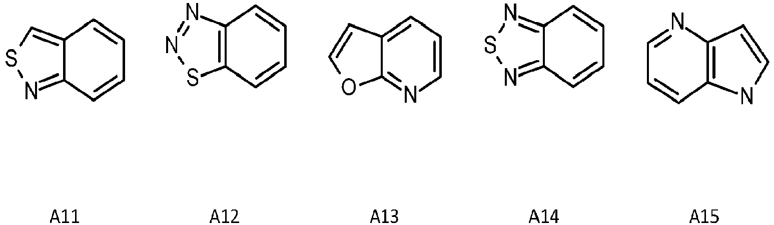 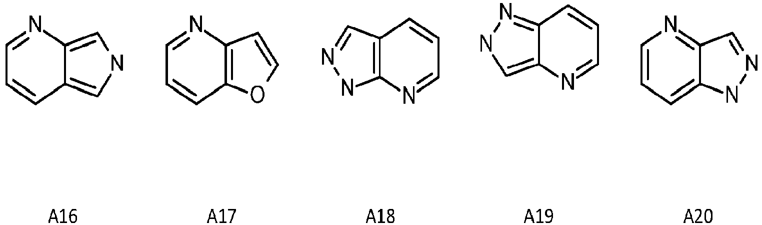 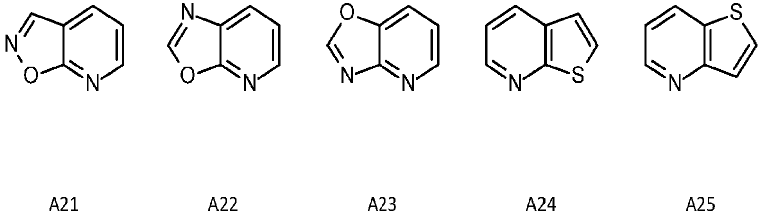 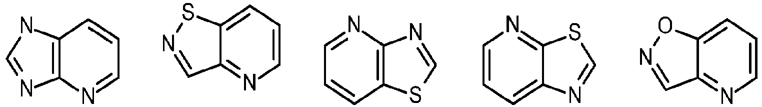 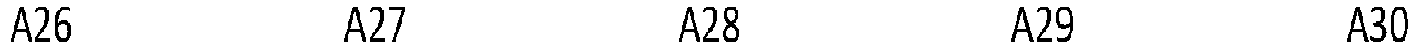 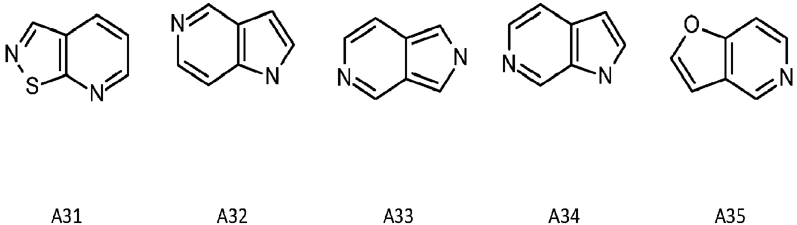 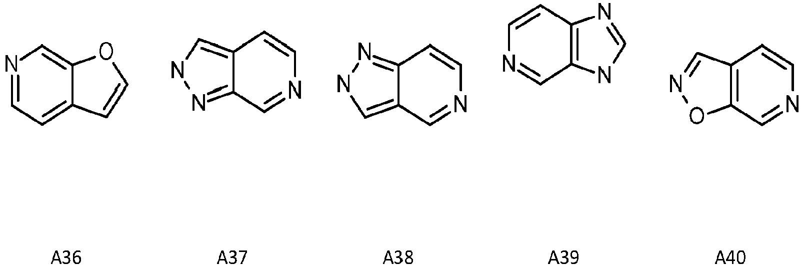 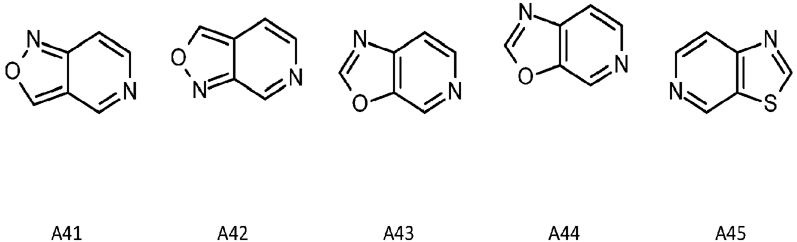 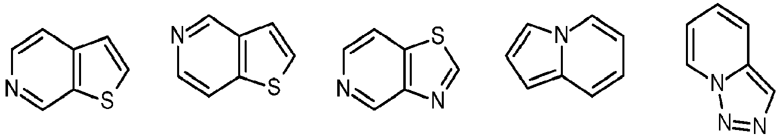 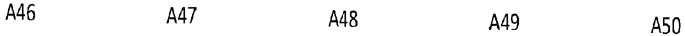 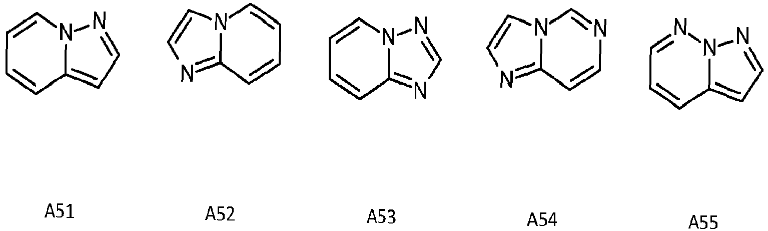 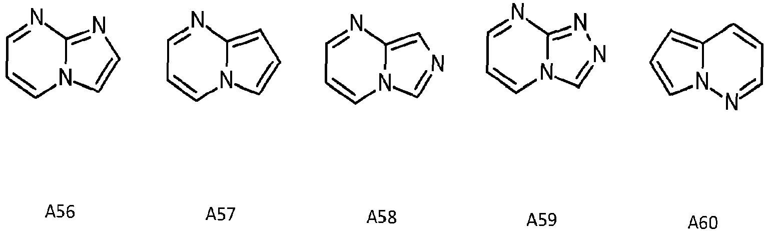 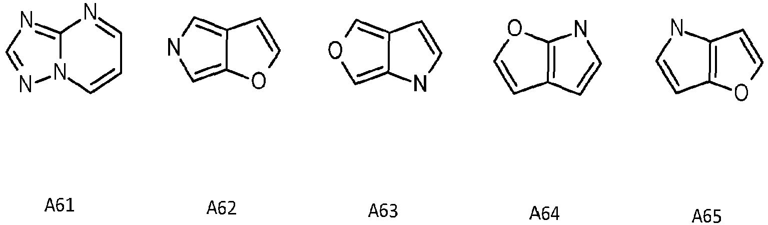 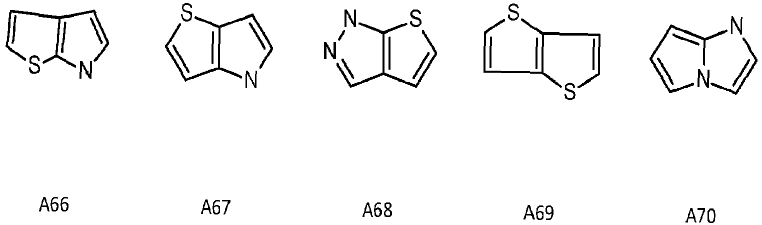 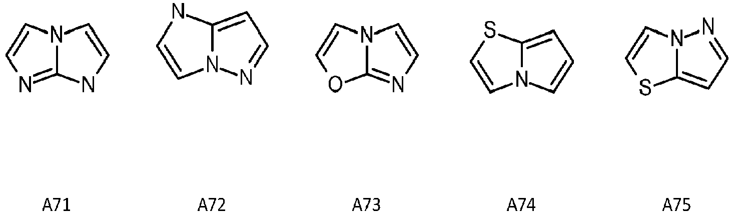 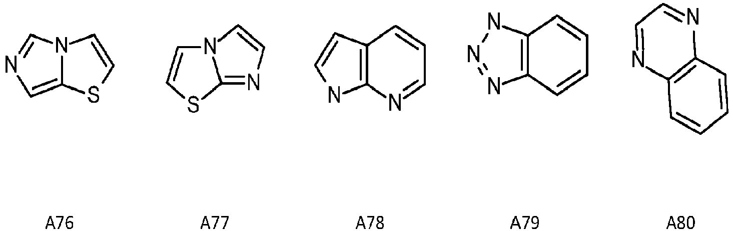 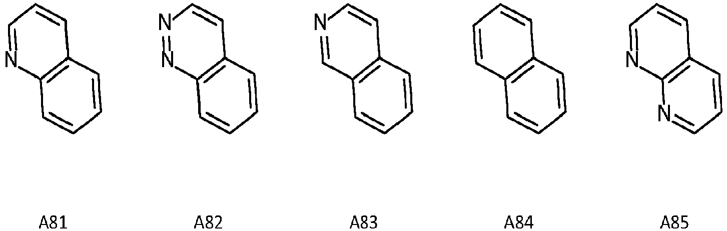 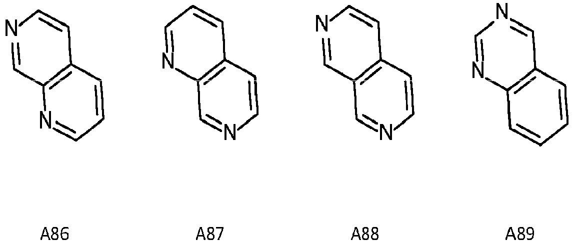 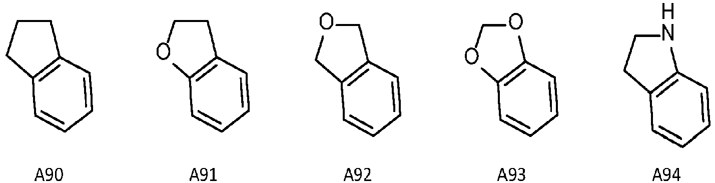 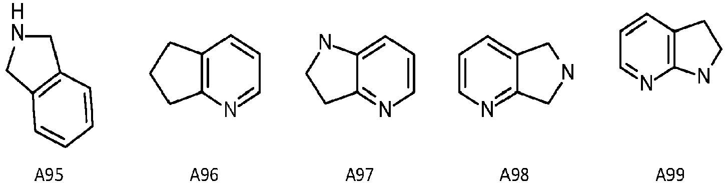 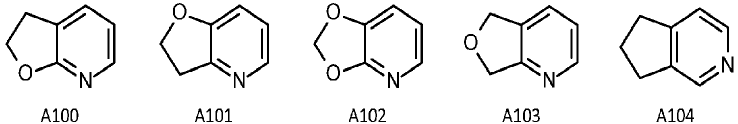 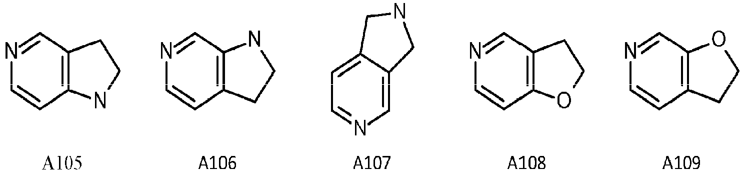 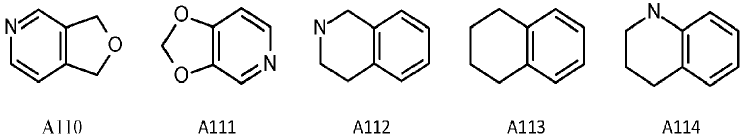 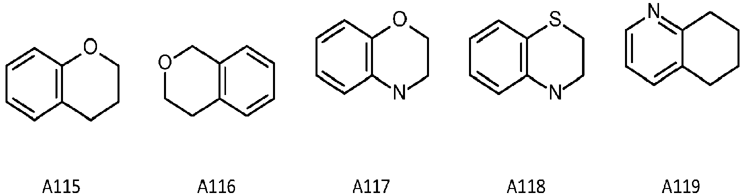 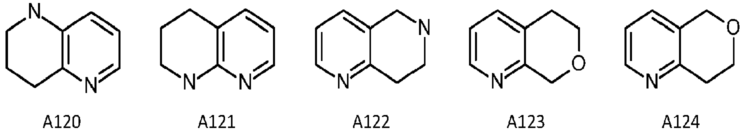 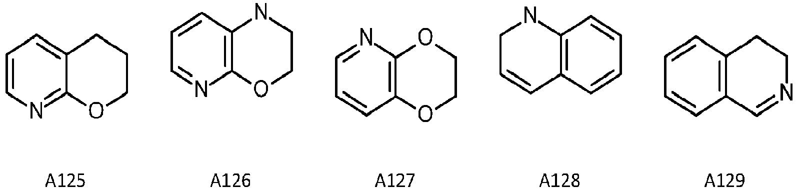 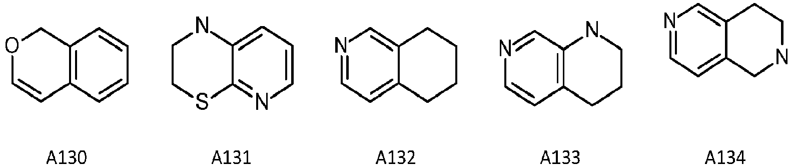 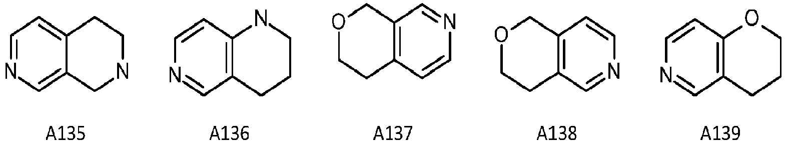 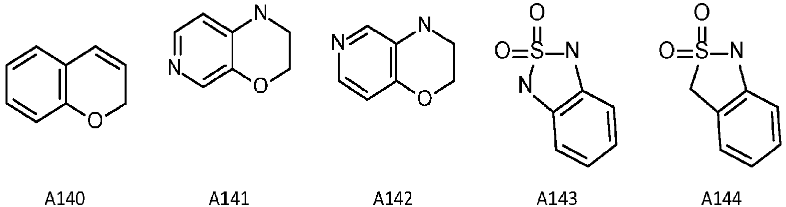 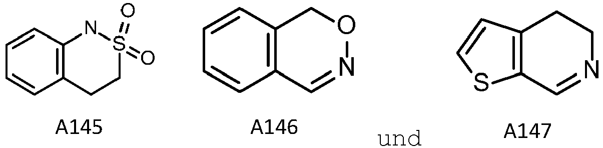 ausgewählt ist.

11. Verfahren zur Bekämpfung von unerwünschtem phytopathogenen Mikroorganismen, das den Schritt des Ausbringens einer oder mehrerer Verbindungen der Formel (I) gemäß Anspruch 9 oder 10 auf die Pflanzen, Pflanzenteile, Samen, Früchte oder auf den Boden, in dem die Pflanzen wachsen, umfasst.

12. Verfahren zur Herstellung einer Verbindung der Formel (I') nach einem der Ansprüche 1 bis 7, wobei n für 1 steht, das den Schritt des Umsetzens von Amidoximen der Formel (II) mit Difluorhalogenalkylessigsäureanhydrid oder Difluorhalogenalkylacetylchlorid in einem Lösungsmittel zu einer Verbindung der Formel (I) umfasst: 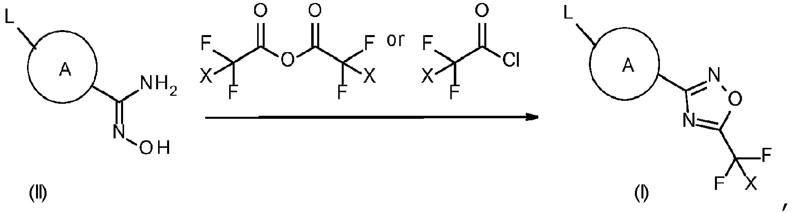 wobei Formel (I) der Formel (I'), wobei n für 1 steht, entspricht und wobei L, A und X in den Formeln (II) und (I) wie in der Verbindung der Formel (I') definiert sind.

## Revendications

1. Composé de la formule (I') ou sel, N-oxyde ou solvate correspondant : 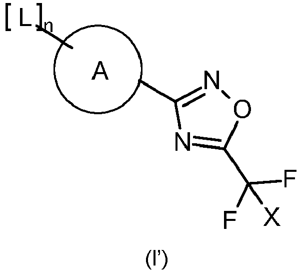
**X** étant F ou Cl ;
**A** étant un cycle C₉-C₁₀-carbocyclyle bicyclique condensé partiellement insaturé ou un cycle hétérocyclyle à 9 ou 10 chaînons bicyclique condensé aromatique ou partiellement insaturé choisi dans le groupe constitué par 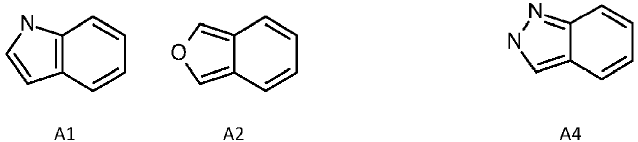 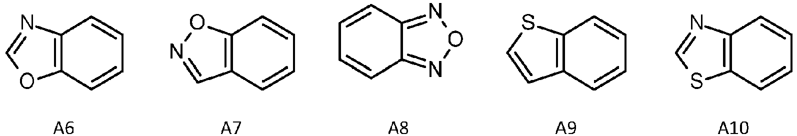 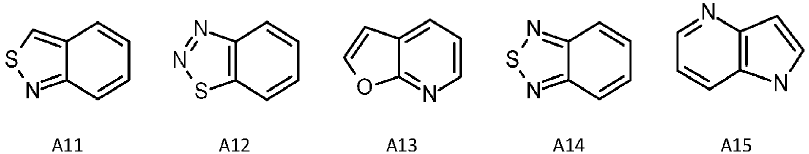 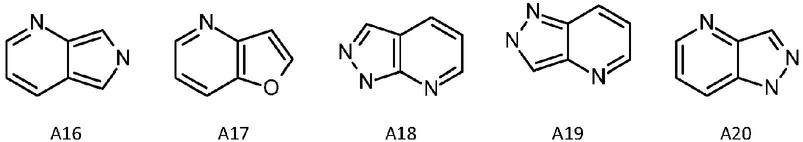 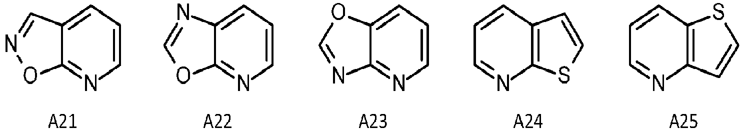 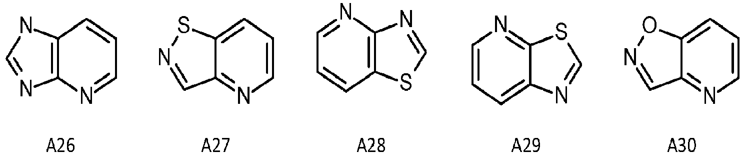 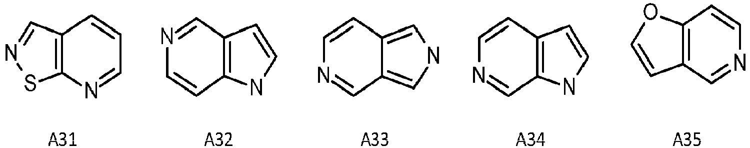 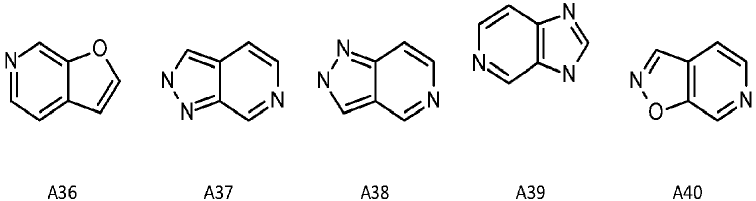 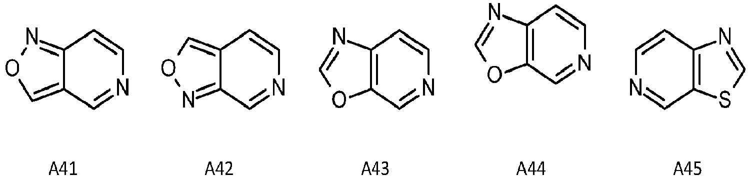 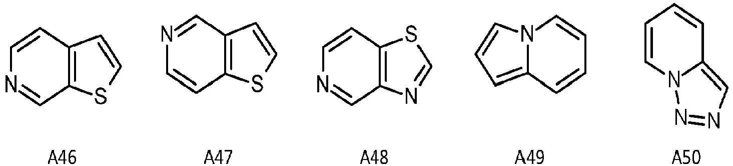 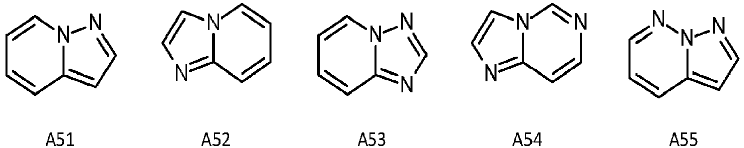 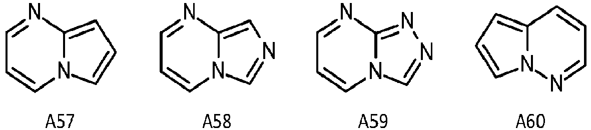 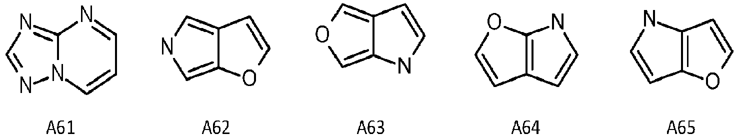 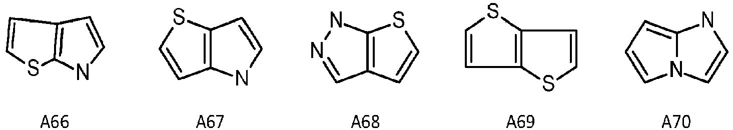 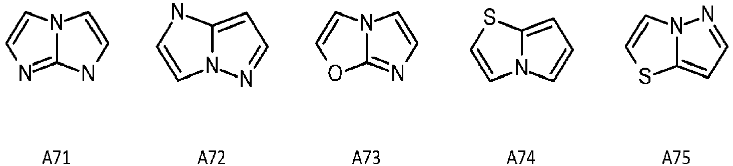 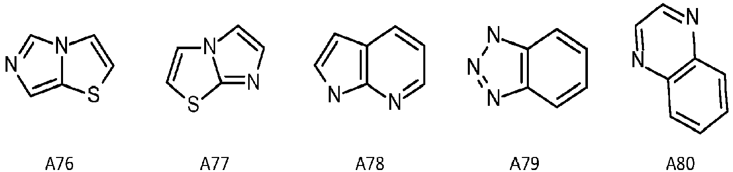 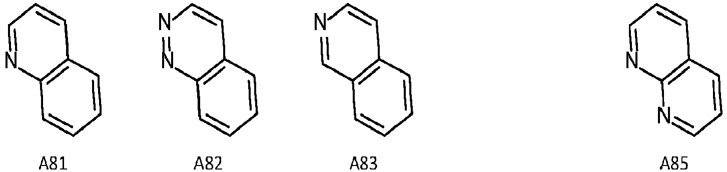 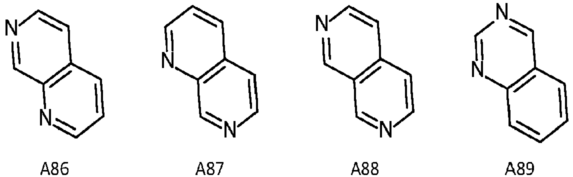 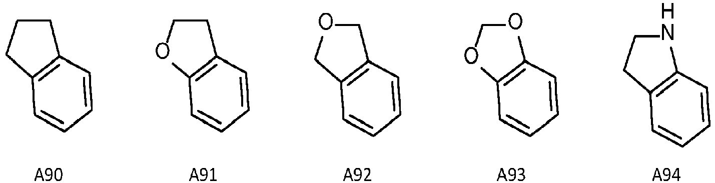 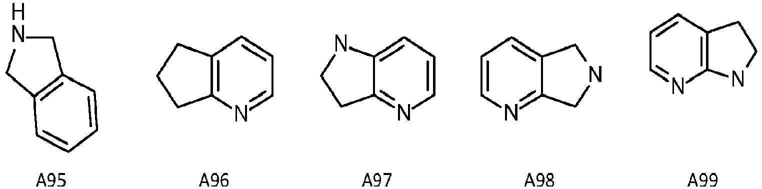 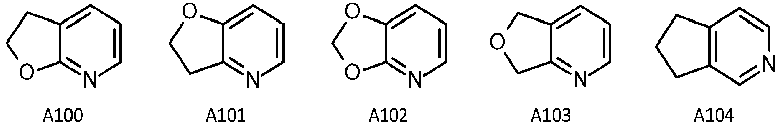 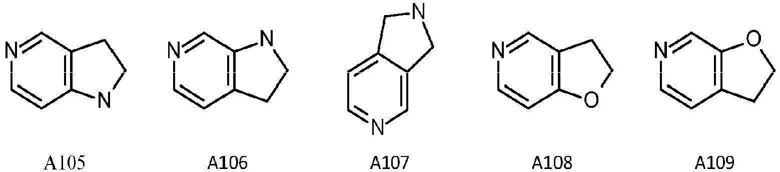 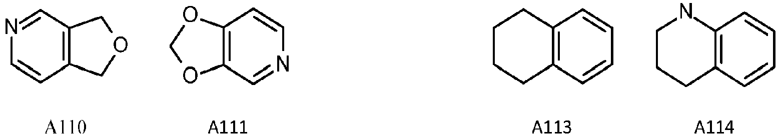 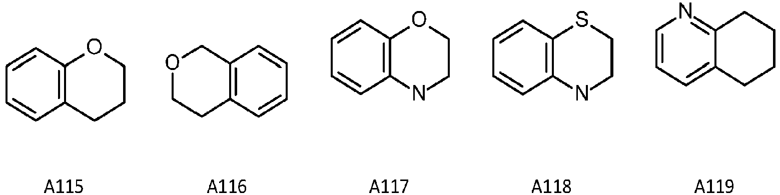 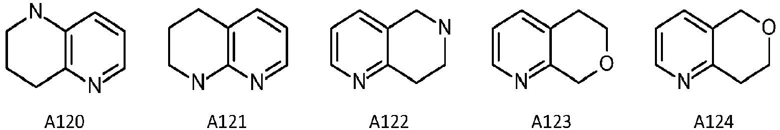 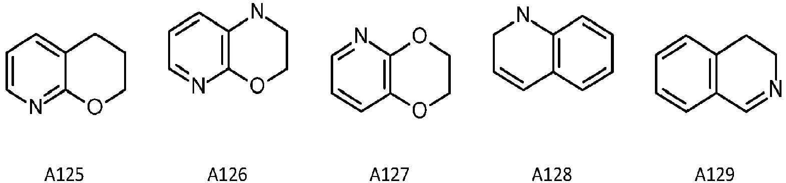 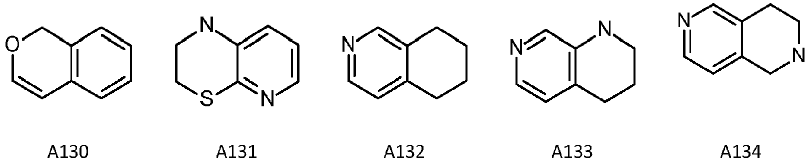 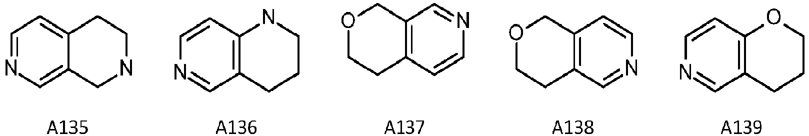 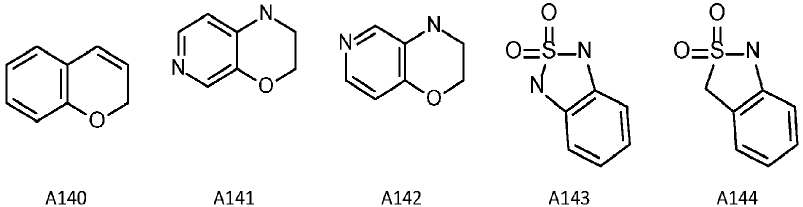 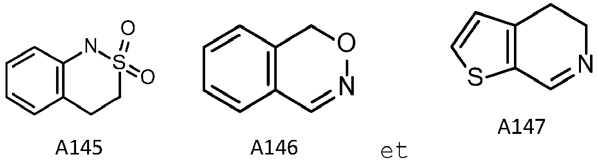
lorsque **n** est 1, ledit cycle C₉-C₁₀-carbocyclyle ou cycle hétérocyclyle à 9 ou 10 chaînons peut être substitué par jusqu'à quatre substituants en plus de **L,** lesdits substituants étant indépendamment choisis dans le groupe constitué par halogène et C₁-C₃-alkyle ;
**n** étant 0 ou 1 ;
**L** étant un substituant choisi dans le groupe constitué par halogène, cyano, C₁-C₆-alkyle, C₁-C₆-halogénoalkyle, C₁-C₆-alcoxy, C₁-C₆-alkylthio, arylsulfényle, C₁-C₆-alkylsulfinyle, arylsulfinyle, C₁-C₆-alkylsulfonyle, arylsulfonyle, C₃-C₁₀-carbocyclyle, hétérocyclyle à 3 à 10 chaînons, aryle, hétéroaryle, =N(OR^{a}), N(OR^{a})C(=O)OR^{a}, N(OR^{a})C(=O)R^{a}, -SO₂R^{a}, -SO₂N(R^{a})₂, -OC(=O)R^{a}, -N(R^{a})₂, - NR^{a}C(=O)OR^{a}, -NR^{a}C(=O)N(R^{a})₂, -NR^{a}C(=O)R^{a}, -NR^{a}C(=S)R^{a}, - NR^{a}C(=S)N(R^{a})₂, -NR^{a}C(=NR^{a})R^{a}, -N=C-N(R^{a})₂, -NR^{a}S(=O)₂R^{a}, - OC(=O)N(R^{a})₂, -C(=NR^{a})R^{a}, -C(=O)R^{a}, -C(=O)(OR^{a}), - C(=O)N(R^{a})₂, -C(=O)NR^{a}N(R^{a})₂, -C(=O)N(OR^{a})R^{a}, -C=NOR^{a}, - C(=S)N(R^{a})₂, , -C₁-C₆-alkyl-N(R^{a})₂, -C₁-C₆-alkyl-C(=O)N(R^{a})₂, -C₁-C₆-alkyl-NR^{a}C(=O)OR^{a}, -C₁-C₆-alkyl-NR^{a}C(=O)R^{a}, -C₁-C₆-alkyl-NR^{a}C(=O)N(R^{a})₂, -C₁-C₆-alkyl-N(OR^{a})C(=O)R^{a}, -C₁-C₆-alkyl-C(=O)N(OR^{a})R^{a}, -C₁-C₆-alkyl-NR^{a}C(=S)N(R^{a})₂, -C₁-C₆-alkyl-NR^{a}C(=O)R^{a} et -C₁-C₆-alkyl-NR^{a}S(=O)₂R^{a}, ledit substituant C₁-C₆-alkyle, C₁-C₆-halogénoalkyle, C₁-C₆-alcoxy, C₁-C₆-alkylthio, arylsulfényle, C₁-C₆-alkylsulfinyle, arylsulfinyle, C₁-C₆-alkylsulfonyle, arylsulfonyle, C₃-C₁₀-carbocyclyle, hétérocyclyle à 3 à 10 chaînons, aryle, hétéroaryle, - C₁-C₆-alkyl-N(R^{a})₂, -C₁-C₆-alkyl-C(=O)N(R^{a})₂, -C₁-C₆-alkyl-NR^{a}C(=O)OR^{a}, -C₁-C₆-alkyl-NR^{a}C(=O)R^{a}, -C₁-C₆-alkyl-NR^{a}C(=O)N(R^{a})₂, -C₁-C₆-alkyl-N(OR^{a})C(=O)R^{a}, -C₁-C₆-alkyl-C(=O)N(OR^{a})R^{a}, -C₁-C₆-alkyl-NR^{a}C(=S)N(R^{a})₂, -C₁-C₆-alkyl-NR^{a}C(=O)R^{a} et -C₁-C₆-alkyl-NR^{a}S(=O)₂R^{a} étant lui-même éventuellement substitué, une ou plusieurs fois, de la même manière ou différemment, par R5 ;
**R5** étant un substituant choisi dans le groupe constitué par halogène, cyano, hydroxy, mercapto, sulfinyle, sulfonyle, amino, C₁-C₆-alkyle, C₁-C₆-alcoxy, C₁-C₆-alkylthio, C₁-C₆-alkylsulfinyle, C₁-C₆-alkylsulfonyle, C₁-C₆-alkylamino, C₂-C₆-alcényle, C₂-C₆-alcynyle, C₃-C₁₀-carbocyclyle, hétérocyclyle à 3 à 10 chaînons, C₁-C₆-halogénoalkyle, hydroxy-C₁-C₆-alkyle, aryle, aryloxy, hétéroaryle, hétéroaryloxy, nitro, -C(=O)R^{a}, -C(=O)OR^{a}, - C(=O)N(R^{a})₂, -C(=S)N(R^{a})₂, -C(=NR^{a})R^{a}, -NR^{a}C(=O)OR^{a}, - NR^{a}C(=O)N(R^{a})₂, -NR^{a}C(=O)R^{a}, -NR^{a}C(=S)R^{a}, -NR^{a}C(=S)N(R^{a})₂, -NR^{a}C(=NR^{a})R^{a}, -OC(=O)R^{a}, -OC(=O)N(R^{a})₂, -NR^{a}S(=O)₂R^{a}, - S(=O)₂R^{a}, -S(=O)₂N(R^{a})₂ et -P(=O)(OR^{a})₂ ; ledit substituant C₁-C₆-alkyle, C₁-C₆-alcoxy, C₁-C₆-alkylthio, C₁-C₆-alkylsulfinyle, C₁-C₆-alkylsulfonyle, C₁-C₆-alkylamino, C₂-C₆-alcényle, C₂-C₆-alcynyle, C₃-C₁₀-carbocyclyle, hétérocyclyle à 3 à 10 chaînons, C₁-C₆-halogénoalkyle, hydroxy-C₁-C₆-alkyle, aryle, aryloxy, hétéroaryle, hétéroaryloxy étant lui-même éventuellement substitué, une ou plusieurs fois, de la même manière ou différemment, par R^{b} ; lorsque deux substituants R5 sont liés à un carbone commun, il peuvent former ensemble C=O, C₃-C₁₀-carbocyclyle ou hétérocyclyle à 3 à 10 chaînons ; R^{a} étant, indépendamment l'un de l'autre, un substituant, qui est identique ou différent, choisi dans le groupe constitué par hydrogène, halogène, cyano, hydroxy, mercapto, sulfinyle, sulfonyle, amino, C₁-C₆-alkyle, C₁-C₆-alcoxy, C₁-C₆-alkylthio, C₁-C₆-alkylsulfinyle, C₁-C₆-alkylsulfonyle, C₁-C₆-alkylamino, C₂-C₆-alcényle, C₂-C₆-alcynyle, C₃-C₁₀-carbocyclyle, hétérocyclyle à 3 à 10 chaînons, C₁-C₆-halogénoalkyle, hydroxy-C₁-C₆-alkyle, aryle, aryloxy, hétéroaryle, hétéroaryloxy, nitro, -C₁-C₆-alkyl-C₃-C₁₀-carbocyclyle, -C₁-C₆-alkyl-aryle, -C₁-C₆-alkyl-C₁-C₆-alcoxy, -C(=O)R^{b}, -C(=O)OR^{b}, -C(=O)N(R^{b})₂, - C(=S)N(R^{b})₂, -NR^{b}C(=O)OR^{b}, -NR^{b}C(=O)N(R^{b})₂, -NR^{b}C(=O)R^{b}, - NR^{b}C(=S)R^{b}, -OC(=O)N(R^{b})₂, -NR^{b}S(=O)₂R^{b}, -S(=O)₂R^{b}, - S(=O)₂N(R^{b})₂ et -P(=O)(OR^{b})₂ ; ledit substituant C₁-C₆-alkyle, C₁-C₆-alcoxy, C₁-C₆-alkylthio, C₁-C₆-alkylsulfinyle, C₁-C₆-alkylsulfonyle, C₁-C₆-alkylamino, C₂-C₆-alcényle, C₂-C₆-alcynyle, C₃-C₁₀-carbocyclyle, hétérocyclyle à 3 à 10 chaînons, C₁-C₆-halogénoalkyle, hydroxy-C₁-C₆-alkyle, aryle, aryloxy, hétéroaryle, hétéroaryloxy étant lui-même éventuellement substitué, une ou plusieurs fois, de la même manière ou différemment, par R^{b} ;
R^{b} étant, indépendamment l'un de l'autre, un substituant, qui est identique ou différent, choisi dans le groupe constitué par hydrogène, halogène, cyano, -OR^{c}, -N(R^{c})₂, -SR^{c}, -S(=O)R^{c}, -S(=O)OR^{c}, -S(=O)₂R^{c}, -S(=O)₂OR^{c}, C₁-C₆-alkyle, C₁-C₆-alcoxy, C₁-C₆-alkylthio, C₁-C₆-alkylsulfinyle, C₁-C₆-alkylsulfonyle, C₁-C₆-alkylamino, C₂-C₆-alcényle, C₂-C₆-alcynyle, C₃-C₁₀-carbocyclyle, hétérocyclyle à 3 à 10 chaînons, C₁-C₆-halogénoalkyle, hydroxy-C₁-C₆-alkyle, aryle, aryloxy, hétéroaryle, hétéroaryloxy, nitro, -C(=O)R^{c}, -C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, - NR^{c}C(=S)R^{c}, -OC(=O)N(R^{c})₂, -NR^{c}S(=O)₂R^{c}, -S(=O)₂N(R^{c})₂ et - P(=O)(OR^{c})₂ ; ledit substituant C₃-C₁₀-carbocyclyle, hétérocyclyle à 3 à 10 chaînons, aryle, hétéroaryle étant lui-même éventuellement substitué, une ou plusieurs fois, de la même manière ou différemment, par CN, halogène, C₁-C₆-alkyle, C₁-C₆-halogénoalkyle ou C₁-C₆-alcoxy ;
R^{c} étant, indépendamment l'un de l'autre, un substituant, qui est identique ou différent, choisi dans le groupe constitué par hydrogène, aryle et C₁-C₆-alkyle, entendu que
(a) lorsque **X** est F, **n** est 1 et **A** est 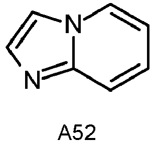 **L** n'est pas un substituant choisi dans le groupe constitué par C₁-C₆-alkyle, aryle, hétérocyclyle à 3 à 10 chaînons et -C(=O)R^{a}, R^{a} étant un hétérocyclyle à 3 à 10 chaînons,
(b) lorsque **X** est F et **n** est 0, **A** n'est pas choisi dans le groupe constitué par : 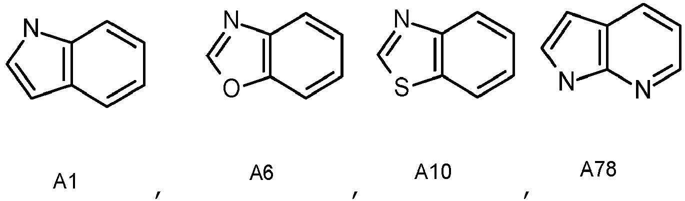 et 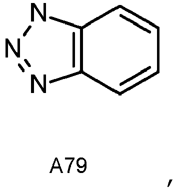
(c) lorsque **X** est F, n est 1 et **A** est choisi dans le groupe constitué par 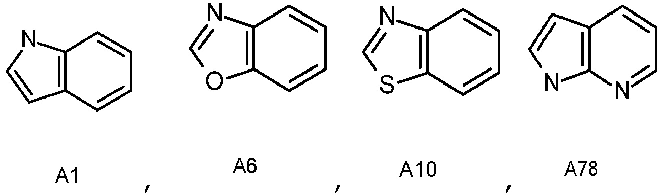 et 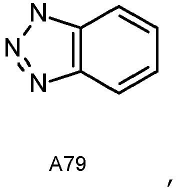 **L** n'est pas un substituant choisi dans le groupe constitué par C₁-C₆-alkyle, -C(=O)(OR^{a}), R^{a} étant un C₁-C₆-alkyle et -SO₂R^{a}, R^{a} étant un aryle,
(d) lorsque **X** est F et n est 0, **A** n'est pas 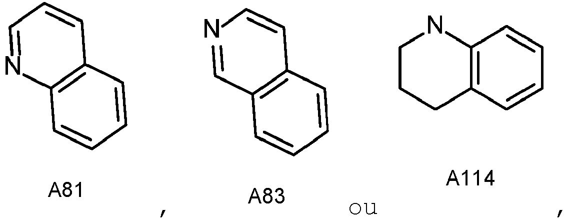
(e) lorsque **X** est F, **n** est 1 et **A** est 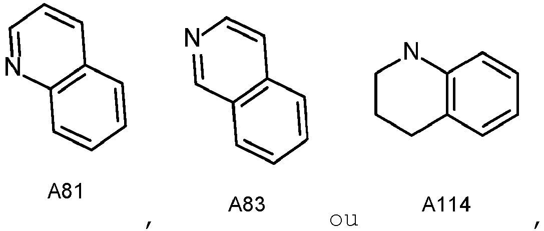 **L** n'est pas un substituant choisi dans le groupe constitué par C₁-C₆-alkyle non substitué ; C₁-C₆-alkyle substitué par un ou plusieurs **R5**, **R5** étant choisi dans le groupe constitué par aryle non substitué, hétéroaryle non substitué, aryle substitué par un à trois Rb, Rb étant C₁-C₆-alkyle non substitué, et hétéroaryle substitué par un à trois Rb, Rb étant C₁-C₆-alkyle non substitué ; C₁-C₆-halogénoalkyle ; halogène ; cyano ; - SO₂R^{a}, R^{a} étant un C₁-C₆-alkyle non substitué ; C₃-C₁₀-carbocyclyle non substitué ; aryle non substitué ; hétéroaryle non substitué ; aryle substitué par un à trois **R5**, **R5** étant un C₁-C₆-alkyle non substitué ; et hétéroaryle substitué par un à trois **R5**, **R5** étant un C₁-C₆-alkyle non substitué,
(f) lorsque **L** est -N(Ra)₂, les deux Ra sont hydrogène, L n'est pas fixé à l'atome de **A** qui lie A au groupement oxadiazole,
(g) le composé de formule (I') n'est pas 2-Pyridinecarboxamide,N-méthyl-4-[[3-méthyl-2-[5-(trifluorométhyl)-1,2,4-oxadiazol-3-yl]benzo[b]thién-6-yl]oxy]- (CAS-2222313-38-2),
Benzothiazole, 6-méthoxy-2-[5-(trifluorométhyl)-1,2,4-oxadiazol-3-yl]- (CAS-897805-25-3),
Thiéno[2,3-c]pyridine, 4-(4-chlorophénoxy)-2-[5-(trifluorométhyl)-1,2,4-oxadiazol-3-yl]- (CAS-251995-30-9),
Pyrazolo[1,5-a]pyrimidine, 3-[5-(trifluorométhyl)-1,2,4-oxadiazol-3-yl]-7-[3-(trifluorométhyl)phényl]-(CAS-159224-00-7),
1H-Indole, 3-(2-chloro-3-thiényl)-5-[5-(trifluorométhyl)-1,2,4-oxadiazol-3-yl]- (CAS-2252389-71-0),
Imidazo[1,2-a]pyridine, 2-(phénylméthyl)-7-[5-(trifluorométhyl)-1,2,4-oxadiazol-3-yl]- (CAS-2170611-35-3),
1H-Pyrrolo[2,3-b]pyridine, 5-[5-(trifluorométhyl)-1,2,4-oxadiazol-3-yl]- (CAS-2170611-21-7), 1H-Pyrrolo[2,3-b]pyridine, 1-(phénylsulfonyl)-5-[5-(trifluorométhyl)-1,2,4-oxadiazol-3-yl]- (CAS-2170611-01-3),
2,1,3-Benzoxadiazole, 4-[3-(4-morpholinylméthyl)-1-azetidinyl]-6-[5-(trifluorométhyl)-1,2,4-oxadiazol-3-yl]- (CAS-1807986-73-7), Tricyclo[3.3.1.13,7]décan-1-ol, 4-[[5-[5-(trifluorométhyl)-1,2,4-oxadiazol-3-yl]-1H-pyrrolo[2,3-b]pyridin-4-yl]amino]- (CAS-944120-75-6),
1H-Indén-1-amine, 2,3-dihydro-5-[5-(trifluorométhyl)-1,2,4-oxadiazol-3-yl]-, (1S)- (CAS-916303-83-8), 1H-Indén-1-amine, 2,3-dihydro-5-[5-(trifluorométhyl)-1,2,4-oxadiazol-3-yl]-, chlorhydrate (1:1), (1S)- (CAS-916210-97-4),
Acide carbamique, ester N-[(1S)-2,3-dihydro-5-[5-(trifluorométhyl)-1,2,4-oxadiazol-3-yl]-1H-indén-1-yl]-, 1,1-diméthyléthylique (CAS-916209-96-6), 1H-Pyrrolo[2,3-b]pyridine, 2-[1-méthyl-5-[5-(trifluorométhyl)-1,2,4-oxadiazol-3-yl]-1H-indol-3-yl]-(CAS-479551-10-5),
1H-Indole, 2,3-dihydro-7-méthyl-1-[3-(3-méthyl-5-isoxazolyl)propyl]-5-[5-(trifluorométhyl)-1,2,4-oxadiazol-3-yl]- (CAS-178396-27-5),
1H-Indole, 7-méthyl-1-[5-(3-méthyl-5-isoxazolyl)pentyl]-5-[5-(trifluorométhyl)-1,2,4-oxadiazol-3-yl]-(CAS-178396-24-2),
1H-Indole, 7-méthyl-1-[4-(3-méthyl-5-isoxazolyl)butyl]-5-[5-(trifluorométhyl)-1,2,4-oxadiazol-3-yl]-(CAS-178396-22-0),
1H-Indole, 1-[3-(3-méthyl-5-isoxazolyl)propyl]-5-[5-(trifluorométhyl)-1,2,4-oxadiazol-3-yl]- (CAS-178396-20-8),
Pyrazino[2,3-c]pyridazine, 5-[(1R)-1-(2,5-dichlorophényl)éthyl]-5,6,7,8-tétrahydro-3-[5-(trifluorométhyl)-1,2,4-oxadiazol-3-yl]- (CAS-1690421-46-5),
Pyrazino[2,3-c]pyridazine, 5-[(2,5-dichlorophényl)méthyl]-5,6,7,8-tétrahydro-3-[5-(trifluorométhyl)-1,2,4-oxadiazol-3-yl]- (CAS-1690421-40-9),
1,2,4-Oxadiazole, 3-[7-(bromométhyl)-2-naphtalényl]-5-(trifluorométhyl)- (CAS-1172849-62-5), 1,6-Naphtyridine, 5,6,7,8-tétrahydro-3-[5-(trifluorométhyl)-1,2,4-oxadiazol-3-yl]-, 2,2,2-trifluoroacétate (1:1) (CAS-741736-97-0), 1,6-Naphtyridine, 5,6,7,8-tétrahydro-3-[5-(trifluorométhyl)-1,2,4-oxadiazol-3-yl]- (CAS-741736-96-9),
1-Butanone, 3-amino-4-(2,5-difluorophényl)-1-[7,8-dihydro-3-[5-(trifluorométhyl)-1,2,4-oxadiazol-3-yl]-1,6-naphtyridin-6(5H)-yl]-, (3R)- (CAS-741736-75-4), Phtalazine, 1-phényl-4-[5-(trifluorométhyl)-1,2,4-oxadiazol-3-yl]- (CAS-863601-14-3).

2. Composé de formule (I') selon la revendication 1, A étant un cycle C₉-C₁₀-carbocyclyle bicyclique condensé aromatique ou un cycle hétérocyclyle à 9 ou 10 chaînons bicyclique condensé aromatique choisi dans le groupe constitué par A1, A2, A4, A6 à A55, A57 à A83, A85 à A89 tel que décrit dans la revendication 1.

3. Composé de formule (I') selon l'une quelconque des revendications précédentes, **A** étant choisi dans le groupe constitué par A1, A4, A10, A52, A77, A79 et A83 tel que décrit dans la revendication 1.

4. Composé de formule (I') selon la revendication 1, **A** étant un cycle C₉-C₁₀-carbocyclyle bicyclique condensé partiellement insaturé ou un cycle hétérocyclyle à 9 10 chaînons bicyclique condensé partiellement insaturé choisi dans le groupe constitué par A90 à A111 et A113 à A147 tel que décrit dans la revendication 1.

5. Composé de formule (I') selon la revendication 1 ou 4, **A** étant choisi dans le groupe constitué par A90, A91, A93, A113, A114 et A140 tel que décrit dans la revendication 1.

6. Composé de formule (I') selon l'une quelconque des revendications précédentes, **L** étant choisi dans le groupe constitué par halogène, C₁-C₆-alkyle, C₁-C₆-alcoxy, C₁-C₆-alkylsulfonyle, aryle, -N(R^{a})₂, =N(OR^{a}), -N(OR^{a})C(=O)OR^{a}, -NR^{a}C(=O)OR^{a}, -N(OR^{a})C(=O)R^{a}, -NR^{a}C(=O)R^{a}, -OC(=O)N(R^{a})₂, - C(=O)R^{a}, -C(=O)(OR^{a}), -C(=O)N(R^{a})₂, ledit substituant C₁-C₆-alkyle, C₁-C₆-alcoxy, C₁-C₆-alkylsulfonyle et aryle étant lui-même éventuellement substitué, une ou plusieurs fois, de la même manière ou différemment, par **R5** tel que décrit dans la revendication 1.

7. Composé de formule (I') selon la revendication 6, **R5** étant halogène, C₁-C₆-alcoxy ou aryle.

8. Composition comprenant un ou plusieurs composés de formule (I') selon l'une quelconque des revendications 1 à 7 et un ou plusieurs supports acceptables.

9. Utilisation non thérapeutique d'un composé de la formule (I) ou d'un sel, d'un N-oxyde ou d'un solvate correspondant pour la lutte contre des champignons phytopathogènes : 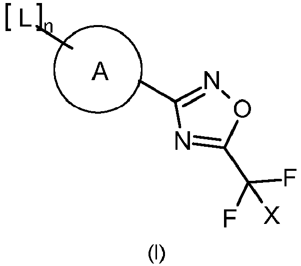
**X** étant F ou Cl ;
**A** étant un cycle C₇-C₁₂-carbocyclyle bicyclique condensé aromatique ou partiellement insaturé ou un cycle hétérocyclyle à 7 à 12 chaînons bicyclique condensé aromatique ou partiellement insaturé, lorsque n est 1, ledit cycle C₇-C₁₂-carbocyclyle ou hétérocyclyle à 7 à 12 chaînons peut être substitué par jusqu'à quatre substituants en plus de L, lesdits substituants étant indépendamment choisis dans le groupe constitué par halogène et C₁-C₃-alkyle ;
**n** étant 0 ou 1 ;
**L** étant un substituant choisi dans le groupe constitué par halogène, cyano, C₁-C₆-alkyle, C₁-C₆-halogénoalkyle, C₁-C₆-alcoxy, C₁-C₆-alkylthio, arylsulfényle, C₁-C₆-alkylsulfinyle, arylsulfinyle, C₁-C₆-alkylsulfonyle, arylsulfonyle, C₃-C₁₀-carbocyclyle, hétérocyclyle à 3 à 10 chaînons, aryle, hétéroaryle, =N(OR^{a}), N(OR^{a})C(=O)OR^{a}, N(OR^{a}) C(=O)R^{a}, -SO₂R^{a}, -SO₂N(R^{a})₂, -OC(=O)R^{a}, -N(R^{a})₂, - NR^{a}C(=O)OR^{a}, -NR^{a}C(=O)N(R^{a})₂, -NR^{a}C(=O)R^{a}, -NR^{a}C(=S)R^{a}, - NR^{a}C(=S)N(R^{a})₂, -NR^{a}C(=NR^{a})R^{a}, -N=C-N(R^{a})₂, -NR^{a}S(=O)₂R^{a}, - OC(=O)N(R^{a})₂, -C(=NR^{a})R^{a}, -C(=O)R^{a}, -C(=O)(OR^{a}), - C(=O)N(R^{a})₂, -C(=O)NR^{a}N(R^{a})₂, -C(=O)N(OR^{a}) R^{a}, -C=(NOR^{a})R^{a}, -C(=S)N(R^{a})₂, , -C₁-C₆-alkyl-N(R^{a})₂, -C₁-C₆-alkyl-C(=O)N(R^{a})₂, -C₁-C₆-alkyl-NR^{a}C(=O)OR^{a}, -C₁-C₆-alkyl-NR^{a}C(=O)R^{a}, -C₁-C₆-alkyl-NR^{a}C(=O)N(R^{a})₂, -C₁-C₆-alkyl-N(OR^{a}) C(=O)R^{a}, -C₁-C₆-alkyl-C(=O)N(OR^{a}) R^{a}, -C₁-C₆-alkyl-NR^{a}C(=S)N(R^{a})₂, -C₁-C₆-alkyl-NR^{a}C(=O)R^{a} et -C₁-C₆-alkyl-NR^{a}S(=O)₂R^{a}, ledit substituant C₁-C₆-alkyle, C₁-C₆-halogénoalkyle, C₁-C₆-alcoxy, C₁-C₆-alkylthio, arylsulfényle, C₁-C₆-alkylsulfinyle, arylsulfinyle, C₁-C₆-alkylsulfonyle, arylsulfonyle, C₃-C₁₀-carbocyclyle, hétérocyclyle à 3 à 10 chaînons, aryle, hétéroaryle, - C₁-C₆-alkyl-N(R^{a})₂, -C₁-C₆-alkyl-C(=O)N(R^{a})₂, -C₁-C₆-alkyl-NR^{a}C(=O)OR^{a}, -C₁-C₆-alkyl-NR^{a}C(=O)R^{a}, -C₁-C₆-alkyl-NR^{a}C(=O)N(R^{a})₂, -C₁-C₆-alkyl-N(OR^{a})C(=O)R^{a}, -C₁-C₆-alkyl-C(=O)N(OR^{a}) R^{a}, -C₁-C₆-alkyl-NR^{a}C(=S)N(R^{a})₂, -C₁-C₆-alkyl-NR^{a}C(=O)R^{a} et -C₁-C₆-alkyl-NR^{a}S(=O)₂R^{a} étant lui-même éventuellement substitué, une ou plusieurs fois, de la même manière ou différemment, par R5 ;
**R5** étant un substituant choisi dans le groupe constitué par halogène, cyano, hydroxy, mercapto, sulfinyle, sulfonyle, amino, C₁-C₆-alkyle, C₁-C₆-alcoxy, C₁-C₆-alkylthio, C₁-C₆-alkylsulfinyle, C₁-C₆-alkylsulfonyle, C₁-C₆-alkylamino, C₂-C₆-alcényle, C₂-C₆-alcynyle, C₃-C₁₀-carbocyclyle, hétérocyclyle à 3 à 10 chaînons, C₁-C₆-halogénoalkyle, hydroxy-C₁-C₆-alkyle, aryle, aryloxy, hétéroaryle, hétéroaryloxy, nitro, -C(=O)R^{a}, -C(=O)OR^{a}, - C(=O)N(R^{a})₂, -C(=S)N(R^{a})₂, -C(=NR^{a})R^{a}, -NR^{a}C(=O)OR^{a}, - NR^{a}C(=O)N(R^{a})₂, -NR^{a}C(=O)R^{a}, -NR^{a}C(=S)R^{a}, -NR^{a}C(=S)N(R^{a})₂, -NR^{a}C(=NR^{a})R^{a}, -OC(=O)R^{a}, -OC(=O)N(R^{a})₂, -NR^{a}S(=O)₂R^{a}, - S(=O)₂R^{a}, -S(=O)₂N(R^{a})₂ et -P(=O)(OR^{a})₂ ; ledit substituant C₁-C₆-alkyle, C₁-C₆-alcoxy, C₁-C₆-alkylthio, C₁-C₆-alkylsulfinyle, C₁-C₆-alkylsulfonyle, C₁-C₆-alkylamino, C₂-C₆-alcényle, C₂-C₆-alcynyle, C₃-C₁₀-carbocyclyle, hétérocyclyle à 3 à 10 chaînons, C₁-C₆-halogénoalkyle, hydroxy-C₁-C₆-alkyle, aryle, aryloxy, hétéroaryle, hétéroaryloxy étant lui-même éventuellement substitué, une ou plusieurs fois, de la même manière ou différemment, par R^{b} ; lorsque deux substituants R5 sont liés à un carbone commun, ils peuvent former ensemble C=O, C₃-C₁₀-carbocyclyle ou hétérocyclyle à 3 à 10 chaînons ;
R^{a} étant, indépendamment l'un de l'autre, un substituant, qui est identique ou différent, choisi dans le groupe constitué par hydrogène, halogène, cyano, hydroxy, mercapto, sulfinyle, sulfonyle, amino, C₁-C₆-alkyle, C₁-C₆-alcoxy, C₁-C₆-alkylthio, C₁-C₆-alkylsulfinyle, C₁-C₆-alkylsulfonyle, C₁-C₆-alkylamino, C₂-C₆-alcényle, C₂-C₆-alcynyle, C₃-C₁₀-carbocyclyle, hétérocyclyle à 3 à 10 chaînons, C₁-C₆-halogénoalkyle, hydroxy-C₁-C₆-alkyle, aryle, aryloxy, hétéroaryle, hétéroaryloxy, nitro, -C₁-C₆-alkyl-C₃-C₁₀-carbocyclyle, -C₁-C₆-alkyl-aryle, -C₁-C₆-alkyl-C₁-C₆-alcoxy, -C(=O)R^{b}, -C(=O)OR^{b}, -C(=O)N(R^{b})₂, - C(=S)N(R^{b})₂, -NR^{b}C(=O)OR^{b}, -NR^{b}C(=O)N(R^{b})₂, -NR^{b}C(=O)R^{b}, - NR^{b}C(=S)R^{b}, -OC(=O)N(R^{b})₂, -NR^{b}S(=O)₂R^{b}, -S(=O)₂R^{b}, - S(=O)₂N(R^{b})₂ et -P(=O)(OR^{b})₂ ; ledit substituant C₁-C₆-alkyle, C₁-C₆-alcoxy, C₁-C₆-alkylthio, C₁-C₆-alkylsulfinyle, C₁-C₆-alkylsulfonyle, C₁-C₆-alkylamino, C₂-C₆-alcényle, C₂-C₆-alcynyle, C₃-C₁₀-carbocyclyle, hétérocyclyle à 3 à 10 chaînons, C₁-C₆-halogénoalkyle, hydroxy-C₁-C₆-alkyle, aryle, aryloxy, hétéroaryle, hétéroaryloxy étant lui-même éventuellement substitué, une ou plusieurs fois, de la même manière ou différemment, par R^{b} ;
R^{b} étant, indépendamment l'un de l'autre, un substituant, qui est identique ou différent, choisi dans le groupe constitué par hydrogène, halogène, cyano, -OR^{c}, -N(R^{c})₂, -SR^{c}, -S(=O)R^{c}, -S(=O)OR^{c}, -S(=O)₂R^{c}, -S(=O)₂OR^{c}, C₁-C₆-alkyle, C₁-C₆-alcoxy, C₁-C₆-alkylthio, C₁-C₆-alkylsulfinyle, C₁-C₆-alkylsulfonyle, C₁-C₆-alkylamino, C₂-C₆-alcényle, C₂-C₆-alcynyle, C₃-C₁₀-carbocyclyle, hétérocyclyle à 3 à 10 chaînons, C₁-C₆-halogénoalkyle, hydroxy-C₁-C₆-alkyle, aryle, aryloxy, hétéroaryle, hétéroaryloxy, nitro, -C(=O)R^{c}, -C(=O)OR^{c}, -C(=O)N(R^{c})₂, -C(=S)N(R^{c})₂, -NR^{c}C(=O)OR^{c}, -NR^{c}C(=O)N(R^{c})₂, -NR^{c}C(=O)R^{c}, - NR^{c}C(=S)R^{c}, -OC(=O)N(R^{c})₂, -NR^{c}S(=O)₂R^{c}, -S(=O)₂N(R^{c})₂ et - P(=O)(OR^{c})₂ ; ledit substituant C₃-C₁₀-carbocyclyle, hétérocyclyle à 3 à 10 chaînons, aryle, hétéroaryle étant lui-même éventuellement substitué, une ou plusieurs fois, de la même manière ou différemment, par CN, halogène, C₁-C₆-alkyle, C₁-C₆-halogénoalkyle ou C₁-C₆-alcoxy ;
R^{c} étant, indépendamment l'un de l'autre, un substituant, qui est identique ou différent, choisi dans le groupe constitué par hydrogène, aryle et C₁-C₆-alkyle,
étant entendu que lorsque A est naphtyle, n est 1 et X est F, L n'est pas un substituant choisi dans le groupe constitué par
- C₁-C₆-alkyle substitué par un ou plusieurs R5, au moins l'un parmi lesdits R5 étant choisi dans le groupe constitué par C₁-C₆-alcoxy, aryle, hétéroaryle, C₃-C₁₀-carbocyclyle, hétérocyclyle à 3 à 10 chaînons, aryloxy et hétéroaryloxy,
- C₁-C₆-alcoxy substitué par un ou plusieurs R5, au moins l'un parmi lesdits R5 étant choisi dans le groupe constitué par aryle, hétéroaryle, C₃-C₁₀-carbocyclyle, hétérocyclyle à 3 à 10 chaînons, aryloxy et hétéroaryloxy,
- -N(R^{a})₂, au moins l'un parmi R^{a} étant aryle, hétéroaryle, C₃-C₁₀-carbocyclyle ou hétérocyclyle à 3 à 10 chaînons,
- N(R^{a})₂, au moins l'un parmi R^{a} étant C₁-C₆-alkyle substitué par un ou plusieurs R^{b}, au moins l'un parmi lesdits R^{b} étant un aryle, hétéroaryle, C₃-C₁₀-carbocyclyle ou hétérocyclyle à 3 à 10 chaînons,
- C₁-C₆-alkyl-N(R^{a})₂, au moins l'un parmi R^{a} étant aryle, hétéroaryle, C₃-C₁₀-carbocyclyle ou hétérocyclyle à 3 à 10 chaînons,
- C₃-C₁₀-carbocyclyle,
- hétérocyclyle à 3 à 10 chaînons et
- hétéroaryle.

10. Utilisation non thérapeutique d'un composé de formule (I) pour la lutte contre des champignons phytopathogènes selon la revendication 9, A étant choisi dans le groupe constitué par

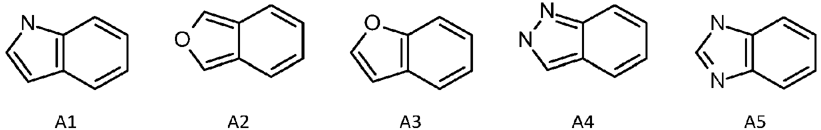

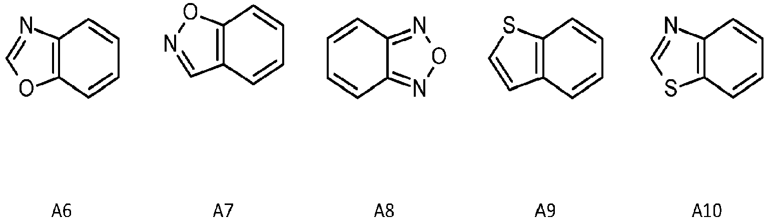

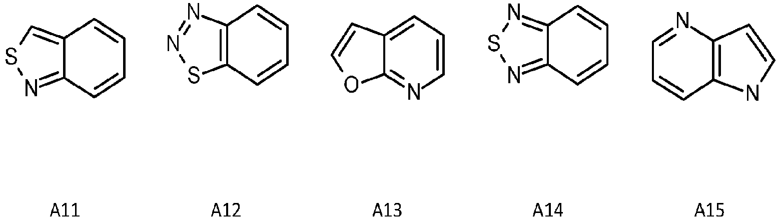

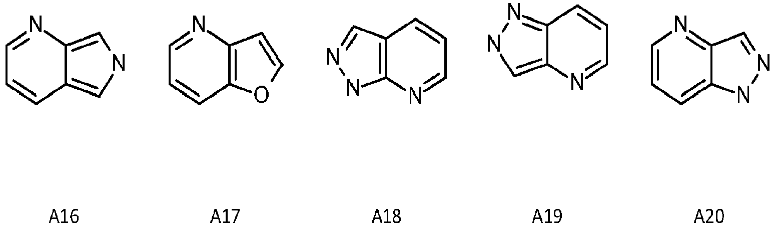

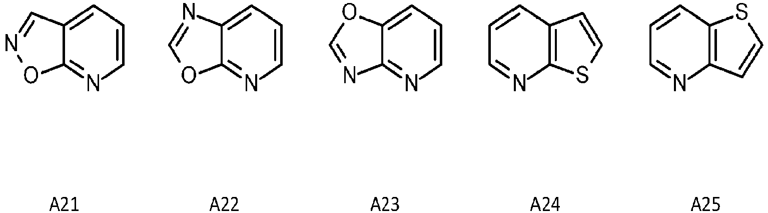

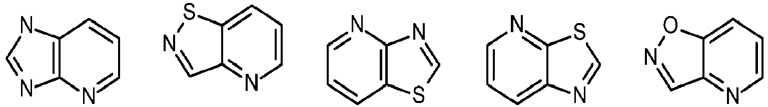

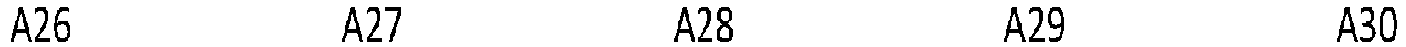

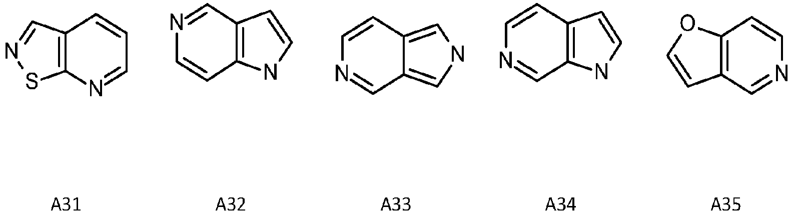

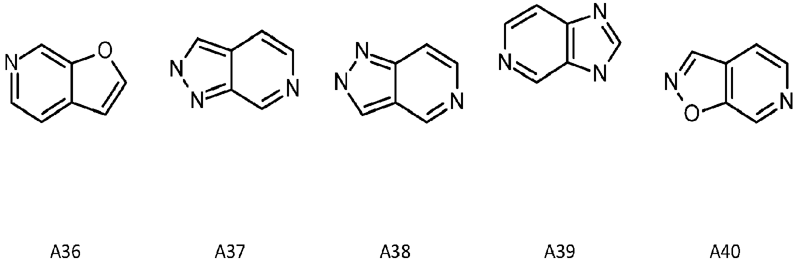

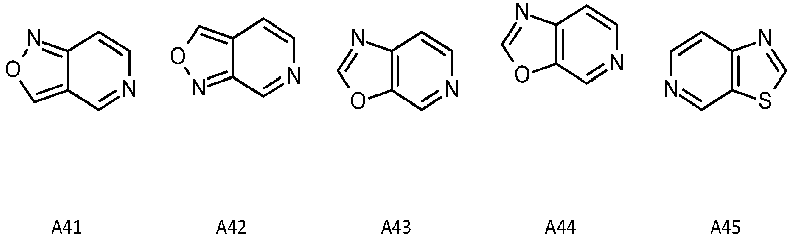

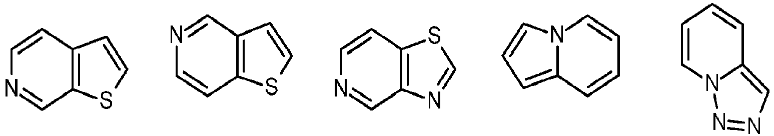

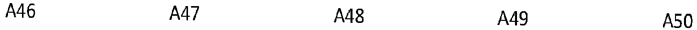

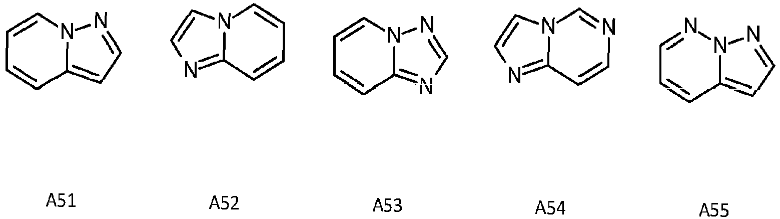

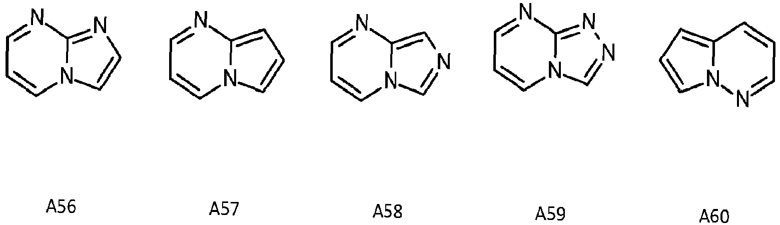

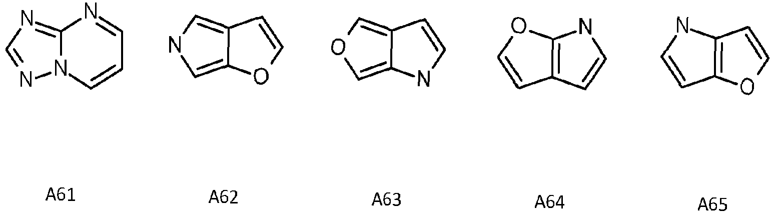

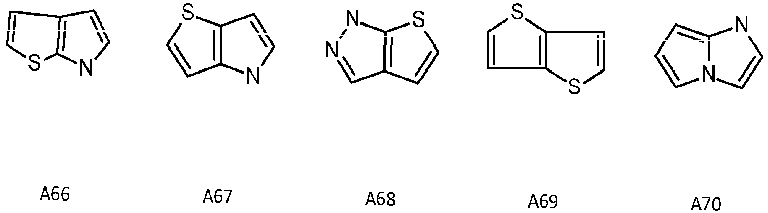

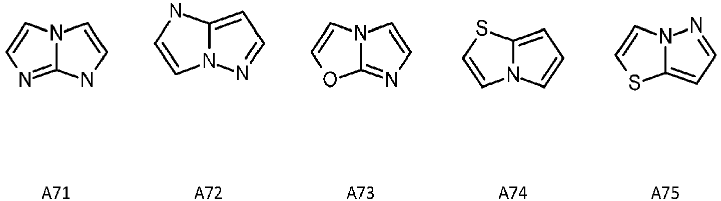

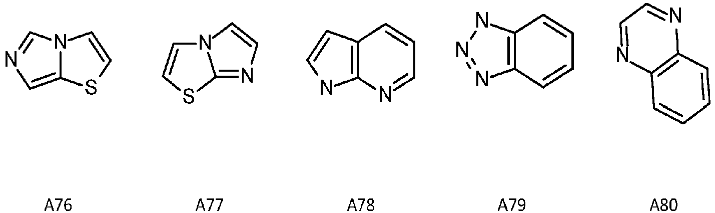

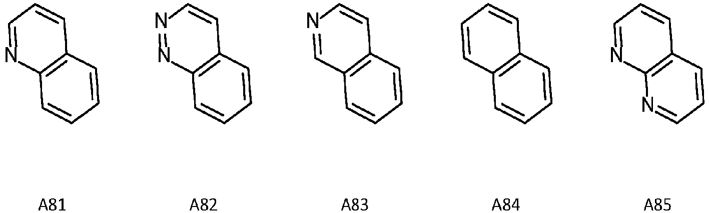

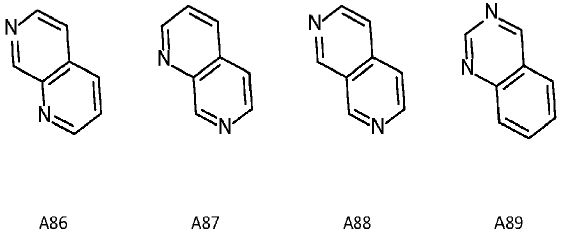

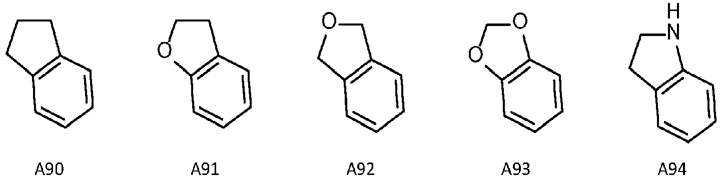

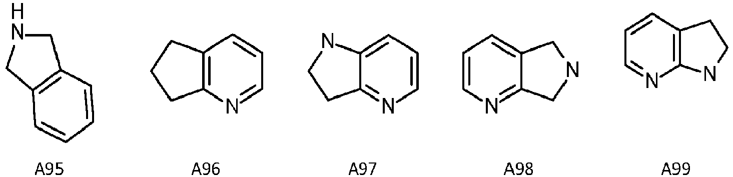

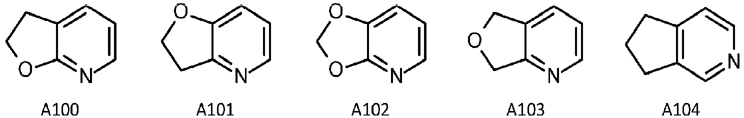

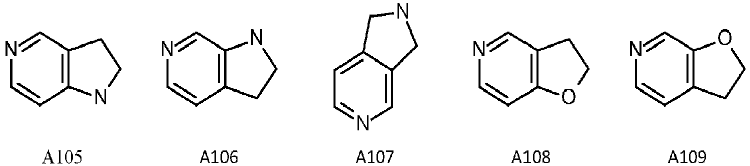

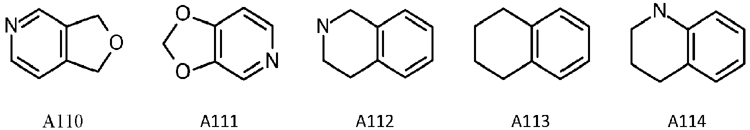

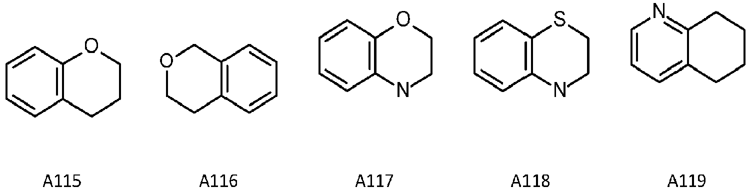

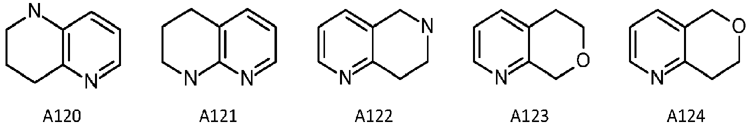

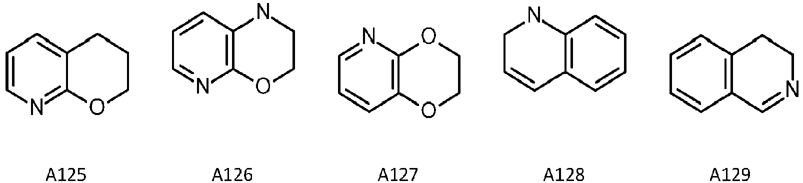

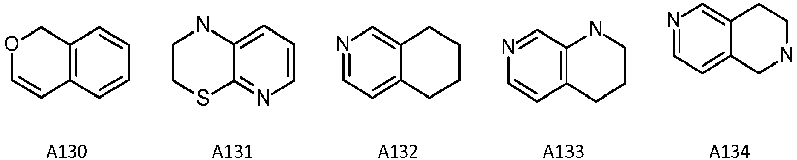

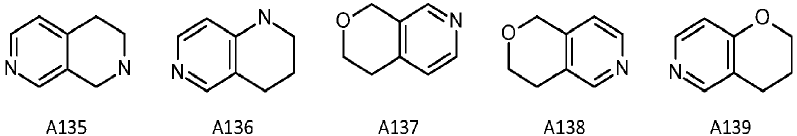

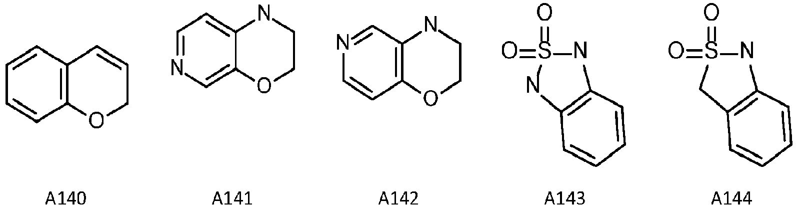

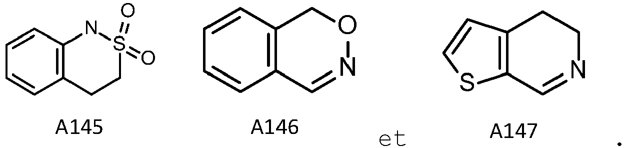

11. Procédé pour la lutte contre des micro-organismes phytopathogènes indésirables qui comprend l'étape d'application d'un ou plusieurs composés de formule (I) tels que décrits dans la revendication 9 ou 10 sur des végétaux, des parties de végétaux, des semences, des fruits ou sur le sol dans lequel les végétaux croissent.

12. Procédé pour la préparation d'un composé de formule (I') selon l'une quelconque des revendications 1 à 7, n étant 1, qui comprend l'étape de mise en réaction d'amidoximes de formule (II) avec un anhydride difluorohalogénoalkylacétique ou un chlorure de difluorohalogénoalkylacétyle dans un solvant pour donner un composé de formule (I) : 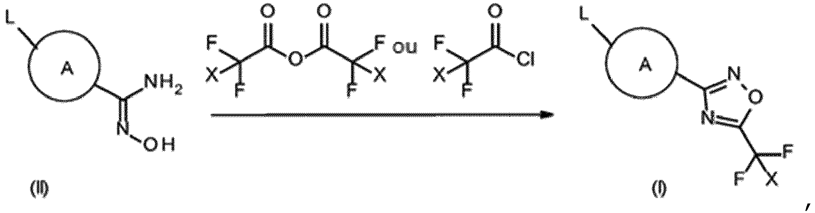 la formule (I) correspondant à la formule (I'), n étant 1, et L, A et X dans les formules (II) et (I) étant tels que définis dans le composé de formule (I').
